# EUROPEAN PATENT APPLICATION

(11) **EP 1 468 688 A2**
(43) Date of publication of application: **20.10.2004**
(21) Application number: 04251451.3
(22) Date of filing: 12.03.2004
(51) Int. Cl.: A61K 31/517, A61K 31/53, A61P 35/00

(54) **Benzoazine mono-N-oxides and benzoazine 1,4 dioxides and compositions therefrom for the therapeutic use in cancer treatments**

(30) Priority: 14.03.2003 NZ 52477003
(71) Applicant: Auckland Uniservices Limited, Auckland (NZ)
(72) Inventor: Wilson, William Robert, RD2 Waiuku (NZ); Pruijn, Frederik Bastiaan, Swanson Auckland 1008 (NZ); Siim, Bronwyn Gae, Mt Eden Auckland 1003 (NZ); Hay, Michazel Patrick, Epsom Auckland 1003 (NZ); Denny, William Alexander, Pakuranga Auckland 1706 (NZ); Gamage, Swarnalatha Akuratiya, Hillsborough Auckland (NZ)
(74) Representative: Watson, Robert James

(57) **Abstract**

The present invention relates to a synergetistic composition comprising one or more benzoazine-mono-N-oxides, and one or more benzoazine 1,4 dioxides for use in cancer therapy.

The invention also provides a range of novel 1,2,4 benzoazine-mono-N-oxides and related analogues. These can be used as potentiators of the cytotoxicity of existing anticancer drugs and therapies for cancer treatment.

## Description

### TECHNICAL FIELD

The present invention relates generally to a cytotoxic synergistic composition comprising one or more benzoazine-mono-N-oxides, and an effective amount of one or more benzoazine 1,4 dioxides for use as anticancer drugs and as radiosensitizers for cancer therapy in combination with radiation and/or other anticancer drugs.

The present invention also relates to the provision of a range of novel 1,2,4-benzoazine-mono-N-oxides and related analogues, and to their use as potentiators of the cytotoxicity of anticancer drugs and as radiosensitizers for cancer therapy in combination with radiation and/or with other anticancer agents, or to at least provide the public with a useful choice.

The term benzoazine used throughout this specification is to be understood as a term used to encompass the mono and di N- oxide derivatives of benzotriazines and quinoxalines.

### BACKGROUND TO THE INVENTION

Hypoxic cells in tumours are resistant to ionising radiation, and are a major cause of treatment failure in radiation therapy (Movsas et al., *Cancer,* **2000**, 89, 2018; Rudat et al., *Radiother. Oncol.,* 2000, 57, 31). Hypoxic cells are also considered to compromise response of solid tumours to cytotoxic chemotherapy (Brown and Giaccia, *Cancer Res.,* **1998,** 58, 1408). The benzotriazine di-N-oxide tirapazamine (TPZ) is selectively toxic to hypoxic cells because of its metabolic activation to a cytotoxic species by one-electron reduction (Baker et al., *Cancer Res.,* 1988, 48, 5947; Laderoute et al., *Biochem. Pharmacol.,* **1988**, 37, 1487; Brown, Br. *J. Cancer* **1993**, 67, 1163). As shown below, the initial one-electron reduction product TPZ^{*} is reoxidised to the starting compound by dioxygen, thereby preventing cytotoxicity in oxic cells.

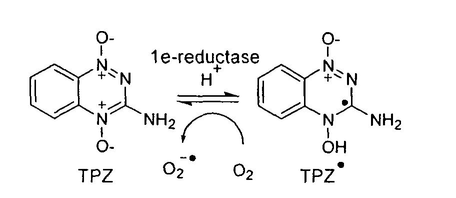

TPZ is therefore of interest for killing hypoxic cells in tumours, thereby improving overall response during radiation therapy. TPZ also has potential for combination with standard cytotoxic chemotherapy (Dorie and Brown, Cancer Res., 1993, 53, 4633; Langmuir et al., Cancer Res., **1994,** 54, 2845; Dorie and Brown, Cancer Chemother. Pharmacol., **1997,** 39, 361), with (at least) two mechanisms of therapeutic synergy. The first mechanism is the killing of resistant hypoxic cells (analogous to the mechanism of interaction with radiotherapy), and the second is the interference with repair of chemotherapy-induced DNA damage in hypoxic cells as has been demonstrated for cisplatin (Kovacs et al., *Br. J. Cancer* **1999,** 80, 1245; Peters et al., Cancer Res., 2001, 61: 5425.

TPZ has already demonstrated significant antitumour activity in early phase human clinical trials in combination with ionising radiation and/or cisplatin chemotherapy (for a review, see Denny and Wilson, *Exp. Opin. Invest. Drugs,* 2000, 9, 2889), and a multicentre phase III trial of TPZ in combination with cisplatin and radiation for treatment of head and neck tumours is in progress. While TPZ shows promising indications of clinical activity, it also displays considerable toxicity, such as neutropenia, thrombocytopenia, nausea, vomiting, diarrhoea and muscle cramping. These toxicity limitations preclude administration of doses high enough to exploit hypoxia fully during cancer treatment. Although the mechanisms of TPZ toxicity to normal tissues are not fully understood, it is considered that the toxicity arises at least in part because of redox cycling. (Elwell et al., *Biochem. Pharmacol.,* **1997**, 54, 249; Wouters et al., *Cancer Res.,* **2001**, 61, 145) The mechanisms of TPZ toxicity are therefore considered to be distinct from the mechanism of hypoxic cell killing. There would be value in identifying agents capable of enhancing the hypoxic cytotoxic potency of TPZ, without increasing its toxicity to oxic cells, in order to improve its therapeutic selectivity for hypoxic tumour cells.

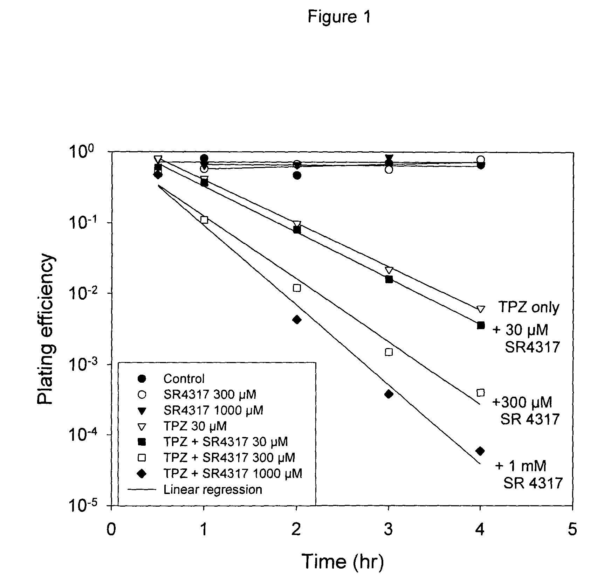

Recent studies indicate that the cytotoxic species arising from reduction of TPZ under hypoxia is an oxidising radical derived from the initial benzotriazine radical (TPZ^{*}); the ultimate cytotoxin has been variously suggested to be the hydroxyl radical OH^{*} (Daniels and Gates, *J*. *Am. Chem. Soc,* **1996,** 118, 3380; Patterson and Taiwo, *Biochem. Pharmacol*., **2000,** 60, 1933) or the benzotriazinyl radical TPZ^{*} shown in Figure 1 below. (Anderson et al., *J. Am. Chem. Soc*, **2003,** 125, 748). Whatever its identity, the oxidising radical generates DNA radicals which give rise to complex DNA lesions responsible for cytotoxicity in hypoxic cells (Wang et al., *Cancer Res.,* **1992,** 52, **4473;** Siim et al., *Br. J. Cancer* 1996, 73, 952; Kotandeniya et al., Bioorg. Med. *Chem. Lett.,* **2002,** 12, 2325; Peters and Brown, *Cancer Res.,* **2002,** 62, 5248).

Published studies (Jones and Weinfeld, *Cancer Res.,* **1996,** 56, 1584; Daniels and Gates, *Chem. Res. Toxicol*., **1998,** 11, 1254) have suggested that TPZ has a dual mechanism of action, with the parent drug being involved in two distinct steps in the generation of DNA damage as represented below in Scheme A.

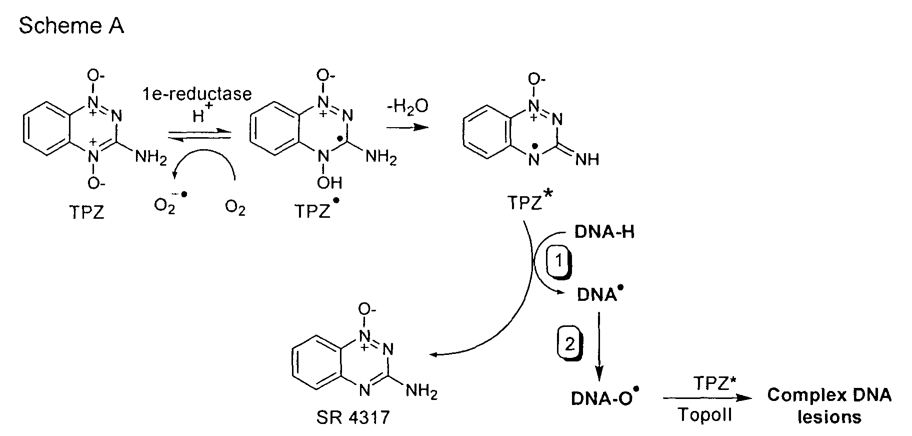

In the first step, the DNA-damaging species, TPZ^{*} or OH^{*}, is considered to generate DNA radicals by hydrogen abstraction. In the second step, TPZ itself can further oxidise the initial DNA radicals to generate a more cytotoxic lesion (DNA break). Certain other agents, such as the 1-N-oxide metabolite derived from TPZ (SR 4317, as illustrated in Fig 1), have also been shown to be capable of oxidising DNA radicals of the kind formed by TPZ^{*} (Hwang et al., *Biochemistry,* **1999,** 38, 14248).

It is an object of the present invention to provide a cytotoxic synergistic composition comprising one or more benzoazine-mono-N-oxides, and an effective amount of one or more benzoazine 1,4 dioxides for use as anticancer drugs and as radiosensitizers for cancer therapy in combination with radiation and/or other anticancer drugs, or to at least provide the public with a useful choice.

It is also an object of the present invention to provide a range of novel 1,2,4-benzoazine-mono-N-oxides and related analogues, and to their use as potentiators of the cytotoxicity of anticancer drugs and as radiosensitizers for cancer therapy in combination with radiation and/or with other anticancer agents, or to at least provide the public with a useful choice.

### SUMMARY OF THE INVENTION

In a first aspect, the present invention provides a cytotoxic synergistic composition, comprising an effective amount of a benzoazine N-mono oxide compound of Formula A or a pharmacologically acceptable salt thereof and an effective amount of a benzoazine 1,4 dioxide compound of Formula B or a pharmacologically acceptable salt thereof 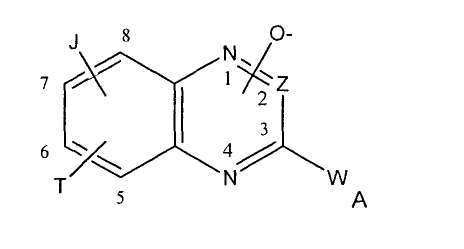 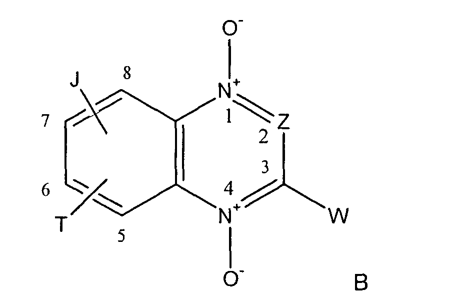 wherein in Formulae A or B
Z is selected from N or C-CN, and
wherein in Formula A when Z represents N, the N-oxide moiety occupies one of the 1-, 2-, or 4-positions; and when Z represents C-CN, the N-oxide moiety occupies one of the 1-, or 4-positions;
wherein J in Formulae A or B represents at one or more of the available carbons 5-8 on the benzo ring the following groups:
halo, H, R, OH, OR, NO₂, NH₂, NHR, NR₂, SH, SR, SO₂R, CF₃, CN, CO₂H, CO₂R, CHO, COR, CONH₂, CONHR or CONRR, cyclic alkylamino, imidazolyl, alkylpiperazinyl, morpholino;
wherein each R is independently selected from an optionally substituted C₁₋₆ aliphatic or an optionally substituted C₃₋₆ cyclic alkyl group, and wherein the said optional substituents are each independently selected from; halo, OH, OR¹, NO₂, NH₂, NHR¹, NR¹R¹, SH, SR¹, imidazolyl, R¹-piperazinyl, morpholino, SO₂R¹, CF₃, CN, CO₂H, CO₂R¹, CHO, COR¹, CONH₂, CONHR¹, CONR¹R¹;
R can also represent an optionally substituted aryl or an optionally substituted heteroaryl group having up to 12 carbon atoms, and wherein the optional substituents are each independently selected from; halo, OH, OR¹, NH_{2,} NHR¹, NR¹R¹, SH, SR¹, imidazolyl, R¹-piperazinyl, morpholino, SO₂R¹, CF_{3,} CN, CO₂H, CO₂R¹, CHO, COR¹, CONH_{2,} CONHR¹, CONR¹R¹, and each heteroaryl group contains one or more heteroatoms in its ring system which are each independently selected from O, N or S;
wherein each R¹ is independently selected from an optionally substituted C₁₋₄ alkyl or an optionally substituted C₂₋₄ alkenyl group and wherein the optional substituents are each independently selected from OH, OR, NH₂, NHR², NR²₂ or N(OH)R² wherein each R² is independently selected from C₁₋₄ alkyl, C₂₋₄ alkenyl, OH, NO₂ NH₂, CF₃, CN, CO₂H or SH, and
wherein W in Formulae A or B represents -X-A, wherein -X-A together may represent H, or halogen; or
X can represent O, S, NH, NMe, CH₂, SO, SO₂, CONH, NHCO, CO or CO₂, and
A represents H, an optionally substituted C₁₋₁₂ alkyl group wherein the optional substituents are each independently selected from OH, OR³, NH₂, NHR³, NR³₂, or N(OH)R³ wherein each R³ is independently selected from C₁₋₄ alkyl, C₂₋₄ alkenyl, OH, NO₂, NH₂, CF₃, CN, CO₂H or SH; and wherein the optionally substituted C₁₋₁₂ alkyl chain can be optionally interrupted or extended by one or more heteroatom containing linkage moieties selected from O, NH, NR⁴, CONH, CONR⁴, NHCO, NR⁴CO, where each R⁴ is independently selected from an optionally substituted C₁₋₄ alkyl or an optionally substituted C₂₋₄ alkenyl group and wherein the optional R⁴ substituents are each independently selected from OH, OR, NH₂, NHR⁵, NR⁵₂ or N(OH)R⁵ wherein each R⁵ is independently selected from C₁₋₄ alkyl, C₂₋₄ alkenyl, OH, NO₂ NH₂, CF₃, CN, CO₂H or SH, or a pharmacologically acceptable salt thereof, or
W represents a group of Formula C 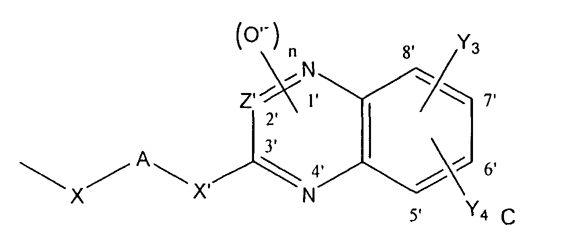 wherein in a group of Formula C
n represents either 1 or 2,
Z' is selected from N or C-CN, and when Z' represents N, and n represents 1 the N-oxide moiety may occupy one of the 1'-, 2'-, or 4'-positions and when Z' represents C-CN, the N-oxide moiety may occupy one of the 1'-, or 4'-positions; and when Z' represents N or C-CN, and n represents 2 the N-oxide moieties occupy the 1'and 4'-positions
Y₃ and Y₄ may each represent at one or more of the available carbons 5'-8' on the benzo ring the following groups:
halo, H, R, OH, OR, NO₂, NH_{2,} NHR, NR_{2,} SH, SR, SO₂R, CF_{3,} CN, CO₂H, CO₂R, CHO, COR, CONH_{2,} CONHR or CONRR, cyclic alkylamino, imidazolyl, alkylpiperazinyl, morpholino;
wherein each R is independently selected from an optionally substituted C₁₋₆ aliphatic or an optionally substituted C₃₋₆ cyclic alkyl group, and wherein the said optional substituents are each independently selected from; halo, OH, OR¹, NO_{2,} NH_{2,} NHR¹, NR¹R¹, SH, SR¹, imidazolyl, R¹-piperazinyl, morpholino, SO₂R¹, CF_{3,} CN, CO₂H, CO₂R¹, CHO, COR¹, CONH_{2,} CONHR¹, CONR¹R¹;
R may also represent an optionally substituted aryl or an optionally substituted heteroaryl group having up to 12 carbon atoms, and wherein the optional substituents are each independently selected from; halo, OH, OR¹, NH₂, NHR¹, NR¹R¹, SH, SR¹, imidazolyl, R¹-piperazinyl, morpholino, SO₂R¹, CF₃, CN, CO₂H, CO₂R¹, CHO, COR¹, CONH₂, CONHR¹, CONR¹R¹, and each heteroaryl group contains one or more heteroatoms in its ring system which are each independently selected from O, N or S;
wherein each R¹ is independently selected from an optionally substituted C₁₋₄ alkyl or an optionally substituted C₂₋₄ alkenyl group and wherein the optional substituents are each independently selected from OH, OR, NH₂, NHR², NR²₂ or N(OH)R² wherein each R² is independently selected from C₁₋₄ alkyl, C₂₋₄ alkenyl, OH, NO₂, NH₂, CF₃, CN, CO₂H or SH, and
X' may represent O, NH, NMe, or CH₂,

A may represent an optionally substituted C₁₋₁₂alkyl group wherein the optional substituents are each independently selected from OH, OR³, NH₂, NHR³, NR³₂, or N(OH)R³ wherein each R³ is independently selected from C₁-₄ alkyl, C₂₋₄ alkenyl, OH, NO₂, NH₂, CF₃, CN, CO₂H or SH; and wherein the optionally substituted C₁₋₁₂ alkyl chain is optionally interrupted or extended by one or more heteroatom containing linkage moieties selected from O, NH, NR⁴, CONH, CONR⁴, NHCO, NR⁴CO, where each R⁴ is independently selected from an optionally substituted C₁₋₄ alkyl or an optionally substituted C₂₋₄ alkenyl group and wherein the optional R⁴ substituents are each independently selected from OH, OR, NH₂, NHR⁵, NR⁵₂ or N(OH)R⁵ wherein each R⁵ is independently selected from C₁₋₄ alkyl, C₂₋₄ alkenyl, OH, NO₂, NH₂, CF₃, CN, CO₂H or SH, or
W may represent a group of Formula D 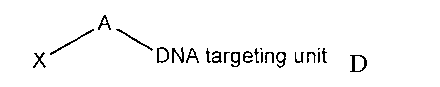 wherein X may represent NH, NMe, CH_{2,} or O;
A may represent an optionally substituted C₁₋₁₂ alkyl group wherein the optional substituents are each independently selected from OH, OR, NH_{2,} NHR³, NR³₂ , or N(OH)R³ wherein each R³ is independently selected from C₁₋₄ alkyl, C₂₋₄ alkenyl, OH, NO_{2,} NH_{2,} CF_{3,} CN, CO₂H or SH; and wherein the optionally substituted C₁₋₁₂ alkyl chain is optionally interrupted or extended by one or more heteroatom containing linkage moieties selected from O, NH, NR⁴, CONH, CONR⁴, NHCO, NR⁴CO, where each R⁴ is independently selected from an optionally substituted C₁₋₄ alkyl or an optionally substituted C₂₋₄ alkenyl group and wherein the optional R⁴ substituents are each independently selected from OH, OR, NH_{2,} NHR⁵, NR⁵₂ or N(OH)R⁵ wherein each R⁵ is independently selected from C₁₋₄ alkyl, C₂₋₄ alkenyl, OH, NO_{2,} NH_{2,} CF_{3,} CN, CO₂H or SH, and
wherein the DNA-targeting unit is any moiety of a molecular weight below 700 Daltons that has an association constant (K) for binding to double-stranded random sequence DNA of >10³ M⁻¹ at an ionic strength of 0.01 M at 20 °C,.
wherein T in Formulae A or B, may represent at one of carbons 5-8 on the benzo ring the following groups:
halo, H, R, OH, OR, NO₂, NH₂, NHR, NR₂, SH, SR, SO₂R, CF₃, CN, CO₂H, CO₂R, CHO, COR, CONH₂, CONHR or CONRR, cyclic alkylamino, imidazolyl, alkylpiperazinyl, morpholino;
wherein each R is independently selected from an optionally substituted C₁₋₆ aliphatic or an optionally substituted C₃₋₆ cyclic alkyl group, and wherein the said optional substituents are each independently selected from; halo, OH, OR¹, NO₂, NH₂, NHR¹, NR¹R¹ SH, SR¹, imidazolyl, R¹-piperazinyl, morpholino, SO₂R¹, CF₃, CN, CO₂H, CO₂R¹, CHO, COR¹, CONH₂, CONHR¹, CONR¹R¹;
R may also represent an optionally substituted aryl or an optionally substituted heteroaryl group having up to 12 carbon atoms, and wherein the optional substituents are each independently selected from; halo, OH, OR¹, NH₂, NHR¹, NR¹R¹, SH, SR¹, imidazolyl, R¹-piperazinyl, morpholino, SO₂R¹, CF₃, CN, CO₂H, CO₂R¹, CHO, COR¹, CONH₂, CONHR¹, CONR¹R¹, and each heteroaryl group contains one or more heteroatoms in its ring system which are each independently selected from O, N or S;
wherein each R¹ is independently selected from an optionally substituted C₁₋₄ alkyl or an optionally substituted C₂₋₄ alkenyl group and wherein the optional substituents are each independently selected from OH, OR, NH₂, NHR², NR²₂ or N(OH)R² wherein each R² is independently selected from C₁₋₄ alkyl, C₂₋₄ alkenyl, OH, NO₂, NH₂, CF₃, CN, CO₂H or SH, or
T may represent a group of Formula E 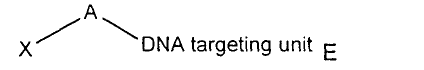 wherein X may represent O, S, NH, NMe, CH₂, SO, SO₂, CONH, NHCO, CO, CO₂, or O and
A may represent an optionally substituted C₁₋₁₂ alkyl group wherein the optional substituents are each independently selected from OH, OR, NH₂, NHR³, NR³₂ , or N(OH)R³ wherein each R³ is independently selected from C₁₋₄ alkyl, C₂₋₄ alkenyl, OH, NO₂, NH₂, CF₃, CN, CO₂H or SH; and wherein the optionally substituted C₁₋₁₂ alkyl chain is optionally interrupted or extended by one or more heteroatom containing linkage moieties selected from O, NH, NR⁴, CONH, CONR⁴, NHCO, NR⁴CO, where each R⁴ is independently selected from an optionally substituted C₁₋₄ alkyl or an optionally substituted C₂₋₄ alkenyl group and wherein the optional R⁴ substituents are each independently selected from OH, OR, NH₂, NHR⁵, NR⁵₂ or N(OH)R⁵ wherein each R⁵ is independently selected from C₁₋₄ alkyl, C₂₋₄ alkenyl, OH, NO₂, NH₂, CF₃, CN, CO₂H or SH, and
wherein the DNA-targeting unit is any moiety of a molecular weight below 700 Daltons that has an association constant (K) for binding to double-stranded random-sequence DNA of >10³ M⁻¹ at an ionic strength of 0.01 M at 20 °C.

Preferably, the DNA targeting agent defined above for a group of Formula D or Formula E is independently selected from any one of the following wherein the DNA-targeting unit is selected from one of formulae **III- XVII**, 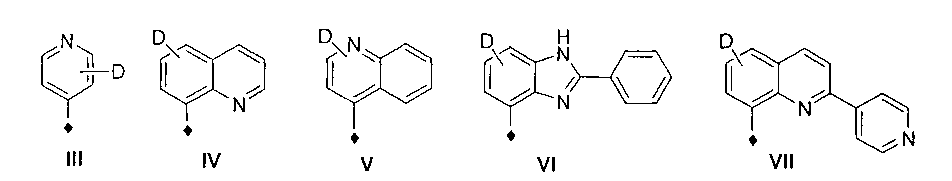 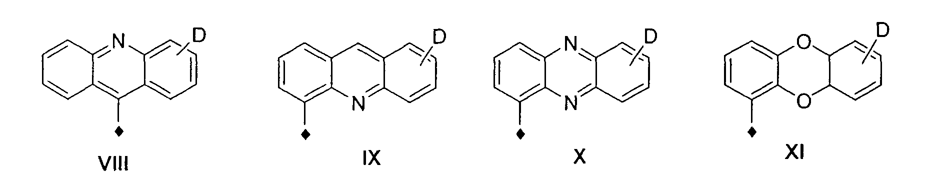 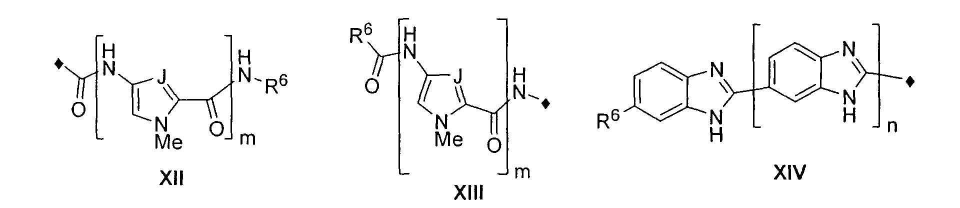 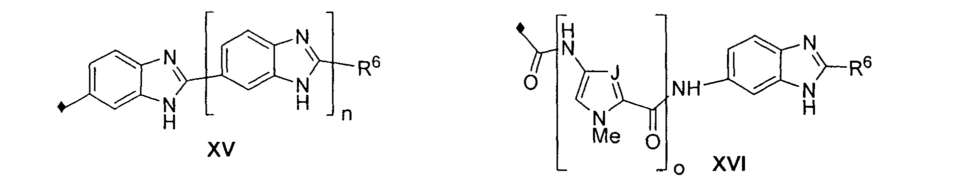 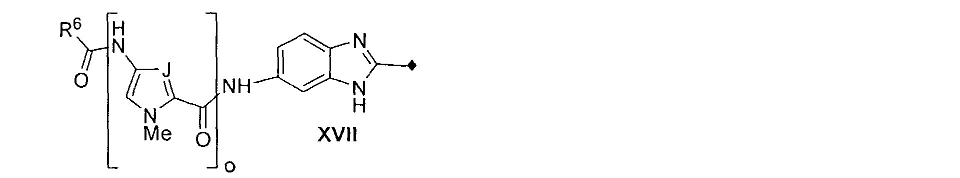 wherein in structures **XII-XVII** R⁶ is independently selected from an optionally substituted C₁₋₆ aliphatic or an optionally substituted C₃₋₆ cyclic alkyl group, and wherein the optional substituents are each independently selected from; halo, OH, OR⁷ NO_{2,} NH₂, NHR⁷, NR⁷R⁷, SR⁷, imidazolyl, R⁷-piperazinyl, morpholino, SO₂R⁷, CF_{3,} CN, CO₂H, CO₂R⁷, CHO, COR⁷, CONH_{2,} CONHR⁷, CONR⁷R⁷;
R⁶ may also be represent an optionally substituted aryl or an optionally substituted heteroaryl group having up to 12 carbon atoms, and wherein the optional substituents are each independently selected from; halo, OH, OR⁷, NH_{2,} NHR⁷, NR⁷R⁷, SH, SR⁷, imidazolyl, R⁷-piperazinyl, morpholino, SO₂R⁷, CF_{3,} CN, CO₂H, CO₂R⁷, CHO, COR⁷, CONH_{2,} CONHR⁷, CONR⁷R⁷, and each heteroaryl group contains one or more heteroatoms in its ring system which are each independently selected from O, N or S;
wherein each R⁷ is independently selected from an optionally substituted C₁₋₄ alkyl or an optionally substituted C₂₋₄ alkenyl group and wherein the optional substituents are each independently selected from OH, OR⁸, NH_{2,} NHR⁸, NR⁸₂or N(OH)R⁸ wherein each R⁸ is independently selected from C₁₋₄ alkyl, C₂₋₄ alkenyl, OH, NO₂ NH_{2,} CF_{3,} CN, CO₂H or SH;
D may represent up to four of the following groups as substituents at any available ring carbon position; H, R⁹, hydroxy, alkoxy, halogen, NO_{2,} NH_{2,} NHR⁹, NR⁹_{2,} SH, SR⁹, SO₂R⁹, CF_{3,} CN, CO₂H, CO₂R⁹, CHO, COR⁹, CONH_{2,} CONHR⁹ or CONR⁹R⁹, cyclic alkylamino, imidazolyl, alkylpiperazinyl, morpholino, wherein each R⁹ is independently selected from an optionally substituted C₁₋₄ alkyl or an optionally substituted C₂₋₄ alkenyl group and wherein the optional substituents are each independently selected from OH, OR¹⁰, NH_{2,} NHR¹⁰, NR¹⁰₂ or N(OH)R¹⁰ wherein each R¹⁰ is independently selected from C₁₋₄ alkyl, C₂₋₄ alkenyl, OH, NO_{2,} NH_{2,} CF_{3,} CN, CO₂H or SH;
and wherein any available ring carbon position of formulae **III** - **XVII** may also be optionally replaced by -N- when the valency and configuration of the formula allows, the point of attachment of formulae **III- XVII** to the A group defined above is represented by ◆; and
wherein in formulae **XII, XIII,** , m is selected from 2, 3 or 4, and
wherein in formulae **XII, XIII, XVI** and **XVII,** J is selected from CH or N;
and wherein in formulae **XIV** and **XV** n is selected from 0, 1 or 2;
and wherein in formulae **XVI** and **XVII** o is selected from 1 and 2.

More preferably the DNA targeting unit is selected from one of formulae **V**, **VI, VII, VIII, IX** or **X.** Most preferably, D of the DNA targeting unit of Formulae **III - XI** is H or Me.

Preferably W in Formula A represents NH(C₁-C₁₂) optionally substituted alkyl or a O(C₁-C₁₂) optionally substituted alkyl.

Preferably W in the compound of Formula A represents NH₂, NHCH₂CH₂NHCH₃, NHCH₂CH₂N(CH₃)₂ or OCH₃.

Preferably, the composition includes a pharmaceutically acceptable excipient, adjuvant, carrier, buffer or stabiliser. The pharmaceutically acceptable excipient, adjuvant, carrier, buffer or stabiliser should be non-toxic and should not interfere with the efficacy of the active composition. The precise nature of the carrier or other material will depend on the intended route of administration, which may be oral, or by injection such as cutaneous, subcutaneous or by intravenous injection. Pharmaceutical compositions for oral administration may be in tablet, capsule, powder or liquid form. A tablet may comprise a solid carrier or an adjuvant. Liquid pharmaceutical compositions generally comprise a liquid such as water, petroleum, animal or vegetable oils, mineral oil or synthetic oil. Physiological saline solution, dextrose or other saccharide solution or glycols such as ethylene glycol, propylene glycol may be included. A capsule may comprise a solid carrier such as a gelatine. For intravenous, cutaneous or subcutaneous injection, the active composition will be in the form of a parenterally acceptable aqueous solution that is pyrogen-free and has a suitable pH, isotonicity and stability. Those of skill in the art would be able to prepare suitable solutions using for example, isotonic vehicles such as sodium chloride injection, Ringer's injection, lactated Ringer's injection. Preservatives, stabilisers, buffers, antioxidants and/or other additives may be included as required.

It is to be appreciated that the compounds of Formula B are to be taken as including all the DNA-targeted benzotriazine 1,4-dioxides and the methods disclosed for making these compounds as specified in co-pending PCT application PCT/NZ03/00210 to Auckland Uniservices and the Board of Trustees of the Leland Stanford Junior University. The disclosure of PCT/NZ03/00210 is hereby incorporated in its entirety.

In a second aspect, there is provided a method of treating a subject in need of cancer therapy, said method comprising the steps of administering to said subject a cytotoxic effective amount of a composition including an effective amount of one or more compounds of Formula A and one or more compounds of formula B as defined above to the tumour cells in said subject.

Preferably, the steps of administration of a compound of Formula A and B may be simultaneous or sequential.

Preferably the tumour cells are in a hypoxic environment.

Preferably, the method includes the further step of administering said composition defined above in combination with one or more other chemotherapeutic agents or treatments, including radiotherapy, either simultaneously, or sequentially, depending on the cancerous condition to be treated.

More preferably the method includes the step of administering radiotherapy to the tumour cells before, during or after the administration of the composition as defined above.

Preferably, the chemotherapeutic agents are selected from Cisplatin or other platinum-based derivatives, Temozolomide or other DNA methylating agents, cyclophosphamide or other DNA alkylating agents, doxorubicin, mitoxandrone, camptothecin or other topoisomerase inhibitors, methotrexate, gemcitabine or other antimetabolites.

While the compositions of the invention will typically be used in cancer therapy of human subjects, they may be used to target tumour cells in other warm blooded animal subjects such as other primates, farm animals such as cattle, and sports animals and pets such as horses, dogs, and cats.

A "cytotoxic effective amount", is to be understood as an amount of the composition including one or more compounds of Formula A and one or more compounds of Formula B as defined above that is sufficient to show benefit to a patient. The actual amount, rate and time-course of administration, will depend on the nature and severity of the disease being treated. Prescription of treatment is within the responsibility of general practitioners and other medical doctors.

A hypoxic environment is to be understood as tissue environments at an oxygen concentration of < 10 µM.

In a third aspect there is provided, the use in the manufacture of a medicament of an effective amount of a composition including an effective amount of one or more compounds of Formula A or one or more compounds of formula B as defined above for the treatment of a subject in need of cancer therapy.

In a fourth aspect, the present invention provides a compound of Formula I, 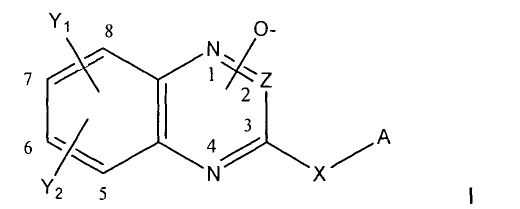 wherein
Z may be selected from N or C-CN, and when Z represents N, the N-oxide moiety occupies one of the 1-, 2-, or 4-positions; and when Z represents C-CN, the N-oxide moiety occupies one of the 1-, or 4-positions;
Y₁ and Y₂ may each represent at one or more of the available carbons 5-8 on the benzo ring the following groups:
halo, H, R, OH, OR, NO₂, NH₂, NHR, NR₂, SH, SR, SO₂R, CF₃, CN, CO₂H, CO₂R, CHO, COR, CONH₂, CONHR or CONRR, cyclic alkylamino, imidazolyl, alkylpiperazinyl, morpholino;
wherein each R may be independently selected from an optionally substituted C₁₋₆ aliphatic or an optionally substituted C₃₋₆ cyclic alkyl group, and wherein the said optional substituents are each independently selected from; halo, OH, OR¹, NO₂, NH₂, NHR¹, NR¹R¹, SH, SR¹, imidazolyl, R¹-piperazinyl, morpholino, SO₂R¹, CF₃, CN, CO₂H, CO₂R¹, CHO, COR¹, CONH₂, CONHR¹, CONR¹R¹;
R may also represent an optionally substituted aryl or an optionally substituted heteroaryl group having up to 12 carbon atoms, and wherein the optional substituents are each independently selected from; halo, OH, OR¹, NH₂, NHR¹, NR¹R¹, SH, SR¹, imidazolyl, R¹-piperazinyl, morpholino, SO₂R¹, CF₃, CN, CO₂H, CO₂R¹, CHO, COR¹, CONH₂, CONHR¹, CONR¹R¹, and each heteroaryl group contains one or more heteroatoms in its ring system which are each independently selected from O, N or S;
wherein each R¹ is independently selected from an optionally substituted C₁₋₄ alkyl or an optionally substituted C₂₋₄ alkenyl group and wherein the optional substituents are each independently selected from OH, OR, NH₂, NHR², NR²₂ or N(OH)R² wherein each R² is independently selected from C₁₋₄ alkyl, C₂₋₄ alkenyl, OH, NO₂ NH₂, CF₃, CN, CO₂H or SH, and
wherein A and X together may represent H, or halogen; or
X may represent O, S, NH, NMe or CH₂ and
A may represent H, an optionally substituted C₁₋₁₂ alkyl group wherein the optional substituents are each independently selected from OH, OR³, NH₂, NHR³, NR³₂, or N(OH)R³ wherein each R³ is independently selected from C₁₋₄ alkyl, C₂₋₄ alkenyl, OH, NO₂, NH₂, CF₃, CN, CO₂H or SH; and wherein the optionally substituted C₁₋₁₂ alkyl chain is optionally interrupted or extended by one or more heteroatom containing linkage moieties selected from O, NH, NR⁴, CONH, CONR⁴, NHCO, NR⁴CO, where each R⁴ is independently selected from an optionally substituted C₁₋₄ alkyl or an optionally substituted C₂₋₄ alkenyl group and wherein the optional R⁴ substituents are each independently selected from OH, OR, NH₂, NHR⁵, NR⁵₂ or N(OH)R⁵ wherein each R⁵ is independently selected from C₁₋₄ alkyl, C₂₋₄ alkenyl, OH, NO₂, NH₂, CF₃, CN, CO₂H or SH, or a pharmacologically acceptable salt thereof,
with the proviso that the following compounds are excluded
3-Amino-1,2,4-benzotriazine-1-oxide,
3-Amino-7-trifluoromethyl-1,2,4-benzotriazine-1-oxide,
3-Amino-7-carbamyl-1,2,4-benzotriazine-1-oxide,
3-Amino-7-chloro-1,2,4-benzotriazine-1-oxide,
3-Amino-7-nitro-1,2,4-benzotriazine-1-oxide
3-Chloro-1,2,4-benzotriazine-1-oxide,
3-(3-N,N-Diethylaminopropylamino)- 3-amino-1,2,4-benzotriazine-1-oxide,
3-Chloro-7-nitro-1,2,4-benzotriazine-1-oxide,
7-Nitro-(3-(2-N,N-diethylamino-ethylamino)-1,2,4-benzotriazine-1-oxide,
8-Methoxy-1,2,4-benzotriazin-3-amine 1-oxide,
8-Methyl-1,2,4-benzotriazin-3-amine 1-oxide,
8-Fluoro-1,2,4-benzotriazin-3-amine 1-oxide,
8-Chloro-1,2,4-benzotriazin-3-amine 1-oxide,
8-Trifluoromethyl-1,2,4-benzotriazin-3-amine 1-oxide,
8-(Methylsulfanyl)-1,2,4-benzotriazin-3-amine 1-oxide,
8-(Butylsulfanyl)-1,2,4-benzotriazin-3-amine 1-oxide,
3-Amino-1,2,4-benzotriazin-7-ol 1-oxide,
3-Amino-1,2,4-benzotriazin-7-ol 1-oxide,
7-Methyl-1,2,4-benzotriazin-3-amine 1-oxide,
7-Fluoro-1,2,4-benzotriazin-3-amine 1-oxide,
7-Chloro-1,2,4-benzotrazin-3-amine 1-oxide,
7-Trifluoromethyl-1,2,4-benzotriazin-3-amine 1-oxide,
7-(Methylsulfanyl)-1,2,4-benzotriazin-3-amine 1-oxide,
7-(Butylsulfanyl)-1,2,4-benzotriazin-3-amine 1-oxide,
7-Nitro-1,2,4-benzotriazin-3-amine 1-oxide,
6-Methoxy-1,2,4-benzotriazin-3-amine 1-oxide,
6-Methyl-1,2,4-benzotriazin-3-amine 1-oxide,
6-Phenyl-1,2,4-benzotriazin-3-amine 1-oxide,
6-Fluoro-1,2,4-benzotriazin-3-amine 1-oxide,
6-Chloro-1,2,4-benzotrazin-3-amine 1-oxide,
6-Trifluoromethyl-1,2,4-benzotriazin-3-amine 1-oxide,
6-(Methylsulfanyl)-1,2,4-benzotriazin-3-amine 1-oxide,
6-(Butylsulfanyl)-1,2,4-benzotriazin-3-amine 1-oxide,
5-Methoxy-1,2,4-benzotriazin-3-amine 1-oxide,
5-Methyl-1,2,4-benzotriazin-3-amine 1-oxide,
5-Chloro-1,2,4-benzotriazin-3-amine 1-oxide,
5-Fluoro-1,2,4-benzotriazin-3-amine 1-oxide,
*N*^{*7*}*,N*^{*7*}-Dimethyl-1,2,4-benzotriazine-3,7-diamine 1-oxide,
3-Chloro-1,2,4-benzotriazine 1-oxide,
3-Methyl-1,2,4-benzotriazine 1-oxide,
3-Ethyl-1,2,4-benzotriazine 1-oxide,
3-Phenyl-1,2,4-benzotriazine 1-oxide,
3-(4-Methoxyphenyl)-1,2,4-benzotriazine 1-oxide,
3-Vinyl-1,2,4-benzotriazine 1-oxide,
3-Allyl-1,2,4-benzotriazine 1-oxide,
3-(2-Hydroxyethyl)-1,2,4-benzotriazine 1-oxide,
3-(2-Methoxyethyl)-1,2,4-benzotriazine 1-oxide,
*N*-Phenyl-1,2,4-benzotriazin-3-amine 1-oxide,
3-Methoxy-1,2,4-benzotriazine 1-oxide,
3-Chloro-7-methyl-1,2,4-benzotriazine 1-oxide,
3-Chloro-7-methoxy-1,2,4-benzotriazine 1-oxide,
1,2,4-benzotriazine 1-oxide,
1,2,4-benzotriazin-3-amine 2-oxide, and
1,2,4-benzotriazin-3-amine 4-oxide.

Preferably, Z is N.

A preferred compound of Formula I is one in which X is NH or CH₂.

A more preferred compound of Formula I is one in which -X-A represents a NH(C₁-C₁₂) optionally substituted alkyl or an O(C₁-C₁₂) optionally substituted alkyl, such as NHCH₂CH₂NHCH₃, NHCH₂CH₂N(CH₃)₂ or OCH₃.

A further preferred compound of Formula I is one in which Y₁ and Y₂ each represent H.

A further preferred compound of Formula I' is one in which the N-oxide moiety occupies the 1-position.

In a fifth aspect, there is provided a method of treating a subject in need of cancer therapy, said method comprising the steps of administering to said subject a cytotoxic effective amount of a compound of Formula I 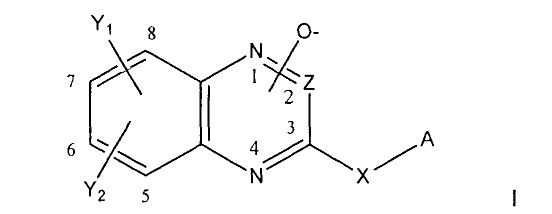 wherein
Z is selected from N or C-CN, and when Z represents N, the N-oxide moiety occupies one of the 1-, 2-, or 4-positions; and when Z represents C-CN, the N-oxide moiety occupies one of the 1-, or 4-positions;
Y₁ and Y₂ may each represent at one or more of the available carbons 5-8 on the benzo ring the following groups:
halo, H, R, OH, OR, NO₂, NH₂, NHR, NR₂, SH, SR, SO₂R, CF₃, CN, CO₂H, CO₂R, CHO, COR, CONH₂, CONHR or CONRR, cyclic alkylamino, imidazolyl, alkylpiperazinyl, morpholino;
wherein each R is independently selected from an optionally substituted C₁₋₆ aliphatic or an optionally substituted C₃₋₆ cyclic alkyl group, and wherein the said optional substituents are each independently selected from; halo, OH, OR¹, NO₂, NH_{2,} NHR¹, NR¹R¹, SH, SR¹, imidazolyl, R¹-piperazinyl, morpholino, SO₂R¹, CF_{3,} CN, CO₂H, CO₂R¹, CHO, COR¹, CONH_{2,} CONHR¹, CONR¹R¹;
R may also represent an optionally substituted aryl or an optionally substituted heteroaryl group having up to 12 carbon atoms, and wherein the optional substituents are each independently selected from; halo, OH, OR¹, NH_{2,} NHR¹, NR¹R¹, SH, SR¹, imidazolyl, R¹-piperazinyl, morpholino, SO₂R¹, CF_{3,} CN, CO₂H, CO₂R¹, CHO, COR¹, CONH₂, CONHR¹, CONR¹R¹, and each heteroaryl group contains one or more heteroatoms in its ring system which are each independently selected from O, N or S;
wherein each R' is independently selected from an optionally substituted C₁₋₄ alkyl or an optionally substituted C₂₋₄ alkenyl group and wherein the optional substituents are each independently selected from OH, OR, NH₂, NHR², NR²₂ or N(OH)R² wherein each R² is independently selected from C₁₋₄ alkyl, C₂₋₄ alkenyl, OH, NO_{2,} NH₂, CF₃, CN, CO₂H or SH, and
wherein A and X together may represent H, or halogen; or
X may represent O, S, NH, NMe or CH₂ and
A may represent H, an optionally substituted C₁₋₁₂ alkyl group wherein the optional substituents are each independently selected from OH, OR, NH₂, NHR³, NR³₂ , or N(OH)R³ wherein each R³ is independently selected from C₁₋₄ alkyl, C₂₋₄ alkenyl, OH, NO₂, NH₂, CF₃, CN, CO₂H or SH; and wherein the optionally substituted C₁₋₁₂ alkyl chain is optionally interrupted or extended by one or more heteroatom containing linkage moieties selected from O, NH, NR⁴, CONH, CONR⁴, NHCO, NR⁴CO, where each R⁴ is independently selected from an optionally substituted C₁₋₄ alkyl or an optionally substituted C₂₋₄ alkenyl group and wherein the optional R⁴ substituents are each independently selected from OH, OR, NH₂, NHR⁵, NR⁵₂ or N(OH)R⁵ wherein each R⁵ is independently selected from C₁₋₄ alkyl, C₂₋₄ alkenyl, OH, NO_{2,} NH₂, CF₃, CN, CO₂H or SH, or a pharmacologically acceptable salt thereof
to the tumour cells in said subject.

Preferably the tumour cells are in a hypoxic environment.

Preferably, the method includes the further step of administering the compound of Formula I in combination with one or more other chemotherapeutic agents or treatments, including radiotherapy, either simultaneously, or sequentially, depending on the cancerous condition to be treated.

More preferably the method includes the step of administering radiotherapy to the tumour cells before, during or after the administration of the composition as defined above.

Preferably, the chemotherapeutic agents are selected from Cisplatin or other platinum-based derivatives, Temozolomide or other DNA methylating agents, cyclophosphamide or other DNA alkylating agents, doxorubicin, mitoxandrone, camptothecin or other topoisomerase inhibitors, methotrexate, gemcitabine or other antimetabolites.

While the method of the invention will typically be used in cancer therapy of human subjects, they may be used to target tumour cells in other warm blooded animal subjects such as other primates, farm animals such as cattle, and sports animals and pets such as horses, dogs, and cats.

Preferably, the compound of Formula I is administered with a pharmaceutically acceptable excipient, adjuvant, carrier, buffer or stabiliser. The pharmaceutically acceptable excipient, adjuvant, carrier, buffer or stabiliser should be non-toxic and should not interfere with the efficacy of the active composition. The precise nature of the carrier or other material will depend on the intended route of administration, which may be oral, or by injection such as cutaneous, subcutaneous or by intravenous injection. Pharmaceutical compositions of Formula I for oral administration may be in tablet, capsule, powder or liquid form. A tablet may comprise a solid carrier or an adjuvant. Liquid pharmaceutical compositions generally comprise a liquid such as water, petroleum, animal or vegetable oils, mineral oil or synthetic oil. Physiological saline solution, dextrose or other saccharide solution or glycols such as ethylene glycol, propylene glycol may be included. A capsule may comprise a solid carrier such as a gelatine. For intravenous, cutaneous or subcutaneous injection, the active composition will be in the form of a parenterally acceptable aqueous solution that is pyrogen-free and has a suitable pH, isotonicity and stability. Those of skill in the art would be able to prepare suitable solutions using for example, isotonic vehicles such as sodium chloride injection, Ringer's injection, lactated Ringer's injection. Preservatives, stabilisers, buffers, antioxidants and/or other additives may be included as required.

A "cytotoxic effective amount", is to be understood as an amount of a compound of Formula defined above that is sufficient to show benefit to a patient. The actual amount, rate and time-course of administration, will depend on the nature and severity of the disease being treated. Prescription of treatment is within the responsibility of general practitioners and other medical doctors.

A hypoxic environment is to be understood as tissue environments at an oxygen concentration of < 10 mM.

In a sixth aspect there is provided, the use in the manufacture of a medicament of an effective amount of a compound of Formula I as defined above for the treatment of a subject in need of cancer therapy.

In a seventh aspect the present invention provides a compound of Formula I', 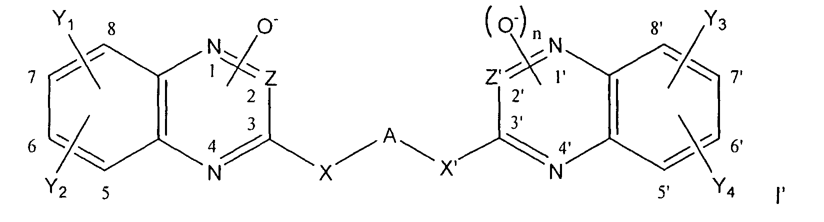 wherein
n may represent either 1 or 2,
Z or Z' is selected from N or C-CN, and when Z or Z' represents N, and n represents 1 each N-oxide moiety may occupy one of the 1-, 2-, or 4-positions or 1'-, 2'-, or 4'-positions respectively and when Z or Z' represents C-CN, each N-oxide moiety may occupy one of the 1-, or 4-positions or 1'-, or 4'-positions respectively; and when Z' represents N, and n represents 2, the N'-oxide moieties occupy the 1'- and 4'-positions and when Z' represents C-CN, and n represents 2 the N'-oxide moieties occupy the 1'-, and 4'-positions;
Y_{1,} Y_{2,} Y₃ and Y₄ may each represent at one or more of the available carbons 5-8 or one or more of the available carbons 5'-8' on the respective benzo ring the following groups:
halo, H, R, OH, OR, NO₂, NH₂, NHR, NR₂, SH, SR, SO₂R, CF₃, CN, CO₂H, CO₂R, CHO, COR, CONH₂, CONHR or CONRR, cyclic alkylamino, imidazolyl, alkylpiperazinyl, morpholino;
wherein each R is independently selected from an optionally substituted C₁₋₆ aliphatic or an optionally substituted C₃₋₆ cyclic alkyl group, and wherein the said optional substituents are each independently selected from; halo, OH, OR¹, NO₂, NH₂, NHR¹, NR¹R¹, SH, SR¹, imidazolyl, R¹-piperazinyl, morpholino, SO₂R¹, CF₃, CN, CO₂H, CO₂R¹, CHO, COR¹, CONH₂, CONHR¹, CONR¹R¹;
R may also represent an optionally substituted aryl or an optionally substituted heteroaryl group having up to 12 carbon atoms, and wherein the optional substituents are each independently selected from; halo, OH, OR¹, NH₂, NHR¹, NR¹R¹, SH, SR¹, imidazolyl, R¹-piperazinyl, morpholino, SO₂R¹, CF₃, CN, CO₂H, CO₂R¹, CHO, COR¹, CONH₂, CONHR¹, CONR¹R¹, and each heteroaryl group contains one or more heteroatoms in its ring system which are each independently selected from O, N or S;
wherein each R¹ is independently selected from an optionally substituted C₁₋₄ alkyl or an optionally substituted C₂₋₄ alkenyl group and wherein the optional substituents are each independently selected from OH, OR, NH₂, NHR², NR²₂ or N(OH)R² wherein each R² is independently selected from C₁₋₄ alkyl, C₂₋₄ alkenyl, OH, NO₂, NH₂, CF₃, CN, CO₂H or SH, and
wherein X may represent NH, NMe, CH₂, or O;

A may represent an optionally substituted C₁₋₁₂ alkyl group wherein the optional substituents are each independently selected from OH, OR³, NH₂, NHR³, NR³₂, or N(OH)R³ wherein each R³ is independently selected from C₁₋₄ alkyl, C₂₋₄ alkenyl, OH, NO₂, NH₂, CF₃, CN, CO₂H or SH; and wherein the optionally substituted C₁₋₁₂ alkyl chain is optionally interrupted or extended by one or more heteroatom containing linkage moieties selected from O, NH, NR⁴, CONH, CONR⁴, NHCO, NR⁴CO, where each R⁴ is independently selected from an optionally substituted C₁₋₄ alkyl or an optionally substituted C₂₋₄ alkenyl group and wherein the optional R⁴ substituents are each independently selected from OH, OR, NH₂, NHR⁵, NR⁵₂ or N(OH)R⁵ wherein each R⁵ is independently selected from C₁₋₄ alkyl, C₂₋₄ alkenyl, OH, NO₂, NH₂, CF₃, CN, CO₂H or SH, or a pharmacologically acceptable salt thereof.

A preferred compound of Formula I' is one in which X is NH or CH₂.

A further preferred compound of Formula I' is one in which Y₁ and Y₂ each represent H.

A further preferred compound of Formula I' is one in which A is -(CH₂)₂NMe(CH₂)₂-

A further preferred compound of Formula I' is one in which the N-oxides are positioned at the 1-position.

In an eighth aspect, there is provided a method of treating a subject in need of cancer therapy, said method comprising the steps of administering to said subject a cytotoxic effective amount of a compound of Formula I' as defined above to the tumour cells in said subject.

Preferably the tumour cells are in a hypoxic environment.

Preferably, the method includes the further step of administering the compound of Formula I' in combination with one or more other chemotherapeutic agents or treatments, including radiotherapy, either simultaneously, or sequentially, depending on the cancerous condition to be treated.

More preferably the method includes the step of administering radiotherapy to the tumour cells before, during or after the administration of the composition as defined above.

Preferably, the chemotherapeutic agents are selected from Cisplatin or other platinum-based derivatives, Temozolomide or other DNA methylating agents, cyclophosphamide or other DNA alkylating agents, doxorubicin, mitoxandrone, camptothecin or other topoisomerase inhibitors, methotrexate, gemcitabine or other antimetabolites.

While the method of the invention will typically be used in cancer therapy of human subjects, they may be used to target tumour cells in other warm blooded animal subjects such as other primates, farm animals such as cattle, and sports animals and pets such as horses, dogs, and cats.

Preferably, the compound of Formula I' is administered with a pharmaceutically acceptable excipient, adjuvant, carrier, buffer or stabiliser. The pharmaceutically acceptable excipient, adjuvant, carrier, buffer or stabiliser should be non-toxic and should not interfere with the efficacy of the active composition. The precise nature of the carrier or other material will depend on the intended route of administration, which may be oral, or by injection such as cutaneous, subcutaneous or by intravenous injection. Pharmaceutical compositions of Formula I' for oral administration may be in tablet, capsule, powder or liquid form. A tablet may comprise a solid carrier or an adjuvant. Liquid pharmaceutical compositions generally comprise a liquid such as water, petroleum, animal or vegetable oils, mineral oil or synthetic oil. Physiological saline solution, dextrose or other saccharide solution or glycols such as ethylene glycol, propylene glycol may be included. A capsule may comprise a solid carrier such as a gelatine. For intravenous, cutaneous or subcutaneous injection, the active composition will be in the form of a parenterally acceptable aqueous solution that is pyrogen-free and has a suitable pH, isotonicity and stability. Those of skill in the art would be able to prepare suitable solutions using for example, isotonic vehicles such as sodium chloride injection, Ringer's injection, lactated Ringer's injection. Preservatives, stabilisers, buffers, antioxidants and/or other additives may be included as required.

A "cytotoxic effective amount", is to be understood as an amount of a compound of Formula I' defined above that is sufficient to show benefit to a patient. The actual amount, rate and time-course of administration, will depend on the nature and severity of the disease being treated. Prescription of treatment is within the responsibility of general practitioners and other medical doctors.

A hypoxic environment is to be understood as tissue environments at an oxygen concentration of < 10 mM.

In a ninth aspect there is provided, the use in the manufacture of a medicament of an effective amount of a compound of Formula I' as defined above for the treatment of a subject in need of cancer therapy.

In a tenth aspect, the present invention provides a compound of Formula II, 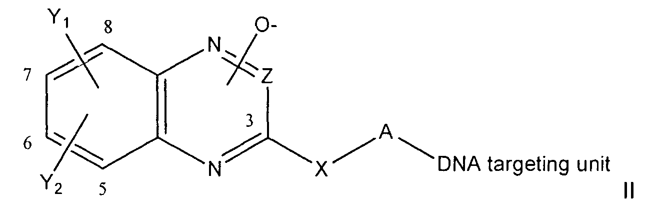 wherein
Z is selected from N or C-CN, and when Z represents N, the N-oxide moiety occupies one of the 1-, 2-, or 4-positions; and when Z represents C-CN, the N-oxide moiety occupies one of the 1-, or 4-positions;
Y₁ and Y₂ may each represent at one or more of the available carbons 5-8 on the benzo ring the following groups:
halo, H, R, OH, OR, NO₂, NH₂, NHR, NR₂, SH, SR, SO₂R, CF₃, CN, CO₂H, CO₂R, CHO, COR, CONH₂, CONHR or CONRR, cyclic alkylamino, imidazolyl, alkylpiperazinyl, morpholino;
wherein each R is independently selected from an optionally substituted C₁₋₆ aliphatic or an optionally substituted C₃₋₆ cyclic alkyl group, and wherein the said optional substituents are each independently selected from; halo, OH, OR¹, NO₂, NH₂, NHR¹, NR¹R¹, SH, SR¹, imidazolyl, R¹-piperazinyl, morpholino, SO₂R¹, CF₃, CN, CO₂H, CO₂R¹, CHO, COR¹, CONH₂, CONHR¹, CONR¹R¹;
R may also represent an optionally substituted aryl or an optionally substituted heteroaryl group having up to 12 carbon atoms, and wherein the optional substituents are each independently selected from; halo, OH, OR¹, NH_{2,} NHR¹, NR¹R¹, SH, SR¹, imidazolyl, R¹-piperazinyl, morpholino, SO₂R¹, CF_{3,} CN, CO₂H, CO₂R¹, CHO, COR¹ CONH_{2,} CONHR¹, CONR¹R¹, and each heteroaryl group contains one or more heteroatoms in its ring system which are each independently selected from O, N or S;
wherein each R¹ is independently selected from an optionally substituted C₁₋₄ alkyl or an optionally substituted C₂₋₄ alkenyl group and wherein the optional substituents are each independently selected from OH, OR, NH_{2,} NHR², NR ²₂ or N(OH)R² wherein each R² is independently selected from C₁₋₄ alkyl, C₂₋₄ alkenyl, OH, NO_{2,} NH_{2,} CF_{3,} CN, CO₂H or SH, and
wherein X may represent NH, NMe, CH_{2,} or O;

A may represent an optionally substituted C₁₋₁₂ alkyl group wherein the optional substituents are each independently selected from OH, OR³, NH_{2,} NHR³, NR³₂ , or N(OH)R³ wherein each R³ is independently selected from C₁₋₄ alkyl, C₂₋₄ alkenyl, OH, NO_{2,} NH_{2,} CF_{3,} CN, CO₂H or SH; and wherein the optionally substituted C₁₋₁₂ alkyl chain is optionally interrupted or extended by one or more heteroatom containing linkage moieties selected from O, NH, NR⁴, CONH, CONR⁴, NHCO, NR⁴CO, where each R⁴ is independently selected from an optionally substituted C₁₋₄ alkyl or an optionally substituted C₂₋₄ alkenyl group and wherein the optional R⁴ substituents are each independently selected from OH, OR, NH_{2,} NHR⁵, NR⁵₂ or N(OH)R⁵ wherein each R⁵ is independently selected from C₁₋₄ alkyl, C₂₋₄ alkenyl, OH, NO_{2,} NH_{2,} CF_{3,} CN, CO₂H or SH, and
wherein the DNA-targeting unit is any moiety of a molecular weight below 700 Daltons that has an association constant (K) for binding to double-stranded random-sequence DNA of >10³ M⁻¹ at an ionic strength of 0.01 M at 20 °C,
or a pharmacologically acceptable salt thereof.

The definition of the DNA targeting unit above refers to double-stranded random-sequence DNA. An example of such double-stranded random-sequence DNA is DNA extracted from calf thymus.

Preferably, Z is N.

A preferred compound of Formula II is one in which X is NH or CH₂.

A further preferred compound of Formula II is one in which the N-oxide is at the 1-position

A further preferred compound of Formula II is one in which Y₁ and Y₂ each represent H.

A further preferred compound of Formula II is one in which Y₁ represents Me

A preferred embodiment of Formula II are compounds wherein A is selected from -(CH₂)₆NH-, -(CH₂)₃NH(CH₂)₃NHCO-, -(CH₂)₃NMe(CH₂)₃NHCO-, -(CH₂)₃NH-, - (CH₂)₂NH(CH₂)₂NHCO- or -(CH₂)₂NMe(CH₂)₂NHCO-.

A further preferred embodiment of Formula II are compounds wherein the DNA-targeting unit is selected from one of formulae **III- XVII,** 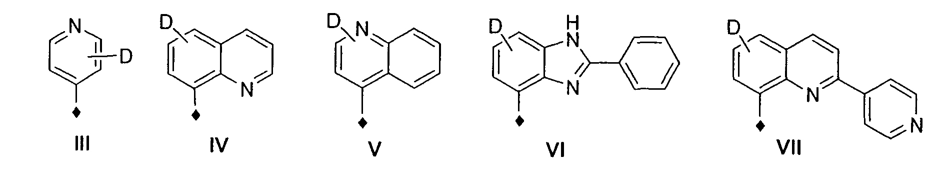 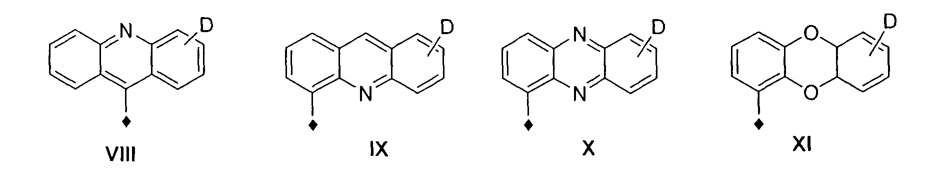 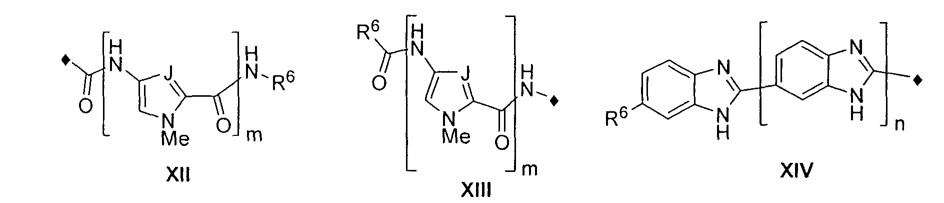 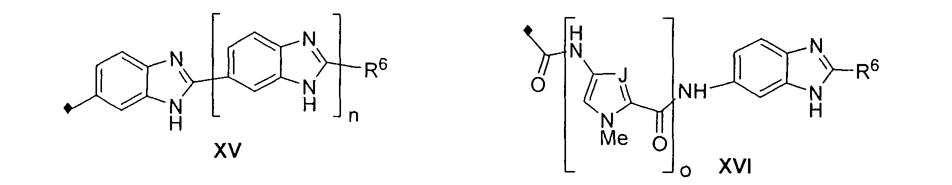 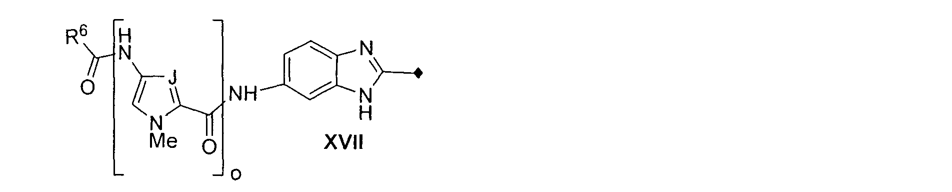 wherein in structures **XII-XVII** R⁶ is independently selected from an optionally substituted C₁₋₆ aliphatic or an optionally substituted C₃₋₆ cyclic alkyl group, and wherein the optional substituents are each independently selected from; halo, OH, OR⁷ NO_{2,} NH_{2,} NHR⁷, NR⁷R⁷, SR⁷, imidazolyl, R⁷-piperazinyl, morpholino, SO₂R⁷, CF_{3,} CN, CO₂H, CO₂R⁷, CHO, COR⁷, CONH_{2,} CONHR⁷, CONR⁷R⁷;
R⁶ may also be represent an optionally substituted aryl or an optionally substituted heteroaryl group having up to 12 carbon atoms, and wherein the optional substituents are each independently selected from; halo, OH, OR⁷, NH_{2,} NHR⁷, NR⁷R⁷, SH, SR⁷, imidazolyl, R⁷-piperazinyl, morpholino, SO₂R⁷, CF_{3,} CN, CO_{2Z}H, CO₂R⁷, CHO, COR⁷, CONH_{2,} CONHR⁷, CONR⁷R⁷, and each heteroaryl group contains one or more heteroatoms in its ring system which are each independently selected from O, N or S;
wherein each R⁷ is independently selected from an optionally substituted C₁₋₄ alkyl or an optionally substituted C₂₋₄ alkenyl group and wherein the optional substituents are each independently selected from OH, OR⁸, NH₂, NHR⁸, NR⁸₂ or N(OH)R⁸ wherein each R⁸ is independently selected from C₁₋₄ alkyl, C₂₋₄ alkenyl, OH, NO₂, NH₂, CF₃, CN, CO₂H or SH;
D may represent up to four of the following groups as substituents at any available ring carbon position; H, R⁹, hydroxy, alkoxy, halogen, NO₂, NH₂, NHR⁹, NR⁹₂, SH, SR⁹, SO₂R⁹, CF₃, CN, CO₂H, CO₂R⁹, CHO, COR⁹, CONH₂, CONHR⁹ or CONR⁹R⁹, cyclic alkylamino, imidazolyl, alkylpiperazinyl, morpholino, wherein each R⁹ is independently selected from an optionally substituted C₁₋₄ alkyl or an optionally substituted C₂₋₄ alkenyl group and wherein the optional substituents are each independently selected from OH, OR¹⁰, NH₂, NHR¹⁰, NR¹⁰₂ or N(OH)R¹⁰ wherein each R¹⁰ is independently selected from C₁₋₄ alkyl, C₂₋₄ alkenyl, OH, NO₂, NH₂, CF₃, CN, CO₂H or SH;
and wherein any available ring carbon position of formulae **III - XVII** may also be optionally replaced by -N- when the valency and configuration of the formula allows, the point of attachment of formulae **III- XVII** to the A group defined above is represented by ◆; and
wherein in formulae **XII, XIII,** m is selected from 2, 3 or 4, and
wherein in formulae **XII, XIII, XVI** and **XVII,** J is selected from CH or N;
and wherein in formulae **XIV** and **XV** n is selected from 0, 1 or 2;
and wherein in formulae **XVI** and **XVII** o is selected from 1 and 2.

A preferred embodiment of formula II is one in which the DNA targeting unit is selected from one of formulae **V, VI, VII, VIII, IX or X.**

A preferred embodiment of formula II is one in which D of the DNA targeting unit of Formulae **III - XI** is H or Me.

Further preferred compounds of formula II include the following
wherein X is NH-, Y is H, Z is N, position 1-oxide, A is -(CH₂)₂NH(CH₂)₂NHCO-, the DNA targeting unit represents formula IX and D is H;
wherein X is NH-, Y is H, Z is N, position 1-oxide, A is -(CH₂)₃NH(CH₂)₃NHCO-, the DNA targeting unit represents formula IX and D is H;
wherein X is NH-, Y is H, Z is N, position 1-oxide, A is -(CH₂)₂NMe(CH₂)₂NHCO-, the DNA targeting unit represents formula IX and D is H;
wherein X is NH-, Y is 6-Me, Z is N, position 1-oxide, A is -(CH₂)₂NMe(CH₂)₂NHCO-. the DNA targeting unit represents formula IX and D is H;
wherein X is NH-, Y is H, Z is N, position 1-oxide, A is -(CH₂)₃NMe(CH₂)₃NHCO-, the DNA targeting unit represents formula IX and D is H;
wherein X is NH-, Y is 6-Me, Z is N, position 1-oxide, A is -(CH₂)₃NMe(CH₂)₃NHCO-, the DNA targeting unit represents formula IX and D is H;
wherein X is NH-, Y is H, Z is N, position 1-oxide, A is -(CH₂)₂NMe(CH₂)₂NHCO-, the DNA targeting unit represents formula IX and D is Me;
wherein X is NH-, Y is H, Z is N, position 1-oxide, A is -(CH₂)₃NMe(CH₂)₃NHCO-, the DNA targeting unit represents formula IX and D is Me.

In an eleventh aspect, there is provided a method of treating a subject in need of cancer therapy, said method comprising the steps of administering to said subject a cytotoxic effective amount of a compound of Formula II as defined above to the tumour cells in said subject.

Preferably the tumour cells are in a hypoxic environment.

Preferably, the method includes the further step of administering the compound of Formula II in combination with one or more other chemotherapeutic agents or treatments, including radiotherapy, either simultaneously, or sequentially, depending on the cancerous condition to be treated.

More preferably the method includes the step of administering radiotherapy to the tumour cells before, during or after the administration of the composition as defined above.

Preferably, the chemotherapeutic agents are selected from Cisplatin or other platinum-based derivatives, Temozolomide or other DNA methylating agents, cyclophosphamide or other DNA alkylating agents, doxorubicin, mitoxandrone, camptothecin or other topoisomerase inhibitors, methotrexate, gemcitabine or other antimetabolites.

While the method of the invention will typically be used in cancer therapy of human subjects, they may be used to target tumour cells in other warm blooded animal subjects such as other primates, farm animals such as cattle, and sports animals and pets such as horses, dogs, and cats.

Preferably, the compound of Formula II is administered with a pharmaceutically acceptable excipient, adjuvant, carrier, buffer or stabiliser. The pharmaceutically acceptable excipient, adjuvant, carrier, buffer or stabiliser should be non-toxic and should not interfere with the efficacy of the active composition. The precise nature of the carrier or other material will depend on the intended route of administration, which may be oral, or by injection such as cutaneous, subcutaneous or by intravenous injection. Pharmaceutical compositions of Formula II for oral administration may be in tablet, capsule, powder or liquid form. A tablet may comprise a solid carrier or an adjuvant Liquid pharmaceutical compositions generally comprise a liquid such as water, petroleum, animal or vegetable oils, mineral oil or synthetic oil. Physiological saline solution, dextrose or other saccharide solution or glycols such as ethylene glycol, propylene glycol may be included. A capsule may comprise a solid carrier such as a gelatine. For intravenous, cutaneous or subcutaneous injection, the active composition will be in the form of a parenterally acceptable aqueous solution that is pyrogen-free and has a suitable pH, isotonicity and stability. Those of skill in the art would be able to prepare suitable solutions using for example, isotonic vehicles such as sodium chloride injection, Ringer's injection, lactated Ringer's injection. Preservatives, stabilisers, buffers, antioxidants and/or other additives may be included as required.

A "cytotoxic effective amount", is to be understood as an amount of a compound of Formula II defined above that is sufficient to show benefit to a patient. The actual amount, rate and time-course of administration, will depend on the nature and severity of the disease being treated. Prescription of treatment is within the responsibility of general practitioners and other medical doctors.

A hypoxic environment is to be understood as tissue environments at an oxygen concentration of < 10 mM.

In a twelth aspect there is provided, the use in the manufacture of a medicament of an effective amount of a compound of Formula II as defined above for the treatment of a subject in need of cancer therapy.

In a thirteenth aspect, the present invention provides a compound of Formula **II'**, 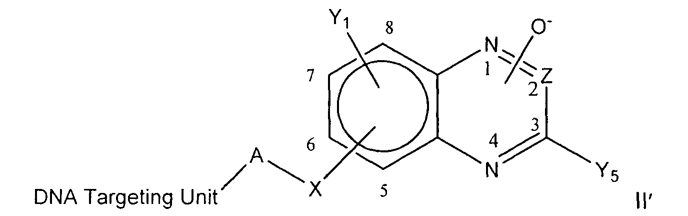 wherein
Z is selected from N or C-CN, and when Z represents N, the N-oxide moiety occupies one of the 1-, 2-, or 4-positions; and when Z represents C-CN, the N-oxide moiety occupies one of the 1-, or 4-positions;
Y₁ may represent at one or more of the available carbons 5-8 on the benzo ring the following groups:
halo, H, R, OH, OR, NO₂, NH₂, NHR, NR₂, SH, SR, SO₂R, CF₃, CN, CO₂H, CO₂R, CHO, COR, CONH₂, CONHR or CONRR, cyclic alkylamino, imidazolyl, alkylpiperazinyl, morpholino;
Y₅ is selected from the following groups halo, H, R, OR, NH₂, NHR, NR₂, SO₂R, CF₃, CN, CO₂H, CO₂R, CHO, COR, CONH₂, CONHR or CONRR, cyclic alkylamino, imidazolyl, alkylpiperazinyl, morpholino;
wherein each R of groups Y₁ and Y₅ is independently selected from an optionally substituted C₁₋₆ aliphatic or an optionally substituted C₃₋₆ cyclic alkyl group, and wherein the optional substituents are each independently selected from; halo, OH, OR', NO_{2,} NH₂, NHR¹, NR¹R¹, SH, SR¹, imidazolyl, R¹-piperazinyl, morpholino, SO₂R¹ CF₃, CN, CO₂H, CO₂R¹, CHO, COR', CONH₂, CONHR¹, CONR¹R¹;
R may also be represent an optionally substituted aryl or an optionally substituted heteroaryl group having up to 12 carbon atoms, and wherein the optional substituents are each independently selected from; halo, OH, OR¹, NH₂, NHR¹, NR¹R¹, SH, SR¹, imidazolyl, R'-piperazinyl, morpholino, SO₂R¹, CF₃, CN, CO₂H, CO₂R¹, CHO, COR¹, CONH₂, CONHR¹, CONR¹R¹, and each heteroaryl group contains one or more heteroatoms in its ring system which are each independently selected from O, N or S;
wherein each R¹ is independently selected from an optionally substituted
C₁₋₄ alkyl or an optionally substituted C₂₋₄ alkenyl group and wherein the optional substituents are each independently selected from OH, OR, NH₂, NHR², NR²₂ or N(OH)R² wherein each R² is independently selected from C₁₋₄ alkyl, C₂₋₄ alkenyl, OH, NO₂, NH₂, CF₃, CN, CO₂H or SH, and
wherein X may represent NH, NMe, CH₂, S, SO, SO₂, CONH, NHCO, CO, CO₂, or O;

A may represent an optionally substituted C₁₋₁₂ alkyl group wherein the optional substituents are each independently selected from OH, OR³, NH₂, NHR³, NR³₂ or N(OH)R³ wherein each R³ is independently selected from C₁₋₄ alkyl, C₂₋₄ alkenyl, OH, NO₂, NH₂, CF₃, CN, CO₂H or SH; and wherein the optionally substituted C₂₋₁₂ alkyl chain is optionally interrupted by one or more heteroatom containing linkage moieties selected from O, NH, NR⁴, CONH, CONR⁴, NHCO, NR⁴CO, where each R⁴ is independently selected from an optionally substituted C₁₋₄ alkyl or an optionally substituted C₂₋₄ alkenyl group and wherein the optional R⁴ substituents are each independently selected from OH, OR, NH₂, NHR⁵, NR⁵₂ or N(OH)R⁵ wherein each R⁵ is independently selected from C₁₋₄ alkyl, C₂₋₄ alkenyl, OH, NO₂, NH₂, CF₃, CN, CO₂H or SH; and
wherein the DNA-targeting unit is any moiety of a molecular weight below 700 Daltons that has an association constant (K) for binding to double-stranded random-sequence DNA of >10³ M⁻¹ at an ionic strength of 0.01 M at 20 °C,
or a pharmacologically acceptable salt thereof.

The definition of the DNA targeting unit above refers to double-stranded random-sequence DNA. An example of such double-stranded random-sequence DNA is DNA extracted from calf thymus.

Preferably, Z is N.

A preferred compound of Formula II' is one in which X is O or CH₂

A further preferred compound of Formula II' is one in which the N-oxide is at the 1-position

A further preferred compound of Formula II' is one in which Y₁ represents H

A further preferred compound of Formula II' is one in which Y₅ represents NHR

A preferred embodiment of Formula II are compounds wherein A is selected from -(CH₂)₆NH-, -(CH₂)₃NH(CH₂)₃NHCO-, -(CH₂)₃NMe(CH₂)₃NHCO-, -(CH₂)₃NH-, -(CH₂)₂NH(CH₂)₂NHCO- or -(CH₂)₂NMe(CH₂)₂NHCO-.

A further preferred embodiment of Formula II are compounds wherein the DNA-targeting unit is selected from one of formulae **III**- **XVII**, 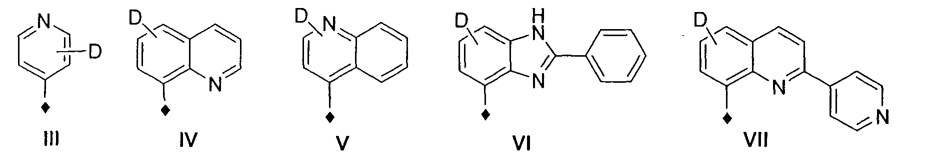 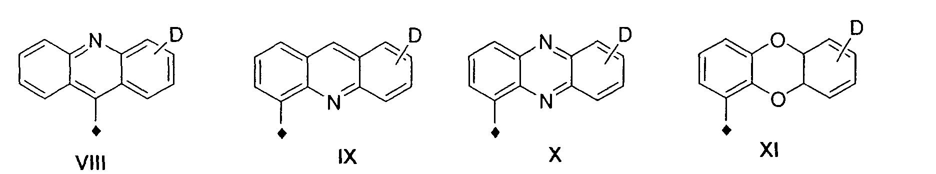 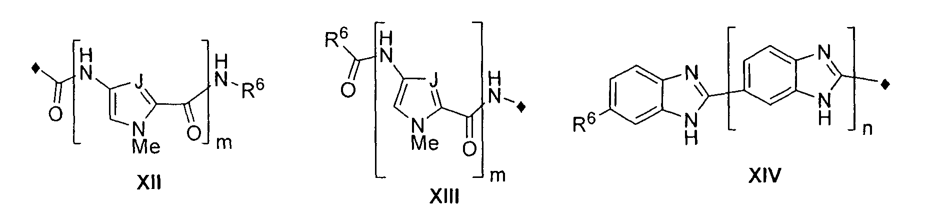 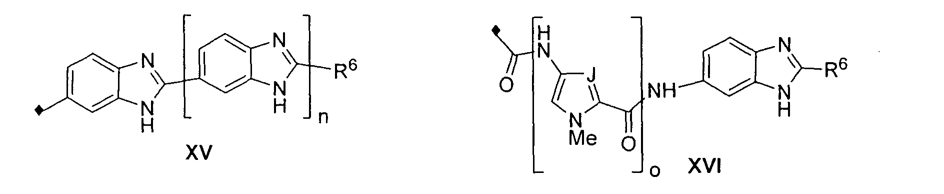 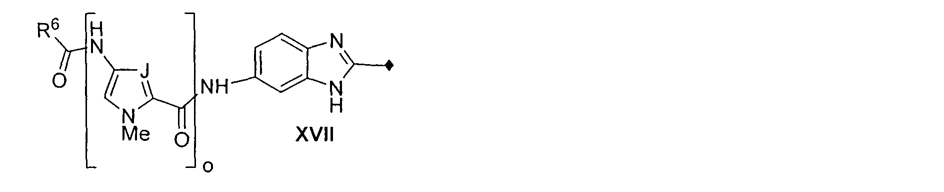 wherein in structures **XII - XVII** R⁶ is independently selected from an optionally substituted C₁₋₆ aliphatic or an optionally substituted C₃₋₆ cyclic alkyl group, and wherein the optional substituents are each independently selected from; halo, OH, OR⁷ NO_{2,} NH_{2,} NHR⁷, NR⁷R⁷, SR⁷, imidazolyl, R⁷-piperazinyl, morpholino, SO₂R⁷, CF_{3,} CN, CO₂H, CO₂R⁷, CHO, COR⁷, CONH_{2,} CONHR⁷, CONR⁷R⁷;
R⁶ may also be represent an optionally substituted aryl or an optionally substituted heteroaryl group having up to 12 carbon atoms, and wherein the optional substituents are each independently selected from; halo, OH, OR⁷, NH_{2,} NHR⁷, NR⁷R⁷, SH, SR⁷, imidazolyl, R⁷-piperazinyl, morpholino, SO₂R⁷, CF_{3,} CN, CO₂H, CO₂R⁷, CHO, COR⁷, CONH_{2,} CONHR⁷, CONR⁷R⁷, and each heteroaryl group contains one or more heteroatoms in its ring system which are each independently selected from O, N or S;
wherein each R⁷ is independently selected from an optionally substituted C₁₋₄ alkyl or an optionally substituted C₂₋₄ alkenyl group and wherein the optional substituents are each independently selected from OH, OR⁸, NH_{2,} NHR⁸, NR⁸₂ or N(OH)R⁹⁸ wherein each R⁸ is independently selected from C₁₋₄ alkyl, C₂₋₄ alkenyl, OH, NO_{2,} NH₂, CF_{3,} CN, CO₂H or SH;
D may represent up to four of the following groups as substituents at any available ring carbon position; H, R⁹, hydroxy, alkoxy, halogen, NO_{2,} NH_{2,} NHR⁹, NR⁹_{2,} SH, SR⁹, SO₂R⁹, CF_{3,} CN, CO₂H, CO₂R⁹, CHO, COR⁹, CONH_{2,} CONHR⁹ or CONR⁹R⁹, cyclic alkylamino, imidazolyl, alkylpiperazinyl, morpholino, wherein each R⁹ independently selected from an optionally substituted C₁₋₄ alkyl or an optionally substituted C₂₋₄ alkenyl group and wherein the optional substituents are each independently selected from OH, OR¹⁰, NH_{2,} NHR¹⁰, N̊R¹⁰ ₂ or N(OH)R¹⁰ wherein each R¹⁰ is independently selected from C₁₋₄ alkyl, C₂₋₄ alkenyl, OH, NO_{2,} NH_{2,} CF_{3,} CN, CO₂H or SH;
and wherein any available ring carbon position of formulae **III- XVII** may also be optionally replaced by -N- when the valency and configuration of the formula allows, the point of attachment of formulae **III- XVII** to the A group defined above is represented by ◆; and
wherein in formulae **XII** and **XIII**, m is selected from 2, 3 or 4,
and wherein in formulae **XII, XIII, XVI** or **XVII** J is selected from CH or N; and
wherein in formulae **XIV** and **XV** n is selected from 0, 1 or 2, and
wherein in formulae **XVI** and **XVII** o is selected from 1 or 2.

A preferred embodiment of formula II' is one in which the DNA targeting unit is selected from one of formulae IV - X.

A preferred embodiment of formula II' is one in which D of the DNA targeting unit of Formulae III - XI is H or Me.

Preferred compounds of formula II' include the following
wherein X is CH₂-, Y₁ is H, Y₅ is NHCH₂CH₂OMe, Z is -N-, A is -(CH₂)NMe(CH₂)₂NHCO-, the DNA targeting unit represents formula IX and D is H;
wherein X is CH₂-, Y₁ is H, Y₅ is NHCH₂CH₂OMe, Z is -N-, A is -(CH₂)₂NH(CH₂)₃NHCO-, the DNA targeting unit represents formula IX and D is H;
wherein X is CH₂-, Y₁ is H, Y₅ is NHCH₂CH₂OMe, Z is -N-, A is -(CH₂)NMe(CH₂)₂NHCO-, the DNA targeting unit represents formula IX and D is Me;
wherein X is CH₂-, Y₁ is H, Y₅ is NHCH₂CH₂OMe, Z is -N-, A is -(CH₂)₂NMe(CH₂)₃NHCO-, the DNA targeting unit represents formula IX and D is Me;
wherein X is CH₂-, Y₁ is H, Y₅ is NHCH₂CH₂OMe, Z is -N-, A is-(CH₂)NMe(CH₂)₂NHCO-, the DNA targeting unit represents formula X and D is Me;
wherein X is CH₂-, Y₁ is H, Y₅ is NHCH₂CH₂OMe, Z is -N-, A is-(CH₂)₂NH(CH₂)₃NHCO-, the DNA targeting unit represents formula X and D is Me;

In a fourteenth aspect, there is provided a method of treating a subject in need of cancer therapy, said method comprising the steps of administering to said subject a cytotoxic effective amount of a compound of Formula II' as defined above to the tumour cells in said subject.

Preferably the tumour cells are in a hypoxic environment.

Preferably, the method includes the further step of administering the compound of Formula II' in combination with one or more other chemotherapeutic agents or treatments, including radiotherapy, either simultaneously, or sequentially, depending on the cancerous condition to be treated.

More preferably the method includes the step of administering radiotherapy to the tumour cells before, during or after the administration of the composition as defined above.

Preferably, the chemotherapeutic agents are selected from Cisplatin or other platinum-based derivatives, Temozolomide or other DNA methylating agents, cyclophosphamide or other DNA alkylating agents, doxorubicin, mitoxandrone, camptothecin or other topoisomerase inhibitors, methotrexate, gemcitabine or other antimetabolites.

While the method of the invention will typically be used in cancer therapy of human subjects, they may be used to target tumour cells in other warm blooded animal subjects such as other primates, farm animals such as cattle, and sports animals and pets such as horses, dogs, and cats.

Preferably, the compound of Formula II' is administered with a pharmaceutically acceptable excipient, adjuvant, carrier, buffer or stabiliser. The pharmaceutically acceptable excipient, adjuvant, carrier, buffer or stabiliser should be non-toxic and should not interfere with the efficacy of the active composition. The precise nature of the carrier or other material will depend on the intended route of administration, which may be oral, or by injection such as cutaneous, subcutaneous or by intravenous injection. Pharmaceutical compositions of Formula II' for oral administration may be in tablet, capsule, powder or liquid form. A tablet may comprise a solid carrier or an adjuvant Liquid pharmaceutical compositions generally comprise a liquid such as water, petroleum, animal or vegetable oils, mineral oil or synthetic oil. Physiological saline solution, dextrose or other saccharide solution or glycols such as ethylene glycol, propylene glycol may be included. A capsule may comprise a solid carrier such as a gelatine. For intravenous, cutaneous or subcutaneous injection, the active composition will be in the form of a parenterally acceptable aqueous solution that is pyrogen-free and has a suitable pH, isotonicity and stability. Those of skill in the art would be able to prepare suitable solutions using for example, isotonic vehicles such as sodium chloride injection, Ringer's injection, lactated Ringer's injection. Preservatives, stabilisers, buffers, antioxidants and/or other additives may be included as required.

A "cytotoxic effective amount", is to be understood as an amount of a compound of Formula II' defined above that is sufficient to show benefit to a patient. The actual amount, rate and time-course of administration, will depend on the nature and severity of the disease being treated. Prescription of treatment is within the responsibility of general practitioners and other medical doctors.

A hypoxic environment is to be understood as tissue environments at an oxygen concentration of < 10 mM.

In a fifteenth aspect there is provided, the use in the manufacture of a medicament of an effective amount of a compound of Formula II' as defined above for the treatment of a subject in need of cancer therapy.

In a sixteenth aspect of the invention, there is provided a method of potentiating the cytotoxicity of an amount of a compound of Formula B or a composition including Formula B as defined above, which has been administered to a subject in need of cancer therapy, by administering to said subject a compound of Formula A or a composition including Formula A as defined above.

Preferably the method potentiates the hypoxic cytotoxicity of an amount of a compound of Formula B.

Preferably, the method includes the further step of administering to said subject the compound of Formula A or a composition including Formula A in combination with one or other chemotherapeutic agents or treatments defined above, including radiotherapy, either simultaneously, or sequentially depending on the cancerous condition to be treated.

More preferably, the method includes the step of administering radiotherapy to the subject, before, during or after the administration of said compound of Formula A or said composition including Formula A.

In a seventeenth aspect of the invention, there is provided a method of potentiating the cytotoxicity of one or more chemotherapeutic agents as defined above, administered to a subject, by further administering to said subject a compound of Formula A or a composition including Formula A as defined above.

Preferably the method potentiates the hypoxic cytotoxicity of the one or more chemotherapeutic agents.

Preferably, the method includes the further step of administering radiotherapy to said subject, either simultaneously, or sequentially depending on the cancerous condition to be treated.

More preferably, the method includes the step of administering radiotherapy to the subject, before, during or after the administration of said compound of Formula A or said composition including Formula A.

It is to be recognised that certain compounds of the present invention may exist in one of more different enantiomeric or diastereomeric forms. It is to be understood that the enantiomeric or diasteriomeric forms are included in the above aspects of the invention.

The term halo or halogen group used throughout the specification is to be taken as meaning a fluoro, chloro, bromo or iodo group.

Further aspects of the present invention will become apparent from the following description given by way of example only and with reference to the accompanying Figures and synthetic schemes, in which:
**Figure 1** shows the potentiation of the anoxic cytotoxicity of the benzotriazine di-N-oxide tirapazamine (TPZ; 30 µM) by the corresponding 1-oxide SR4317 in stirred single cell suspensions of HT29 human colon carcinoma cells at 5×10⁵ cells/ml. Cultures were maintained at <10 ppm O₂ under a continuously-flowing stream of 5%CO₂ in nitrogen and were sampled at intervals to determine plating efficiency. SR4317 alone was non-toxic up to its solubility limit (ca 1 mM).

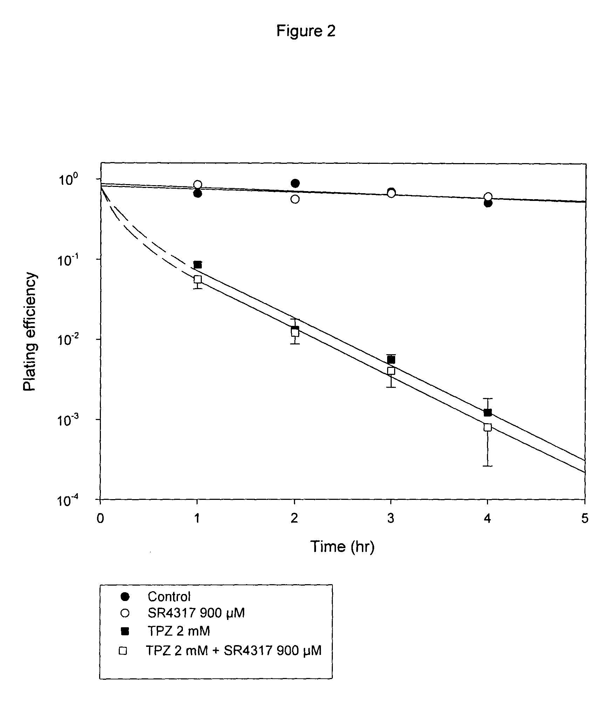

**Figure 2** shows lack of potentiation of the cytotoxicity of TPZ by SR4317 under aerobic conditions. Experimental conditions were as for Figure 2, except that the gas phase was 5% CO₂ in air. Values are means and error bars are ranges for duplicate cultures.

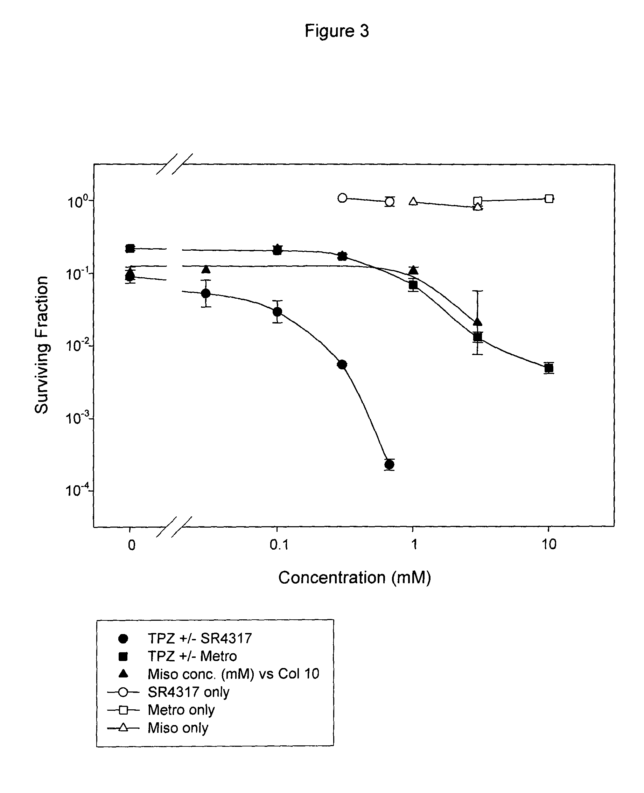

**Figure 3** shows potentiation of the cytotoxicity of TPZ (30 µM) against anoxic HT29 cells (5×10⁵/ml) by SR 4317, misonidazole and metronidazole. Drug exposure time was 1 hr. Error bars represent the range for duplicate determinations.

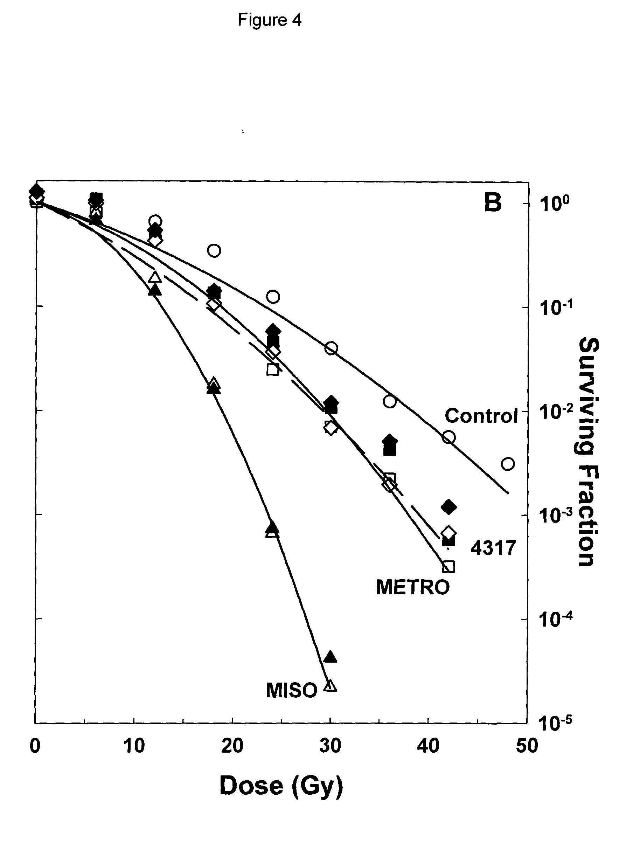

**Figure 4** shows radiosensitisation of anoxic HT29 cells (5×10⁵/ml) by 0.6 mM SR 4317 (squares), 0.6 mM misonidazole (triangles), and 0.6 mM metronidazole (diamonds). Anoxic cell suspensions were irradiated 5 min after addition of pre-equilibrated anoxic drug solutions. Data are shown for duplicate determinations, and are fitted using a linear-quadratic model

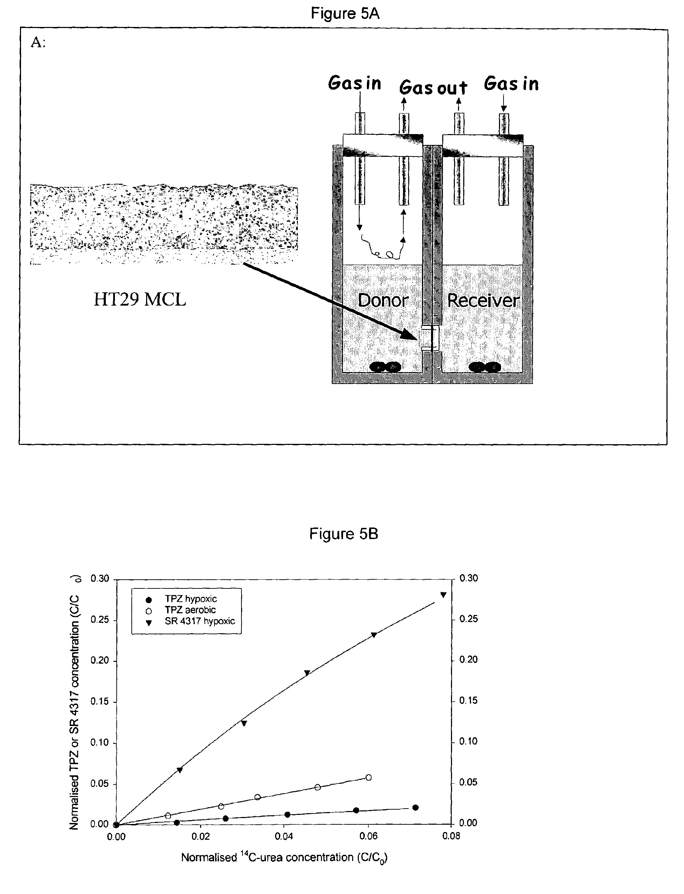

**Figure 5A** shows histology of an HT29 MCL, stained with haematoxylin and eosin, and apparatus (diffusion chamber) for measurement of transport through MCLs. Compounds are added to the donor compartment, along with ¹⁴C-urea as an internal standard, and diffusion into the receiver compartment is monitored by HPLC and scintillation counting.
**Figure 5B** shows transport of SR 4317 (200 µM) and TPZ (50 µM) through oxic and hypoxic HT-29 MCLs (ca 160 µm in thickness). The concentrations in the Receiver compartment (normalised against the initial concentration in the Donor compartment) are plotted against those of the flux marker ¹⁴C-urea to account for small differences in thickness of the MCLs. The fitted diffusion coefficients for TPZ (n=12) and SR 4317 (n=2) are: 3.97 and 32 7×10⁻⁷ cm²sec⁻¹, respectively.

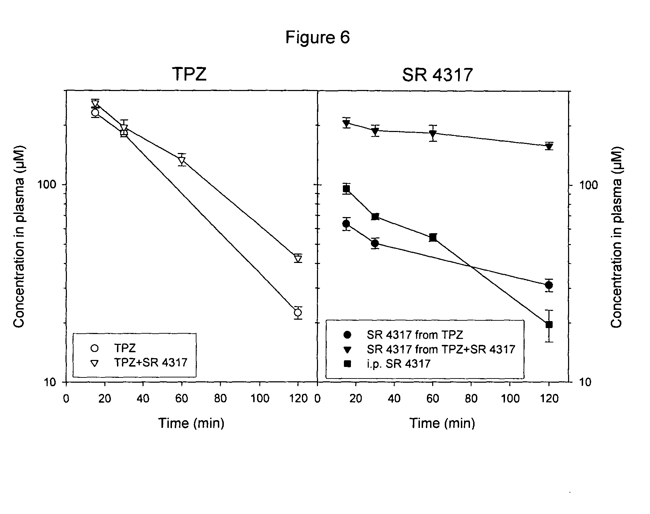

**Figure 6** shows plasma pharmacokinetics of TPZ (left panel) and SR 4317 (right panel) after intraperitoneal administration of TPZ (270 µmol/kg; circles), SR 4317 (750 µmol/kg; squares), or co-administration of TPZ + SR 4317 (triangles) at these doses to CD-1 mice bearing HT29 human tumour xenografts.

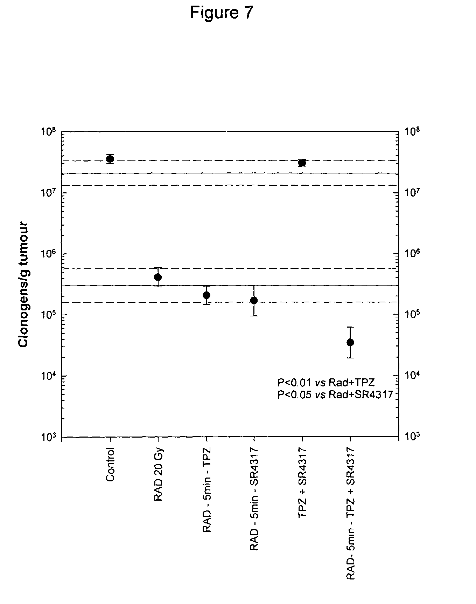

**Figure 7** shows potentiation of the cytotoxicity of TPZ (133 µmol/kg) against hypoxic (radioresistant) cells in HT29 tumours. Animals with subcutaneous tumours (ca 300 mg) received whole body radiation (RAD; 20 Gy) to sterilise oxygenated tumour cells. Activity of drugs against the hypoxic survivors was determined by intraperitoneal administration of solutions in 5% DMSO/saline 5 min after radiation, using TPZ alone (133 µmol/kg) or in combination with SR 4317 (1000 µmol/kg). Tumours were excised 18 hr after treatment, dissociated enzymatically, and plated to determine the number of clonogenic survivors. Values are geometric means and error bars are standard errors of the mean. Horizontal lines show historical values for untreated controls and radiation only (solid lines are means, dashed lines are 95% confidence limits. p values were determined by one-way ANOVA using only data within this experiment.

### DETAILED DESCRIPTION OF THE INVENTION

The inventors have demonstrated that the cytotoxicity of tirapazamine (TPZ) to hypoxic tumour cells can surprisingly be increased quite markedly by simultaneous exposure to SR 4317, as illustrated for HT29 tumour cells in culture in Fig.1. Advantageously, SR 4317 does not potentiate the aerobic toxicity of TPZ (Fig 2), and therefore can be used to increase the hypoxic selectivity of the latter.

This observation provides evidence that the second (DNA radical oxidation) step in the dual action of TPZ as illustrated in Scheme A in the background of the invention is a limiting factor for its hypoxic cytotoxicity. It also demonstrates that the therapeutic utility of TPZ and related analogues could, in principle, be improved by simultaneous exposure to SR 4317 or an analogous DNA radical oxidant.

The inventors have also shown that DNA radical oxidising agents other than TPZ (illustrated by the nitroimidazoles metronidazole and misonidazole) are able to potentiate the hypoxic cytotoxicity of TPZ, although with lower dose potency than SR 4317 (Fig 4). Unexpectedly, comparison of the ability of these agents to radiosensitize hypoxic HT29 cells (Fig 5), under the same conditions as for the TPZ potentiation experiments, shows that the structure-activity relationship for TPZ potentiation (potency SR 4317 > metronidazole = misonidazole) is different from that for radiosensitization (misonidazole > metronidazole = SR4317). The inventors therefore consider that there is a special feature of the benzoazine N-oxide system for potentiation of the hypoxic cytotoxicity of TPZ and its related analogues.

The inventors have also investigated the extravascular transport properties (tissue diffusion characteristics) of SR 4317 to assess whether it can diffuse well enough to reach hypoxic cells in tumours efficiently. This study used the multicellular layer (MCL) assay (Hicks et al., *Int. J. Radiat. Oncol., Biol., Phys.,* **1998,** 42, 641; Hicks et al., *J. Pharmacol. Exper. Ther.,* **2001,** 297, 1088), developed in this laboratory. Transport of SR 4317 through hypoxic MCLs grown from HT29 cells was faster than for TPZ Fig 5, which indicates its ability to reach hypoxic cells in tumours.

The plasma pharrmacokinetics of SR 4317 and TPZ were determined in C₃H mice, alone and in combination (Fig 6), to assess whether therapeutic concentrations of SR 4317 can be achieved in mice. The maximum SR 4317 concentration in plasma, after co-administration of the two compounds, was ca 200 µM with a slow clearance over the first two hours. Based on the *in vitro* results in Fig 3 (showing significant potentiation of TPZ cytotoxicity with SR 4317 at 100 µM for 1 hr), and the efficient tissue penetration of SR 4317 (Fig 4), the plasma concentration profile would appear to be high enough to cause potentiation of TPZ hypoxic cytotoxicity in tumours.

The utility of SR 4317 as a potentiator of the hypoxic cytotoxicity of TPZ was assessed in an *in vivo* model (HT29 tumour xenografts) as illustrated in Fig 7. In this experiment tumour response was determined by excising tumours 18 hr after treatment and quantifying the number of clonogenic survivors by plating *in vitro.* TPZ was administered at a sub-efficacious dose (0.133 mmol/kg), 5 minutes after whole body radiation (20 Gy). As anticipated from earlier experiments, this dose of TPZ did not result in statistically significant killing of the (hypoxic) cells surviving radiation. Adding SR 4317 (1 mmol/kg) to this combination provided activity that was now greater than radiation alone (p <0.01 by one way ANOVA), and the difference between radiation + TPZ vs radiation + TPZ + SR 4317 was also significant (p <0.05). No significant cytotoxicity was observed when the two compounds were administered in the absence of irradiation, indicating lack of activity against aerobic cells in tumours (Fig 7). This experiment demonstrates selective potentiation of the hypoxic cytotoxicity of TPZ in tumours.

It is envisaged that further potentiation over and above that seen with SR 4317 could be readibly achieved. This is because SR4317 has only modest aqueous solubility (ca 800 µM) and provides relatively low plasma concentrations in mice.

The following table shows potentiation of anoxic cytotoxicity results using TPZ and benzotriazine-1-oxides.

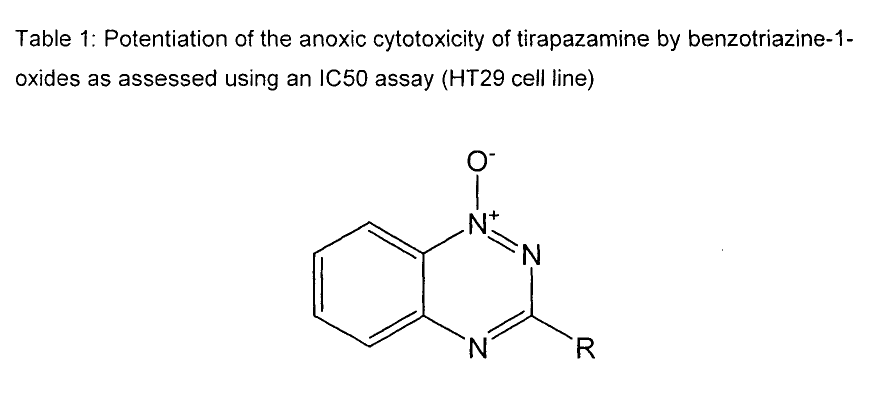

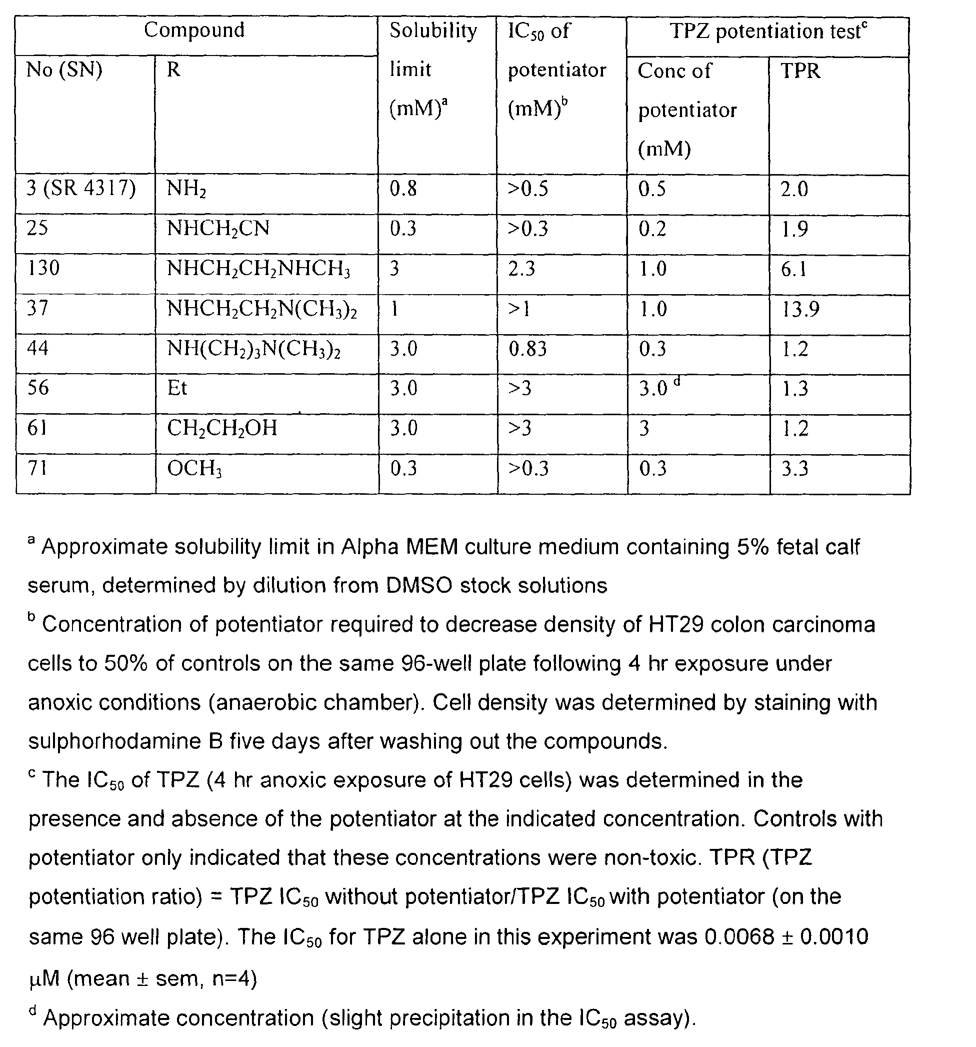

### Methods for preparinq compounds of Formulas I, I', II, and II' of the invention.

Reaction of the appropriately substituted 2-nitroanilines **1** with cyanamide, followed by cyclization of the intermediate guanidine with NaOH (Method A) gave corresponding 1-oxides **3**. In several instances (7-CF₃, 7-NO₂, 5-Cl, 8-F), it was necessary to use an alternative method (Method B) involving reaction of 2-halonitrobenzenes (**2**) with guanidine, followed by cyclization under basic conditions. This gave 1-oxides **3** in modest yield (Scheme 1).

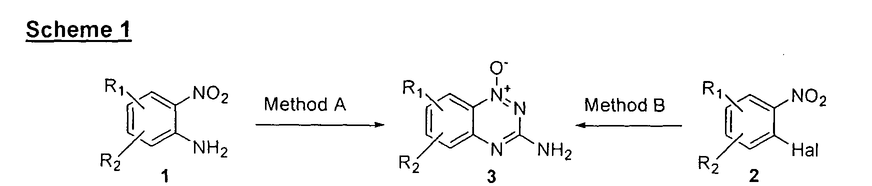

### Method A Reagents:

a) NH₂CN, HCl;
b) NaOH;

### Method B Reagents:

a) Guanidine.HCl, tBuOK.

The sulfides **1f** and **1g** were prepared by substitution of 3-chloro-2-nitroaniline **1d** (Scheme 2).

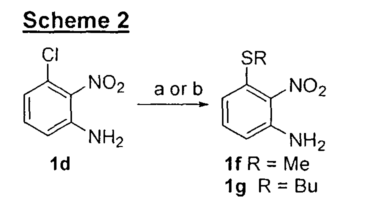

### Reagents:

a) MeSLi, DMF;
b) BuSLi, DMF.

The sulfide 1 n and 1o were prepared using the Newman-Kwart rearrangement (Newman & Karnes, *J. Org. Chem.* 1966, *31*, 3980-3984) and vicarious nucleophilic substitution (V_{N}S) (Seko, et al., J. *Chem. Soc. Perkin Trans. 1* **1999,** 1437-1444; Makosza & Bialecki, *J*. *Org. Chem.* **1998,** *63*, 4878-4888) (Scheme 3). Thus, isomerisation of O-thiocarbamate **4** gave S-thiocarbamate **5,** which was hydrolysed, and the intermediate thiol alkylated with Mel to give sulfide **6.** V_{N}S Reaction of **6** with NH₂OMe.HCl gave nitroanilines **1n** and **1ad.** A similar sequence from **5** gave butylsulfanylnitroaniline **1o** as well as the isomeric **8** and **9.**

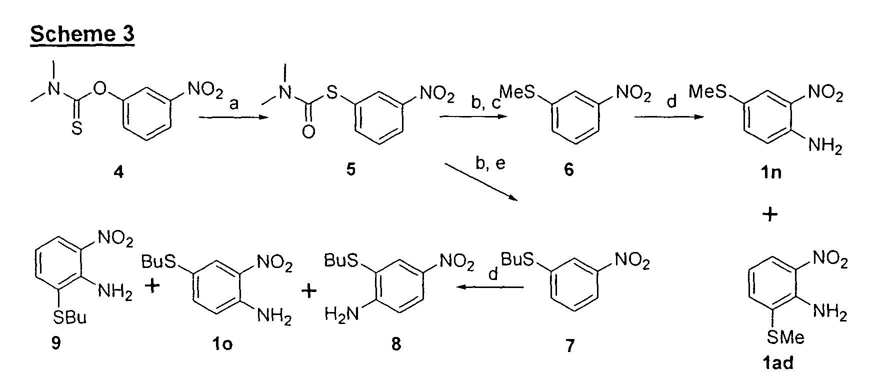

### Reagents:

a) neat, 220 °C;
b) KOH, MeOH;
c) Me₂SO₄, KOH, MeOH;
d) NH₂OMe.HCl, KOtBu, CuCl, DMF;
e) nBuBr, K₂CO₃, DMF.

Nitroaniline **1q** was prepared by Curtius rearrangement of 5-methoxy-2-nitrobenzoic acid (**10**) (Scheme 4). Nitroaniline **1x** was prepared by nucleophilic displacement of **1u** (Scheme 4).

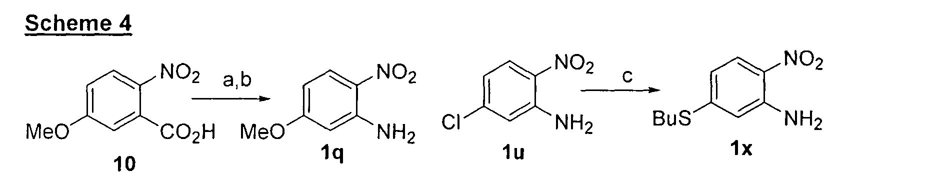

### Reagents:

a) Diphenylphosphorylazide, Et₃N, tBuOH;
b) HCl, MeOH;
c) NBuSLi, DMF.

Displacement of the fluoride **3k** with dimethylamine gave 1-oxide **11** (Scheme 5) and similarly reaction of fluoride **3t** with either dimethylamine or diethylamine gave **12** and **13**, respectively.

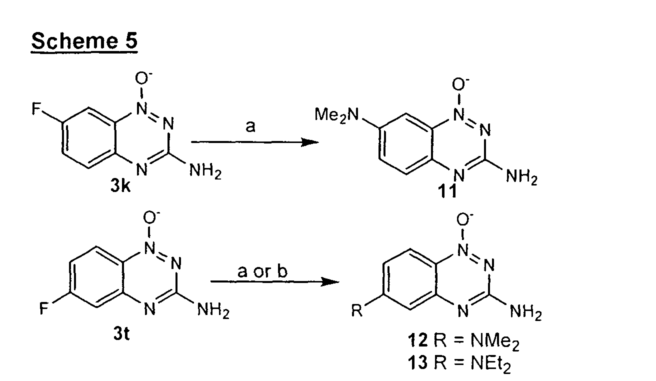

### Reagents:

a) NMe₂, MeCN;
b) NEt₂, MeCN.

Alkylation of the 7-hydroxy-1-oxide **3h** with bromides gave compounds **14-17** (Scheme 6).

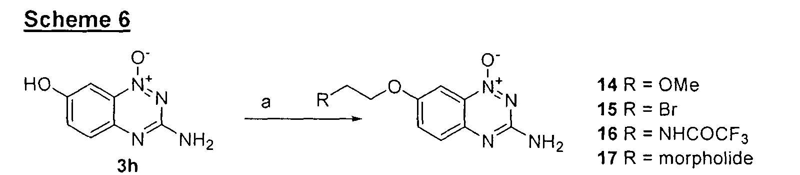

### Reagents:

For **14:** BrCH₂CH₂OMe K₂CO₃, DMF;
For **15:** BrCH₂CH₂Br, K₂CO₃, DMF;
For **16:** BrCH₂CH₂NHCOCF₃, K₂CO₃, DMF;
For **17:** BrCH₂CH₂morpholide, K₂CO₃, DMF.

1,2,4-Benzotriazin-3-amine 1-oxide (**3**) (Scheme 1: R₁ =R₂ =H) was synthesized from. nitroaniline using Method A. Diazotisation gave the phenol **18** which was chlorinated to give chloride **19** (Scheme 7). Reaction of chloride **19** with glycine ethyl ester gave the ester **20.** Base catalysed hydrolysis of the ester gave the acid **21**.

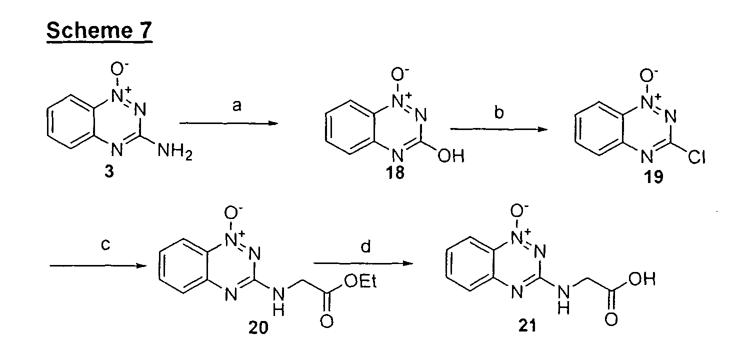

### Reagents

a) NaNO₂, HCl;
b) POCl₃, PhNMe₂;
c) NH₂CH₂CO₂Et, Et₃N, DME;
d) NaOH, MeOH.

Reaction of chloride **19** with various alkylamines in refluxing DME gave 1-oxides **22-29** and **32** in good yields (Scheme 8)

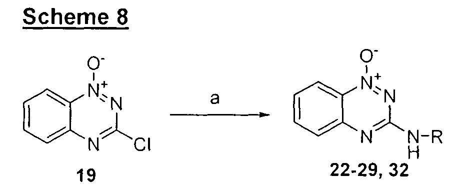

| Compound | Reagent | R = |
|---|---|---|
| **22** | NH₂(CH₂)₂OH | -(CH₂)₂OH |
| **23** | NH₂(CH₂)₂OMe | -(CH₂)₂OMe |
| **24** | NH₂(CH₂)₃OH | -(CH₂)₃OH |
| **25** | NH₂CH₂CN | -CH₂CN |
| **26** | NH₂(CH₂)₂CN | -(CH₂)₂CN |
| **27** | NH₂(CH₂)₃CN | -(CH₂)₃CN |
| **28** | NH₂(CH₂)₃N₃ | -(CH₂)₃N₃ |
| **29** | NH₂(CH₂)₃NHCO₂tBu | -(CH₂)₃NHCO₂tBu |
| **32** | NH₂(CH₂)₃N(Et)CO₂tBu | -(CH₂)₃N(Et)CO₂tBu |

Reaction of chloride **19** with 2-(aminoethoxy)ethanol gave 1-oxide **33** (Scheme 9) which was converted to the azide **34** by reaction with methanesulfonyl chloride and displacement with azide. Reduction with propanedithiol gave selective reduction of the azide **34** to an amine which was protected as carbamate **35.** Deprotection of carbamate **35** under acidic conditions gave amine **36**.

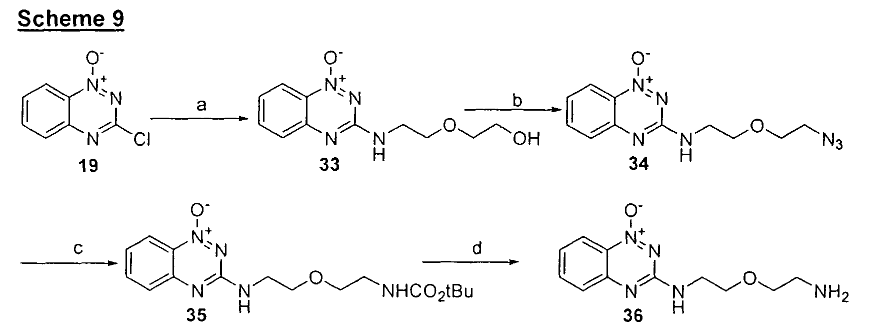

### Reagents:

a) NH₂(CH₂)₂O(CH₂)₂OH, Et₃N, DCM;
b) MsCl, Et₃N, DCM;
c) HS(CH₂)₃SH, Et₃N, MeOH; then di-t-butyldicarbonate, THF;
d) HCl, MeOH.

Reaction of chloride **19** with a variety of amines gave 1-oxides **37, 38, 40-45** (Scheme 10). Reaction of alcohol **22** with methanesulfonyl chloride and displacement with di-n-propylamine gave 1-oxide **39**.

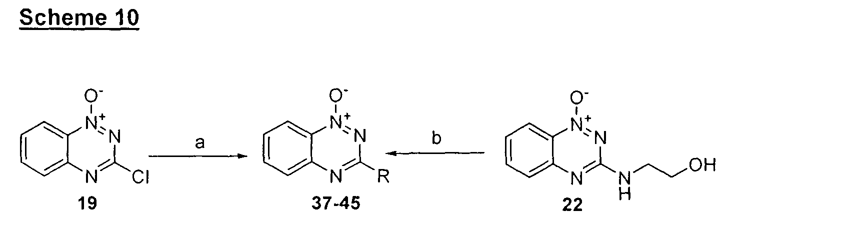

| Compound | Reagents | R = |
|---|---|---|
| **37** | NH₂(CH₂)₂NMe₂, DME | -NH(CH₂)₂NMe₂ |
| **38** | NHMe(CH₂)₂NMe₂, DME | -NMe(CH₂)₂NMe₂ |
| **39** | MsCl, Et₃N, DCM; then HNPr₂ | -NH(CH₂)₂NPr₂ |
| **40** | NH₂(CH₂)₂N-Pyrrolidine, DME | -NH₂(CH₂)₂N-Pyrrolidine |
| **41** | NH₂(CH₂)₂N-Morpholine, DME | -NH(CH₂)₂N-Morpholine |
| **42** | NH₂(CH₂)₂N-piperidine, DME | -NH(CH₂)₂N-piperidine |
| **43** | NH₂(CH₂)₂N-2,6-dimethylpiperidine, DME | -NH(CH₂)₂N-2,6-dimethylpiperidine |
| **44** | NH₂(CH₂)₃NMe₂, DME | -NH(CH₂)₃NMe₂ |
| **45** | Aniline, HCl, DME | -NHPhenyl |

Aniline **48** was prepared by methylation of 3-nitrophenethyl alcohol **46** (Scheme 11) and reduction of the ether **47**. Aniline **48** was coupled to chloride **19** to give 1-oxide 49

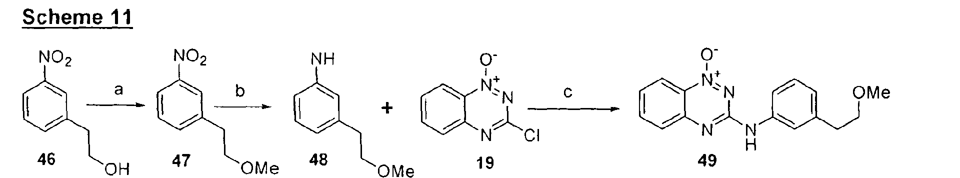

### Reagents:

a) NaH, Mel, THF;
b) H₂, Pd/C, EtOH;
c) **19 + 48**, DMSO.

Reaction of chloride **19** and aniline **50** gave ester **51** (Scheme 12). Hydrolysis of the ester **51** gave acid **52** which was condensed with methoxyethylamine and CDI to give amide **53** Condensation of **52** with N,N-dimethylethanediamine gave **54**

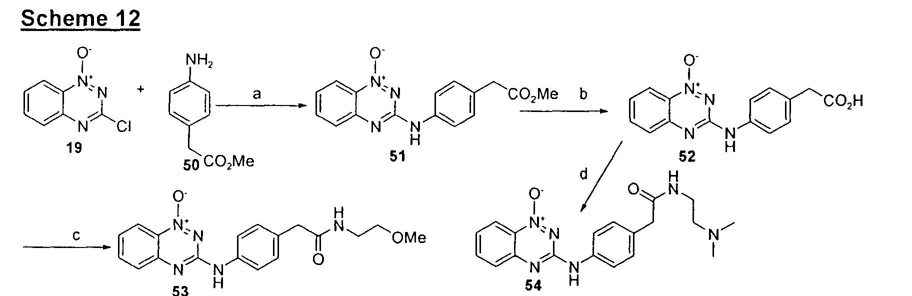

### Reagents:

a) 19 + 50, DMSO;
b) aqueous NaOH, MeOH;
c) CDI, Methoxyethylamine, DMF;
d) CDI, NH₂CH₂CH₂NMe₂, DMF.

Stille reaction of chloride **19** with tetramethyltin and Pd(PPh)₃ in refluxing DME gave the 3-methyl 1-oxide **55** (Scheme 13). Similarly, reaction of **19** with tetraethyltin gave 3-ethyl 1-oxide **56**, reaction with vinyltributyltin gave 3-vinyl 1-oxide **59**, and reaction with allyltributyltin gave 3-allyl 1-oxide **60**. Suzuki reaction of chloride **19** with phenylboronic acid and 4-methoxyphenylboronic acid gave 3-aryl derivatives **57** and **58**, respectively.

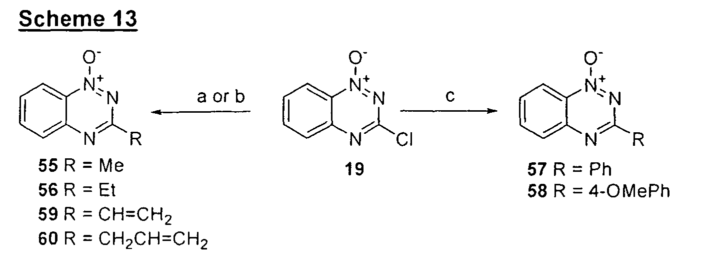

### Reagents:

a) R₄Sn, Pd(PPh₃)₄, DME;
b) NBu₃SnR, Pd(PPh₃)₄, DME;
c) RB(OH)₂, Pd(PPH₃)₄, DME.

Oxidation of alkene **60** with MCPBA gave epoxide **62** (Scheme **14).** Ozonolysis of **60,** followed by a reductive workup gave **61.** Treatment of alcohol **61** with TMS-diazomethane and HBF₄ gave the ether **63.** Treatment of alcohol **61** with methanesulfonyl chloride followed by either dimethylamine or morpholine gave 1-oxides **64** and **65,** respectively.

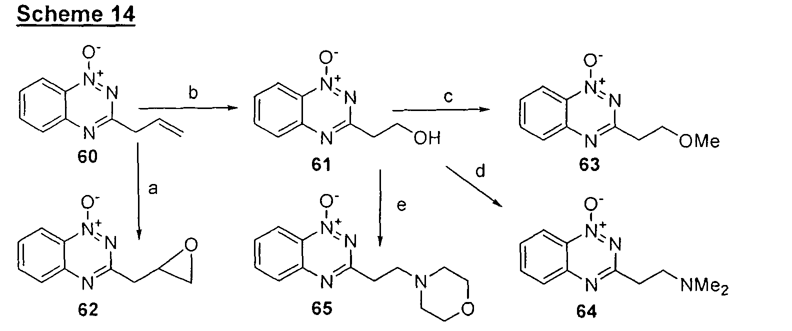

### Reagents:

a) MCPBA, DCM;
b) O₃, DCM, MeOH; then NaBH₄;
c) TMSCH₂N₂, HBF₄, DCM;
d) MsCl, Et₃N, DCM; then HNMe₂.HCl, Et₃N, THF;
e) MsCl, Et₃N, DCM; then morpholine, THF.

Hydroboration and oxidation of alkene **60** gave alcohol **66** which was methylated with TMS-diazomethane and HBF₄ to give ether **67** (Scheme 15). Treatment of alcohol **66** with methanesulfonyl chloride and displacement with secondary amines gave 1-oxides **68-70**.

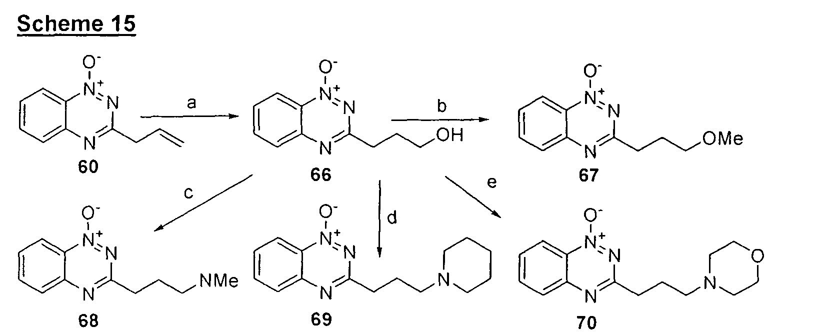

### Reagents.

a) 9-BBN, THF; then 30% H₂O₂, NaOH;
b) TMSCH₂N₂, HBF_{4,} DCM;
c) MsCl, Et₃N, DCM; then HNMe_{2,} DMF;
d) MsCl, Et₃N, DCM; then piperidine, DMF;
e) MsCl, Et₃N, DCM; then morpholine, DMF.

Reaction of chloride **19** with sodium methoxide gave ether **71** (Scheme 16) and similarly reaction of **19** with sodium 2-methoxyethoxide gave ether **72**.

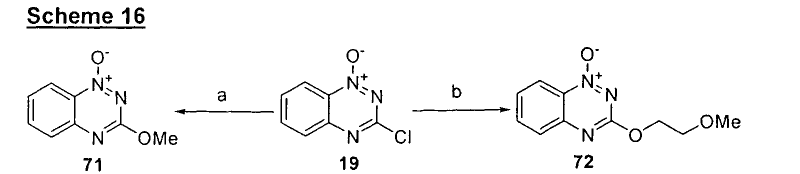

### Reagents:

a) Na, MeOH;
b) Na, MeOCH₂CH₂OH;

Diazotisation of amine **3r** in trifluoroacetic acid and chlorination of the intermediate phenol gave chloride **73** (Scheme 17). Nucleophilic displacement of chloride **73** with a variety of amines gave the 1-oxides **74-78.**

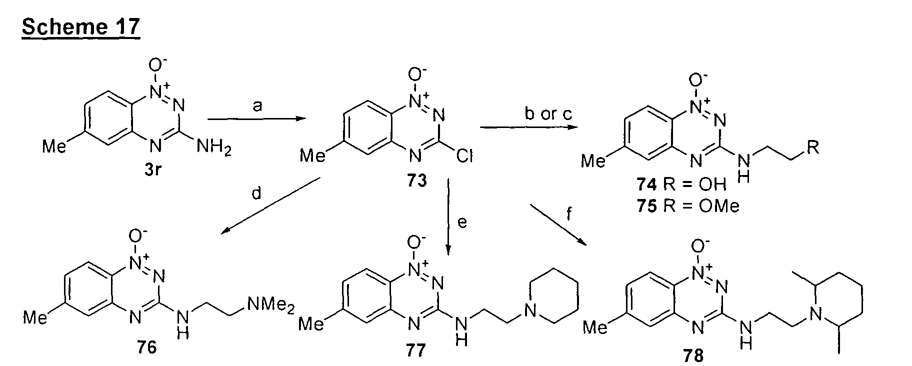

### Reagents:

a) NaNO₂, TFA; then POCl₃, DMF;
b) NH₂CH₂CH₂OH, DME;
c) NH₂CH₂CH₂OMe, DME;
d) NH₂CH₂CH₂NMe₂, DME;
e) NH₂CH₂CH₂N-piperidine, DME;
f) NH₂CH₂CH₂N-2,6-dimethylpiperidine, DME.

Stille reaction of chloride **73** with tetraethyltin and Pd(PP₃)₄ gave 1-oxide **79**, while reaction with allyltributyltin under similar conditions gave 1-oxide **80** (Scheme 18). Ozonolysis of **80** with a reductive workup gave alcohol **81** which was methylated with TMS-diazomethane and HBF₄ to give ether **82.**

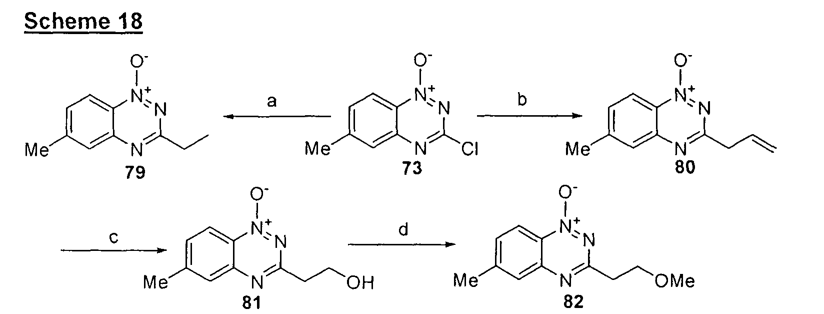

### Reagents:

a) Et₄Sn, Pd(PPh₃)₄, DME;
b) nBu₃Snallyl, Pd(PPh₃)₄, DME;
c) O₃, DCM, MeOH, NaBH₄;
d) TMSCH₂N₂, HBF₄, DCM.

Diazotisation of amine **3q** in trifluoroacetic acid and chlorination of the intermediate phenol gave chloride **83** (Scheme 19). Nucleophilic displacement of chloride **83** with a variety of amines gave the 1-oxides **84-86**.

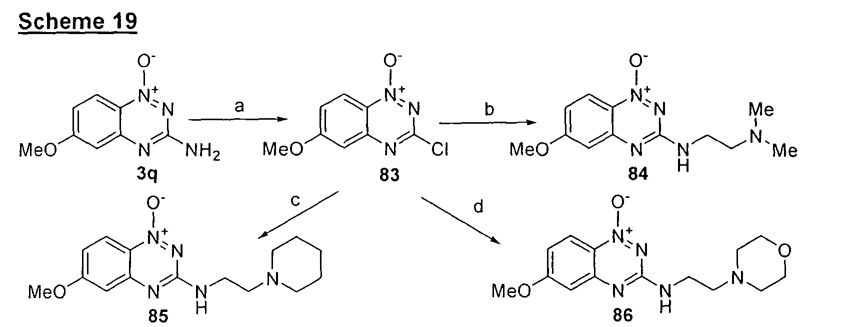

### Reagents:

a) NaNO₂, TFA, then POCl₃, DMF;
b) NH₂CH₂CH₂NMe₂, DME;
c) NH₂CH₂CH₂N-piperidine, DME;
d) NH₂CH₂CH₂N-morpholine, DME.

Diazotisation of amine **3j** and chlorination of the intermediate phenol gave chloride **87** (Scheme 20). Nucleophilic displacement of chloride **87** with a variety of amines gave the 1-oxides **88-90.**

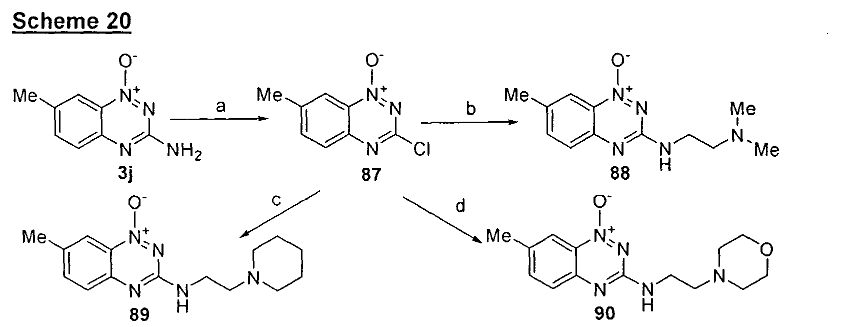

### Reagents:

a) NaNO₂, aq. HCl; then POCl₃, DMF;
b) NH₂CH₂CH₂NMe₂, DME;
c) NH₂CH₂CH₂N-piperidine, DME;
d) NH₂CH₂CH₂N-morpholine, DME.

Diazotisation of amine **3i** and chlorination of the intermediate phenol gave chloride **91** (Scheme 21). Nucleophilic displacement of chloride **91** with a variety of amines gave the 1-oxides **92-94**.

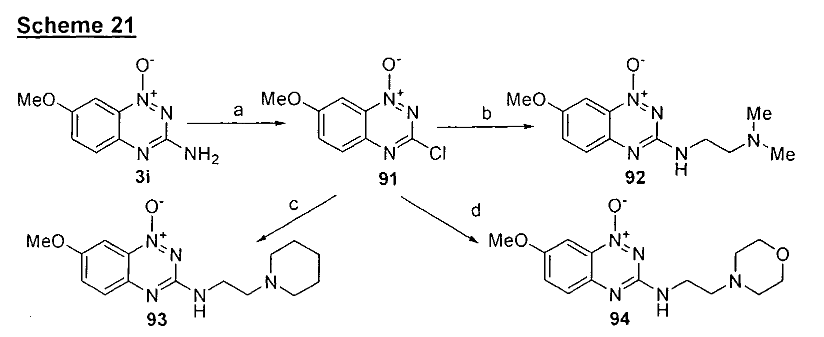

### Reagents:

a) NaNO₂, aq. HCl; then POCl₃, DMF;
b) NH₂CH₂CH₂NMe₂, DME;
c) NH₂CH₂CH₂N-piperidine, DME;
d) NH₂CH₂CH₂N-morpholine, DME.

Diazotisation of amine **14** and chlorination of the intermediate phenol gave chloride **96** (Scheme 22). Stille reaction of chloride **96** with tetraethyltin and Pd(PPh₃)₄ in DMF gave the 1-oxide **97**

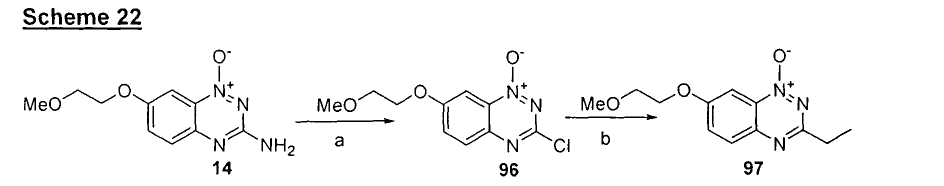

### Reagents:

a) NaNO₂, aq. HCl; then POCl₃, DMF;
b) Et₄Sn, Pd(PPh₃)₄, DMF.

Similarly, diazotisation of amine **17** and chlorination of the intermediate phenol gave chloride **98** (Scheme 23). Stille reaction of chloride **98** with tetraethyltin and Pd(PPh₃)₄ in DMF gave the 1-oxide **99.**

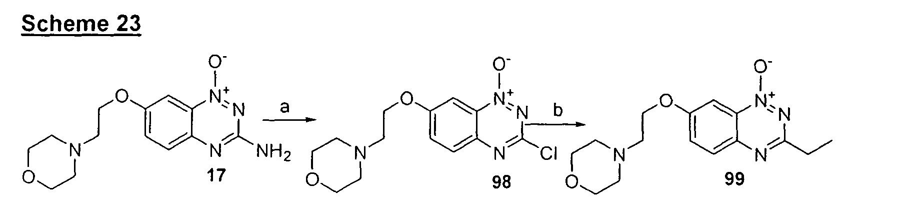

### Reagents:

c) NaNO₂, aq HCl; then POCl₃, DMF;
d) Et₄Sn, Pd(PPh₃)₄, DMF

Diazotisation of amine **3b** and chlorination of the intermediate phenol gave chloride **100** (Scheme 24). Nucleophilic displacement with amines gave 1-oxides **101** and **102**.

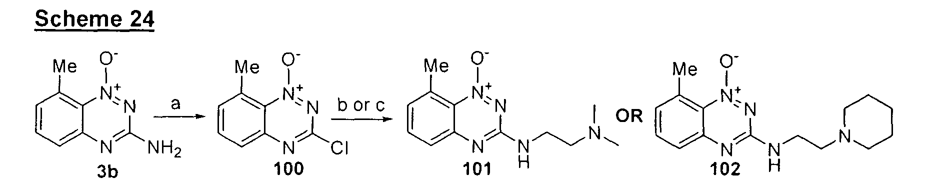

### Reagents:

a) NaNO₂, TFA; then POCl₃, DMF;
b) NHMe(CH₂)₂NMe₂, DME;
c) NH₂(CH₂)₂N-piperidine, DME.

Reaction of nitroaniline **103** with cyanamide and condensation of the intermediate guanidine under basic conditions, followed by diazotisation and chlorination gave chloride **104** (Scheme 25). Displacement of **104** with amines gave 1-oxides **105** and **106**.

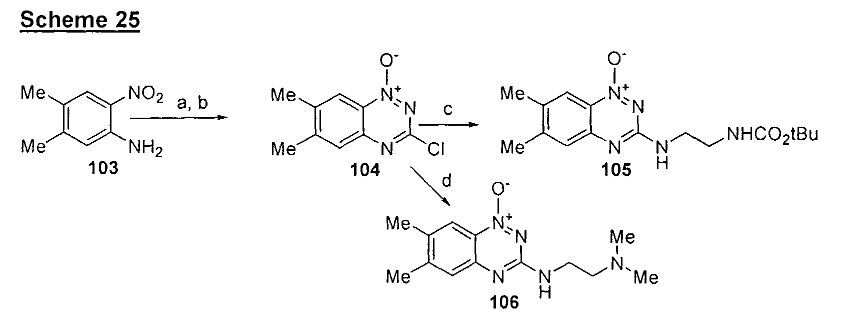

### Reagents:

a) NH₂CN, HCl; then NaOH;
b) NaNO₂, TFA; then POCl₃, DMF;
c) NH₂CH₂CH₂N(Et)CO₂tBu, DME;
d) NHMe(CH₂)₂NMe₂, DME.

Reaction of nitroaniline **107** with cyanamide and condensation of the intermediate guanidine under basic conditions, followed by diazotisation and chlorination gave chloride **108** (Scheme 26). Displacement of **108** with amines gave 1-oxides **109** and **110.**

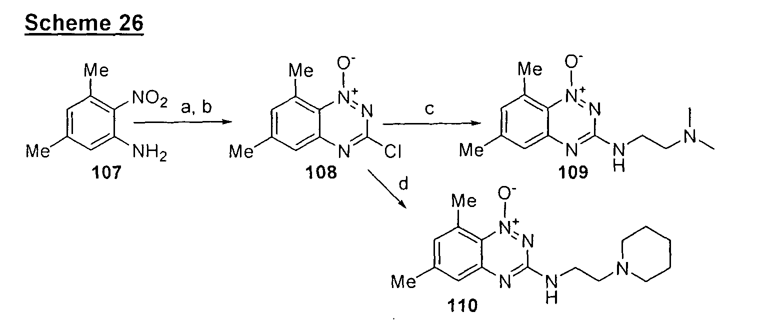

### Reagents:

a) NH₂CN, HCl; then NaOH;
b) NaNO₂, TFA; then POCl₃, DMF;
c) NH₂CH₂CH₂NMe₂, DME;
d) NHMe(CH₂)₂Npiperidine, DME.

Reaction of nitroaniline **111** with cyanamide and condensation of the intermediate guanidine under basic conditions gave amine **112** (Scheme 27). Diazotisation and chlorination of **112** gave chloride **113.** Displacement of **113** with *N*,*N*-dimethylethylenediamine gave 1-oxide **114.**

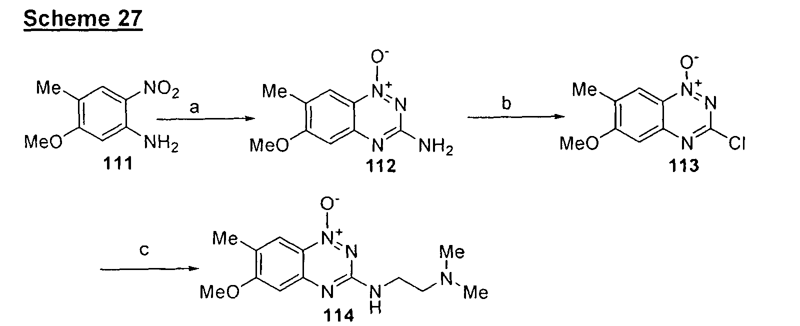

### Reagents:

a) NH₂CN, HCl; then NaOH;
b) NaNO₂, TFA; then POCl₃, DMF;
c) NH₂CH₂CH₂NMe₂, DME.

Reaction of nitroaniline **115** with cyanamide and condensation of the intermediate guanidine under basic conditions gave amine 116 (Scheme 28). Diazotisation and chlorination of **116** gave chloride **117** Displacement of **117** with dimethylethylenediamine gave 1-oxide **118,** while reaction of **117** with 2-(1-piperidinyl)ethylamine gave 1-oxide **119.**

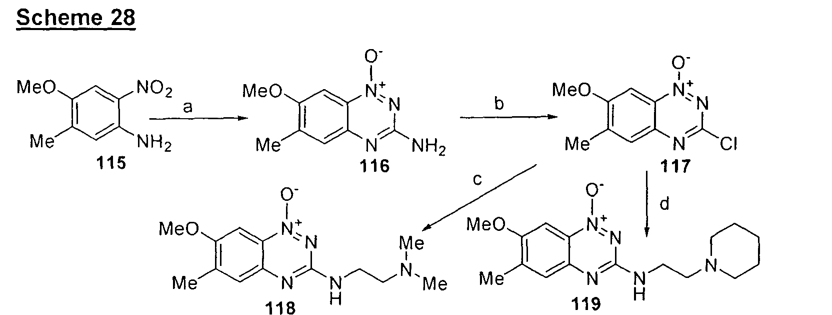

### Reagents:

a) NH₂CN, HCl; then NaOH;
b) NaNO₂, TFA; then POCl₃, DMF;
c) NH₂CH₂CH₂NMe₂, DME;
d) NH₂CH₂CH₂Npiperidine, DME.

The imidazolide of **120** was formed using CDI in DMF and was coupled to amine **36** to give 1-oxide **121** (Scheme 29).

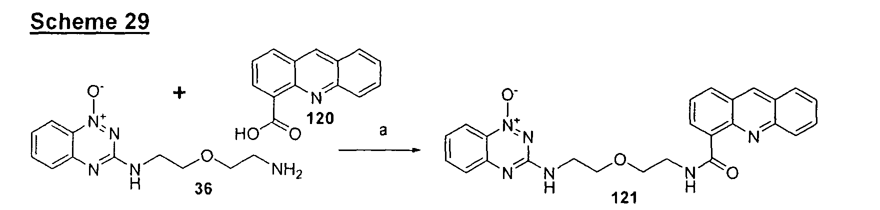

### Reagents.

a) **120**, CDI, DMF; then **36**.

Reaction of the chloride **122** with methoxyethylamine gave 1-oxide **123** (Scheme 30).

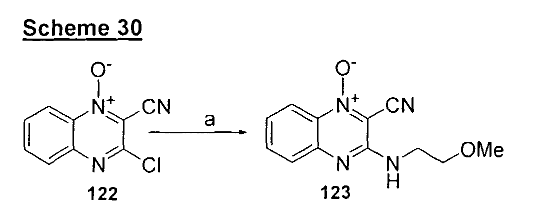

### Reagents:

a) MeOCH₂CH₂NH₂, DME.

Amine **3** was deaminated with isoamyl nitrite in DMF to give 1-oxide **124** (Scheme 31) Reduction of amine **3** with sodium dithionite in aqueous ethanol gave benzotriazine **125** which was oxidised with MCPBA to 1-oxide **3**, 2-oxide **126,** and 4-oxide **127.**

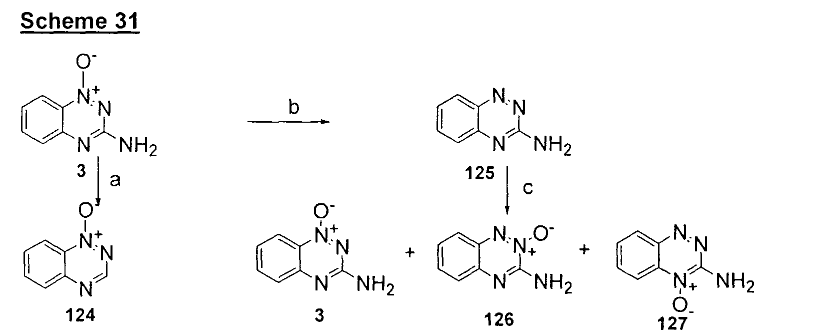

### Reagents

a) Isoamylnitrite, DMF;
b) Na₂S₂O_{4,} 70% EtOH;
c) MCPBA, DCM

Reaction of chloride **19** with diamine **128** gave bis-1-oxide **129** (Scheme 32).

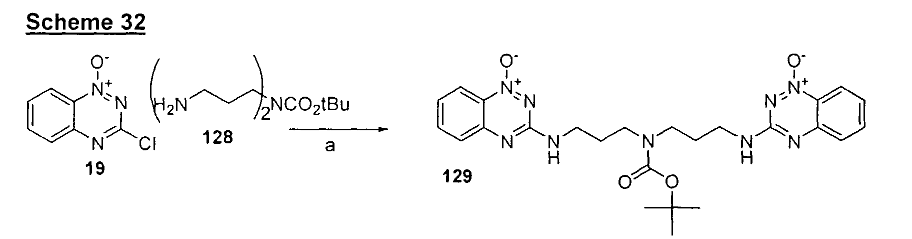

### Reagents:

a) **19 + 128**, Et₃N, DCM.

Reaction of chloride **19** with *N*-methylethylenediamine gave 1-oxide **130** (Scheme 33)

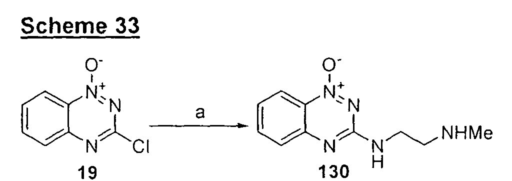

### Reagents:

a) NH₂CH₂CH₂NHMe, Et₃N, DCM

Treatment of 1-oxide **3t** with NaNO₂ in trifluoroacetic acid, followed by chlorination in POCl₃ gave the chloride **131** (Scheme 34). Nucleophilic displacement of chloride **131** with *N*,*N*-dipropylethylenediamine gave the 1-oxide **132.**

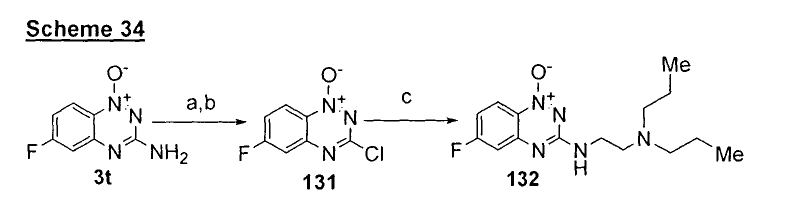

### Reagents:

a) NaNO₂, TFA;
b) POCl₃, DMF;
c) NH₂CH₂CH₂NPr₂, DME.

Similarly, amine **3u** was treated with NaNO₂ in trifluoroacetic acid and the intermediate chlorinated with POCl₃ to give chloride **133** (Scheme 35). Reaction of **133** with a range of amines gave 1-oxides **134-136.**

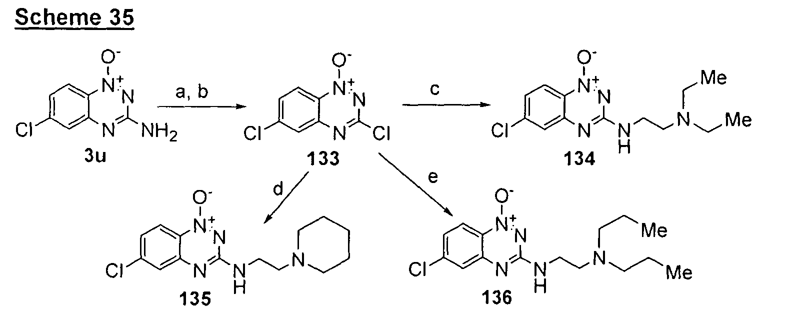

### Reagents:

a) NaNO₂, TFA;
b) POCl₃, DMF;
c) NH₂CH₂CH₂NEt₂, DME,
d) NH₂CH₂CH₂Npipendine, DME;
e) NH₂CH₂CH₂NPr₂, DME.

Reaction of chloride **73** with a range of amines gave 1-oxides **137-140** (Scheme 36).

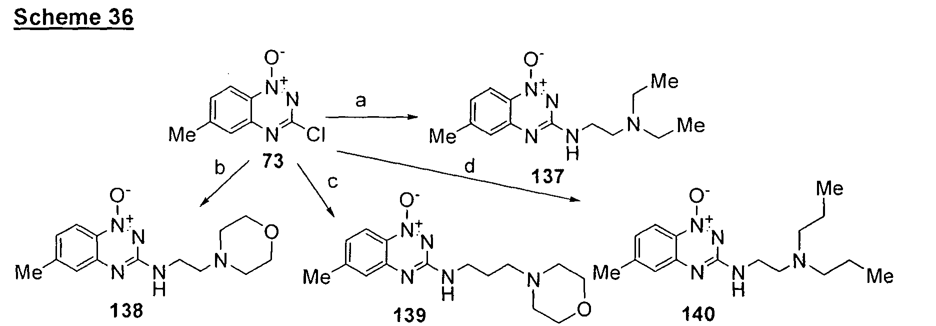

### Reagents:

a) NH₂CH₂CH₂NEt₂, DME;
b) NH₂CH₂CH₂NMorpholine, DME;
c) NH₂CH₂CH₂CH₂NMorpholine, DME;
d) NH₂CH₂CH₂NPr₂, DME.

Reaction of the chloride **83** with the amine gave 1-oxide **141** (Scheme 37).

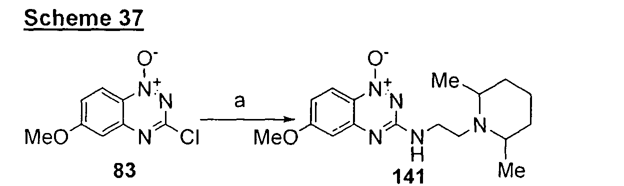

### Reagents

a) NH₂CH₂CH₂N-2,6-Me₂-piperidine, DME.

Treatment of 1-oxide **3v** with NaNO₂ in trifluoroacetic acid, followed by chlorination in POCl₃ gave the chloride **142** (Scheme 38). Nucleophilic displacement of chloride **142** with N,N-dipropylethylenediamine gave the 1-oxide **143**.

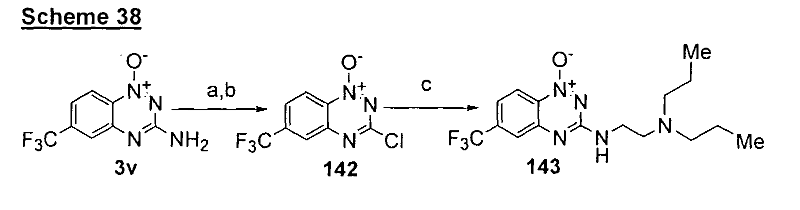

### Reagents:

a) NaNO₂, TFA;
b) POCl₃, DMF;
c) NH₂CH₂CH₂NMe₂, DME.

Treatment of nitroaniline **144** with cyanamide under acidic conditions followed by cyclisation under basic conditions gave 1-oxide **145** (Scheme 39). Diazotization and chlorination of **145** gave chloride **146** Displacement of **146** with a range of amines gave 1-oxides **147-151.**

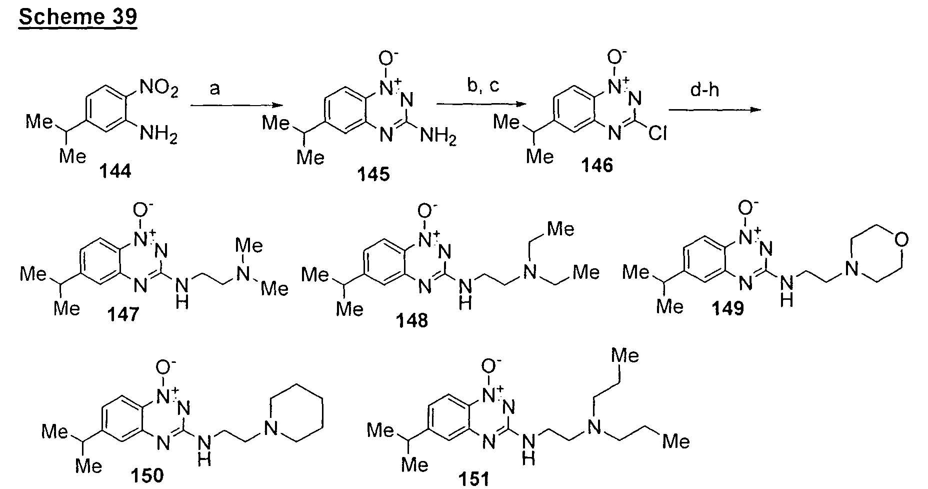

### Reagents

a) NH₂CN, HCl; then NaOH;
b) NaNO_{2,} TFA;
c) POCl₃, DMF;
d) NH₂CH₂CH₂NMe_{2,} DME;
e) NH₂CH₂CH₂NEt_{2,} DME;
f) NH₂CH₂CH₂Nmorpholine, DME;
g) NH₂CH₂CH₂Npiperidine, DME;
h) NH₂CH₂CH₂NPr_{2,} DME.

Treatment of nitroaniline **152** with cyanamide under acidic conditions followed by cyclisation under basic conditions gave 1-oxide **153** (Scheme 40). Diazotization and chlorination of **153** gave chloride **154**. Displacement of **154** with a range of amines gave 1-oxides **155-159**.

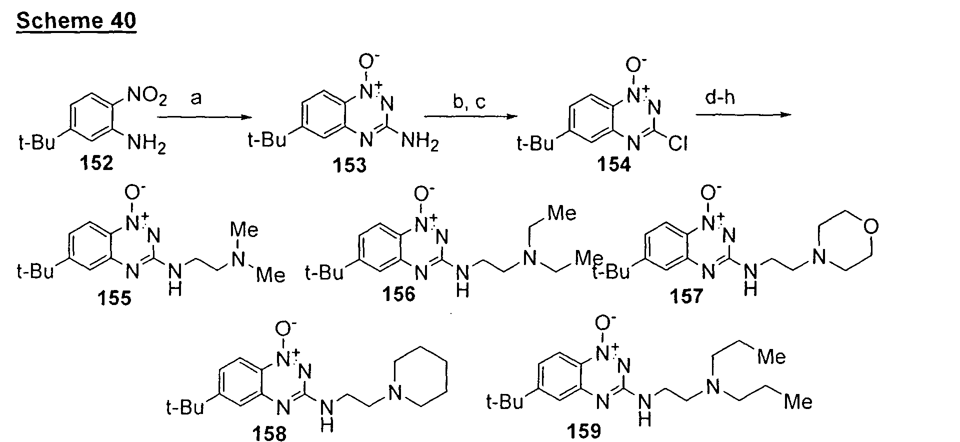

### Reagents:

a) NH₂CN, HCl; then NaOH;
b) NaNO₂, TFA;
c) POCl₃, DMF;
d) NH₂CH₂CH₂NMe₂, DME;
e) NH₂CH₂CH₂NEt₂, DME;
f) NH₂CH₂CH₂Nmorpholine, DME;
g) NH₂CH₂CH₂Npiperidine, DME,
h) NH₂CH₂CH₂NPr₂, DME.

Reaction of chloride **131** under Stille conditions gave 1-oxide **160** which underwent nucleophilic displacement to give 1-oxide **161** (Scheme 41).

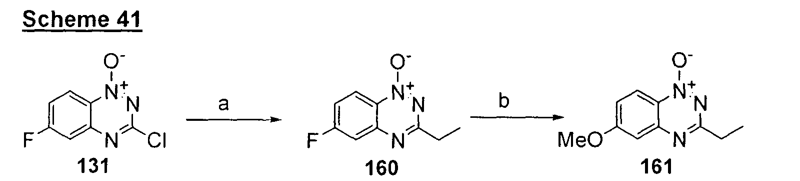

### Reagents:

a) Et₄Sn, Pd(PPh₃)₄, DME;
b) NaOMe, MeOH.

Reaction of the chloride **100** with a range of amines gave 1-oxides **162-165** (Scheme 42)

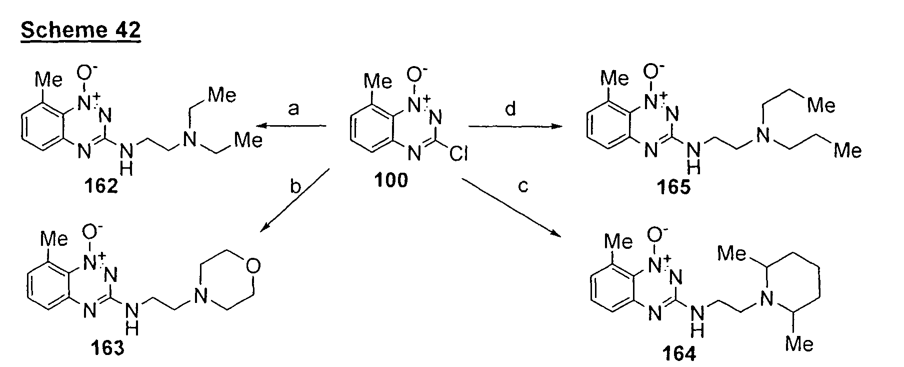

### Reagents:

a) NH₂CH₂CH₂NEt₂, DME;
b) NH₂CH₂CH₂Nmorpholine, DME;
c) NH₂CH₂CH₂N(2,6-Me₂-piperidine), DME;
d) NH₂CH₂CH₂NPr₂, DME.

Reaction of the chloride **104** with a range of amines gave 1-oxides **166-172** (Scheme 43)

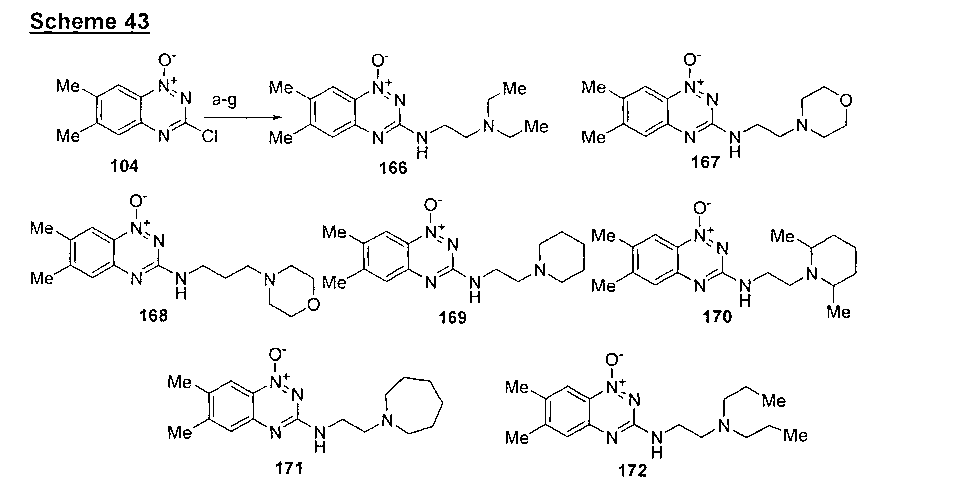

### Reagents:

a) NH₂CH₂CH₂NEt₂, DME;
b) NH₂CH₂CH₂Nmorpholine, DME;
c) NH₂CH₂CH₂CH₂Nmorpholine, DME;
d) NH₂CH₂CH₂CH₂Npiperidine, DME;
e) NH₂CH₂CH₂N(2,6-Me₂-piperidine), DME;
f) NH₂CH₂CH₂CH₂Nazepane, DME;
g) NH₂CH₂CH₂NPr₂, DME.

Reaction of the chloride **108** with *N*',*N*'-dipropyl-1,2-ethanediamine gave 1-oxide **173** (Scheme 44).

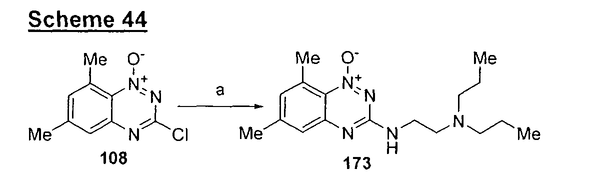

### Reagents:

a) NH₂CH₂CH₂NPr₂, DME.

### Examples of the compounds of the invention

The following examples are representative of the invention and the detailed methods for preparing them, however, the scope of the invention is not to be taken as being limited to these examples.

Analyses were carried out in the Microchemical Laboratory, University of Otago, Dunedin, NZ. Melting points were determined on an Electrothermal 2300 Melting Point Apparatus. NMR spectra were obtained on a Bruker AM-400 spectrometer at 400 MHz for ¹H and 100 MHz for ¹³C spectra Spectra were obtained in CDCl₃ unless otherwise specified, and are referenced to Me₄Si. Chemical shifts and coupling constants were recorded in units of ppm and Hz, respectively. Assignments were determined using COSY, HSQC, and HMBC two-dimensional experiments. Mass spectra were determined on a VG-70SE mass spectrometer using an ionizing potential of 70 eV at a nominal resolution of 1000. High-resolution spectra were obtained at nominal resolutions of 3000, 5000, or 10000 as appropriate. All spectra were obtained as electron impact (EI) using PFK as the reference unless otherwise stated. Solutions in organic solvents were dried with anhydrous Na₂SO₄. Solvents were evaporated under reduced pressure on a rotary evaporator. Thin-layer chromatography was carried out on aluminum-backed silica gel plates (Merck 60 F₂₅₄) with visualization of components by UV light (254 nm) or exposure to I₂. Column chromatography was carried out on silica gel, (Merck 230-400 mesh). All compounds designated for biological testing were analysed at >99% purity by reverse phase HPLC using a Philips PU41 00 liquid chromatograph, a Phenomenex BondClone 10-C18 stainless steel column (300mm x 3.9 mm i.d.) and a Philips PU4120 diode array detector. Chromatograms were run using various gradients of aqueous (1 M NaH₂PO₄, 0.75 M heptanesulfonic acid, 0.5 M dibutylammonium phosphate, and MilliQ water in a 1:1:1:97 ratio) and organic (80% MeOH/MilliQ water) phases. DCM refers to dichloromethane; DME refers to dimethoxyethane, DMF refers to dry dimethylformamide; ether refers to diethyl ether; EtOAc refers to ethyl acetate; EtOH refers to ethanol; MeOH refers to methanol; pet. ether refers to petroleum ether, boiling range 40-60 °C; THF refers to tetrahydrofuran dried over sodium benzophenone ketyl. All solvents were freshly distilled.

### Example 1

**Method A: Condensation of 2-nitroanilines (1) with cyanamide.** 2-Nitroaniline **(1)** (4.3 mmol) and cyanamide (22 mmol) were melted together at 100 °C, cooled to ca. 50 °C, and cHCl (5 mL) added carefully. The mixture was stirred until the exotherm subsided then stirred at 100 °C for 2 h. If necessary, more cyanamide (22 mmol) was added and the mixture stirred at 100 °C for 4 h. The mixture was cooled to 20 °C, made strongly basic with 7.5 M NaOH solution (ca. 50 mL) and the mixture heated at 100 °C for 1 h then cooled to 20 °C and diluted with water (100 mL). The precipitate was filtered, washed with water (2 × 10 mL), ether (2 × 10 mL) and dried. If necessary, the solid was chromatographed, eluting with a gradient (2-5%) of MeOH/CHCl₃, to give the corresponding 1,2,4-benzotriazin-3-amine 1-oxide (3).

### Example 2

**Method B: Condensation of 2-nitrohalobenzenes (2) with guanidine**. Guanidine hydrochloride (104 mmol) was added to a stirred solution of KOtBu (104 mmol) in abs. EtOH (80 mL) and the mixture stirred at 20 °C for 1 h. The mixture was filtered, and the filtrate added slowly to a stirred solution of 2-nitrohalobenzene (**2**) (26 mmol) in absolute EtOH (50 mL). The mixture was heated at reflux temperature for 72 h then cooled, acidified with cHCl and the solvent evaporated. The residue was suspended in 0.5 M HCl and the precipitate was filtered. The aqueous fraction was washed with CHCl₃, basified with aqueous NH₃, and extracted into EtOAc. The organic fraction was dried and the solvent evaporated. The residue was suspended in 10% aq. NaOH and heated at 100 °C for 2 h. The precipitate was filtered, washed with water (2 × 10 mL), ether (2 × 10 mL) and dried. If necessary, the solid was chromatographed, eluting with a gradient (2-5%) of MeOH/CHCl₃, to give the corresponding 1,2,4-benzotriazin-3-amine 1-oxide (3).

### Example 3

**8-Methoxy-1,2,4-benzotriazin-3-amine 1-oxide (3a).** Method A using 3-methoxy-2-nitroaniline **(1a)** (Shigyo et. al., *Chem. Pharm. Bull.* **1993**, 41, 1573) gave **3a** (51%) as a yellow powder, mp (H₂O) 235-239 °C; ¹H NMR [(CD₃)₂SO] δ 7.62 (dd, *J* = 8.3, 8.0 Hz, 1 H, H-6), 7.15 (br s, 2 H, NH₂), 7.02 (d, *J* = 8.3 Hz, 1 H, H-7), 6 80 (d, *J* = 8.0 Hz, 1 H, H-5), 3.83 (s, 3 H, OCH₃); ¹³ C NMR [(CD₃)₂SO] δ 160.0, 153.3, 151.4, 135.4, 122.6, 117.0, 105.1, 56.4; Anal. calc. for C₈H₈N₄O₂: C, 50.0; H, 4.2; N, 29.2; found C, 50.3, H, 4.1, N, 29.2%.

### Example 4

**8-Methyl-1,2,4-benzotriazin-3-amine 1-oxide (3b).** Method A using 3-methyl-2-nitroaniline (**1b**) gave **3b** (100%) as a yellow powder, mp (DMF) 265 °C (dec.); ¹H NMR [(CD₃)_{Z}SO] δ 7.59 (dd, *J* = 8 3, 7.3 Hz, 1 H, H-6), 7.35 (d, *J* = 8.0 Hz, 1 H, H-5), 7.18 (s, 2 H, NH₂), 7.10 (dd, *J* = 7.2, 0.8 Hz, 1 H, H-7), 2.79 (s, 3 H, CH₃); Anal. calc. for C₈H₈N₄O: C, 54 5; H, 4.6; N, 31 8; found C, 54.6; H, 4.7; N, 31.9%.

### Example 5

**8-Fluoro-1,2,4-benzotriazin-3-amine 1-oxide (3c).** Method B using 2,6-difluoroaniline (**2c**) gave **3c** (49%) as a yellow powder, mp (DCM/pet. ether) 270-278 °C (dec) [lit. (Suzuki & Kawakami, *Synthesis* **1977**, 855) mp 271 °C (dec.)]; ¹H NMR [(CD₃)₂SO] δ 7.69 (ddd, *J* = 10.9, 8.3, 5.2 Hz, 1 H, H-6), 7.45 (br s, 2 H, NH₂), 7.31 (dd, J = 9.6, 1.0 Hz, 1 H, H-5), 7.09 (ddd, *J* = 12.0, 8.0, 1.0 Hz, 1 H, H-7); ¹³C NMR [(CD₃)₂SO]δ 160 2 (d, *J* = 5.3 Hz), 153 5 (d, *J* = 264.3 Hz), 151.1 (d, *J* = 3 2 Hz), 135.2 (d, *J* = 4.4 Hz), 121.7 (d, J = 4.5 Hz), 121.1, 110.0 (d, *J* = 20.8 Hz); HRMS (EI⁺) calc. for C₇H₅FN₄O (M⁺) m/z 180.0360, found 180.0441.

### Example 6

**8-Chloro-1,2,4-benzotriazin-3-amine 1-oxide (3d).** Method A using 3-chloro-2-nitroaniline (**1d**) gave **3d** (30%) as a yellow powder, mp (DMF) 280-290 °C (dec.); ¹H NMR [(CD₃)₂SO] δ 7.63 (dd, *J* = 8.4, 7.8 Hz, 1 H, H-6), 7.45 (dd, *J* = 8.6, 1.0 Hz, 1 H, H-7), 7.42 (br s, 2 H, NH₂), 7.36 (dd, *J* = 7.6, 1.1 Hz, 1 H, H-5); HRMS (EI) calc. for C₇H₅N₄O³⁵Cl (M⁺) *m*/*z* 196.0152, found 196.0152; calc for C₇H₅N₄O³⁷Cl (M⁺) m/z 198.0122, found 198.0124.

### Example 7

**8-Trifluoromethyl-1,2,4-benzotriazin-3-amine 1-oxide (3e).** Method A using 3-trifluoromethyl-2-nitroaniline **(1e)** gave **3e** (14%) as a yellow powder, mp (DCM/pet. ether) 280-286 °C (dec.); ¹H NMR [(CD₃)₂SO] δ 7.78-7 87 (m, 3 H, H-5, H-6, H-7), 7 55 (br s, 2 H, NH₂); Anal. calc. for C₈H₅F₃N₄O: C, 41.8; H, 2 2; N, 24.3; F, 24.8; found C, 41.6; H, 2.1; N, 24.3; F, 24.9%.

### Example 8

**8-(Methylsulfanyl)-1,2,4-benzotriazin-3-amine 1-oxide (3f).**
**3-(Methylsulfanyl)-2-nitroaniline (1f).** A solution of LiSMe (1.19 g, 22.0 mmol) in DMF (20 mL) was added dropwise to a stirred solution of 3-chloro-2-nitroaniline (**1d**) (3.17 g, 18.4 mmol) in DMF (80 mL) at 20 °C and the mixture stirred for 2 h. The mixture was poured into water (300 mL) and extracted with EtOAc (2 × 150 mL). The combined organic fraction was washed with water (2 × 100 mL), brine (50 mL), dried, and the solvent evaporated. The residue was chromatographed, eluting with 20% EtOAc/pet ether, to give (i) starting material (0.51 g, 16%) and (ii) sulfide **(1f)** (2.36 g, 70%) as red crystals, mp (EtOAc/pet. ether) 70-72 °C; ¹H NMR δ 7.21 (t, *J* = 8.2 Hz, 1 H, H-5), 6.55 (d, *J* = 8 2 Hz, 2 H, H-4, H-6), 5.92 (br s, 2 H, NH₂), 2.42 (s, 3 H, SCH₃); ¹³C NMR δ 146.0, 141.6, 133.3, 131.0, 113.9 (2), 17.0; Anal. calc. for C₇H₈N₂O₂S: C, 45.6; H, 4 4; N, 15.2; found C, 45.8; H, 4.4; N, 15.1 %.

**8-(Methylsulfanyl)-1,2,4-benzotriazin-3-amine 1-oxide (3f).** Method A using **1f** gave **3f** (68%) as a yellow powder, mp (H₂O) 271-275 °C; ¹H NMR [(CD₃)₂SO] δ 7.63 (dd, *J* = 8.3, 8.0 Hz, 1 H, H-6), 7.28 (s, 2 H, NH₂), 7.17 (d, *J* = 8.3 Hz, 1 H, H-5), 6.98 (d, *J* = 8.0 Hz, 1 H, H-7), 2.39 (s, 3 H, SCH₃); ¹³C NMR [(CD₃)₂SO] δ 159.9, 151.1, 137.1, 134.7, 127.9, 120.1, 118.7, 15.7; Anal. calc. for C₈H₈N₄OS: C, 46.1; H, 3.9; N, 26 9; found C, 45.9; H, 3.9; N, 26.7%.

### Example 9

**8-(Butylsulfanyl)-1,2,4-benzotriazin-3-amine 1-oxide (3g).**
**3-(Butylsulfanyl)-2-nitroaniline (1g).** A solution of LiSBu (1.39 g, 14.5 mmol) in DMF (10 mL) was added dropwise to a stirred solution of 3-chloro-2-nitroaniline **(1d)** (2.08 g, 12.05 mmol) in DMF (50 mL) at 20 °C and the mixture stirred for 2 h. The mixture was poured into water (300 mL) and extracted with EtOAc (2 x 150 mL). The combined organic fraction was washed with water (2 × 100 mL), brine (50 mL), dried, and the solvent evaporated. The residue was chromatographed, eluting with 20% EtOAc/pet. ether, to give sulfide **1g** (2.47 g, 91%) as a red oil, ¹H NMR δ 7.17 (dd, *J* = 8.2, 8.0 Hz, 1 H, H-5), 6 62 (d, *J* = 8.0 Hz, 1 H, H-4), 6.54 (dd, *J* = 8.2, 1.0 Hz, 1 H, H-6), 5.74 (br s, 2 H, NH₂), 2.87 (t, *J* = 7.4 Hz, 2 H, CH₂S), 1.64-1.72 (m, 2 H, CH₂), 1 45-1.53 (m, 2 H, CH₂), 0.95 (s, 3 H, CH₃);¹³C NMR δ 145.3, 140.1, 133.0, 132.4, 115.0, 113 9, 33.0, 29.8, 22.2, 13.6; MS (EI) *m*/*z* 226 (M⁺, 35%), 106 (100); HRMS (EI) calc. for C₁₀H₁₄N₂O₂S (M⁺) m/z 226.0776, found 226 0773.

**8-(Butylsulfanyl)-1,2,4-benzotriazin-3-amine 1-oxide (3g).** Method A using **1g** gave **4i** (26%) as a red/brown solid, mp (MeOH/CHCl₃) 233-236 °C; ¹H NMR [(CD₃)₂SO] δ 7.61 (dd, *J* = 8.2, 7.4 Hz, 1 H, H-6), 7.27 (s, 2 H, NH₂), 7.16 (dd, *J* = 8.2, 0 8 Hz, 1 H, H-5), 7.04 (dd, *J* = 7.4, 0 8 Hz, 1 H, H-7), 2.86 (t, *J* = 7.3 Hz, 2 H, CH₂S), 1 62-1.70 (m, 2 H, CH₂), 1.44-1.52 (m, 2 H, CH₂), 0.93 (s, 3 H, CH₃); ¹³C NMR [(CD₃)₂SO] δ 159.8, 151.1, 136.4, 134.7, 128.0, 120.1, 119 1, 31.0, 28.8, 21.7, 13.5; Anal calc for C₁₁H₁₄N₄OS: C, 52.8; H, 5.6; N, 22.4; found C, 52.7; H, 5.6; N, 22.6%.

### Example 10

**3-Amino-1,2,4-benzotriazin-7-ol 1-oxide (3h).** Method A using 4-amino-3-nitrophenol (**1h)** gave **3h** (97%) as a yellow powder, mp > 300°C [lit. (Friebe et. al., *US Patent* 5,856,325, Jan 5, 1999) mp (HOAc) >270 °C]; ¹H NMR [(CD₃)₂SO] δ 10.37 (br s, 1 H, OH), 7 48 (dd, *J* = 7.7, 2.6 Hz, 1 H, H-6), 7.40-7 37 (m, 2 H, H-5, H-8), 6.96 (br s, 2 H, NH₂).

### Example 11

**3-Amino-1,2,4-benzotriazin-7-ol 1-oxide (3h).** Method A using 4-hydroxy-2-nitroaniline **(1i)** gave **3i** (93%) as a yellow powder, mp (HOAc) 269-271 °C [lit. (Mason & Tennant, *J. Chem.* Soc. *(B)* **1970,** 911) mp (HOAc) 271°C]; ¹H NMR [(CD₃)₂SO] δ 7.48-7.53 (m, 3 H, H-5, H-6, H-8), 7.10 (br s, 2 H, NH₂), 3.88 (s, 3 H, OCH₃); ¹³C NMR [(CD₃)₂SO] δ 159.3, 156.3, 144.9, 129.7, 128.3, 127.3, 97.9, 55.8.

### Example 12

**7-Methyl-1,2,4-benzotriazin-3-amine 1-oxide (3j).** Method A using 4-methyl-2-nitroaniline (**1j**) gave **3j** (74%) as a yellow powder, mp (DMF) 270 °C (dec.) [lit. (Pazdera & Potacek, *Chem. Papers* 1988, 42, 527) mp (Methylcellosolve) 282°C]; ¹H NMR [(CD₃)₂SO] δ 7 94 (s, 1 H, H-8); 7.64 (dd, *J* = 8.7, 1.9 Hz, 1 H, H-6), 7.46 (d, *J* = 8.6 Hz, 1 H, H-5), 7.21 (s, 2 H, NH₂), 2.42 (s, 3 H, CH₃); Anal. calc. for C₈H₈N₄O: C, 54.5; H, 4.6; N, 31.8; found C, 54.8; H, 4.5; N, 31.9%.

### Example 13

**7-Fluoro-1,2,4-benzotriazin-3-amine 1-oxide (3k).** Method A using 4-fluoro-2-nitroaniline (**1k**) gave **3k** (78%) as a yellow powder, mp (DMF) 280-290 °C (dec.) [lit. (Suzuki & Kawakami, *Synthesis* **1977**, 855) mp 290 °C (dec )]; ¹H NMR [(CD₃)₂SO] δ 7.89 (dd, *J* = 8.6, 2 9 Hz, 1 H, H-8), 7.76 (ddd, *J* = 9.3, 8.8, 2.9 Hz, 1 H, H-6), 7.62 (dd, *J* = 9.3, 5.2 Hz, 1 H, H-5), 7.35 (br s, 2 H, NH₂); Anal. calc. for C₇H₅FN₄O: C, 46.7, H, 2 8; N, 31.1, F, 10.6; found C, 46.7; H, 2.7; N, 31.1; F, 10.7%.

### Example 14

**7-Chloro-1,2,4-benzotriazin-3-amine 1-oxide (31).** Method A using 4-chloro-2-nitroaniline **(1l)** gave **3l** (39%) as a yellow powder, mp (DCM/pet. ether) 309 °C (dec.) [lit (Pazdera & Potacek, *Chem. Papers* **1988**, 42, 527) mp (HOAc) 306-308 °C]; ¹H NMR [(CD₃)₂SO] δ 8.14 (d, *J* = 1.7 Hz, 1 H, H-8), 7.80 (dd, *J* = 8.8, 1.9 Hz, 1 H, H-6), 7.56 (d, *J* = 9.0 Hz, 1 H, H-5), 7.48 (br s, 2 H, NH₂).

### Example 15

**7-Trifluoromethyl-1,2,4-benzotriazin-3-amine 1-oxide (3m).** Method B using 1-chloro-2-nitro-4-(trifluoromethyl)benzene **(2m)** gave **3m** (30%) as a yellow powder, mp (DCM/pet. ether) 290 °C (dec.) [lit. (Suzuki & Kawakami, *Synthesis* **1977,** 855) mp (acetone/toluene) 301-302 °C]; ¹H NMR [(CD₃)₂SO] δ 8.38 (br s, 1 H, H-8), 8.01 (dd, *J* = 8 9, 2.0 Hz, 1 H, H-6), 7.72 (br s, 2 H, NH₂), 7.68 (d, *J* = 8.9 Hz, 1 H, H-5); ¹³C NMR [(CD₃)₂SO] δ 161.1, 150.6, 130.7 (*J* = 2.9 Hz), 129.3, 127.5, 123.4 (q, *J* = 272.0 Hz), 123.6 (q, *J* = 32.1 Hz), 118.0 (q, *J* = 10.9 Hz).

### Example 16

**7-(Methylsulfanyl)-1,2,4-benzotriazin-3-amine 1-oxide (3n).**
**4-(Methylsulfanyl)-2-nitroaniline (1n) and 6-(methylsulfanyl)-2-nitroaniline (1ad).** *O*-(3-Nitrophenyl)dimethylthiocarbamate (Newman & Karnes, *J. Org. Chem.* **1966,** *31*, 3980) **(4)** (14.05 g, 62.1 mmol) was heated at 235-240 °C for 3 h under N₂, cooled to 20 °C to give crude S-(3-nitrophenyl)dimethylthiocarbamate **(5)** which was heated at reflux temperature with KOH solution (7.5 M, 410 mL, 3.1 mol) and MeOH (200 mL) for 2 h. The mixture was cooled to 20 °C and Me₂SO₄ (59 mL, 0.62 mol) added dropwise and the mixture stirred at 20 °C for 16 h. The mixture was partitioned between EtOAc (300 mL) and water (300 mL), the organic fraction washed with water (3 × 100 mL), brine (100 mL), dried, and the solvent evaporated. The residue was chromatographed, eluting with 10% EtOAc/pet. ether, to give 1-(methylsulfanyl)-3-nitrobenzene (**6**) (9.6 g, 91 %) as a soft solid, ¹H NMR δ 8 05 (dd, *J* = 2.0, 1.9 Hz, 1 H, H-2), 7.96 (dd, *J* = 8.2, 2.0, 0.9 Hz, 1 H, H-4), 7.53 (ddd, *J* = 7.9, 1.9, 1.0 Hz, 1 H, H-6), 7 43 (dd, *J* = 8.2, 7.9 Hz, 1 H, H-5), 2.56 (s, 3 H, SCH₃); ¹³C NMR δ 148.6, 141.6, 131.9, 129.4, 120.3, 119.6, 15.4
A solution of NH₂OMe HCl (2.83 g, 34.0 mmol) and nitrobenzene **6** (4.79 g, 28.3 mmol) in DMF (100 mL) was added dropwise to a stirred mixture of KOtBu (13.0 g, 116 6 mmol) and CuCl (0.28 g, 2.83 mmol) in DMF (50 mL) at 5 °C. The mixture was stirred at 20 °C for 3 h, quenched with saturated aqueous NH₄Cl solution (100 mL). The mixture was extracted with EtOAc (3 × 100 mL), the combined organic fraction dried, and the solvent evaporated. The residue was chromatographed, eluting with 10% EtOAc/pet. ether, to give (i) 6-(methylsulfanyl)-2-nitroaniline (**1ad**) (2.11 g, 40%) as a red oil, ¹H NMR δ 8 11 (dd, *J* = 8.7, 1.5 Hz, 1 H, H-3), 7.66 (dd, *J* = 7.4, 1.5 Hz, 1 H, H-5), 6.92 (br s, 2 H, NH₂), 6.66 (dd, *J* = 8.7, 7.4 Hz, 1 H, H-4), 2.38 (s, 3 H, SCH₃); ¹³C NMR δ 145.0, 140.7, 132.5, 126.6, 124.5, 115.9, 18.2; MS (El) m/z 184 (100, M⁺), 169 (10), 150 (30); HRMS calc. for C₇H₈N₂O₂S (M⁺) m/z 184.0307, found 184.0304; (ii) 4-(methylsulfanyl)-2-nitroaniline **(1n)** (0.8 g, 15%) as a red oil, ¹H NMR δ 8.05 (d, *J* = 2.3 Hz, 1 H, H-3), 7.34 (dd, *J* = 8.7, 2.3 Hz, 1 H, H-5), 6.76 (d, *J* = 8.7 Hz, 1 H, H-6), 6 05 (br s, 2 H, NH₂), 2.46 (s, 3 H, SCH₃); ¹³C NMR δ 143.1, 136.8, 132 2, 125.8, 125.1, 119 5, 18 0.

**7-(Methylsulfanyl)-1,2,4-benzotriazin-3-amine 1-oxide (3n).** Method A using 1n gave 3n (56%) as a red solid, mp (MeOH/CHCl₃) 245-247 °C; ¹H NMR [(CD₃)₂SO] δ 7 79 (d, *J* = 2.1 Hz, 1 H, H-8), 7.67 (dd, *J* = 8.9, 2.1 Hz, 1 H, H-6), 7.47 (d, *J* = 8.9 Hz, 1 H, H-5), 7.28 (s, 2 H, NH₂), 2.58 (s, 3 H, SCH₃); ¹³C NMR [(CD₃)₂SO] δ 159.9, 147.0, 135.7, 134.6, 130.1, 126.5, 113.4, 14.6; Anal. calc. for C₈H₈N₄OS: C, 46.1; H, 3 9; N, 26.9; found C, 46.1; H, 3.8; N, 26.6%.

### Example 17

**7-(Butylsulfanyl)-1,2,4-benzotriazin-3-amine 1-oxide (3o).**
**4-(Butylsulfanyl)-2-nitroaniline (1o) and 6-(butylsulfanyl)-2-nitroaniline (9).** A mixture of crude **5** (7.0 g, 31 mmol) and KOH (7.5 M, 41 mL, 310 mmol) in MeOH (200 mL) was heated at reflux temperature for 2 h. The mixture was cooled to 5 °C and the pH adjusted to 2 with cHCl. The precipitate was collected, washed with water (20 mL), dissolved in EtOAc (200 mL), dried, and the solvent evaporated. The residue was dissolved in DMF (100 mL) and K₂CO₃ (5.15 g, 37.3 mmol) added and the mixture stirred at 20 °C for 30 min. n-Butylbromide (4.0 mL, 37.3 mmol) was added and the mixture stirred at 80 °C for 16 h. The mixture was cooled and the solvent evaporated. The residue was partitioned between EtOAc (300 mL) and water (300 mL), the organic fraction washed with water (2 × 100 mL), brine (100 mL), dried, and the solvent evaporated. The residue was chromatographed, eluting with 10% EtOAc/pet. ether, to give 3-(butylsulfanyl)nitrobenzene (**7**) (5.53 g, 84%) as a yellow oil, ¹H NMR δ 8.10 (dd, *J* = 2.0, 2.0 Hz, 1 H, H-2), 7.97 (ddd, *J* = 8.1, 2.0, 0.9 Hz, 1 H, H-4), 7.57 (ddd, *J* = 8.1, 2.0, 0.9 Hz, 1 H, H-6), 7.42 (dd, *J* = 8.1, 8.1 Hz, 1 H, H-5), 3.01 (dd, *J* = 7.4, 7.3 Hz, 2 H, CH₂S), 1.64-1.71 (m, 2 H, CH₂), 1.43-1.53 (m, 2 H, CH₂), 0.95 (t, *J* = 7.3 Hz, 3 H, CH₃); ¹³ C NMR δ 148.6, 140.5, 133.5, 129.4, 121.9, 120.1, 32.6, 30.7, 21.9, 13.6; MS (EI) m/z 211 (M⁺, 60%), 155 (100); HRMS (EI) calc. for C₁₀H₁₃NO₂S (M⁺) m/z 211 0667, found 211.0661.
A solution of NH₂OMe HCl (2 61 g, 31.2 mmol) and **7** (5.5 g, 26.0 mmol) in DMF (40 mL) was added dropwise to a stirred mixture of KOtBu (12.0 g, 106.7 mmol) and CuCl (0.26 g, 2.6 mmol) in DMF (50 mL) at 5 °C. The mixture was stirred at 20 °C for 6 h, quenched with saturated aqueous NH₄Cl solution (300 mL). The mixture was extracted with EtOAc (3 × 100 mL), the combined organic fraction dried, and the solvent evaporated The residue was chromatographed, eluting with 5% EtOAc/pet ether, to give (i) 6-(butylsulfanyl)-2-nitroaniline (**9**) (2.34 g, 40%) as a red oil, ¹H NMR δ 8.12 (dd, *J* = 8.7, 1.5 Hz, 1 H, H-3), 7.66 (dd, *J* = 7.3, 1.5 Hz, 1 H, H-5), 7.00 (br s, 2 H, NH₂), 6.64 (dd, *J* = 8.7, 7.3 Hz, 1 H, H-4), 2.75 (dd, *J* = 7.5, 7.2 Hz, 2 H, CH₂S), 1.51-1.58 (m, 2 H, CH₂), 1.37-1.46 (m, 2 H, CH₂), 0.90 (dd, 7.4, 7.2 Hz, 3 H, CH₃); ¹³C NMR δ 145.8, 142.5, 132.5, 127.0, 122.7, 115.6, 35.0, 31.6, 21.7, 13.6; MS (EI) m/z 226 (M⁺, 100%), 209 (15), 192 (25); HRMS (EI) calc. for C₁₀H₁₄N₂O₂S (M⁺) m/z 226.0776, found 226.0770; (ii) 4-(butylsulfanyl)-2-nitroaniline (**1o**) (1.12 g, 19%) as a red oil, ¹H NMR δ 8.16 (d, *J* = 2.2 Hz, 1 H, H-3), 7.39 (dd, *J* = 8.7, 2.2 Hz, 1 H, H-5), 6.76 (d, *J* = 8.7 Hz, 1 H, H-6), 6.06 (br s, 2 H, NH₂), 2.83 (dd, *J* = 7.3, 7.2 Hz, 2 H, CH₂S), 1 56-1.62 (m, 2 H, CH₂), 1.38-1.48 (m, 2 H, CH₂), 0.91 (dd, J = 7.4, 7.3 Hz, 3 H, CH₃); ¹³C NMR δ 143.5, 139.1, 132.2, 128.3, 123.9, 119.3, 35.3, 31.2, 21.8, 13.6; MS (EI) m/z 226 (M⁺, 100%), 170 (80); HRMS (EI) calc. for C₁₀H₁₄N₂O₂S (M⁺) m/z 226.0776, found 226.0772; and (iii) 2-(butylsulfanyl)-4-nitroaniline (**8**) (0.76 g, 13%) as a red oil, ¹H NMR δ 8.16 (d, *J* = 2.6 Hz, 1 H, H-3), 8.00 (dd, *J* = 9.1, 2.6 Hz, 1 H, H-5), 6 68 (d, *J* = 9.1 Hz, 1 H, H-6), 5.07 (br s, 2 H, NH₂), 2.79 (dd, *J* = 7.2, 6.6 Hz, 2 H, CH₂S), 1.52-1.60 (m, 2 H, CH₂), 1.38-1.47 (m, 2 H CH₂), 0.91 (t, *J* = 7.3 Hz, 3 H, CH₃); ¹³C NMR δ 153.5, 138.7, 131.4, 125.7, 118.1, 113.0, 34.7, 31.5, 21.7, 13.6. **7-(Butylsulfanyl)-1,2,4-benzotriazin-3-amine 1-oxide (3o).** Method A using **1o** gave **3o** (68%) as a red solid, mp (MeOH/CHCl₃) 215-217 °C; ¹H NMR [(CD₃)₂SO] δ 7.87 (d, *J* = 2.1 Hz, 1 H, H-8), 7.68 (dd, *J* = 8.9, 2.1 Hz, 1 H, H-6), 7.46 (d, *J* = 8.9 Hz, 1 H, H-5), 7.07 (br s, 2 H, NH₂), 3.04 (dd, *J* = 7.3, 7.2 Hz, 2 H, CH₂S), 1.55-1.63 (m, 2 H, CH₂), 1.37-1.45 (m, 2 H, CH₂), 0.88 *(t, J* = 7.3 Hz, 3 H, CH₃); ¹³C NMR [(CD₃)₂SO] δ 160.0, 147.3, 135 9, 133.9, 130.0, 126.4, 115.8, 31.6, 30.1, 21.2, 13.4; Anal. calc. for C₁₁H₁₄N₄OS: C, 52 8; H, 5.6; N, 22.4; found C, 52.9; H, 5.8; N, 22.2%.

### Example 18

**7-Nitro-1,2,4-berizotriazin-3-amine 1-oxide (3p).** Method B using 1-chloro-2,4-dimtrobenzene (**2p**) gave **3p** (15%) as a yellow powder, mp (DMF) 269-272 °C [lit.( Pazdera & Potacek, *Chem. Papers* 1988, 42, 527-537) mp (pyridine/EtOH) 290 °C]; ¹H NMR [(CD₃)₂SO] δ 8.82 (d, *J* = 2.6 Hz, 1 H, H-8), 8.44 (dd, *J* = 9 4, 2.6 Hz, 1 H, H-6), 8.03 (br s, 2 H, NH₂), 7.64 (d, *J* = 9.3 Hz, 1 H, H-5).

### Example 19

**6-Methoxy-1,2,4-benzotriazin-3-amine 1-oxide (3q).**
**5-Methoxy-2-nitroaniline (1q)**. A mixture of 5-methoxy-2-nitrobenzoic acid (**10**) (10 g, 50 7 mmol) diphenylphosphorylazide (DPPA) (11.5 mL, 53.3 mmol) and Et₃N (7.4 mL, 53.3 mmol) in t-BuOH (200 mL) was heated at reflux temperature for 16 h. The solution was cooled to 20 °C and the solvent evaporated. The residue was dissolved in DCM (300 mL) and washed with water (2 × 100 mL), saturated aqueous KHCO₃ (100 mL), brine (50 mL), dried, and the solvent evaporated. The residue was suspended in MeOH (250 mL), cHCl (50 mL) added, and the mixture stirred at 20 °C for 96 h. The solvent was evaporated and the residue suspended in saturated aqueous KHCO₃ (400 mL) and stirred for 30 min. The suspension was filtered, the solid washed with water (20 mL) and dried at 80 °C under reduced pressure. The solid was chromatographed, eluting with a gradient (20-30%) of EtOAc/pet. ether, to give **1q** (8.26 g, 98%); as a yellow solid, mp 128-130 °C [lit. (Seko et. al., *J*. *Chem. Soc. Perkin Trans. 1* **1999,** 1437) mp 130-132 °C], ¹H NMR δ 8.07 (d, *J* = 9.5 Hz, 1 H, H-3), 6.28 (dd, *J* = 9.5, 2.6 Hz, 1 H, H-4), 6.21 (br s, 2 H, NH₂), 6.15 (d, *J* = 2.6 Hz, 1 H, H-6), 3.83 (s, 3 H, OCH₃); ¹³C NMR δ 165.4, 147.1, 128.5, 126.9, 106.7, 99.4, 55.7.

**6-Methoxy-1,2,4-benzotriazin-3-amine** 1-oxide **(3q).** Method A using 5-methoxy-2-nitroaniline (**1q**) gave **3q** (63%) as a yellow powder, mp (CHCl₃) 265-270 °C; ¹H NMR [(CD₃)₂SO] δ 8 04 (d, *J* = 9 5 Hz, 1 H, H-8), 7.24 (br s, 2 H, NH₂), 6.95 (dd, *J* = 9.5, 2.6 Hz, 1 H, H-7), 6 86 (d, *J* = 2.6 Hz, 1 H, H-5), 3.91 (s, 3 H, OCH₃); ¹³C NMR [(CD₃)₂SO] δ 164.7, 160.7, 151.3, 125 0, 121.5, 117.0, 103.8, 56.0; MS (El⁺) *m*/*z* 192 (M⁺, 100%), 176 (5); HRMS (EI) calc. for C₈H₈N₄O₂ (M⁺) *m*/*z* 192.0647, found 192.0653, Anal calc. for C₈H₈N₄O₂: C, 50.0; H, 4 2; N, 29.2, found C, 50.0; H, 4.0; N, 290%

### Example 20

**6-Methyl-1,2,4-benzotriazin-3-amine 1-oxide (3r).** Method A using 5-methyl-2-nitroaniline **(1r)** gave **3r** (87%) as a yellow powder, mp (DMF) 263 °C (dec) [lit. (Friebe et al , *US Patent* 5,856,325, Jan 1999) mp (HOAc) 284-286 °C]; ¹H NMR [(CD₃)₂SO] δ 8 02 (d, *J* = 8 8 Hz, 1 H, H-8), 7 33 (s, 1 H, H-5), 7.27 (br s, 2 H, NH_{z}), 7.18 (dd, *J* = 8.8, 1.7 Hz, 1 H, H-7), 2.42 (s, 3 H, CH₃); Anal. calc. for C₈H₈N₄O: C, 54 5; H, 4 6; N, 31.8; found C, 54 9; H, 4 6; N, 31.9%.

### Example 21

**6-Phenyl-1,2,4-benzotriazin-3-amine 1-oxide (3s).** Method A using 4-nitro[1,1'-biphenyl]-3-amine **(1s)** gave **3s** (50%) as a yellow powder, mp (MeOH/DCM) 256-258 °C; ¹H NMR [(CD₃)₂SO] δ 8.11 (d, *J* = 8.9 Hz, 1 H, H-8), 7.82 (br d, *J* = 7.2 Hz, 2 H, H-2', H-6'), 7.75 (d, *J* = 1.9 Hz, 1 H, H-5), 7.67 (dd, *J* = 8.9, 1.8 Hz, 1 H, H-7), 7.47-7.50 (m, 2 H H-3', H-5'), 7.47-7 50 (m, 1 H, H-4'), 7.38 (br s, 2 H, NH₂); ¹³C NMR [(CD₃)₂SO] δ 160.5, 149.1, 146.9, 138.0, 129.1 (2), 129.0, 128.9, 127.2 (2), 123.7, 122.5, 120.5; Anal. calc for C₁₃H₁₀N₄O: C, 65.5; H, 4.2; N, 23.5; found C, 65.4; H, 4.2; N, 23.7%.

### Example 22

**6-Fluoro-1,2,4-benzotriazin-3-amine 1-oxide (3t).** Method A using 5-fluoro-2-nitroaniline **(1t)** gave **3t** (61 %) as a yellow powder, mp (DCM/pet. ether) 276-280 °C [lit. (Suzuki & Kawakami, Synthesis **1977,** 855) mp 268 °C]; ¹H NMR [(CD₃)₂SO] δ 8.21 (dd, *J* = 9.5, 5 9 Hz, 1 H, H-8), 7.50 (br s, 2 H, NH₂), 7.30 (dd, *J* = 10.0, 2.6 Hz, 1 H, H-5), 7.21 (ddd, *J* = 8.8, 7 5, 2.6 Hz, 1 H, H-7); ¹³C NMR [(CD₃)₂SO] δ 164.5 (d, *J* = 254.6 Hz), 160.8, 150.6 (d, *J* = 16.1 Hz), 127.4, 123.4 (d, *J* = 11.1 Hz), 114.1 (d, *J* = 26.2 Hz), 109.5 (d, *J* = 23.1 Hz); Anal. calc. for C₇H₅FN₄O: C, 46.7; H, 2.8; N, 31.1; F, 10.6; found C, 46.5; H, 2.7; N, 31.3; F, 10.8%.

### Example 23

**6-Chloro-1,2,4-benzotrazin-3-amine 1-oxide (3u).** Method A using 5-fluoro-2-nitroaniline (1u) gave 3u (53%) as a yellow solid, mp (DCM/pet. ether) >320 °C [lit. (Fnebe et al., *US Patent* 5,856,325, Jan 1999) mp (HOAc) >300 °C], ¹H NMR [(CD₃)₂SO] δ 8.13 (d, *J* = 9.2 Hz, 1 H, H-8), 7.60 (d, *J* = 2.11 Hz, 1 H, H-5), 7.53 ( br s, 2 H, NH₂), 7.33 (dd, *J* = 9.2, 2.1 Hz, 1 H, H-7).

### Example 24

**6-Trifluoromethyl-1,2,4-benzotriazin-3-amine 1-oxide (3v)**. Method A using 5-trifluoromethyl-2-nitroaniline (**1v**) gave **3v** (29%) as a yellow solid, mp (DCM/pet. ether) 280-284 °C (dec ); ¹H NMR [(CD₃)₂SO] δ 8.31 (d, *J* = 8 9 Hz, 1 H, H-8), 7.85 (br s, 1 H, H-5), 7.65 (br s, 2 H, NH₂), 7.56 dd, *J* = 8.9, 1.6 Hz, 1 H, H-7); ¹³C NMR [(CD₃)₂SO] δ 160.8, 148.4, 134.8 (q, *J* = 32.7 Hz), 131.5, 123.5 (q, *J* = 4.0 Hz), 123.1 (q, *J* = 273.1 Hz), 122.0, 119.3; Anal. calc. for C₈H₅F₃N₄O: C, 41.8; H, 2.2; N, 24.3; F, 24 8; found C, 41.1; H, 2.1; N, 24.3; F, 24.6%.

### Example 25

**6-(Methylsulfanyl)-1,2,4-benzotriazin-3-amine 1-oxide (3w).** Method A using 5-methylsulfanyl-2-nitroaniline (**1w**) (Seko et. al., *J*. *Chem. Soc. Perkin Trans. 1* **1999**, 1437) gave 3w (55%) as a yellow powder, mp (MeOH/CHCl₃) 248-250 °C; ¹H NMR [(CD₃)₂SO] δ 7.99 (d, *J* = 8.3 Hz, 1 H, H-8), 7.30 (br s, 2 H, NH₂), 7.16-7.19 (m, 2 H, H-5, H-7), 2.59 (s, 3 H, SCH₃); ¹³ C NMR [(CD₃)₂SO] δ 160.7, 149.2, 149.1, 127.3, 122.9, 119 8, 118.0, 14.0; Anal. calc. for C₈H₈N₄OS: C, 46.1; H, 3.9; N, 26.9; found C, 46.0; H, 3.8; N, 27.0%.

### Example 26

**6-(Butylsulfanyl)-1,2,4-benzotriazin-3-amine 1-oxide (3x).**
**5-(Butylsulfanyl)-2-nitroaniline (1x).** A solution of LiSnBu (3.34 g, 34.8 mmol) in DMF (30 mL) was added dropwise to a stirred solution of 5-chloro-2-nitroaniline (**1u**) (5 0 g, 30.0 mmol) in DMF (50 mL) at 20 °C and the mixture stirred for 2 h. The mixture was poured into water (300 mL) and extracted with EtOAc (2 × 150 mL). The combined organic fraction was washed with water (2 × 100 mL), brine (50 mL), dried, and the solvent evaporated. The residue was chromatographed, eluting with 20% EtOAc/pet. ether, to give **1x** (6.12 g, 90%) as a red solid, mp (EtOAc/pet. ether) 91-93°C; ¹H NMR δ 8.00 (d, *J* = 7.5 Hz, 1 H, H-3), 6.52-6.56 (m, 2 H, H-4, H-6), 6.11 (br s, 2 H, NH₂), 2.96 (dd, *J* = 7.4, 7.3 Hz, 2 H, CH₂S), 1.66-1.73 (m, 2 H, CH₂), 1.44-1 53 (m, 2 H, CH₂), 0.96 (s, 3 H, CH₃); ¹³C NMR δ 149.0, 144.8, 129.6, 126.4, 115.2, 113.4, 31.3, 30.6, 22.0, 13.6; Anal. calc. for C₁₀H₁₄N₂O₂S: C, 53.1; H, 6.2; N, 12.4; found C, 53.2; H, 6.3; N, 12.4%.

**6-(Butylsulfanyl)-1,2,4-benzotriazin-3-amine 1-oxide (3x).** Method A using **1x** gave (i) starting material **(1x)** (60%) and (ii) **3x** (30%) as a red solid, mp (MeOH/CHCl₃) 180-182 °C; ¹H NMR [(CD₃)₂SO) δ 7.99 (d, *J* = 9.1 Hz, 1 H, H-8), 7.31 (br s, 2 H, NH₂), 7 21 (d, *J* = 2.0 Hz, 1 H, H-5), 7.17 (dd, *J* = 9.1, 2.0 Hz, 1 H, H-7), 3.12 (dd, *J* = 7.3, 7.2 Hz, 2 H, CH₂S), 1.61-1.68 (m, 2 H, CH₂), 1.41-1.49 (m, 2 H, CH₂), 0 92 (s, 3 H, CH₃); ¹³C NMR [(CD₃)₂SO) δ 160.6, 149.1, 147.9, 127.3, 123.4, 119.9, 118 8, 30.1, 29.9, 21.3, 13.4; Anal. calc. for C₁₁H₁₄N₄OS: C, 52.8; H, 5.6; N, 22.4; found C, 52.5; H, 5.6; N, 22 5%.

### Example 27

**5-Methoxy-1,2,4-benzotriazin-3-amine 1-oxide (3y).** Method A using 6-methoxy-2-nitroaniline (**1y**) (Seko et. al., *J*. *Chem. Soc. Perkin Trans. 1* **1999,** 1437) gave **3y** (66%) as a yellow powder, mp (HOAc) 267 °C (dec.) [lit. (Friebe et. al., *US Patent* 5,856,325, Jan 1999) mp (HOAc) >270 °C]; ¹H NMR [(CD₃)₂SO] δ 7.65-7 69 (m, 1 H, H-7), 7 39 (br s, 2 H, NH₂), 7.23-7.27 (m, 2 H, H-6, H-8), 3.92 (s, 3 H, OCH₃); ¹³C NMR [(CD₃)₂SO] δ 159.7, 153 3, 141.5, 130.0,123.9, 113.4, 110.7, 55.9; Anal. calc. for C₈H₈N₄O₂: C, 50.0; H, 4.2; N, 29.2; found C, 50.2; H, 4.1; N, 29.1 %.

### Example 28

**5-Methyl-1,2,4-benzotriazin-3-amine 1-oxide (3z).** Method A using 6-methyl-2-nitroaniline **(1z)** gave **3z** (89%) as a yellow solid, mp (DCM/pet. ether) 253-255 °C; ¹H NMR [(CD₃)₂SO] δ 7.98 (d, *J* = 8.6 Hz, 1 H, H-8), 7.64 (d, *J* = 7.1 Hz, 1 H, H-6), 7.33 (br s, 2 H, NH₂), 7 23 (dd, *J* = 8 6, 7.1 Hz, 1 H, H-7), 2.49 (s, 3 H, CH₃); ¹³C NMR [(CD₃)₂SO] δ 156.7, 148.0, 135.1, 134.3, 129.8, 124.0, 117.4, 16.8; Anal. calc. for C₈H₈N₄O: C, 54.5; H, 4.6; N, 31.8; found C, 54.7; H, 4.7; N, 32.1 %.

### Example 29

**5-Chloro-1 ,2,4-benzotriazin-3-amine 1-oxide (3aa).** Method B using 1,2-dichloro-3-nitrobenzene **(2aa)** gave **3aa** (45%) as a yellow solid, mp (HOAc) 251-254 °C; ¹H NMR [(CD₃)₂SO] δ 8.11 (dd, *J* = 8.7, 1.0 Hz, 1 H, H-8), 7.95 (dd, *J* = 7.6, 1.0 Hz, 1 H, H-6), 7.67 (br s, 2 H, NH₂), 7.29 (dd, J = 8.7, 7.6 Hz, 1 H, H-7); ¹³C NMR [(CD₃)₂SO] δ 160.1, 145.7, 135.1, 131.1, 128.6, 123.6, 119.1; Anal. calc. for C₇H₅ClN₄O: C, 42.8; H, 2.6; N, 28.5; Cl, 18.0; found C, 43.0; H, 2.5; N, 28.3; Cl, 17.0%.

### Example 30

**5-Fluoro-1,2,4-benzotriazin-3-amine 1-oxide (3ab).** Method A using 6-fluoro-2-nitroaniline (**1ab**) gave **3ab** (43%) as a yellow powder, mp (DMF) 252-256 °C [lit. (Suzuki & Kawakami, *Synthesis* 1977, 855-857) mp 278 °C] ¹H NMR [(CD₃)₂SO] δ 7.96 (dd, *J* = 8 7, 1.0 Hz, 1 H, H-8), 7.66 (ddd, *J* = 10.3, 7.9, 1.2 Hz, 1 H, H-6), 7.61 (br s, 2 H, NH₂), 7.28 (ddd, *J* = 8.6, 8.0, 5.1 Hz, 1 H, H-7); ¹³C NMR [(CD₃)_{z}SO] δ 160 1 (d, *J* = 5.6 Hz), 154 9 (d, *J* = 253 1 Hz), 139.7 (d, *J* = 16 1 Hz), 131.1 (d, *J* = 4 2 Hz), 122.8 (d, *J* = 7.6 Hz), 119.4 (d, *J* = 17.7 Hz), 115.8 (d, *J* = 4.4 Hz); Anal. calc. for C₇H₅FN₄O: C, 46 7; H, 2.8; N, 31.1; F, 10.6; found C, 46.6; H, 2.7; N, 31.2; F, 10 3%.

### Example 31

**5-Nitro-1,2,4-benzotriazin-3-amine-1-oxide (3ac).** Method B using 2,6-dinitrofluorobenene (**2ac**) gave 1-oxide **3ac** (27%) as a yellow powder, mp (DCM/pet. ether) 269-272 °C; ¹H NMR [(CD₃)₂SO] δ 8.38 (dd, *J* = 8.5, 1.1 Hz, 1 H, H-6), 8.32 (dd, *J* = 7.7, 1.2 Hz, 1 H, H-8), 7.88 (br s, 2 H, NH₂),7.39 (dd, *J* = 8.5, 7 7 Hz, 1 H, H-7); ¹³C NMR [(CD₃)₂SO] δ 160.5, 144 2, 141.5, 130 9, 129.7, 124.2, 122.0; HRMS (EI) calc for C₇H₅N₅O₃ (M⁺) m/z 207.0392, found 207.0393.

### Example 32

**5-Methylsulfanyl-1,2,4-benzotriazin-3-amine 1-oxide (3ad).** Method A using 6-methylsulfanyl-2-nitroaniline **(1ad)** gave **3ad** (7%) as a yellow solid, mp (MeOH/DCM) 248-252 °C; ¹H NMR [(CD₃)₂SO] δ 7.85 (dd, *J* = 8.7, 1.4 Hz, 1 H, H-8), 7.45-7.48 (m, 3 H, H-6, NH₂), 7.28 (dd, *J* = 8.7, 7.7 Hz, 1 H, H-7), 2.49 (s, 3 H, SCH₃), ¹³C NMR [(CD₃)₂SO] δ 159 4, 145.9, 136.8, 129.4, 127.6, 124.3, 114.6, 13.5; Anal. calc. for C₈H₈N₄OS: C, 46.1; H, 3.9; N, 26.9; found C, 46.4; H, 3.8; N, 26.8%.

### Example 33

**N**^{**7**}**,N**^{**7**}**-Dimethyl-1,2,4-benzotriazine-3,7-diamine 1-oxide (11)**. A solution of 7-fluoro-1,2,4-benzotriazine-3-amine 1-oxide (**3k**) (114 mg, 0.63 mmol) and 40% aqueous dimethylamine (5 mL) in CH₃CN (15 mL) was stirred at 90 °C for 4 days. The solvent was evaporated and the residue was partitioned between dilute aqueous NH₃ (10 mL) and DCM (10 mL). The aqueous fraction was extracted with DCM (3 × 15 mL), the combined organic fraction dried, and the solvent evaporated. The residue was chromatographed, eluting with a gradient (0-2%) of MeOH/DCM, to give 11 (30 mg, 61%) as an orange powder, mp (DCM/hexane) 231-233 °C; ¹H NMR [(CD₃)₂SO] δ 7.58 (dd, *J* = 9.4, 2.9 Hz, 1 H, H-6), 7.45 (d, *J* = 9 4 Hz, 1 H, H-5), 7.02 (d, J = 2.9 Hz, 1 H, H-8), 6.97 (br s, 2 H, NH₂), 3.05 [s, 6 H, N(CH₃)₂]; ¹³C NMR [(CD₃)₂SO] δ 158.3, 147.6, 130.3, 126 5, 125.6, 95.3, 40.0; Anal. calc. for C₉H₁₁N₅O: C, 52.7; H, 5.4, N, 34.2; found, C, 52.4; H, 5.3; N, 34.2%.

### Example 34

***N***^{**6**}**,*N***^{**6**}**-Dimethyl-1,2,4-benzotriazine-3,6-diamine 1-oxide (12).** A solution of 6-fluoro-1,2,4-benzotriazine-3-amine 1-oxide **(3t)** (0.1 g, 0.55 mmol) and 40% aqueous solution of dimethylamine (5 mL) in CH₃CN (15 mL) was stirred at 20 °C for 5 days. The solvent was evaporated and the residue was partitioned between dilute aqueous NH₃ (10 mL) and DCM (10 mL). The aqueous layer was extracted with DCM (3 × 15 mL), the combined organic extract dried, and the solvent evaporated. The residue was chromatographed, eluting with a gradient (0-3%) of MeOH/DCM, to give **12** (93 mg, 82%) as an orange powder, mp (DCM/hexane) 264-267 °C; ¹H NMR [(CD₃)₂SO] δ 7 92 (d, *J* = 9.7 Hz, 1 H, H-8), 6.97 (dd, *J* = 9.7, 2.7 Hz, 1 H, H-7), 6.88 (s, 2 H, NH₂), 6.33 (d, *J* = 2.7 Hz, 1 H, H-5), 3 09 [s, 6 H, N(CH₃)₂]; Anal. calc. for C₉H₁₁N₅O: C, 52.7; H, 5.4; N, 34.1; found C, 52.5; H, 5.40; N, 34.2%.

### Example 35

***N***^{**6**}**,*N***^{**6**}**-Diethyl-1,2,4-benzotriazine-3,6-diamine 1-oxide (13).** 6-Fluoro-1,2,4-benzotriazine-3-amine 1-oxide (**3t**) (0.1 g, 0.55 mmol) and diethylamine (3 mL) in CH₃CN (15 mL) was heated at 90 °C for 2 days.The solvent was evaporated and the residue was stirred in dilute ammonia (10 mL) and the resulting precipitate was filtered and chromatographed eluting with a gradient of 0-2% MeOH/DCM, to give **13** (83 mg, 65%) as an orange powder, mp (DCM/Hexane) 247-251 °C; ¹H NMR [(CD₃)₂SO) δ 7.91 (d, *J* = 9.8 Hz, 1 H, H-8), 6.93 (dd, *J* = 9.7, 2.8 Hz, 1 H, H-7), 6.83 (s, 2 H, NH₂), 6.31 (d, *J* = 2.6 Hz, 1 H, H-5), 3.47 (q, *J* = 7.0 Hz, 4 H, 2 x CH₂) 1.14 (t, *J*= 7.0 Hz, 6 H, 2 × CH₃); ¹³C NMR [(CD₃)₂SO] δ 160.6, 152.1, 150.9, 121.8, 121.2, 113.9, 99.0, 44.2, 12.3; Anal. calc. for C₁₁H₁₅N₅O: C, 56.6; H, 6.5; N, 30.0; found C, 56.6; H, 6.6; N, 30.1%.

### Example 36

**7-(2-Methoxyethoxy)-1,2,4-benzotriazin-3-amine 1-oxide (14).** A mixture of 7-hydroxy-1-oxide **3h** (1.00 g, 5.8 mmol), dry K₂CO₃ (2.40 g, 17.4 mmol) and 2-bromoethylmethylether (2.42 g, 17 4 mmol) in DMF (20 mL) was heated at 80 °C for 2 h. The solvent was evaporated and the residue chromatographed, eluting with a gradient (0-3%) MeOH/DCM, to give compound **14** (1.06 g, 77 %) as a yellow powder, mp (DCM/pet ether) 201-203 °C; ¹H NMR [(CD₃)₂SO] δ 8.07 (d, *J* = 9.5 Hz, 1 H, H-5), 7 82 (br s, 2 H, NH₂), 7.76 (dd, *J* = 9.5, 2.6 Hz, 1 H, H-6), 7.50 (d, *J* = 2.6 Hz, 1 H, H-8), 4.26, (t, *J* = 4.3 Hz, 2 H, CH₂), 3.72 (t, *J* = 4.3 Hz, 2 H, CH₂), 3.33 (s, 3 H, OCH₃); Anal. calc. for C₁₀H₁₂N₄O₅: C, 50.8; H, 5.1; N, 23.7; found C, 51.1; H, 5.0; N, 23.7%.

### Example 37

**7-(2-Bromoethoxy)-1,2,4-benzotriazin-3-amine 1-oxide (15).** A mixture of 7-hydroxy-1-oxide **3h** (1 23 g, 7 15 mmol), K₂CO₃ (1.97 g, 14.3 mmol) and 1,2-dibromoethane (4 0 ml) in DMF (20 ml) was heated at 80 °C for 20 h. The solvent was evaporated, and the residue stirred in water (100 mL). The resulting precipitate was filtered, washed with water (3 × 50 mL) and dried to give a yellow solid, which was chromatographed, eluting with 0-3% MeOH/DCM, to give 1-oxide **15** (1.0 g, 49%) as a yellow powder, mp (DCM/pet. ether) 228-230 °C; ¹H NMR δ 7.52-7.50 (m, 3 H, H-5, H-6, H-8), 7.12 (br s, 2 H, NH₂), 4.45 (t, *J* = 5.2 Hz, 2 H, CH₂), 3.85 (t, *J* = 5.2 Hz, 2 H, CH₂), HRMS (EI) calc. for C₉H₉⁷⁹BrN₄O₂ (M⁺) *mlz* 283.9909, found 283.9902; calc. for C₉H₉⁸¹BrN₄O₂ (M⁺) *m*/*z* 285.9888, found 285.9881.

### Example 38

***N*-{2-[(3-Amino-1 -oxido-1,2,4-benzotriazin-7-yl)oxy]ethyl)-2,2,2-trifluoroacetamide (16).** A mixture of 7-hydroxy-1-oxide **3h** (520 mg, 3.02 mmol), K₂CO₃ (883 mg, 6.04 mmol) and *N*-(2-bromoethyl)-2,2,2-trifluoroacetamide (1.25 g, 6.03 mmol) in DMF (20 ml) was heated at 80 °C for 3 h. The solvent was evaporated and residue stirred in water (100 mL) the resulted precipitate was filtered, washed with water (3 x 50 mL), and dried to give a yellow solid, which was chromatographed, eluting with a gradient (0-3%) of MeOH/DCM, to give 1-oxide **16** (639 mg, 66%) as a yellow powder, mp (DCM/pet. ether) 234-246 °C; ¹H NMR [(CD₃)₂SO] δ 9.66 (br s, 1 H, CONH), 7.52 (d, *J* = 9.2 Hz, 1 H, H-5), 7.51 (d, *J* = 2.3 Hz, 1 H, H-8), 7.42 (dd, *J* = 9.1, 2.9 Hz, 1 H, H-6) 7.12 (br s, 2 H, NH₂), 4.23 (t, *J* = 5.5 Hz, 2 H, CH₂), 3.63 (t, *J* = 5.4 Hz, 2 H, CH₂); ¹³C NMR [(CD₃)₂SO] δ 159.5, 156.7 (q, *J* = 36.3 Hz), 155.1, 144.9, 129 7, 128.4, 127.4, 119.8, 115.8 (q, *J* = 287.8 Hz), 99.0, 65.9; Anal. calc. for C₁₁H₁₀F₃N₅O₃: C, 41.7; H, 3.2; N, 22.2; F, 18.0; found C, 42.0; H, 3.0; N, 21.9; F, 17.5%.

### Example 39

**7-(2-(4-Morpholinyl)ethoxy]-1,2,4-benzotriazin-3-amine 1-oxide (17).** A mixture of 7-hydroxy-1-oxide **3h** (1.15 g, 6 7 mmol), K₂CO₃ (3.77 g, 20.0 mmol) and 4-(2-chloroethyl)morpholine hydrochloride (2.49 g, 13.4 mmol) in DMF (25 ml) was heated at 80 °C for 2 h The solvent was evaporated, the residue stirred in water (100 mL), the resulting precipitate filtered, washed with water (3 × 50 mL), and dried to give 1-oxide **17** (1.53 g, 79%) as a yellow solid, mp (DCM/pet. ether) 175-181 °C; ¹H NMR [(CD₃)₂SO] δ 7.52-7.46 (m, 3 H, H-5, H-6, H-8), 7 09 (br s, 2 H, NH₂), 4.20 (t, *J* = 5.6 Hz, 2 H, CH₂), 3.58 (t, *J* = 4.7 Hz, 4 H, 2 × CH₂), 2.73 (t, *J* = 5.6 Hz, 2 H, CH₂), 2.49 (t, *J* = 4.6Hz, 4H, 2 × CH₂), ¹³C NMR [(CD₃)₂SO] δ 159.5, 155.5, 144.8, 129.7, 128.5, 127.3, 98.8, 66.1 (2), 56 7, 53.5 (2); Anal calc. for C₁₃H₁₇N₅O₃: C, 53.6; H, 5 9; N, 24 0; found C, 53.5; H, 6.0; N, 23.8%.

### Example 40

**1,2,4-Benzotriazin-3-amine 1-oxide (3).** A mixture of 2-nitroaniline (1) (10.0 g, 72.4 mmol) and cyanamide (15.2 g, 0.36 mmol) was melted at 100 °C, cooled to ca. 40 °C, cHCl (20 mL) added carefully. The exotherm was allowed to subside and the mixture was heated at 100 °C for 1 h. The mixture was cooled to ca. 40 °C and 30% NaOH solution (30 mL) added carefully The mixture was stirred at 100 °C for 2 h, cooled to 25 °C, diluted with water (50 mL) and stirred for 30 min. The suspension was filtered, washed with water (2 × 10 mL), ether (2 × 5 mL) and dried under vacuum to give 1-oxide **3** (10.3 g, 88%) as a yellow powder, mp (MeOH/EtOAc) 267-269 °C [lit. (Arndt, Ber. **1913,** 46, 3522) mp (EtOH) 269°C]; ¹H NMR δ 8.13 (d, *J* = 8.7 Hz, 1 H, H-8), 7.79 (dd, *J* = 8.6, 7.0 Hz, 1 H, H-6), 7.54 (d, *J* = 8.6 Hz, 1 H, H-5), 7.32-7.38 (m, 3 H, H-7, NH₂).

### Example 41

**3-Chloro-1,2,4-benzotriazine 1-oxide (19).** A solution of NaNO₂ (10 g, 0.145 mol) in water (100 mL) added dropwise to a suspension of 1-oxide **3** (11.7 g, 72.2 mmol) in 2 M HCl (300 mL) at 5 °C and the mixture stirred vigorously until the foaming subsided (2 h). The resulting precipitate was filtered, dissolved in dilute aqueous NH₃, filtered, and acidified with cHCl. The precipitate was filtered, washed with water and dried to give 3-hydroxy-1,2,4-benzotriazine 1-oxide (**18**) (5.77 g, 49%) as a yellow powder, mp 209-212 °C; [lit. (Robbins *et al., J. Chem.* Soc. **1957**, 3186) mp (H₂O) 219 °C);¹H NMR δ 8.14 (d, *J* = 8.4 Hz, 1 H, H-8), 7.77-7.81 (m, 1 H, H-6), 7.54 (d, *J* = 8.4 Hz, 1 H, H-5), 7.90 (m, 3 H, H-7, NH₂); ¹³C NMR δ 160 2, 148.7, 135.6, 129.8, 125.8, 124.6, 119.8.
A mixture of alcohol **18** (5.7 g, 34.9 mmol), N,N-dimethylaniline (11 mL, 87.3 mmol), and POCl₃ (23 mL, 244 mmol) was heated at reflux temperature for 1 h then poured on to ice. The resulting solid was filtered and recrystallized to give chloride **19** (3.77 g, 59%) as a pale yellow powder, mp (MeOH) 119-119.5 °C [lit. (Robbins *et al., J. Chem.* Soc., **1957**, 3186) mp (MeOH) 117-118 °C]; ¹H NMR δ 8.38 (dd, J = 8.7, 1.0 Hz, 1 H, H-8), 8.16 (ddd, *J* = 8.3, 7.0, 1.3 Hz, 1 H, H-6), 8 06 (dd, *J* = 8.2, 1.0 Hz, 1 H, H-5), 7.90 (ddd, *J* = 8 7, 6 9, 1 3 Hz, 1 H, H-7); ¹³C NMR δ 155.3, 146 9, 137.2, 133.9, 131 5, 128.0, 119 9.

### Example 42

**Ethyl [(1-oxido-1,2,4-benzotriazin-3-yl)amino]acetate (20).** A mixture of chloride **19** (2 02 g, 11 1 mmol), glycine ethyl ester hydrochloride (2.33 g, 16.7 mmol) and Et₃N (4 2 mL, 30 mmol) in DME (100 mL) was heated at reflux temperature for 6 h. The solvent was evaporated and the residue partitioned between DCM (100 mL) and water (100 mL), the aqueous fraction extracted with DCM (2 x 50 mL), the combined organic fraction dried , and the solvent evaporated. The residue was chromatographed, eluting with 10%EtOAc/DCM, to give ester **20** (2.75 g, 99%) as a yellow solid, mp (EtOAc/DCM) 136-138 °C; ¹H NMR δ 8.27 (dd, *J* = 8.6, 1.0 Hz, 1 H, H-8), 7 72 (ddd, *J* = 8.5, 7.0, 1 4 Hz, 1 H, H-6), 7.62 (dd, J = 8.5, 1.0 Hz, 1 H, H-5), 7.34 (ddd, *J* = 8.6, 7.0, 1.0 Hz, 1 H, H-7), 5.87 (br s, 1 H, NH), 4.30 (d, *J* = 5.7 Hz, 2 H, CH₂N), 4.26 (q, *J* = 7.2 Hz, 2 H, CH₂O), 1.31 (t, *J* = 7.2 Hz, 3 H, CH₃); ¹³C NMR δ 169.9 (CO₂), 158 4 (C-3), 148.5 (C-4a), 135.6 (C-6), 131.2 (C-8a), 126.7 (C-5), 125.5 (C-7), 120.4 (C-8), 61.6 (CH₂O), 43.2 (CH₂N), 14.2 (CH₃); Anal. calc. for C₁₁H₁₂N₄O₃: C, 53.2; H, 4.9; N, 22 6; found C, 53.4; H, 5.0; N, 22.6%.

### Example 43

**[(1-Oxido-1,2,4-benzotriazin-3-yl)amino]acetic acid (21).** A solution of ester **20** (0.75 g, 3.0 mmol) and 1 M NaOH (15 mL, 15.0 mmol) in MeOH (50 mL) was stirred at 20 °C for 2 h. The solution was washed with ether (2 × 50 mL), the volume reduced to 20 mL, and the pH adjusted to 3 with 5 M HCl. The precipitate was filtered and recrystallized to give acid **21** (576 mg, 86%) as a pale yellow solid, mp (water) 217-219 °C; ¹H NMR [(CD₃)₂SO] δ 8.14 (d, *J* = 8.6 Hz, 1 H, H-8), 8.08 (br. s, 1 H, NH), 7.80 (ddd, *J* = 8.6, 7.1, 1.1 Hz, 1 H, H-6), 7.59 (d, *J* = 8.6 Hz, 1 H, H-5), 7.37 (ddd, *J* = 8.6, 7 1, 1.1 Hz, 1 H, H-7), 4.01 (br s, 2 H, CH₂N), CO₂H not observed; ¹³C NMR [(CD₃)₂SO] δ 171.3 (CO₂), 158.8 (C-3), 148.0 (C-4a), 135.8 (C-6), 130.2 (C-8a), 126.1 (C-5), 125.0 (C-7), 119.8 (C-8), 42.6 (CH₂N); Anal. calc. for C₉H₈N₄O₃¼H₂O: C, 48 1; H, 3.8; N, 24.9; found C, 48 2; H, 3.8; N, 24.2%.

### Example 44

**2-[(1-Oxido-1,2,4-benzotriazin-3-yl)amino]ethanol (22).** A solution of chloride **19** (536 mg, 3.0 mmol) and ethanolamine (0.53 mL, 8.9 mmol) was heated at reflux temperature in DME (50 mL) for 1 h The mixture was cooled to 20 °C, the solvent evaporated, and the residue partitioned between dilute aqueous NH₃ (50 mL) and DCM (100 mL) The organic fraction was dried , and the solvent evaporated The residue was chromatographed, eluting with 5% MeOH/DCM, to give alcohol **22** (533 mg, 88%) as a yellow solid, mp (MeOH/DCM) 214-218 °C; ¹H NMR [(CD₃)₂SO] δ 8.13 (dd, *J* = 8.5, 1.0 Hz, 1 H, H-8'), 7.82 (br s, 1 H, NH), 7.78 (ddd, *J* = 8.4, 7.0, 1.0 Hz, 1 H, H-6'), 7.57 (d, *J* = 8.4, Hz, 1 H, H-5'), 7.34 (ddd, J = 8.5, 7.0, 1 0 Hz, 1 H, H-7'), 4.74 (t, *J* = 5.6 Hz, 1 H, OH), 3.56-3.61 (m, 2 H, CH₂), 3.40-3.45 (m, 2 H, CH₂N); ¹³C NMR [(CD₃)₂SO] δ 159 0 (C-3'), 148.2 (C-4a'), 135.7 (C-6'), 130.0 (C-8a'), 125.9 (C-5'), 124.4 (C-7'), 119.8 (C-8'), 59 2 (CH₂O), 43.2 (CH₂N); Anal. calc. for C₉H₁₀N₄O₂: C, 52 4; H, 4.9, N, 27.2; found C, 52 3; H, 4.8; N, 26.6%.

### Example 45

***N*-(2-Methoxyethyl)-1,2,4-benzotriazin-3-amine 1-oxide (23).** A solution of chloride 19 (783 mg, 4.3 mmol) and 2-methoxyethylamine (0.82 mL, 9.5 mmol) in DME (70 mL) was heated at reflux temperature for 5 h. The cooled solution was partitioned betwen EtOAc (100 mL) and water (100 mL). The aqueous fraction was extracted with EtOAc (50 mL), the combined organic fractioned dried, and the solvent evaporated. The residue was chromatographed, eluting with a gradient (10-30%) EtOAc/DCM, to give 1-oxide **23** (915 mg, 96%) as a yellow powder, mp (EtOAc/DCM) 154-156 °C; ¹H NMR δ 8.26 (dd, *J =* 8.6, 1.3 Hz, 1 H, H-8), 7.71 (ddd, *J* = 8.4, 7.1, 1.3 Hz, 1 H, H-6), 7.60 (br d, *J* = 8.4 Hz, 1 H, H-5), 7.30 (ddd, *J* = 8.6, 7.1, 1.4 Hz, 1 H, H-7), 5.71 (br s, 1 H, NH), 3.72 (dt, *J* = 5.5, 5.3 Hz, 2 H, CH₂N), 3.61 (dd, *J* = 5.3, 5 0 Hz, 2 H, CH₂O), 3.40 (s, 3 H, CH₃O); ¹³C NMR δ 158.8 (C-3), 148.5 (C-4a), 135.6 (C-6), 131 0 (C-8a), 126.3 (C-5), 125.78 (C-7), 120.5 (C-8), 70.8 (CH₂O), 58.9 (OCH₃), 41.1 (CH₂N); Anal. calc. for C₁₀H₁₂N₄O₂: C, 54.5; H, 5.5; N, 25.4; found C, 54.8; H, 5.3; N, 25.3%.

### Example 46

**3-[(1-Oxido-1,2,4-benzotriazin-3-yl)amino]propanol (24).** A solution of chloride **19** (710 mg, 3.9 mmol) and 3-amino-1-propanol (0.75 mL, 9.8 mmol) was heated at reflux temperature in DME (50 mL) for 1 h. The mixture was cooled to 20 °C, the solvent evaporated, and the residue partitioned between dilute aqueous NH₃ (50 mL) and DCM (100 mL). The organic fraction was dried , and the solvent evaporated. The residue was chromatographed, eluting with 5% EtOAc/DCM, to give alcohol **24** (828 mg, 96%) as a yellow solid, mp (MeOH/DCM) 155-156 °C; ¹H NMR [(CD₃)₂SO] δ 8.13 (dd, *J* = 8.6, 1.1 Hz, 1 H, H-8'), 7.87 (br s, 1 H, NH), 7.78 (ddd, *J* = 8.4, 7.1, 1.1 Hz, 1 H, H-6'), 7.57 (d, *J* = 8.4, Hz, 1 H, H-5'), 7.34 (ddd, *J* = 8.6, 7.1, 1.1 Hz, 1 H, H-7'), 4.51 (t, *J* = 5 1 Hz, 1 H, OH), 3.50-3.55 (m, 2 H, CH₂), 3.40-3.45 (m, 2 H, CH₂N), 1.72-1 79 (m, 2 H, CH₂); ¹³C NMR [(CD₃)₂SO] δ 158.9 (C-3'), 148.3 (C-4a'), 135.6 (C-6'), 129.9 (C-8a'), 125.9 (C-5'), 124.3 (C-7'), 119.8 (C-8'), 58.4 (CH₂O), 37.9 (CH₂N), 31.7 (CH₂); Anal. calc. for C₁₀H₁₂N₄O₂; C, 54.5; H, 5 5; N, 25.5; found C, 54.9; H, 5.6; N, 25.6%.

### Example 47

**2-[(1-Oxido-1,2,4-benzotriazin-3-yl)amino]acetonitrile (25).** A solution of chloride **19** (790 mg, 4.4 mmol), 2-aminoacetonitrile hydrochloride (0.81 g, 8.7 mmol) and Et₃N (1.2 mL, 8.7 mmol) in DME (80 mL) was stirred at reflux temperature for 6 h. The solution was partitioned between DCM (100 mL) and water (100 mL), the aqueous fraction washed with DCM (2 × 50 mL), the combined organic fraction dried and the solvent evaporated. The residue was chromatographed, eluting with a gradient (5-20%) of EtOAc/DCM, to give nitrile **25** (383 mg, 44%) as yellow needles, mp (EtOAc/DCM) 233-237 °C; ¹H NMR δ 8.33 (dd, *J* = 8.5, 1.2 Hz, 1 H, H-8'), 7.83 (ddd, *J* = 8.4, 7.1, 1.2 Hz, 1 H, H-6'), 7.76 (d, *J* = 8.4 Hz, 1 H, H-5'), 7.45 (ddd, *J* = 8.5, 7.1, 1.2 Hz, 1 H, H-7'), 5.68 (br s, 1 H, NH), 4.49 (d, J = 6.3 Hz, 2 H, CH₂); Anal. calc for C₉H₇N₅O: C, 53.7; H, 3.5; N, 34.8; found C, 54.0; H, 3.2; N, 34.9%.

### Example 48

**3-[(1-Oxido-1,2,4-benzotriazin-3-yl)amino]propanenitrile (26).** A solution of chloride **19** (776 mg, 4.3 mmol), 3-aminopropanenitrile fumarate (2.74 g, 21.4 mmol) and Et₃N (3.6 mmol, 25.6 mmol) in DME (50 mL) was stirred at reflux temperature for 6 h. The solution was partitioned between DCM (100 mL) and water (100 mL), the aqueous fraction washed with DCM (2 × 50 mL), the combined organic fraction dried and the solvent evaporated. The residue was chromatographed, eluting with 10% EtOAc/DCM, to give nitrile **26** (771 mg, 84%) as yellow needles, mp (EtOAc/DCM) 191-193 °C; ¹H NMR δ 8.29 (dd, *J* = 8.7, 1.1 Hz, 1 H, H-8'), 7.76 (ddd, *J* = 8.5, 7.0, 1 1 Hz, 1 H, H-6'), 7.64 (dd, *J* = 8 5, 1.0 Hz, 1 H, H-5') 7.37 (ddd, *J* = 8.7, 7.0, 1.0 Hz, 1 H, H-7'), 6.00 (br s, 1 H, NH), 3 87 (q, *J* = 6.5 Hz, 2 H, H-3), 2.85 (t, *J* = 6.5 Hz, 2 H, H-2); ¹³C NMR δ 158 (C-3'), 148.4 (C-4a'), 135.9 (C-6'), 131.3 (C-8a'), 126.7 (C-5'), 120.4 (C-8'), 117.9 (C-1), 37.6 (C-3), 18.1 (C-2); Anal. calc. for C₁₀H₉N₅O: C, 55.8; H, 4 2; N, 32 6; found, C, 55.9; H, 4.3; N, 32 6%.

### Example 49

**4-[(1-Oxido-1,2,4-benzotriazin-3-yl)amino]butanenitrile (27).** A solution of chloride **19** (1.82 g, 10.0 mmol) and 4-aminobutanenitrile (2.11 g, 25.0 mmol) in DME (100 mL) was heated at reflux temperature for 4 h. The cooled solution was partitioned betwen EtOAc (200 mL) and water (200 mL). The aqueous fraction was extracted with EtOAc (100 mL), the combined organic fractioned dried, and the solvent evaporated. The residue was chromatographed, eluting with a gradient (5-10%) EtOAc/DCM, to give 1-oxide **27** (1.80 g, 79%) as a yellow powder, mp (EtOAc/DCM) 184-187 °C, ¹H NMR δ 8.26 (dd, *J* = 8.7, 1.3 Hz, 1 H, H-8'), 7.73 (ddd, *J* = 8.4, 7.0, 1.5 Hz, 1 H, H-6'), 7.63 (d, *J* = 8.4 Hz, 1 H, H-5'), 7.33 (ddd, *J* = 8.7, 7.0, 1.3 Hz, 1 H, H-7'), 6 00 (br s, 1 H, NH), 3.72 (dd, *J* = 6.6, 6.4 Hz, 2 H, H-4), 2.51 (t, *J* = 7.2 Hz, 2 H, H-2), 2.07-2.14 (m, 2 H, H-3); ¹³C NMR δ 158.7 (C-3'), 148.2 (C-4a'),135.8 (C-6'), 131.0 (C-8a'), 126.4 (C-5'), 125.4 (C-7'), 120.4 (C-8'), 119.2 (C-1), 40.0 (C-4), 25.4 (C-2), 14.8 (C-3); Anal. calc. for C₁₁H₁₁N₅O: C, 57.6; H, 4.8; N, 30.6; found C, 57.8; H, 5.1; N, 30.8%.

### Example 50

***N*-(3-Azidopropyl)-1,2,4-benzotriazin-3-amine 1-oxide (28).** A solution of chloride **19** (2.18 g, 12.0 mmol) and 3-azido-1-propanamine hydrochloride (2.46 g, 18.0 mmol) and Et₃N (5.0 mL, 36.0 mmol) in DCM (100 mL) was heated at reflux temperature for 16 h. The solvent was evaporated and the residue chromatographed, eluting with a gradient (5-10%) of EtOAc/DCM, to give 1-oxide **28** (2.49 g, 85%) as a yellow powder, mp (EtOAc/DCM) 128-130 °C; ¹H NMR δ 8.26 (dd, *J* = 8.7, 1.4 Hz, 1 H, H-8'), 7.71 (ddd, *J* = 8.3, 7.1, 1.4 Hz, 1 H, H-6'), 7.61 (d, *J* = 8.3 Hz, 1 H, H-5'), 7.30 *(ddd, J =* 8.7, 7.1, 1.1 Hz, 1 H, H-7'), 5.73 (br s, 1 H, NH), 3.67 (dd, *J =* 6.6, 6.4 Hz, 2 H, CH₂N), 3.47 (t, *J* = 6.5 Hz, 2 H, CH₂N₃), 1.95-2.03 (m, 2 H, CH₂); ¹³C NMR δ 158 9 (C-3'), 148.8 (C-4a'), 135.6 (C-6'), 130.9 (C-8a'), 126.6 (C-5'), 125.0 (C-7'), 120.4 (C-8'), 49 2 (CH₂N₃), 38.8 (CH₂N), 28.6 (CH₂); Anal. calc. for C₁₀H₁₁O₇: C, 50.0; H, 4.5; N, 40.0; found C, 49.1; H, 4.6; N, 40.3%.

### Example 51

***tert*-Butyl 3-[(1-oxido-1,2,4-benzotriazin-3-yl)amino]propylcarbamate (29).** A solution of chloride **19** (4.0 g, 22.0 mmol), tert-butyl 3-aminopropylcarbamate (5.76 g, 33 0 mmol) and Et₃N (4.6 mL, 33.0 mmol) in DCM (150 mL) was stirred at 20 °C for 5 days. The solvent was evaporated and the residue chromatographed, eluting with 20% EtOAc/DCM, to give 1-oxide **29** (5.21 g, 74%) as a yellow powder, mp (EtOAc/DCM) 145-147 °C; ¹H NMR [(CD₃)₂SO] δ 8.13 (dd, *J* = 8.6, 1.1 Hz, 1 H, H-8'), 7.84 (s, 1 H, NH), 7.78 (ddd, *J* = 8.4, 7.1, 1.1 Hz, 1 H, H-6'), 7.56 (d, *J* = 8.4 Hz, 1 H, H-5'), 7 32 (ddd, *J* = 8.6, 7.1, 1.1 Hz, 1 H, H-7'), 6.83 (t, *J* = 5.3 Hz, 1 H, NHCO₂), 3.32-3.36 (m, 2 H, H-1), 2.99-3.04 (m, 2 H, H-3), 1.66-1.73 (m, 2 H, H-2), 1.37 [s, 9 H, C(CH₃)₃]; ¹³C NMR [(CD₃)₂SO] δ 158.9, 155.6, 148.2, 135.7, 130.0, 125.9, 124.4, 119.9, 77.4, 38.2, 37.5, 28.9, 28.2 (3); Anal. calc. for C₁₅H₂₁N₅O₃: C, 56.4; H, 6.6; N, 21 9; found: C, 56.4; H, 6.6; N, 22.1%.

### Example 52

***btert*-Butyl 3-[(1-oxido-1,2,4-benzotriazin-3-yl)amino]propyl(ethyl)carbamate (32). *tert*-Butyl 2-cyanoethyl(ethyl)carbamate (30).** A solution of ethylamine (6.1 mL, 76 mmol) was added dropwise to stirred acrylonitrile (10 mL) at 5 °C and the mixture allowed to warm to 20 °C over 1 h. The excess acrylonitrile was evaporated and the residue dissolved in DCM (100 mL). A solution of di-*tert*-butyldicarbonate (18.3 g, 84 mmol) in DCM (50 mL) added dropwise at 5 °C and then stirred at 20 °C for 16 h. The solution was diluted with DCM (100 mL), washed with dilute Na₂CO₃ solution (100 mL), 0.1 M HCl (100 mL), water (2 × 100 mL), and brine (50 mL). The organic fraction was dried and the solvent evaporated. The residue was chromatographed, eluting with 20% EtOAc/pet. ether, to give nitrile 30 (15.0 g, 99%) as an oil, ¹H NMR δ 3.47 (t, *J* = 6 7 Hz, 2 H, CH₂N), 3.20 (q, *J* = 7.1 Hz, 2 H, CH₂), 2.61 (br s, 2 H, CH₂), 1.48 [s, 9 H, C(CH₃)₃], 1.14 (t, *J* = 7.1 Hz, 3 H, CH₃); MS (EI) m/z 198 (M⁺, 1%), 158 (2), 143 (5), 125 (20), 57 (100); HRMS (El) calc. for C₁₀H₁₈N₂O₂ (M⁺) m/z 198.1368, found 198.1367.

***tert*-Butyl 3-[(1-oxido-1,2,4-benzotriazin-3-yl)amino]propyl(ethyl)carbamate (32).** A mixture of nitrile **30** (4.60 g, 23.2 mmol) and freshly prepared Raney Nickel (3 mL) in EtOH saturated with NH₃ was stirred under H₂ (60 psi) for 16 h. The mixture was filtered through celite, washed with EtOH (4 × 10 mL), and the solvent evaporated to give tert-butyl 3-aminopropyl(ethyl)carbamate (**31**) (4.65 g, 99%) as an oil which was used without further characterization. Amine **31** (2.5 g, 12.3 mmol) was added to a stirred solution of chloride **19** (0 89 g, 4.9 mmol) in DME (50 mL) and the solution heated at 100 °C for 6 h. The solvent was evaporated and the residue partitioned between DCM (150 mL) and water (100 mL). The organic fraction was dried and the solvent evaporated. The residue was chromatographed, eluting with a gradient (20-50%) of EtOAc/pet. ether, to give 1-oxide 32 (1.43 g, 84%) as a yellow solid, mp (EtOAc/pet ether) 64-66 °C; ¹H NMR δ 8.24 (d, *J* = 8.6 Hz, 1 H, H-8), 7.65-7.70 (m, 1 H, H-6), 7.57 (d, *J* = 8.4 Hz, 1 H, H-5), 7.24-7.28 (m, 1 H, H-7), 6.32 (br s, 1 H, NH), 3 50-3 55 (m, 2 H, CH₂N), 3.32-3.35 (m, 2 H, CH₂N), 3.19-3 25 (m, 2 H, CH₂N), 1.82-1 86 (m, 2H, CH₂), 1.48 [s, 9 H, C(CH₃)₃], 1.12 (t, *J* = 7.0 Hz, 3 H, CH₃); ¹³C NMR δ 159.0 (NCO₂), 156.2 (C-3), 148.9 (C-4a), 135.4 (C-6), 130.8 (C-8a), 126.4 (C-5), 124 6 (C-7), 120.4 (C-8), 79.6 [C(CH₃)₃], 43.5 (CH₂N), 42.0 (CH₂N), 37.9 (CH₂N), 27.9 [C(CH₃)₃], 27.4 (CH₂), 13.8 (CH₃); Anal. calc. for C₁₇H₂₅N₅O₃: C, 58.8; H, 7.3; N, 20.2; found C, 59.0; H, 7.3, N, 20.4%.

### Example 53

**3-{[2-(2-Hydroxyethoxy)ethyl]amino}-1,2,4-benzotriazine 1-oxide** (33). A solution of chloride **19** (3.0 g, 16.5 mmol) in DCM (50 mL) was added to a stirred solution of 2-(aminoethoxy)ethanol (2.49 mL, 24.8 mmol) and Et₃N (3.45 mL, 24.8 mmol) in DCM (80 mL) and the solution stirred at 20 °C for 16 h. The solvent was evaporated and the residue chromatographed, eluting with 40% EtOAc/DCM, to give 1-oxide 33 (2.62 g, 63%) as a yellow powder, mp (DCM/EtOAc) 131-131.5 °C; ¹H NMR δ 8.25 (dd, *J* = 8.7, 1 2 Hz, 1 H, H-8), 7.68 (ddd, *J* = 8.4, 7.2, 1.5 Hz, 1 H, H-6), 7.57 (d, *J* = 8.4 Hz, 1 H, H-5), 7 28 (ddd, *J* = 8.7, 7.2, 1.3 Hz, 1 H, H-7), 6.02 (br s, 1 H, NH), 3.74-3.80 (m, 6 H, 3 × CH₂O), 3.64-3.67 (m, 2 H, CH₂N), 2.71 (t, *J* = 5.9 Hz, 1 H, OH); ¹³C NMR δ 158.9, 149.7, 135.5, 130.9, 126.4, 124.9, 120.4, 72.4, 69.5, 61.7, 41.9; Anal. calc. for C₁₁H₁₄N₄O₃: C, 52.8; H, 5.6; N, 22.4; found C, 52.9; H, 5.7; N, 22.6%.

### Example 54

**3-{[2-(2-Azidoethoxy)ethyl]amino}-1,2,4-benzotriazine 1-oxide (34).**
Methanesulfonyl chloride (0.82 mL, 10.6 mmol) was added dropwise to a stirred solution of alcohol **33** (2.41 g, 9.63 mmol) and Et₃N (1.74 mL, 12.5 mmol) in DCM (100 mL) at 5 °C and the solution stirred at 20 °C for 1 h. The solution was diluted with DCM (100 mL) and washed with water (3 × 50 mL), brine (50 mL), dried and the solvent evaporated. The residue was dissolved in DMF (50 mL) and NaN₃ (0.69 g, 10.6 mmol) added. The mixture was heated at 100 °C for 2 h, cooled to 30 °C and the solvent evaporated. The residue was partitioned between EtOAc (100 mL) and water (100 mL). The organic fraction was washed with brine (50 mL), dried, and the solvent evaporated. The residue was chromatographed, eluting with 50% EtOAc/pet. ether, to give azide **34** (2.35 g, 89%) as yellow crystals, mp (EtOAc/pet. ether) 102-104 °C; ¹H NMR δ 8 27 (dd, *J* = 8.7, 1.4 Hz, 1 H, H-8), 7.70 (ddd, *J* = 8.6, 7.1, 1.5 Hz, 1 H, H-6), 7.59 (d, *J* = 8.6 Hz, 1 H, H-5), 7.29 (ddd, *J* = 8.6, 7.1, 1.4 Hz, 1 H, H-7), 5.70 (br s, 1 H, NH), 3 71-3.78 (m, 4 H, 2 × CH₂O), 3.69 (dd, *J* = 5.3, 4.8 Hz, 2 H, CH₂N₃), 3.41 (dd, *J* = 5.1, 4 9 Hz, 2H, CH₂N); ¹³C NMR δ 158 9, 148 7, 135.5, 131.1, 126.5, 125.0, 120.4, 70.0, 69.6, 50.7, 41.1; Anal. calc. for C₁₁H₁₃N₇O₂; C, 48 0; H, 4.8; N, 35.6; found C, 48.3; H, 4.6, N, 35.7%.

### Example 55

**3-{[2-(2-*tert*-Butyloxycarbamoylethoxy)ethyl]amino}-1,2,4-benzotriazine 1-oxide (35).** Propane-1,3-dithiol (5.7 mL, 57.0 mmol) was added dropwise to a stirred solution of azide **34** (1.57 g, 5.70 mmol) and Et₃N (7 95 mL, 57 mmol) in MeOH (100 mL) under N₂ and the solution heated at reflux temperature for 8 h. The solution was cooled to 30 °C and partitioned between 1 M HCl (100 mL) and Et₂O (100 mL). The aqueous fraction was adjusted to pH 12 with 7 M NaOH solution and extracted with DCM (3 × 50 mL). The organic fraction was dried and the solvent evaporated. The residue was dissolved in THF (100 mL) and a solution of di-tert-butyldicarbonate (1 87 g, 8.55 mmol) in THF (50 mL) added dropwise. The solution was stirred at 20 °C for 16 h, the solvent evaporated and the residue chromatographed, eluting with 40% EtOAc/pet. ether, to give carbamate **35** (1.85 g, 93%) as a yellow solid, mp (EtOAc/pet. ether) 134-137 °C; ¹H NMR δ 8.26 *(dd, J* = 8.4, 0.9 Hz, 1 H, H-8), 7.71 (ddd, *J* = 8.3, 7.1, 1.4 Hz, 1 H, H-6), 7 59 (d, *J* = 8.3 Hz, 1 H, H-5), 7.29 (ddd, *J* = 8.4, 7.1, 1.3 Hz, 1 H, H-7), 5 74 (br s, 1 H, NH), 4.93 (br s, 1 H, NH), 3.67-3.73 (m, 4 H, 2 × CH₂O), 3.56 (t, *J* = 5.2 Hz, 2 H, CH₂N), 3.29-3.36 (m, 2 H, CH₂N), 1.45 [s, 9 H, C(CH₃)₃]; ¹³C NMR δ 159.9, 155.9, 148.7, 135.5, 131.0, 126.5, 125.0, 120.4, 79.4, 70 2, 69.2, 41.1, 40.4, 28.4 (3); Anal. calc. for C₁₆H₂₃N₅O₄: C, 55.0; H, 6.6; N, 20.1; found C, 55.3; H, 6.8; N, 20.1 %.

### Example 56

***N*-[2-(2-Aminoethoxy)ethyl]-1,2,4-benzotriazin-3-amine 1-oxide (36).**
A solution of carbamate **35** (0.99 g, 2.8 mmol) in MeOH (30 mL) was treated with HCl gas and stirred at 20 °C for 2 h. The solvent was evaporated and the residue partioned between dilute NH₄OH (50 mL) and CHCl₃ (50 mL). The organic fraction was dried and the solvent evaporated to give amine 36 (618 mg, 88%) as a red solid, mp 116-118 °C; ¹H NMR δ 8.25 (dd, *J* = 8.7, 1.1 Hz, 1 H, H-8), 7.70 (ddd, *J* = 8.4, 7.1, 1.1 Hz, 1 H, H-6), 7.58 (d, *J* = 8.4 Hz, 1 H, H-5), 7.28 (ddd, *J* = 8.7, 7.1, 1.1 Hz, 1 H, H-7), 6.04 (br s, 1 H, NH), 3.68-3.76 (m, 4 H, 2 x CH₂O), 3.54 (t, *J* = 5.1 Hz, 2 H, CH₂N), 2.90 (t, *J* = 5 1 Hz, 2 H, CH₂N), 1 82 (br s, 2 H, NH₂); ¹³C NMR δ 158 9 (C-3), 148.8 (C-4a), 135.5 (C-6), 130.9 (C-8a), 126.4 (C-5), 124.9 (C-7), 120.4 (C-8), 73.1 (CH₂O), 69.2 (CH₂O), 41.7 (CH₂N), 41.2 (CH₂N); Anal. calc. for C₂₅H₂₂N₆O₃.½H₂O: C, 51.15; H, 6.2, N, 27.1; found C, 51.6; H, 6.1, N, 26.8%.

### Example 57

***N***^{**1**}**,*N***^{**1**}**-Dimethyl-*N***^{**2**}**-(1-oxido-1,2,4-benzotriazin-3-yl)-1,2-ethanediamine (37).** *N*,*N*-Dimethylethanediamine (0.66 mL, 6.0 mmol) was added to a stirred solution of chloride **19** (438 mg, 2.4 mmol) in DME (50 mL) and the solution stirred at reflux temperature for 2 h. The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-10%) of MeOH/DCM, to give 1-oxide **37** (514 mg, 91%) as a yellow solid, mp (MeOH/EtOAc) 121-123 °C; ¹H NMR [(CD₃)₂SO] δ 8.13 (dd, *J =* 8.6, 1.1 Hz, 1 H, H-8'), 7.78 (ddd, *J =* 8.5, 7.0, 1.1 Hz, 1 H, H-6'), 7.72 (br s, 1 H, NH), 7.57 (br d, *J* = 8.5 Hz, 1 H, H-5'), 7.33 (ddd, *J* = 8.6, 7.0, 1.3 Hz, 1 H, H-7'), 3.41-3.45 (m, 2 H, CH₂N), 2.45-2.50 (m, 2 H, CH₂N), 2.20 [s, 6 H, N(CH₃)₂]; ¹³C NMR [(CD₃)₂SO] δ 158.8 (C-3'), 148 3 (C-4a'), 135.6 (C-6'), 129.9 (C-8a'), 125.9 (C-5'), 124.4 (C-7'), 119.8 (C-8'), 57.6 (CH₂N), 45.1 [N(CH₃)₂], 38.6 (CH₂N); Anal. calc. for C₁₁H₁₅N₅O; C, 56.6; H, 6.5; N, 30.0; found C, 56 8: H, 6.6; N, 30.4%.

### Example 58

***N***^{***1***}***,N***^{***1***}***,N*** ^{**2**}**-Trimethyl-N**^{**2**}**-(1-oxido-1,2,4-benzotriazin-3-yl)-1,2-ethanediamine (38).** N¹,N¹,N²-Trimethyl-1,2-ethanediamine (0.45 mL, 3.5 mmol) was added to a stirred solution of chloride **19** (210 mg, 1.2 mmol) in DME (50 mL) and the solution stirred at reflux temperature for 16 h. The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-10%) of MeOH/DCM, to give 1-oxide **38** (277 mg, 96%) as a yellow solid which was recrystallized as the hydrochloride salt, mp (MeOH/EtOAc) 220-223 °C; ¹H NMR [(CD₃)₂SO] δ 10.64 (br s, 1 H, NH+Cl⁻), 8.16 (dd, *J* = 8.7, 1 3 Hz, 1 H, H-8'), 7.84 (ddd, *J* = 8.6, 7.1, 1.3 Hz, 1 H, H-6'), 7.64 (d, *J* = 8.6 Hz, 1 H, H-5'), 7.40 (ddd, *J* = 8.7, 7.1, 1.2 Hz, 1 H, H-7'), 4.04 (t, *J* = 6.3 Hz, 2 H, CH₂N), 3.37-3.42 (m, 2 H, CH₂N), 3.21 (s, 3 H, NCH₃), 2.85 [d, *J* = 4.5 Hz, 6 H, N(CH₃)₂]; ¹³C NMR [(CD₃)₂SO] δ 158.2 (C-3'), 148.1 (C-4a'), 136.0 (C-6'), 129.4 (C-8a'), 126.2 (C-5'), 125.3 (C-7'), 119.8 (C-8'), 53.5 (CH₂N), 43.7 (NCH₃), 42.5 [N(CH₃)₂], 35.0 (CH₂N); Anal calc. for C₁₂H₁₈ClN₅O: C, 50.8; H, 6.4; N, 24.7; Cl, 12.5; found C, 51.3: H, 6.7; N, 24.8; Cl, 12.7%.

### Example 59

***N***^{**1**}**-(1-Oxido-1,2,4-benzotriazin-3-yl)-N**^{**2**}**,N**^{**2**}**-dipropyl-1,2-ethanediamine hydrochloride (39)**. MsCl (125 µL, 1.6 mmol) was added to a stirred solution of alcohol **22** (277 mg, 1 3 mmol) and Et₃N (280 µL, 2.0 mmol) in dry DCM (50 mL) at 5 °C and the solution stirred for 2 h at 20 °C. The solution was diluted with DCM (30 mL), washed with water (2 × 20 mL), the organic fraction dried and the solvent evaporated. The residue was dissolved in DMF (5 mL) and di-n-propylamine (9.2 mL, 67 mmol) added and the solution heated at 50 °C for 2 h. The solvent was evaporated and the residue partitioned between EtOAc (50 mL) and water (50 mL). The organic fraction was extracted with water (2 × 25 mL), dried and the solvent evaporated. The residue was chromatographed, eluting with a gradient (2-5%) of MeOH/DCM, to give the amine **39** (152 mg, 39 %) which was dissolved in HCl saturated MeOH, the solvent evaporated and the residue crystallized as a tan solid, mp (MeOH/EtOAc) 159-161 °C; ¹H NMR [(CD₃)₂SO] δ 10.70 (br s, 1 H, NH⁺Cl⁻), 8.17 (dd, *J* = 8.6, 1.0 Hz, 1 H, H-8), 8 14 (br s, 1 H, NH), 7.84 (ddd, *J* = 8.4, 7.0, 1.3 Hz, 1 H, H-6'), 7.59 (d, *J* = 8.4 Hz, 1 H, H-5'), 7.40 (ddd, *J* = 8.6, 7.0, 1.3 Hz, 1 H, H-7'), 3.74-3.81 (m, 2 H, CH₂N), 3.29-3.33 (m, 2 H, CH₂N), 3.03-3.13 (m, 4 H, 2 × CH₂N), 1.70-1.79 (m, 4 H, 2 × CH₂), 0.93 (t, *J* = 7.3 Hz, 6 H, 2 × CH₃); ¹³C NMR [(CD₃)₂SO] δ 158.5 (C-3'), 147.8 (C-4a'), 135.9 (C-6'), 130.3 (C-8a'), 126.0 (C-5'), 125.1 (C-7'), 119.8 (C-8'), 53.6 (2 × CH₂N), 50.1 (CH₂N), 35.3 (CH₂N), 16.3 (2 × CH₂), 10.8 (2 × CH₃); Anal. calc. for C₁₅H₂₃N₅O.2HCl; C, 49.7; H, 7.0; N, 19.3; Cl, 19.6; found C, 50.1; H, 7.0; N, 19.4; Cl, 19.4%.

### Example 60

***N*-[2-(1-Pyrrolidinyl)ethyl]-1,2,4-benzotriazin-3-amine 1-oxide (40).** 2-(1-Pyrrolidinyl)ethylamine (1 25 mL, 9.9 mmol) was added to a stirred solution of chloride **19** (599 mg, 3.3 mmol) in DME (50 mL) and the solution stirred at reflux temperature for 4 h The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-10%) of MeOH/DCM, to give 1-oxide **40** (806 mg, 94%) as a yellow solid, mp (DCM) 141-143 °C; ¹H NMR [(CD₃)₂SO] δ 8.13 (dd, *J* = 8.6, 1.0 Hz, 1 H, H-8), 7.81 (br s, 1 H, NH), 7.78 (ddd, *J* = 8.4, 7.0, 1.0 Hz, 1 H, H-6), 7.57 (d, *J* = 8.4 Hz, 1 H, H-5), 7.33 (ddd, *J* = 8.6, 7.0, 1.0 Hz, 1 H, H-7), 3.42-3.48 (m, 2 H, CH₂N), 2.63 (t, *J* = 6.8 Hz, 2 H, CH₂N), 2.47-2.53 (m, 4 H, 2 × CH₂N), 1.64-1.71 (m, 4 H, 2 × CH₂); ¹³C NMR [(CD₃)₂SO] δ 158.8 (C-3), 148.3 (C-4a), 135.6 (C-6), 128.0 (C-8a), 126.0 (C-5), 124.4 (C-7), 119.8 (C-8), 54.2 (CH₂N), 53.5 (2 × CH₂N), 39.8 (CH₂N), 23 0 (2 × CH₂); Anal calc. for C₁₃H₁₇N₅O; C, 60.2; H, 6.6; N, 27.0; found C, 60.2: H, 6.9; N, 27.3%.

### Example 61

***N*-[2-(4-Morpholinyl)ethyl]-1,2,4-benzotriazin-3-amine 1-oxide (41).** 2-(4-Morpholinyl)ethylamine (1.2 mL, 8.9 mmol) was added to a stirred solution of chloride **19** (541 mg, 3.0 mmol) in DME (50 mL) and the solution stirred at reflux temperature for 4 h. The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-10%) of MeOH/DCM, to give 1-oxide **41** (802 mg, 98%) as a yellow solid, mp (DCM) 170-172 °C; ¹H NMR [(CD₃)₂SO] δ 8.13 (dd, *J* = 8.6,1.2 Hz, 1 H, H-8), 7.79 (ddd, *J* = 8.4, 7.0, 1.2 Hz, 1 H, H-6), 7.76 (br s, 1 H, NH), 7.57 (d, *J* = 8.4 Hz, 1 H, H-5), 7.34 (ddd, *J* = 8 6, 7.0, 1.2 Hz, 1 H, H-7), 3.55-3.58 (m, 4 H, 2 × CH₂O), 3.45-3 50 (m, 2 H, CH₂N), 2.51-2.56 (m, 2 H, CH₂N), 2.41-2.45 (m, 4 H, 2 × CH₂N); ¹³C NMR [(CD₃)₂SO] δ 158.9 (C-3), 148.3 (C-4a), 135.7 (C-6), 130.0 (C-8a), 126.0 (C-5), 124.5 (C-7), 119.8 (C-8), 66.1 (2 × CH₂O), 56.8 (CH₂N), 53.2 (2 × CH₂N), 37.7 (CH₂N); Anal. calc. for C₁₃H₁₇N₅O₂. C, 56.7; H, 6.2; N, 25.4; found C, 56.5; H, 6.3; N, 25.1%.

### Example 62

***N*-[2-(1- Piperidinyl)ethyl]-1 ,2,4-benzotriazin-3-amine 1-oxide (42).** 2-(1-Piperidinyl)ethylamine (1.2 mL, 8.2 mmol) was added to a stirred solution of chloride **19** (499 mg, 2.7 mmol) in DME (50 mL) and the solution stirred at reflux temperature for 1 h The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-4%) of MeOH/DCM, to give 1-oxide **42** (644 mg, 86%) as a yellow solid, mp (DCM) 141-144 °C; ¹H NMR δ 8.24 (d, *J* = 8.6, Hz, 1 H, H-8), 7.69 (ddd, *J* = 8.4, 7.0, 1.4 Hz, 1 H, H-6), 7.58 (d, *J* = 8.4 Hz, 1 H, H-5), 7.27 (ddd, *J* = 8.6, 7.0, 1.3 Hz, 1 H, H-7), 5 30 (br s, 1 H, NH), 3.57-3.64 (m, 2 H, CH₂N), 2.63-2.69 (m, 2 H, CH₂N), 2.47-2 54 (m, 4 H, 2 × CH₂N), 1.60-1 68 (m, 4 H, 2 × CH₂), 1.44-1.50 (m, 2 H, CH₂); ¹³C NMR δ 158.8 (C-3), 148.9 (C-4a), 135.5 (C-6), 130.8 (C-8a), 126.4 (C-5), 124.7 (C-7), 120 5 (C-8), 56.8 (CH₂N), 54.3 (2 × CH₂N), 37.8 (CH₂N), 25.7 (2 × CH₂), 24.2 (CH₂); Anal. calc. for C₁₄H₁₉N₅O: C, 61.5; H, 7.0; N, 25.6; found C, 61.4: H, 7.1; N, 25.6%.

### Example 63

***N*-[2-(2,6-Dimethyl-1-piperidinyl)ethy)]-1,2,4-benzotriazin-3-amine 1-oxide (43).**
2-(2,6-Dimethyl-1-piperidinyl)ethylamine (636 mg, 4.1 mmol) was added to a stirred solution of chloride **19** (493 mg, 2.7 mmol) and Et₃N (0.57 mL, 4.1 mmol) in DME (50 mL) and the solution stirred at reflux temperature for 2 h. The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-10%) of MeOH/DCM, to give 1-oxide **43** (484 mg, 59%) as a yellow solid, mp (MeOH/DCM) 160-163 °C; ¹H NMR δ 8 25 (dd, *J* = 8 6, 1.4 Hz, 1 H, H-8), 7.68 (ddd, J = 8.5, 7.0, 1.4 Hz, 1 H, H-6), 7 57 (dd, *J* = 8.5, 1.3 Hz, 1 H, H-5), 7.27 (ddd, *J* = 8.6, 7.0, 1.3 Hz, 1 H, H-7), 5.68 (br s, 1 H, NH), 3.53-3.58 (m, 2 H, CH₂N), 2.91 (dd, *J* = 7.4, 7.1 Hz, 2 H, CH₂N), 2.50-2.59 (m, 2 H, 2 × CH), 1.65-1.70 (m, 1 H, CH₂), 1.54-1.59 (m, 2 H, CH₂), 1.35-1.40 (m, 1 H, CH₂), 1.25-1.33 (m, 2H, CH₂), 1.20 (d, *J* = 6.3 Hz, 6 H, 2 x CH₃); ¹³C NMR δ 159.0 (C-3), 148.9 (C-4a), 135.4 (C-6), 130.9 (C-8a), 126.5 (C-5), 124.7 (C-7), 120.5 (C-8), 57.3 (2 × CH), 47.4 (CH₂N), 39.5 (CH₂N), 34.2 (CH₂), 24.4 (2 × CH₂), 21.6 (2 × CH₃); Anal. calc. for C₁₆H₂₃N₅O: C, 63.8; H, 7.7; N, 23.2; found C, 63.6; H, 7.6; N, 23.3%.

### Example 64

***N***^{**1**}**-(1-Oxido-1,2,4-benzotriazin-3-yl)-*N***^{**3**}**,*N***^{**3**}**-dimethyl-1,3-propanediamine (44).** N,N-dimethylpropylenediamine (0.9 mL, 6.9 mmol) was added dropwise to a stirred solution of chloride **19** (500 mg, 2.75 mmol) in DME (50 mL) and the solution stirred at reflux temperature for 8 h. The solution was cooled to 20 °C, the solvent evaporated and the residue partitioned between aqueous NH₄OH solution (100 mL) and EtOAc (100 mL) The organic fraction was dried, and the solvent evaporated. The residue was chromatographed, eluting with a gradient (0-10%) of MeOH/DCM, to give 1-oxide **44** (629 mg, 92%) as a yellow solid, mp 137-138 °C; ¹H NMR [(CD₃)₂SO] δ 8.13 (dd, *J* = 8 6, 1 1 Hz, 1 H, H-8'), 7 92 (br s, 1 H, NH), 7.77 (ddd, *J* = 8.4, 7.1, 1.1 Hz, 1 H, H-6'), 7.56 (d, *J* = 8.4 Hz, 1 H, H-5'), 7 32 (ddd, *J* = 8.6, 7.1, 1.1 Hz, 1 H, H-7'), 3.37 (br s, 2 H, H-1), 2.30 (t, *J* = 7.0 Hz, 2 H, H-3), 2.15 [s, 6 H, N(CH₃)₂], 1.70-1.76 (m, 2 H, H-2); ¹³C NMR [(CD₃)₂SO] δ 158.8 (C-3'), 148.3 (C-4a'), 135.6 (C-6'), 129.9 (C-8a'), 125.9 (C-5'), 124.3 (C-7'), 119.8 (C-8'), 56.6 (CH₂N), 45.1 [N(CH₃)₂], 39.0 (CH₂N), 26.3 (CH₂); Anal. calc. for C₁₂H₁₇N₅O: C, 58.3; H, 6.9; N, 28 3; found C, 58.3; H, 7.0; N, 28 5%.

### Example 65

***N*-Phenyl-1,2,4-benzotriazin-3-amine 1-oxide (45)** Two drops of cHCl were added to a solution of chloride **19** (0.52 g, 2.86 mmol) and aniline (0.78 mL, 8.59 mmol) in DME (10 mL) and the solution stirred at reflux temperature for 16 h The solvent was evaporated and the residue chromatographed, eluting with 10% EtOAc/pet. ether, to give 1-oxide **45** (334 mg, 49%) as a yellow powder, mp 197-198.5 °C [lit. (Pazdera & Potacek, *Chem. Papers,* **1989**, 43, 107) mp 199-201 °C]; ¹H NMR δ 8.32 (d, J = 9.0 Hz, 1 H, H-8), 7.70-7.77 (m, 4 H, H-5, H-6, H-2', H-6'), 7.37-7.42 (m, 3 H, H-7, H-3', H-5'), 7.22 (br s, 1 H, NH), 7.13 (dt, *J* = 7.5, 0.9 Hz, 1 H, H-4'); ¹³C NMR δ 156.3 (C-3), 148.1 (C-4a), 138.1 (C-1'), 135.8 (C-6), 131.6 (C-8a), 129.1 (C-3', C-5'), 127.1 (C-5), 126.1 (C-4'), 123.8 (C-7), 120.4 (C-8), 119.7 (C-2', C-6').

### Example 66

***N*-[3-(2-Methoxyethyl)phenyl]-1,2,4-benzotriazin-3-amine 1-oxide (49). 1-(2-Methoxyethyl)-3-nitrobenzene (47).** A solution of 3-nitrophenethyl alcohol (**46**) (1.05 g, 6.3 mmol) in THF (10 mL) was addded dropwise to a stirred suspension of NaH (325 mg, 8.1 mmol) in THF (30 mL) at 5 °C and the mixture warmed to 20 °C and stirred 30 min. lodomethane (3.9 mL, 62.5 mmol) was added and the mixture stirred at 20 °C for 16 h. The solvent was evaporated and the residue partitioned between EtOAc (100 mL) and water (100 mL). The organic fraction was washed with water (2 × 30 mL), brine (30 mL), dried, and the solvent evaporated. The residue was chromatographed, eluting with 20% EtOAc/pet. ether, to give ether **47** (981 mg, 87%) as a clear oil, (Norman & Radda, J. *Chem.* Soc. **1961**, 3030) ¹H NMR δ 8.06-8 11 (m, 2 H, H-2, H-4), 7.57 (d, *J* = 7 6 Hz, 1 H, H-6), 7.47 (dd, *J* = 7.9, 7.6 Hz, 1 H, H-5), 3.65 (t, *J* = 6.5 Hz, 2 H, CH₂O), 3.36 (s, 3 H, OCH₃), 2.98 (t, *J* = 6.5 Hz, 2 H, CH₂); ¹³C NMR δ 148.3 (C-3), 141 3 (C-1), 135.2 (C-6), 129.2 (C-5), 123.7 (C-2), 121.4 (C-4), 72 5 (CH₂O), 58.8 (OCH₃), 35.8 (CH₂).

**3-(2-Methoxyethyl)aniline (48)** A solution of ether **47** (928 mg, 5.1 mmol) in EtOH (50 mL) with Pd/C (100 mg) was stirred under H₂ (60 psi) for 2 h. The mixture was filtered through celite, washed with EtOH (2 × 10 mL) and the solvent evaporated to give aniline **48** (718 mg, 93%) as a pale pink oil, ¹H NMR δ 7.08 (dd, *J* = 7.7, 7.3 Hz, 1 H, H-5), 6.62 (br d, *J* = 7.3 Hz, 1 H, H-4), 6.51-6.55 (m, 2 H, H-2, H-6), 3.50 (br s, 2 H, NH₂), 3.58 (t, *J* = 7.2 Hz, 2 H, CH₂O), 3.35 (s, 3 H, OCH₃), 2.80 (t, *J* = 7.2 Hz, 2 H, CH₂); ¹³C NMR δ 146.4 (C-1), 140.1 (C-3), 129.3 (C-5), 119.1 (C-4), 115.7 (C-2), 113.1 (C-6), 73.6 (CH₂O), 58.6 (OCH₃), 36.2 (CH₂); MS (EI⁺) *m*/*z* 151 (M⁺, 90%), 136 (20), 106 (100); HRMS (EI⁺) calc. for C₉H₁₃NO (M⁺) *m*/*z* 151.0997, found 151.0995.

**N-[3-(2-Methoxyethyl)phenyl]-1,2,4-benzotriazin-3-amine 1-oxide** (49). A solution of chloride **19** (376 mg, 2.07 mmol) and aniline **48** (688 mg, 4.55 mmol) in DMSO (20 mL) was heated at 100 °C for 16 h The solution was partitioned between EtOAc (100 mL) and water (100 mL), the organic fraction washed with water (2 × 50 mL), brine (50 mL), dried , and the solvent evaporated. The residue was chromatographed, eluting with a gradient (20-50%) of EtOAc/pet. ether, to give 1-oxide **49** (590 mg, 96%) as an orange powder, mp (EtOAc/Et₂O) 122-124 °C; ¹H NMR [(CD₃)₂SO] δ 10.18 (s, 1 H, NH), 8.22 (dd, *J* = 8.6, 1.0 Hz, 1 H, H-8), 7.87 (ddd, *J* = 8.5, 7.1, 1.3 Hz, 1 H, H-6), 7.70-7.76 (m, 3 H, H-5, H-2', H-6'), 7.47 (ddd, *J =* 8.6, 7.1, 1.3 Hz, 1 H, H-7), 7.27 (dd, *J* = 7.9, 7.8 Hz, 1 H, H-5'), 6.94 (d, *J* = 7.8 Hz, 1 H, H-4'), 3.58 (t, *J* = 6.8 Hz, 2 H, CH₂O), 3.27 (s, 3 H, OCH₃), 2.82 (t, *J* = 6.8 Hz, 2 H, CH₂); ¹³C NMR [(CD₃)₂SO] δ 156.3 (C-3'), 147.5 (C-3), 139.5 (C-1'), 139.1 (C-4a), 135.9 (C-6), 130.9 (C-8a), 128.4 (C-5'), 126.6 (C-5), 125.8 (C-4'), 123.2 (C-7), 119.8 (C-8), 119.7 (C-2'), 117.3 (C-6'), 72.6 (CH₂O), 57.8 (OCH₃), 35.5 (CH₂); Anal. calc. for C₁₆H₁₆N₄O₂: C, 64.9; H, 5.4; N, 18.9; found C, 65.0; H, 5.5; N, 19.2%.

### Example 67

**Methyl {4-[(1-oxido-1,2,4-benzotriazin-3-yl)amino)phenyl}acetate (51).** A solution of chloride **19** (992 mg, 5 5 mmol) and aniline **50** (1.99 g, 12.0 mmol) in DMSO (30 mL) was heated at 100 °C for 6 h and then 20 °C for 16 h. The solution was partitioned between EtOAc (200 mL) and water (200 mL), the organic fraction washed with water (2 x 100 mL), dried, and the solvent evaporated. The residue was chromatographed, eluting with 10% EtOAc/DCM, to give the 1-oxide **51** (1.05 g, 61%) as a yellow solid, mp (EtOAc/DCM) 216-218 °C; ¹H NMR [(CD₃)₂SO] δ 10.00 (s, 1 H, NH), 8.24 (d, *J* = 8 3 Hz, 1 H, H-8"), 7.82 (d, *J* = 8.4 Hz, 2 H, H-2', H-6'), 7.79 (dd, *J* = 8.2, 7.3 Hz, 1 H, H-6"), 7.70 (d, *J* = 8 2 Hz, 1 H, H-5"), 7.40 (dd, *J* = 8.3, 7.3 Hz, 1 H, H-7"), 7.22 (d, *J* = 8 4 Hz, 2 H, H-3', H-5'), 3.67 (s, 3 H, OCH₃), 3.60 (s, 2 H, H-2), ¹³C NMR [(CD₃)₂SO] δ 171.7 (C-1), 156.3 (C-3"), 147.9 (C-4a"), 138.1 (C-4'), 135.6 (C-6"), 131.9 (C-8a"), 129.4 (C-2', C-6'), 128.3 (C-1'), 126.7 (C-5"), 125.5 (C-7"), 119.9 (C-8"), 119.7 (C-3', C-5'), 51.7 (OCH₃), 40.1 (C-2); Anal. calc. for C₁₆H₁₄N₄O₃: C, 61 9; H, 4.6; N, 18.1; found C, 62 3; H, 4.8; N, 18.1 %.

### Example 68

**{4-[(1-Oxido-1,2,4-benzotriazin-3-yl)amino)phenyl}acetic acid (52).** A solution of NaOH (1 M, 5.2 mL, 5.2 mmol) was added to a stirred suspension of ester **51** (323 mg, 1.0 mmol) in MeOH (30 mL) and the mixture stirred at 20 °C for 2 h. The volume was reduced to ca. 10 mL and the remaining solution washed with Et₂O (2 × 10 mL). The solution was adjusted to pH 1 with 2 M HCl and the suspension extracted with EtOAc (3 x 50 mL), the combined organic fraction dried, and the solvent evaporated. Recrystallization gave the acid **52** (306 mg, 99%) as a yellow powder, mp (EtOAc) 243-245 °C; ¹H NMR [(CD₃)₂SO) δ 12.27 (s, 1 H, CO₂H), 10.21 (s, 1 H, NH), 8.22 (dd, *J =* 8.6, 0 8 Hz, 1 H, H-8"), 7.88 (ddd, *J =* 8.4, 7.1, 1.0 Hz, 1 H, H-6"), 7.79 *(d, J =* 8.5 Hz, 2 H, H-2', H-6'), 7.75 (d, *J* = 8.4 Hz, 1 H, H-5"), 7.46 (ddd, *J=* 8.6, 7.1, 1.0 Hz, 1 H, H-7"), 7.25 (d, *J* = 8.5 Hz, 2 H, H-3', H-5'), 3.54 (s, 2 H, H-2); ¹³C NMR [(CD₃)₂SO] δ 172.7 (C-1), 156.3 (C-3"), 147.6 (C-4a"), 137.7 (C-4'), 135.9 (C-6"), 130.9 (C-8a"), 129.5 (C-2', C-6'), 129.2 (C-1'), 126.5 (C-5"), 125.8 (C-7"), 119.8 (C-8"), 119.3 (C-3', C-5'), 40.0 (C-2); Anal. calc. for C₁₅H₁₂N₄O₃: C, 60.8; H, 4.1; N, 18.9; found C, 61.0; H, 4.0; N, 19.1%.

### Example 69

**2-{4-[(1-Oxido-1,2,4-benzotriazin-3-yl)amino]phenyl}-N-(2-methoxyethyl)acetamide (53).** A solution of acid **52** (259 mg, 0.87 mmol) and CDI (213 mg, 1.3 mmol) in DMF (10 mL) was stirred at 50 °C for 10 min. 2-Methoxyethylamine (152 µL, 1.75 mmol) was added dropwise and the solution stirred at 20 °C for 16 h. The solvent was evaporated and the residue suspended in water (100 mL). The precipitate was filtered, dried, and recrystallized from MeOH to give amide **53** (239 mg, 78%) as a yellow powder, mp (MeOH) 216-218 °C; ¹H NMR [(CD₃)₂SO] δ 10.19 (s, 1 H, NH), 8.21 (dd, *J* = 8.6, 1.0 Hz, 1 H, H-8"), 8.09 (dd, *J* = 5.6, 5.3 Hz, 1 H, CONH), 7.88 (ddd, *J* = 8 3, 7.1, 1.0 Hz, 1 H, H-6"), 7.73-7.78 (m, 3 H, H-2', H-6', H-5"), 7.47 (ddd, *J* = 8.6, 7.1, 1.0 Hz, 1 H, H-7"), 7.25 (d, *J* = 8.5 Hz, 2 H, H-3', H-5'), 3.40 (br s, 2 H, H-2), 3 32-3.37 (m, 2 H, CH₂O), 3.25 (s, 3 H, OCH₃), 3 23 (q, J = 5.7 Hz, 2 H, CH₂N); ¹³C NMR [(CD₃)₂SO] δ 170.3 (C-1), 156.4 (C-3"), 147 6 (C-4a"), 137 5 (C-4'), 135.9 (C-6"), 130 9 (C-8a"), 130.7 (C-1'), 129.1 (C-2', C-6'), 128.5 (C-5"), 125.8 (C-7"), 119.8 (C-8"), 119.4 (C-3', C-5'), 70.6 (CH₂O), 57.8 (OCH₃), 41.6 (CH₂N), 38.4 (C-2); Anal. calc. for C₁₈H₁₉N₅O₃: C, 61.2; H, 5.4; N, 19.8; found C, 61.6; H, 5.3; N, 19.9%.

### Example 70

**N-[2-(Dimethylamino)ethyl]-2-{4-[(1-oxido-1,2,4-benzotriazin-3-yl)amino]phenyl}acetamide (54)**. A solution of acid **52** (476 mg, 1.6 mmol) and CDI (391 mg, 2 4 mmol) in DMF (10 mL) was stirred at 50 °C for 10 min. *N,N*-Dimethylaminoethylamine (353 µL, 3.2 mmol) was added dropwise and the solution stirred at 20 °C for 16 h. The solvent was evaporated and the residue suspended in EtOAc (200 mL). The precipitate was filtered and dried. The mother liquor was evaporated and the residue suspended in water (50 mL), the precipitate filtered, and combined with the previous crop to give amide **54** (562 mg, 95%) as a yellow powder, mp (EtOAc) 225-226 °C; ¹H NMR [(CD₃)₂SO] δ 10.18 (s, 1 H, NH), 8.22 (dd, *J* = 8.6, 1 0 Hz, 1 H, H-8"), 7.92 (t, *J* = 5.4 Hz, 1 H, CONH), 7.87 (ddd, *J* = 8.4, 7.1, 1.0 Hz, 1 H, H-6"), 7.72-7 78 (m, 3 H, H-2', H-6', H-5"), 7.46 (ddd, *J* = 8.6, 7.1, 1.0 Hz, 1 H, H-7"), 7.24 (d, *J* = 8.5 Hz, 2 H, H-3', H-5'), 3.16 (dt, *J* = 6.7, 5.4 Hz, 2 H, CH₂N), 2.28 (t, *J* = 6.7 Hz, 2 H, CH₂N), 2.15 [s, 6 H, N(CH₃)₂]; ¹³C NMR [(CD₃)₂SO) δ 170.1 (C-1), 156.4 (C-3"), 147.6 (C-4a"), 137.5 (C-4'), 135.7 (C-6"), 130.9 (C-8a"), 130.8 (C-1'), 129.1 (C-2', C-6'), 128.5 (C-5"), 125.8 (C-7"), 119.8 (C-8"), 119.4 (C-3', C-5'), 58.2 (CH₂N), 45.1 [N(CH₃)₂], 41.7 (CH₂N), 36.8 (C-2); Anal. calc. for C₁₉H₂₂N₆O₂: C, 62.3; H, 6.1; N, 22.9; found C, 62.6; H, 6.2; N, 22.7%.

### Example 71

**3-Methyl-1,2,4-benzotriazine 1-oxide (55).** Pd(PPh₃)₄ (82 mg, 71 µmol) was added to a stirred, degassed solution of chloride **19** (258 mg, 1.42 mmol) and Me₄Sn (0.39 mL, 2.8 mmol) in DME (20 mL) and the solution stirred under N₂ at reflux temperature for 48 h. The solvent was evaporated and the residue chromatographed, eluting with 20% EtOAc/pet. ether, to give an oil which was chromatographed, eluting with 5% EtOAc/DCM, to give (i) starting material **19** (164 mg, 64%) and (ii) 1-oxide **55** (55 mg, 24%) as a white solid, mp (EtOAc/pet. ether) 99-101 °C [lit. (Atallah & Nazar, *Tetrahedron Lett.,* **1982**, *38*, 1793) mp (benzene/pet ether) 101-102 °C]; ¹H NMR δ 8.44 (d, J = 8.6 Hz, 1 H, H-8), 7.90-7.97 (m, 2 H, H-5, H-6), 7.70 (ddd, J = 8.6, 6.8, 1.8 Hz, 1 H, H-7), 2.80 (s, 3 H, CH₃).

### Example 72

**3-Ethyl-1,2,4-benzotriazine 1-oxide (56).** Pd(PPh₃)₄ (340 mg, 0.30 mmol) was added to a stirred, degassed solution of chloride **19** (539 mg, 2.97 mmol) and Et₄Sn (0.54 mL, 2.7 mmol) in DME (20 mL) and the solution stirred under N₂ at reflux temperature for 4 h The solvent was evaporated and the residue chromatographed, eluting with 20% EtOAc/pet. Ether, to give an oil which was chromatographed, eluting with 5% EtOAc/DCM, to give 1-oxide **56** (448 mg, 86%) as a white solid, mp (EtOAc/pet. ether) 78-80 °C; ¹H NMR δ 8.45 (dd, *J* = 8.7, 1.1 Hz, 1 H, H-8), 7.99 (dd, *J* = 8.5, 1.1 Hz, 1 H, H-5), 7.93 (ddd, *J* = 8.5, 7.1, 1.3 Hz, 1 H, H-6), 7.69 (ddd, *J* = 8.7, 7.1, 1.2 Hz, 1 H, H-7), 3.06 (q, *J* = 7.6 Hz, 2H, CH₂), 1.45 (t, *J* = 7.6 Hz, 3 H, CH₃); ¹³C NMR δ 168.1 (C-3), 147.6 (C-4a), 135.5 (C-6), 133.2 (C-8a), 129.8 (C-5), 128.7 (C-7), 120.1 (C-8), 30.7 (CH₂), 12.2 (CH₃); Anal. calc. for C₉H₉N₃O₃: C, 61.7; H, 5.2, N, 24.0; found C, 62.0; H, 5.0; N, 24.6%.

### Example 73

**3-Phenyl-1,2,4-benzotriazine 1-oxide (57).** Pd(PPh₃)₄ (314 mg, 0.27 mmol) was added to a stirred, degassed solution of chloride **19** (986 mg, 5.43 mmol) and phenylboronic acid (0.73 g, 5.97 mmol) in DME (50 mL) and Cs₂CO₃ (5.3 g, 16.3 mmol) in water (10 mL) and the mixture stirred under N₂ at reflux temperature for 2 h. The mixture was partitioned between EtOAc (100 mL) and water (100 mL), the organic fraction washed with water (2 × 50 mL), dried, and the solvent was evaporated. The residue was chromatographed, eluting with a gradient (20-50%) of EtOAc/pet. ether, to give an oil which was chromatographed, eluting with 5% EtOAc/DCM, to give 1-oxide **57** (743 mg, 61%) as a white solid, mp (EtOAc/pet. ether) 125-127 °C; ¹H NMR δ 8.49-8.54 (m, 3 H, H-8, H-2', H-6'), 8.09 (d, *J* = 8.6 Hz, 1 H, H-5), 7.94 (ddd, *J* = 8.6, 7.1, 1.4 Hz, 1 H, H-6), 7.70 (ddd, *J* = 8.7, 7.1, 1.4 Hz, 1 H, H-7), 7.51-7.57 (m, 3 H, H-3', H-4', H-5'); ¹³C NMR δ 160.7 (C-3), 147.7 (C-4a), 135.6 (C-6), 134.1 (C-1'), 133.5 (C-8a), 131.9 (C-5), 130.5 (C-7), 129.4 (C-4'), 128.8 (C-2', C-6'), 128.5 (C-3', C-5'), 120.3 (C-8); Anal. calc. for C₁₃H₉N₃O: C, 69.9; H, 4.1; N, 18 8; found C, 69.9; H, 4.0; N, 18.7%.

### Example 74

**3-(4-Methoxyphenyl)-1,2,4-benzotriazine 1-oxide (58).** Pd(PPh₃)₄ (162 mg, 0.14 mmol) was added to a stirred, degassed solution of chloride **19** (510 mg, 2.8 mmol) and 4-methoxyphenylboronic acid (0.47 g, 3.1 mmol) in DME (50 mL) and C_{S2}CO₃ (3.0 g, 8.4 mmol) in water (8 mL) and the mixture stirred under N₂ at reflux temperature for 2 h. The mixture was partitioned between EtOAc (100 mL) and water (100 mL), the organic fraction washed with water (2 × 50 mL), dried, and the solvent was evaporated. The residue was chromatographed, eluting with a gradient (20-50%) of EtOAc/pet. ether, to give an oil which was chromatographed, eluting with 5% EtOAc/DCM, to give 1-oxide **58** (408 mg, 57%) as a white solid, mp (EtOAc/pet. ether) 168-170 °C; ¹H NMR δ 8 44-8.49 (m, 3 H, H-8, H-2', H-6'), 8.02 (d, *J* = 8.7 Hz, 1 H, H-5), 7.90 (ddd, *J* = 8.7, 7.2, 1.4 Hz, 1 H, H-6), 7.64 (ddd, *J* = 8.5, 7.2, 1.4 Hz, 1 H, H-7), 7.02 (ddd, *J* = 9.0, 2.9, 2 1 Hz, 2 H, H-3', H-5'), 3.90 (s, 3 H, OCH₃); ¹³C NMR δ 162.8 (C-4'), 160.5 (C-3), 147.8 (C-4a), 135.5 (C-6), 133.2 (C-8a), 130.3 (C-3', C5'), 129.5 (C-5), 129.1 (C-7), 126.5 (C-1'), 120.3 (C-8), 114.3 (C-2', C-6'), 55.4 (OCH₃); Anal. calc. for C₁₄H₁₁N₃O₂: C, 66.4; H, 4.4; N, 16.6; found C, 66.5; H, 4.4; N, 16.7%.

### Example 75

**3-Vinyl-1,2,4-benzotriazine 1-oxide (59).** Pd(PPh₃)₄ (204 mg, 0.18 mmol) was added to a stirred solution of chloride **19** (320 mg, 1 8 mmol) and vinyltributyltin (0.77 mL, 2 6 mmol) in DME (20 mL), the solution degassed and stirred under N₂ at reflux temperature for 6 h. The solvent was evaporated and the residue chromatographed, eluting with 20% EtOAc/pet. ether to give an oil which was chromatographed, eluting with 5% EtOAc/DCM, to give 1-oxide **59** (177 mg, 58%) as a white solid, mp (EtOAc/pet. ether) 85-86 °C; ¹H NMR δ 8.46 (dd, *J* = 8.9, 1.4 Hz, 1 H, H-8), 8.10 (d, *J* = 8.5 Hz, 1 H, H-5), 7.92 (ddd, *J =* 8.5, 7.1, 1.4 Hz, 1 H, H-6), 7.69 (ddd, *J =* 8.9, 7.1, 1 4 Hz, 1 H, H-7), 6.86 *(dd, J =* 17.4, 9.4 Hz, 2 H, H-1'), 6.79 (dd. *J=* 17.4, 2.2 Hz, 1 H, H-2'), 5.92 (dd, *J* = 9 4, 2 2 Hz, 1 H, H-2'); ¹³C NMR δ 160.2 (C-3), 147.4 (C-4a), 135.6 (C-6), 133.6 (C-8a), 133.0 (C-2'), 130.2 (C-5), 129.1 (C-7), 126.6 (C-1'), 120.2 (C-8); Anal. calc. for C₉H₇N₃O: C, 62 4; H, 4.1; N, 24.3; found C, 61.8; H, 4.0; N, 24.4%.

### Example 76

**3-Allyl-1,2,4-benzotriazine 1-oxide (60).** Pd(PPh₃)₄ (340 mg, 0.29 mmol) was added to a stirred solution of chloride **19** (1.1 g, 5 9 mmol) and allyltributyltin (2.0 mL, 6.5 mmol) in DME (60 mL), the solution degassed and stirred under N₂ at reflux temperature for 6 h. The solvent was evaporated and the residue chromatographed, eluting with 20% EtOAc/pet. ether, to give an oil which was chromatographed, eluting with 5% EtOAc/DCM, to give 1-oxide **60** (1.00 g, 90%) as a white solid, mp (EtOAc/pet. ether) 57-58 °C; ¹H NMR δ 8 45 (dd, *J* = 8.6, 1.4 Hz, 1 H, H-8), 8.10 (dd, *J* = 8.4, 1.4 Hz, 1 H, H-5), 7.94 (ddd, *J* = 8.4, 7.1, 1.4 Hz, 1 H, H-6), 7.70 (ddd, *J* = 8.6, 7.1, 1.4 Hz, 1 H, H-7), 6 15-6.24 (m, 1 H, H-2'), 5.31 (dq, *J* = 17.0, 1.5 Hz, 1 H, H-3'), 5 24 (dq, *J* = 10.1, 1.5 Hz, 1 H, H-3'), 3.80 (dq, *J* = 6.8, 1.5 Hz, 2 H, H-1'); ¹³C NMR δ 165.2 (C-3), 147.5 (C-4a), 135 6 (C-6), 133.3 (C-8a), 132.7 (C-2'), 130.1 (C-5), 128.8 (C-7), 120.8 (C-8), 118 5 (C-3'), 41.8 (C-1'); Anal. calc. for C₁₀0H₉N₃O: C, 64.2; H, 4.9; N, 22.5; found C, 63.9; H, 4 9; N, 22.7%.

### Example 77

**3-(2-Hydroxyethyl)-1,2,4-benzotriazine 1-oxide (61).** Ozone was bubbled into a solution of **60** (548 mg, 2.9 mmol) in DCM/MeOH (1:1, 50 mL) at -78 °C until a blue colour persisted. The solution was purged with N₂ to remove excess ozone and a solution of NaBH₄ (111 mg, 2.9 mmol) in EtOH (10 mL) added dropwise and the solution allowed to warm to 20 °C over 1 h. HOAc (1 mL) was added and the solution stirred at 20 °C for 30 min. The solvent was evaporated and the residue partitioned between DCM (50 mL) and water (3 × 50 mL). The organic fraction was dried and the solvent evaporated. The residue was chromatographed, eluting with 50% EtOAc/pet. ether, to give alcohol 61 (392 mg, 70%) as pale yellow needles, mp 105-107 °C; ¹H NMR δ 8.45 (dd, J = 8.6, 1.3 Hz, 1 H, H-8), 7.99 (dd, *J* = 8.5, 1.6 Hz, 1 H, H-5), 7.96 (ddd, *J* = 8.5, 6.7, 1.3 Hz, 1 H, H-6), 7.72 (ddd, *J* = 8.5, 6.7, 1.6 Hz, 1 H, H-7), 4.18-4.20 (m, 2 H, CH₂O), 3.29 (t, *J* = 5.6 Hz, 2 H, CH₂), 3.11 (t, *J* = 5.5 Hz, 1 H, OH); ¹³C NMR δ 165.4 (C-3), 147.0 (C-4a), 135.8 (C-6), 133.6 (C-8a), 130.2 (C-5), 128.6 (C-7), 120.1 (C-8), 60.0 (CH₂O), 39.0 (CH₂); Anal. calc. for C₉H₉N₃O₂: C, 56.5; H, 4.7; N, 22.0; found C, 56.8; H, 4.7; N, 21.8%.

### Example 78

**3-(2-Oxiranylmethyl)-1,2,4-benzotriazine 1-oxide (62).** MCPBA (0.96 g, 3.9 mmol) was added to a stirred solution of alkene **60** (484 mg, 2.6 mmol) in DCM (50 mL) at 20 °C and the mixture stirred for 16 h The solution was diluted with DCM (100 mL), washed with dilute aqeous NH₃ (3 × 50 mL). The organic fraction was dried and the solvent evaporated. The residue was chromatographed, eluting with 50% EtOAc/pet. ether, to give (i) starting material 60 (173 mg, 36%) and (ii) epoxide **62** (251 mg, 48%) as white crystals, mp (EtOAc/pet. ether) 105-107 °C; ¹H NMR δ 8.46 (dd, *J* = 8.7, 1.2 Hz, 1 H, H-8), 8 03 (dd, *J* = 8 5, 1.1 Hz, 1 H, H-5), 7.95 (ddd, *J* = 8.5, 7.1, 1.2 Hz, 1 H, H-6), 7.73 (ddd, *J* = 8.7, 7.1, 1.1 Hz, 1 H, H-7), 3.55-3.60 (m, 1 H, H-2'), 3.27 (dd, *J* = 5.8, 1.9 Hz, 2 H, H-1'), 2.93 (dd, *J* = 4.7, 4.1 Hz, 1 H, H-3'), 2.76 (dd, *J* = 4.7, 2.6 Hz, 1 H, H-3'); ¹³C NMR δ 163.4 (C-3), 147.4 (C-4a), 135.7 (C-6), 133.6 (C-8a), 130.4 (C-5), 128.9 (C-7), 120.1 (C-8), 50.0 (C-2'), 47.1 (C-3'), 40.5 (C-1'); Anal calc. for C₁₀H₉N₃O₂: C, 59.1; H, 4.5; N, 20.7, found C, 59.2; H, 4.6; N, 20.4%.

### Example 79

**3-(2-Methoxyethyl)-1,2,4-benzotriazine 1-oxide (63).** Five aliquots of TMSCH₂N₂ (3 mL, 6.0 mmol) were added to a stirred solution of alcohol **61** (1.14 g, 6.0 mmol) and HBF₄ (1.5 mL, 12 mmol) in DCM (50 mL) over 3 h The solution was stirred at 20 °C for 16 h, the solvent evaporated and the residue chromatographed, eluting with 30% EtOAc/pet. ether, to give (i) methyl ether **63** (375 mg, 30%) as a yellow powder, mp (EtOAC/pet. ether) 56-58°C; ¹H NMR δ 8.45 (dd, *J* = 8.7, 1.2, 1 H, H-8), 8.03 (d, *J =* 8.4 Hz, 1 H, H-5), 7.92 (ddd, *J* = 8.4, 7.0, 1.2 Hz, 1 H, H-6), 7.71 (ddd, *J* = 8.7, 7.0, 1.1 Hz, 1 H, H-7), 3.97 (dd, *J* = 6.5, 6.3 Hz, 2 H, CH₂O), 3.38 (s, 3 H, OCH₃), 3.31 (dd, *J* = 6.5, 6.3 Hz, 2 H, CH₂); ¹³C NMR δ 164.7 (C-3), 147.3 (C-4a), 135.6 (C-6), 130.3 (C-8a), 130.1 (C-5), 128.7 (C-7), 120.1 (C-8), 70.0 (CH₂O), 58.8 (OCH₃), 37.6 (CH₂); Anal. calc. for C₁₀H₁₁N₃O₂: C, 58.5; H, 5.4; N, 20.5; found: C, 58.8; H, 5.4; N, 20.6%; and (ii) starting material **61** (334 mg, 24%), spectroscopically identical to sample prepared above.

### Example 80

**2-(1-Oxido-1,2,4-benzotriazin-3-yl)-*N,N*-dimethylethanamine (64).** MsCl (246 µL, 3.1 mmol) was added to a stirred solution of alcohol **61** (496 mg, 2.6 mmol) and Et₃N (470 µL, 3.4 mmol) in dry DCM (50 mL) at 20 °C and the solution stirred for 2 h. The solution was diluted with DCM (50 mL), washed with water (2 × 10 mL), brine (20 mL), the organic fraction dried and the solvent evaporated. The residue was dissolved in THF (50 mL) and Et₃N (9.0 mL, 64.9 mmol) and dimethylamine hydrochloride (5.3 g, 64.9 mmol) added and the solution heated at reflux temperature for 3 h, then stirred at 20 °C for 16 h. The solvent was evaporated and the residue partitioned between EtOAc (100 mL) and water (100 mL). The organic fraction was extracted with water (2 x 25 mL), dried and the solvent evaporated. The residue was chromatographed, eluting with a gradient (0-20%) of MeOH/EtOAc then 1% Et₃N/20% MeOH/EtOAc, to give amine **64** (528 mg, 93%) as a yellow/orange solid, mp (MeOH/EtOAc) 47-49 °C, ¹H NMR [(CD₃)₂SO] δ 8.36 (d, *J* = 8.5 Hz, 1 H, H-8'), 8 02-8.10 (m, 2 H, H-5', H-6'), 7.83 (ddd, *J* = 8.5, 6.7, 1.6 Hz, 1 H, H-7'), 3.09 (dd, *J* = 7.5, 7.2 Hz, 2 H, H-1), 2.81 (d, *J* = 7 5, 7.2 Hz, 2 H, H-2), 2.22 [s, 6 H, N(CH₃)_{2]}; ¹³C NMR [(CD₃)₂SO] δ 165 1 (C-3'), 146.8 (C-4a'), 136.1 (C-6'), 132.7 (C-8a'), 130.5 (C-5'), 128.3 (C-7'), 119.5 (C-8'), 56.9 (C-1), 44.7 [N(CH₃)₂], 34.6 (C-1); Anal. calc. for C₁₁H₁₄N₄O: C, 60.5; H, 6.5; N, 25 7; found C, 60.7; H, 6.7; N, 25.6%.

### Example 81

**3-[2-(4-Morpholinyl)ethyl]-1,2,4-benzotriazine 1-oxide hydrochloride (65).** MsCl (381 µL, 4.8 mmol) was added to a stirred solution of alcohol 61 (769 mg, 4.0 mmol) and Et₃N (729 µL, 5.2 mmol) in dry DCM (50 mL) at 20 °C and the solution stirred for 2 h. The solution was diluted with DCM (50 mL), washed with water (2 × 30 mL), brine (50 mL), the organic fraction dried and the solvent evaporated. The residue was dissolved in THF (50 mL) and morpholine (8.8 mL, 100 mmol) added and the solution heated at reflux temperature for 3 h, then stirred at 20 °C for 16 h. The solvent was evaporated and the residue partitioned between EtOAc (100 mL) and water (100 mL). The aqueous fraction was extracted with EtOAc (3 × 50 mL), the combined organic fraction dried and the solvent evaporated The residue was chromatographed, eluting with a gradient (0-10%) of MeOH/EtOAc, to give the morpholide 65 (840 mg, 80 %) which was dissolved in HCl saturated MeOH, the solvent evaporated and the residue crystallized as an yellow/orange solid, mp (MeOH/EtOAc) 213-215 °C; ¹H NMR [(CD₃)₂SO] δ 11.37 (br s, 1 H, NH⁺Cl⁻), 8.40 (d, *J* = 8.7 Hz, 1 H, H-8), 8.13 *(ddd, J =* 8.5, 6.9, 1.2 Hz, 1 H, H-6), 8.06 *(dd, J =* 8.5, 1.2 Hz, 1 H, H-5), 7.87 (ddd, *J =* 8.7, 6.9, 1.2 Hz, 1 H, H-7), 3.96-4.01 (m, 2 H, CH₂O), 3.80-3.86 (m, 2 H, CH₂O), 3.63-3.68 (m, 2 H, CH₂N), 3.52-3.58 (m, 4 H, 2 × CH₂N), 3.15-3.25 (m, 2 H, CH₂); Anal. calc. for C₁₃H₁₇ClN₄O₂: C, 52.6; H, 5.8; N, 18.9; Cl, 12.0; found C, 52.6; H, 5.5; N, 18.9; Cl, 12.0%.

### Example 82

**3-(3-Hydroxypropyl)-1 ,2,4-benzotriazine 1-oxide (66).** A solution of 9-BBN in THF (13 7 mL, 6.8 mmol) was added to a stirred solution of alkene **60** (1.07 g, 5.7 mmol) in THF (50 mL) and the solution stirred at 20 °C for 1 h. A solution of NaOH (3 M; 2.9 ml, 8.5 mmol), followed by 35% H₂O₂ (2.6 mL, 25.6 mmol) were carefully added and the mixture stirred at 20 °C for 1 h. The mixture was diluted with brine (100 mL), extracted with EtOAc (3 × 100 mL), the combined organic fraction dried, and the solvent evaporated. The residue was chromatographed, eluting with a gradient (10-50%) of EtOAc/DCM, to give alcohol **66** (1.02 g, 87%) as a white solid, mp (EtOAc/pet. ether) 99-100 °C; ¹H NMR δ 8.46 (dd, *J* = 8.7, 1.0 Hz, 1 H, H-8), 7.99 (dd, *J* = 8.5, 1.2 Hz, 1 H, H-5), 7.93 (ddd, *J* = 8.5, 7.0, 1.0 Hz, 1 H, H-6), 7.70 (ddd, *J* = 8 7, 7.0, 1.2 Hz, 1 H, H-7), 3.80 (t, *J* = 6.1 Hz, 2 H, CH₂O), 3.18 (t, *J* = 7.3 Hz, 2 H, CH₂), 2.15-2 22 (m, 2 H, CH₂), (OH not observed); ¹³C NMR δ 166.9, 147.3, 135.7, 133.3, 130 1, 128.6, 120.1, 62.1, 34.1, 30.5; Anal. calc. for C₁₀H₁₁N₃O₂: C, 58.5; H, 5.4 ;N, 20.5; found C, 58.6; H, 5.5; N, 20.5%.

### Example 83

**3-[3-Methoxypropyl]-1,2,4-benzotriazine 1-oxide (67).** TMSCH₂N₂ (1 1 mL, 2.1 mmol) was added to a stirred solution of alcohol **66** (437 mg, 2.1 mmol) and HBF₄ (0.53 mL, 4.3 mmol) in DCM (20 mL) at 20 °C and the solution stirred for 2 h at 20 °C. More TMSCH₂N₂ (5 × 1.1 mL) was added at hourly intervals and the solution stirred vigorously for 16 h. The solvent was evaporated and the residue chromatographed, eluting with a gradient (20-35%) of EtOAc/DCM, to give methyl ether **67** (310 mg, 66%) as a tan oil, ¹H NMR δ 8.45 (dd, *J* = 8.7, 1.1 Hz, 1 H, H-8), 8.03 (d, *J* = 8.5 Hz, 1 H, H-5), 7.94 (ddd, *J* = 8.5, 7.0, 1.1 Hz, 1 H, H-6), 7.70 (ddd, *J* = 8.7, 7.0, 1.1 Hz, 1 H, H-7), 3.52 (t, *J* = 6.3 Hz, 2 H, CH₂O), 3.33 (s, 3 H, OCH₃), 3.10-3.14 (m, 2 H, CH₂N), 2.16-2.23 (m, 2 H, CH₂); ¹³C NMR δ 166.8 (C-3), 147.0 (C-4a), 135 7 (C-6), 133.3 (C-8a), 130.0 (C-5), 128.4 (C-7), 120.1 (C-8), 71.7 (CH₂O), 58.5 (OCH₃), 34 0 (CH₂), 32.5 (CH₂); MS (EI) *m*/*z* 219 (M⁺, 25%), 202 (90), 101 (100); HRMS (EI) calc. for C₁₁H₁₃N₃O₂(M⁺) *m*/*z* 219.1008, found 219.1006.

### Example 84

***N*,*N*-Dimethyl-3-(1-oxido-1,2,4-benzotriazin-3-yl)-1-propanamine hydrochloride (68).** Methanesulfonyl chloride (175 µL, 2.3 mmol) was added to a stirred solution of alcohol **66** (386 mg, 1.9 mmol) and Et₃N (393 µL, 2.8 mmol) in dry DCM (30 mL) at 5 °C and the solution stirred for 2 h at 20 °C. The solution was diluted with DCM (30 mL), washed with water (2 × 20 mL), the organic fraction dried and the solvent evaporated. The residue was dissolved in DMF (5 mL) and 40% aqueous dimethylamine (12 mL, 94 mmol) added and the solution heated at 50 °C for 2 h. The solvent was evaporated and the residue partitioned between EtOAc (50 mL) and water (50 mL). The organic fraction was extracted with water (2 × 25 mL), dried and the solvent evaporated. The residue was chromatographed, eluting with a gradient of (0-1%) Et₃N/(0-5%) MeOH/DCM, to give amine **68** (348 mg, 80%) which was dissolved in HCl saturated MeOH, the solvent evaporated and the residue crystallized as a tan solid, mp (MeOH/EtOAc) 228-230 °C; ¹H NMR [(CD₃)₂SO] δ 10.72 (br s, 1 H, NH⁺Cl⁻), 8 39 (d, *J* = 8.6 Hz, 1 H, H-8), 8 10 (ddd, *J* = 8.4, 7.0, 1.4 Hz, 1 H, H-6), 8.06 (dd, *J* = 8.4, 1.5 Hz, 1 H, H-5), 7.86 (ddd, *J* = 8.6, 7.0, 1.5 Hz, 1 H, H-7), 3 04-3.09 (m, 2 H, CH₂N), 3.04 (t, J = 7.4 Hz, 2 H, CH₂), 2.74 [s, 6 H, N(CH₃)₂], 2.19-2.27 (m, 2 H, CH₂); ¹³C NMR ((CD₃)₂SO] δ 164.7 (C-3), 146.8 (C-4a), 136 1 (C-6), 133.0 (C-8a), 130.7 (C-5), 128.4 (C-7), 119.5 (C-8), 55.5 (CH₂N), 41.8 [N(CH₃)₂], 33.2 (CH₂), 21.6 (CH₂); Anal. calc. for C₁₂H₁₇ClN₄O; C, 53.6; H, 6.4; N, 20.9; found C, 53.9; H, 6.3; N, 21.0%.

### Example 85

**3-[3-(1-Piperidinyl)propyl)-1,2,4-benzotriazine 1-oxide hydrochloride (69).** MsCl (133 µL, 1 7 mmol) was added to a stirred solution of alcohol 66 (293 mg, 1.4 mmol) and Et₃N (300 µL, 2.1 mmol) in dry DCM (20 mL) at 5 °C and the solution stirred for 1 h at 20 °C The solution was diluted with DCM (30 mL), washed with water (2 × 20 mL), the organic fraction dried and the solvent evaporated. The residue was dissolved in DMF (10 mL) and piperidine (7 mL, 70 mmol) added and the solution heated at 50 °C for 2 h. The solvent was evaporated and the residue partitioned between EtOAc (50 mL) and water (50 mL). The organic fraction was extracted with water (2 x 25 mL), dried and the solvent evaporated. The residue was chromatographed, eluting with a gradient (0-5%) of MeOH/DCM, to give amine **69** (291 mg, 75%) which was dissolved in HCl saturated MeOH, the solvent evaporated and the residue crystallized as a white solid, mp (MeOH/EtOAc) 151-153 °C; ¹H NMR [(CD₃)₂SO] δ 10.71 (br s, 1 H, NH⁺Cl⁻), 8.39 (d, *J* = 8.7 Hz, 1 H, H-8), 8.10 (ddd, *J* = 8.5, 6.9, 1.2 Hz, 1 H, H-6), 8.05 (dd, *J* = 8.5, 1.3 Hz, 1 H, H-5), 7.86 (ddd, *J* = 8.7, 69, 1.3 Hz, 1 H, H-7), 3.37-3.43 (m, 2 H, CH₂N), 3.10-3.17 (m, 2 H, CH₂N), 3.03 (t, *J* = 7.4 Hz, 2 H, CH₂), 2.80-2.89 (m, 2 H, CH₂N), 2.25-2.33 (m, 2 H, CH₂), 1.67-1.83 (m, 6 H, 3 × CH₂); ¹³C NMR [(CD₃)₂SO] δ 164.8 (C-3), 146.8 (C-4a), 136.1 (C-6), 133.0 (C-8a), 130.7 (C-5), 128.4 (C-7), 119.5 (C-8), 54.8 (CH₂N), 51.8 (2 × CH₂N), 33.42 (CH₂), 22.1 (2 x CH₂), 21.3 (CH₂), 21.0 (CH₂); Anal. calc. for C₁₅H₂₀N₄O.2HCl; C, 52.2; H, 6.4; N, 16.3; found C, 52.2; H, 6.4; N, 16.4%.

### Example 86

**3-[3-(4-Morpholinyl)propyl]-1 ,2,4-benzotriazine 1-oxide hydrochloride (70).** MsCl (137 µL, 1.8 mmol) was added to a stirred solution of alcohol **66** (303 mg, 1.5 mmol) and Et₃N (309 µL, 2.2 mmol) in dry DCM (20 mL) at 5 °C and the solution stirred for 2 h at 20 °C. The solution was diluted with DCM (30 mL), washed with water (2 × 20 mL), the organic fraction dried and the solvent evaporated. The residue was dissolved in DMF (5 mL) and morpholine (6.4 mL, 74 mmol) added and the solution heated at 50 °C for 2 h. The solvent was evaporated and the residue partitioned between EtOAc (50 mL) and water (50 mL). The organic fraction was extracted with water (2 x 25 mL), dried and the solvent evaporated The residue was chromatographed, eluting with a gradient (0-5%) of MeOH/30% EtOAc/DCM, to give the morpholide **70** (330 mg, 81%) which was dissolved in HCl saturated MeOH, the solvent evaporated and the residue crystallized as a tan solid, mp (MeOH) 175-177 °C; ¹H NMR [(CD₃)₂SO] δ 11 54 (br s, 1 H, NH⁺Cl⁻), 8 39 (dd, *J* = 8.7, 1.0 Hz, 1 H, H-8), 8.10 (ddd, *J* = 8.4, 6.9, 1.0 Hz, 1 H, H-6), 8.06 (dd, *J* = 8.4, 1.0 Hz, 1 H, H-5), 7.86 (ddd, *J* = 8.7, 6 9, 1 0 Hz, 1 H, H-7), 3.82-3.96 (m, 4 H, 2 × CH₂O), 3.38-3.43 (m, 2 H, CH₂), 3.18-3.23 (m, 2 H, CH₂N), 2.98-3.08 (m, 4 H, 2 × CH₂N), 2.26-2.33 (m, 2 H, CH₂); ¹³C NMR [(CD₃)₂SO] δ 164.7 (C-3), 146.8 (C-4a), 136.2 (C-6), 133.0 (C-8a), 130.7 (C-5), 128.4 (C-7), 119.5 (C-8), 63.0 (2 x CH₂O), 55.0 (CH₂N), 50.8 (2 × CH₂N), 33.4 (CH₂), 20.7 (CH₂).

### Example 87

**3-Methoxy-1,2,4-benzotriazine 1-oxide (71).** A solution of NaOMe [prepared from the dissolution of Na (57 mg, 2.5 mmol) in dry MeOH (10 mL)] and chloride **19** (298 mg, 1.6 mmol) was stirred at 20 °C for 3 h. The solvent was evaporated and the residue partitioned between DCM (100 mL) and water (100 mL). The organic fraction was dried and the solvent evaporated. The residue was chromatographed, eluting with 5% EtOAc/DCM, to give 1-oxide **71** (257 mg, 88%) as pale yellow needles, mp (EtOAc/pet. ether) 123-124 °C [lit (Ergener, Istanbul Univ. Fen. Fak. Mecm. Seri. A, 1950, 15, 91) mp (MeOH) 121-122 °C]; ¹H NMR δ 8.38 (dd, *J* = 8.4, 0.7 Hz, 1 H, H-8), 7.83-7.88 (m, 2 H, H-5, H-6), 7.54 (ddd, *J* = 8.4, 6.2, 2.2 Hz, 1 H, H-7), 4.16 (s, 3 H, OCH₃); ¹³C NMR δ 162.6 (C-3), 147.4 (C-4a), 135.9 (C-6), 132.4 (C-8a), 127.6 (C-5), 127.5 (C-7), 120.5 (C-8), 55.6 (OCH₃).

### Example 88

**3-(2-Methoxyethoxy)-1,2,4-benzotriazine 1-oxide (72).** Na (123 mg, 5.3 mmol) was added to a solution of chloride **19** (645 mg, 3.6 mmol) in 2-methoxyethanol (20 mL) at 5 °C. The mixture was stirred at 20 °C for 2 h, diluted with water (80 mL), extracted with EtOAc (3 × 80 mL), the organic fraction dried , and the solvent evaporated. The residue was chromatographed, eluting with 10%EtOAc/DCM, to give 1-oxide **72** (618 mg, 79%) as white needles, mp (EtOAc/pet ether) 74-76 °C; ¹H NMR δ 8.37 (dd, *J* = 8.7,1.3 Hz, 1 H, H-8), 7.81-7.88 (m, 2 H, H-5, H-6), 7.53 (ddd, *J =* 8.7, 6.7, 1.8 Hz, 1 H, H-7), 4.65-4.68 (m, 2 H, CH₂O), 3.81-3.84 (m, 2 H, CH₂O), 3.46 (s, 3 H, OCH₃); ¹³C NMR δ 162 1 (C-3), 147.4 (C-4a), 135.9 (C-6), 132.5 (C-8a), 127.6 (C-5), 127.5 (C-7), 120.4 (C-8), 70 2 (CH₂O), 67.6 (CH₂O), 59.2 (OCH₃); Anal. calc. for C₁₀H₁₁N₃O₃: C, 54.3; H, 5.0; N, 19.0; found C, 54.4; H, 4.9; N, 19.0%.

### Example 89

**3-Chloro-6-methyl-1,2,4-benzotriazine 1-oxide (73).** Sodium nitrite (7.09 g, 103 mmol) was added in small portions to a stirred solution of 6-methyl-1,2,4-benzotriazin-3-amine 1-oxide (**3r**) (9.05 g, 51.4 mmol) in trifluoroacetic acid (80 mL) at 5 °C and the solution stirred at 20 °C for 3 h. The solution was poured into ice/water, stirred 30 minutes, filtered, washed with water (3 × 30 mL) and dried. The solid was suspended in POCl₃ (100 mL) and DMF (0.5 mL) and stirred at 100 °C for 1 h. The solution was cooled, poured into ice/water, stirred for 30 minutes, filtered, washed with water (3 × 30 mL) and dried. The solid was suspended in DCM (150 mL), dried and the solvent evaporated. The residue was chromatographed, eluting with 5% EtOAc/DCM, to give chloride 73 (7.86 g, 78%) as a pale yellow solid, mp (EtOAc/DCM) 156-158 °C; ¹H NMR δ 8.29 *(d, J =* 8.8 Hz, 1 H, H-8), 7.74 *(d, J =* 1.7 Hz, 1 H, H-5), 7.56 (dd, *J* = 8.8, 1.7 Hz, 1 H, H-7), 2.61 (s, 3 H, CH₃); Anal. calc. for C₈H₆ClN₃O: C, 49.1; H, 3 1; N, 21.5; found C, 49.2; H, 3.4; N, 21.5%.

### Example 90

**2-[(6-Methyl-1-oxido-1,2,4-benzotriazin-3-yl)amino]ethanol (74).** 2-Aminoethanol (0.61 mL, 10.1 mmol) was added to a stirred solution of chloride **73** (657 mg, 3.4 mmol) in DME (50 mL) and the solution stirred at reflux temperature for 16 h. The solvent was evaporated and the residue partitioned between DCM (100 mL) and dilute aqueous NH₃ (50 mL). The organic fraction was dried and the solvent evaporated. The residue was chromatographed, eluting with a gradient (5-10%) of MeOH/DCM, to give 1-oxide **74** (700 mg, 95%) as a yellow powder, mp (MeOH) 198-202 °C; ¹H NMR δ 8.00 (d, *J* = 8 8 Hz, 1 H, H-8), 7.70 (br s, 1 H, NH), 7.35 (d, *J* = 1.7 Hz, 1 H, H-5), 7 15 (dd, *J* = 8.8, 1.7 Hz, 1 H, H-7), 4.70 (t, *J* = 5.6 Hz, 1 H, OH), 3.54-3.60 (m, 2 H, CH₂O), 3.38-3.43 (m, 2 H, CH₂N), 2.47 (s, 3 H, CH₃); ¹³C NMR δ 159.1 (C-3), 148.4 (C-4a), 146.6 (C-6), 128.2 (C-8a), 126.4 (C-5), 125.7 (C-7), 119.5 (C-8), 59.2 (CH₂O), 43.2 (CH₂N), 21.3 (CH₃); Anal. calc. for C₁₀H₁₂N₄O₂: C, 54.5; H, 5.5, N, 25.4; found C, 54.7; H, 5.4; N, 25.7%.

### Example 91

***N*-(2-Methoxyethyl)-6-methyl-1,2,4-benzotriazin-3-amine 1-oxide (75).** 2-Methoxyethylamine (0 44 mL, 5 0 mmol) was added to a stirred solution of chloride **73** (329 mg, 1 7 mmol) in DME (50 mL) and the solution stirred at reflux temperature for 2 h. The solvent was evaporated and the residue partitioned between DCM (100 mL) and dilute aqueous NH₃ (50 mL) The organic fraction was dried and the solvent evaporated. The residue was chromatographed, eluting with a gradient (5-40%) of EtOAc/DCM, to give 1-oxide **75** (368 mg, 93%) as a yellow powder, mp (MeOH) 157-158 °C; ¹H NMR δ 8.14 (d, *J =* 8.8 Hz, 1 H, H-8), 7.36 (d, *J =* 1.7 Hz, 1 H, H-5), 7.10 (dd, *J* = 8.8, 1.7 Hz, 1 H, H-7), 5.58 (br s, 1 H, NH), 3.68-3.72 (m, 2 H, CH₂N), 3.59-3.62 (m, 2 H, CH₂O), 3.39 (s, 3 H, OCH₃), 2.47 (s, 3 H, CH₃); ¹³C NMR δ 159.1 (C-3), 149.1 (C-4a), 147.0 (C-6), 129.3 (C-8a), 127.2 (C-5), 125.4 (C-7), 120.1 (C-8), 70.9 (CH₂O), 58.8 (OCH₃), 41.1 (CH₂N), 22.0 (CH₃); Anal. calc. for C₁₁H₁₄N₄O₂: C, 56.4; H, 6 0; N, 23.9; found C, 56.4, H, 5.9; N, 23.8%.

### Example 92

**N**^{**1**}**,N**^{**1**}**-Dimethyl-*N***^{**2**}**-(6-methyl-1-oxido-1,2,4-benzotriazin-3-yl)-1,2-ethanediamine** (76). N,N-Dimethylethanediamine (705 µL, 6.6 mmol) was added to a stirred solution of chloride **73** (518 mg, 2.7 mmol) in DME (50 mL) and the solution stirred at reflux temperature for 2 h. The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-10%) of MeOH/DCM, to give 1-oxide **76** (603 mg, 92%) as a yellow solid, mp (MeOH/EtOAc) 143-145 °C; ¹H NMR δ 8.11 (d, *J* = 8.8 Hz, 1 H, H-8), 7.35 (d, *J* = 1.7 Hz, 1 H, H-5), 7.07 (dd, *J* = 8.8, 1.7 Hz, 1 H, H-7), 5.89 (br s, 1 H, NH), 3.50-3 56 (m, 2 H, CH₂N), 2.52-2.56 (m, 2 H, CH₂N), 2.45 (s, 3 H, CH₃), 2.26 [s, 6 H, N(CH₃)₂]; ¹³C NMR δ 159.2 (C-3), 149.1 (C-4a), 146.9 (C-6), 129.2 (C-8a), 126.9 (C-5), 125.3 (C-7), 120.1 (C-8), 57.5 (CH₂N), 45.1 [N(CH₃)₂], 38.7 (CH₂N), 22.0 (CH₃); Anal. calc. for C₁₂Hₗ₇N₅O: C, 58.3; H, 6.9; N, 28.3; found C, 58.5: H, 7.1; N, 28.6%.

### Example 93

**6-Methyl-*N*-[2-(1-piperidinyl)ethyl]-1,2,4-benzotriazin-3-amine 1-oxide (77).** 2-(1-Piperidinyl)ethylamine (0.87 mL, 6.1 mmol) was added to a stirred solution of chloride **73** (476 mg, 2.4 mmol) in DME (50 mL) and the solution stirred at reflux temperature for 2 h. The solvent was evaporated and the residue partitioned between DCM (100 mL) and dilute aqueous NH₃ (50 mL). The organic fraction was dried and the solvent evaporated. The residue was chromatographed, eluting with a gradient (0-10%) of MeOH/DCM, to give 1-oxide **77** (656 mg, 94%) as a yellow powder, mp (MeOH/EtOAc) 156-158 °C; ¹H NMR δ 8.13 (d, *J* = 8.7 Hz, 1 H, H-8), 7.36 (d, *J* = 1.7 Hz, 1 H, H-5), 7.08 (dd, *J* = 8.7, 1.7 Hz, 1 H, H-7), 5.98 (br s, 1 H, NH), 3.51-3.56 (m, 2 H, CH₂N), 2.54-2.58 (m, 2 H, CH₂N), 2.47 (s, 3 H, CH₃), 2.39-2.45 (m, 4 H, 2 × CH₂N), 1.55-1 61 (m, 4 H, 2 × CH₂), 1 42-1.48 (m, 2 H, CH₂); ¹³C NMR δ 159.1 (C-3), 149.1 (C-4a), 146.9 (C-6), 129.1 (C-8a), 126.9 (C-7), 125.3 (C-5), 120.1 (C-8), 56.9 (CH₂N), 54.3 (2 × CH₂N), 37.9 (CH₂N), 26.0 (2 × CH₂), 24.4 (CH₂), 22.0 (CH₃); Anal. calc for C₁₅H₂₁N₅O: C, 62.7; H, 7.4; N, 24.4, found C, 62.8; H, 7.7; N, 24.5%.

### Example 94

***N*-[2-(2,6-Dimethyl-1-piperidinyl)ethyl]-6-methyl-1,2,4-benzotriazin-3-amine 1-oxide (78)**. 2-(2,6-Dimethyl-1-piperidinyl)ethylamine (834 mg, 5.3 mmol) was added to a stirred solution of chloride **73** (418 mg, 2.1 mmol) in DME (50 mL) and the solution stirred at reflux temperature for 2 h. The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-10%) of MeOH/DCM, to give 1-oxide **78** (597 mg, 93%) as a yellow solid, mp (MeOH/EtOAc) 162-165 °C; ¹H NMR δ 8.12 (d, *J* = 8.9 Hz, 1 H, H-8), 7.35 (d, *J* = 1.7 Hz, 1 H, H-5), 7.09 (dd, *J* = 8.9, 1.7 Hz, 1 H, H-7), 5.57 (br s, 1 H, NH), 3.50-3.56 (m, 2 H, CH₂N), 2.87-2.91 (m, 2 H, CH₂N), 2.49-2.57 (m, 2 H, 2 × CH), 2.47 (s, 3 H, CH₃), 1.65-1.69 (m, 1 H, CH₂), 1.53-1.58 (m, 2 H, CH₂), 1.25-1.41 (m, 3 H, CH₂), 1.19 (d, *J* = 6.3 Hz, 6 H, 2 × CH₃); ¹³C NMR δ 159.2 (C-3), 149.1 (C-4a), 146.9 (C-6), 129.2 (C-8a), 127.0 (C-5), 125.4 (C-7), 120.8 (C-8), 57.3 (2 x CH), 47.4 (CH₂N), 39.5 (CH₂N), 34.2 (CH₂), 24.4 (2 × CH₂), 22.0 (2 × CH₃), 21.6 (CH₃); Anal. calc. for C₁₇H₂₅N₅O: C, 64.7; H, 8.0; N, 22.2; found C, 64.3: H, 7.3; N, 22.0%.

### Example 95

**3-Ethyl-6-methyl-1,2,4-benzotriazine 1-oxide (79).** Pd(PPh₃)₄ (410 mg, 0.35 mmol) was added to a stirred solution of chloride **73** (728 mg, 3.6 mmol) and tetraethyltin (1.4 mL, 7.1 mmol), the solution degassed, and stirred under N₂ at reflux temperature for 16 h. The solvent was evaporated and the residue chromatographed, eluting with 20% EtOAc/pet ether to give an oil which was chromatographed, eluting with 5% EtOAc/DCM, to give (i) starting material **73** (412 mg, 56%) and (ii) 1-oxide **79** (250 mg, 37%) as a white solid, mp (EtOAc/DCM) 68-70 °C; ¹H NMR δ 8.33 (d, *J* = 8.8 Hz, 1 H, H-8), 7 74 (br s, 1 H, H-5), 7.49 (dd, *J* = 8.8, 1.7 Hz, 1 H, H-7), 3.02 (q, *J* = 7.6 Hz, 2 H, CH₂), 2.59 (s, 3 H, CH₃), 1.44 (t, *J* = 7.6 Hz, 3 H, CH₃); ¹³C NMR δ 168.2 (C-3), 147.8 (C-4a), 147.1 (C-6), 132.0 (C-5), 131.6 (C-8a), 127.5 (C-7), 119.8 (C-8), 30.7 (CH₂), 22.1 (CH₃), 12 2 (CH₃); Anal. calc. for C₁₀H₁₁N₃O: C, 63.5; H, 5.9; N, 22.2; found C, 63.5, H, 6 0; N, 22.3%.

### Example 96

**3-Allyl-6-methyl-1,2,4-benzotriazine 1-oxide (80).** Pd(PPh₃)₄ (370 mg, 0.32 mmol) was added to a stirred solution of chloride 73 (1.24 g, 6.3 mmol) and allyltributyltin (2.2 mL, 7.0 mmol), the solution degassed, and stirred under N₂ at reflux temperature for 6 h The solvent was evaporated and the residue chromatographed, eluting with 20% EtOAc/pet ether, to give an oil which was chromatographed, eluting with 5% EtOAc/DCM, to give alkene **80** (0.97 g, 74%) as a white solid, mp (EtOAc/pet. ether) 65-67 °C, ¹ NMR δ 8.32 (d, J = 8.8 Hz, 1 H, H-8), 7.76 (d, *J* = 1.7 Hz, 1 H, H-5), 7.50 (dd, *J* = 8.8, 1.7 Hz, 1 H, H-7), 6.13-6.21 (m, 1 H, H-2'), 5.30 (dq, *J* = 17.0, 1.5 Hz, 1 H, H-3'), 5.22 (dq, *J* = 10.1, 1.5 Hz, 1 H, H-3'), 3.76 (dq, *J* = 6.9, 1.5 Hz, 2 H, H-1'), 2.58 (s, 3 H, CH₃); ¹³C NMR δ 165.3 (C-3), 147.8 (C-4a), 147.3 (C-6), 132.8 (C-2'), 132.5 (C-5), 131.6 (C-8a), 127.6 (C-7), 119.7 (C-8), 118.4 (C-3'), 41.8 (C-1'), 22.1 (CH₃); Anal. calc. for C₁₁H₁₁N₃O: C, 65.7; H, 5.5; N, 20.9; found C, 65.8; H, 5.5; N, 21.0%.

### Example 97

**2-(6-Methyl-1-oxido-1,2,4-benzotriazin-3-yl)ethanol (81).** Ozone was bubbled into a solution of alkene 80 (1.12 g, 5.6 mmol) in DCM/MeOH (1:1, 80 mL) at -78 °C until a blue colour persisted. The solution was purged with N₂ to remove excess ozone, then a solution of NaBH₄ (210 mg, 5.6 mmol) in EtOH (10 mL) added dropwise and the solution allowed to warm to 20 °C over 1 h. HOAc (2 mL) was added and the solution stirred at 20 °C for 30 min. The solvent was evaporated and the residue partitioned between DCM (50 mL) and water (3 × 50 mL). The organic fraction was dried and the solvent evaporated. The residue was chromatographed, eluting with a gradient (50-100%) of EtOAc/pet. ether, to give alcohol **81** (780 mg, 68%) as pale yellow prisms, mp (EtOAc/pet. ether) 121-123 °C; ¹H NMR δ 8.32 (d, J = 8.8 Hz, 1 H, H-8), 7.74 (d, *J* = 1.7 Hz, 1 H, H-5), 7.50 (dd, *J* = 8.8, 1.7 Hz, 1 H, H-7), 4.04-4.10 (m, 2 H, CH₂O), 3 27 (t, *J* = 5.6 Hz, 2 H, CH₂), 3.17 (t, *J* = 6.3 Hz, 1 H, OH), 2.60 (s, 3 H, CH₃), ¹³C NMR δ 165.5 (C-3), 147.6 (C-4a), 147.3 (C-6), 132.4 (C-5), 131.9 (C-8a), 127.4 (C-7), 119.8 (C-8), 60.1 (CH₂O), 39.0 (CH₂), 22.1 (CH₃); Anal. calc for C₁₀H₁₁N₃O₂, C, 58.5; H, 5.4; N, 20.5; found C, 58.8; H, 5.5; N, 20.5%.

### Example 98

**3-(2-Methoxyethyl)-6-methyl-1,2,4-benzotriazine 1-oxide (82).** Three aliquots of TMSCH₂N₂ (1.1 mL, 2.1 mmol) were added to a stirred solution of alcohol **81** (433 mg, 2.1 mmol) and HBF₄ (0 26 mL, 2 1 mmol) in DCM (30 mL) over 3 h. The solution was stirred at 20 °C for 16 h, the solvent evaporated and the residue chromatographed, eluting with 50% EtOAc/pet. ether, to give (i) methyl ether **82** (119 mg, 19%) as a yellow powder, mp (EtOAC/pet. ether) 77-79 °C; ¹H NMR δ 8.32 (d, *J* = 8 8 Hz, 1 H, H-8), 7.56 (d, *J* = 1.7 Hz, 1 H, H-5), 7.50 (dd, *J* = 8.8, 1.7 Hz, 1 H, H-6), 3.95 (t, *J* = 6.5 Hz, 2 H, CH₂O), 3.37 (s, 3 H, OCH₃), 3.27 (t, *J* = 6.5 Hz, 2 H, CH₂), 2 58 (s, 3 H, CH₃); ¹³C NMR δ 164.7 (C-3), 147.7 (C-4a), 147.2 (C-6), 132.2 (C-5), 131.8 (C-8a), 127.6 (C-7), 119.7 (C-8), 70.1 (CH₂O), 58.7 (OCH₃), 37.6 (CH₂), 22.1 (CH₃); and (ii) starting material **81** (360 mg, 62%), spectroscopically identical to sample prepared above.

### Example 99

**3-Chloro-6-methoxy-1,2,4-benzotriazine 1-oxide (83).** Sodium nitrite (7.14 g, 103.4 mmol) was added in portions to a stirred solution of 6-methoxy-1,2,4-benzotriazin-3-amine 1-oxide 3q (9.94 g, 51.7 mmol) in trifluoroacetic acid (50 mL) at 5 °C and the solution stirred at 20 °C for 1 h. The solution was poured into ice/water, filtered, washed with water (2 × 50 mL) and dried. The solid was suspended in POCl₃ (80 mL), DMF (2 drops) added, and the mixture stirred at 100 °C for 3 h. The solution was poured into ice/water, stirred for 20 minutes and filtered. The solid was dissolved in DCM (150 mL), dried, and the solvent evaporated. The residue was chromatographed, eluting with 5% EtOAc/DCM, to give chloride **83** (7.42 g, 68%) as a pale yellow solid, mp (EtOAc/DCM) 196-199 °C; ¹H NMR δ 8.30 (d, *J* = 9.6 Hz, 1 H, H-8), 7.32 (dd, *J* = 9.6, 2.7 Hz, 1 H, H-7), 7 19 (d, *J* = 2.7 Hz, 1 H, H-5), 4.01 (s, 3 H, OCH₃); ¹³C NMR δ 166.3 (C-6), 157.8 (C-3), 150.2 (C-4a), 128.9 (C-8a), 123.9 (C-5), 121.9 (C-7), 105.7 (C-8), 56.5 (OCH₃); Anal. calc. for C₈H₆ClN₃O₂: C, 45.4; H, 2.9; N, 19.9; Cl, 16.8; found C, 45.2; H, 2 6; N, 19.9; Cl, 16.9%.

### Example 100

***N***^{**1**}**-(6-Methoxy-1-oxido-1,2,4-benzotriazin-3-yl)-*N***^{**2**},***N***^{**2**}**-dimethyl-1,2-ethanediamine (84).** *N,N*-Dimethyl-1,2-ethanediamine (1.33 mL, 12.1 mmol) was added to a stirred solution of chloride **83** (0.85 g, 4.04 mmol) in DME (50 mL) and the solution stirred at reflux temperature for 16 h. The solvent was evaporated and the residue partitioned between DCM (100 mL) and dilute aqueous NH₃ (50 mL). The organic fraction was dried and the solvent evaporated. The residue was chromatographed, eluting with a gradient (0-5%) of MeOH/DCM, to give amine **84** (0.72 g, 68%) which was dissolved in HCl saturated MeOH, the solvent evaporated and the residue crystallized as a tan solid, mp (MeOH/EtOAc) 236-239 °C; ¹H NMR [(CD₃)₂SO] δ 10 68 (br s, 1 H, NH⁺Cl⁻), 8.07 (d, *J* = 9.3 Hz, 1 H, H-8), 8.03 (br s, 1 H, NH), 6 95-6 99 (m, 2 H, H-5, H-7), 3.92 (s, 3 H, OCH₃), 3.70-3.76 (m, 2 H, CH₂N), 3.30-3.35 (m, 2 H, CH₂N), 2.81 [d, *J* = 4.9 Hz, 6 H, N(CH₃)₂]; ¹³C NMR [(CD₃)₂SO] δ 164.9 (C-6), 159.0 (C-3), 150.4 (C-4a), 125.4 (C-8a), 121.6 (C-8), 117.3 (C-5), 104.3 (C-7), 55 2 (OCH₃), 55.2 (CH₂N), 42.3 [N(CH₃)₂], 35.8 (CH₂N); Anal. calc. for C₁₂H₁₈ClN₅O₂: C, 48.1; H, 6.1; N, 23.4; Cl, 11.8; found C, 48.3; H, 6.1; N, 23.6; Cl, 11.9%.

### Example 101

**6-Methoxy-*N*-[2-(1-piperidinyl)ethyl]-1,2,4-benzotriazin-3-amine 1-oxide** (85). 2-(1-Piperidinyl)ethylamine (0.9 mL, 6.0 mmol) was added to a stirred solution of chloride **83** (509 mg, 2.4 mmol) in DME (50 mL) and the solution stirred at reflux temperature for 2 h. The solvent was evaporated and the residue partitioned between DCM (100 mL) and dilute aqueous NH₃ (50 mL). The organic fraction was dried and the solvent evaporated. The residue was chromatographed, eluting with a gradient (0-10%) of MeOH/DCM, to give 1-oxide **85** (736 mg, 100%) as a yellow powder, mp (MeOH) 133-135 °C; ¹H NMR δ 8.15 (d, *J* = 9.8 Hz, 1 H, H-8), 6.85-6.88 (m, 2 H, H-5, H-7), 6.00 (br s, 1 H, NH), 3.92 (s, 3 H, OCH₃), 3.52-3.56 (m, 2 H, CH₂N), 2.56-2.60 (m, 2 H, CH₂N), 2.38-2.44 (m, 4 H, 2 × CH₂N), 1.56-1.62 (m, 4 H, 2 × CH₂), 1.42-1.48 (m, 2 H, CH₂); ¹³C NMR δ 165.4 (C-7), 159.5 (C-3), 151.5 (C-4a), 126.0 (C-8a), 122 0 (C-7), 117.6 (C-8), 104.5 (C-5), 56.8 (CH₂N), 56.0 (OCH₃), 54.3 (2 × CH₂N), 37 9 (CH₂N), 26.0 (2 x CH₂), 24.4 (CH₂); Anal. calc. for C₁₅H₂₁N₅O₂.H₂O: C, 56.1; H, 7.2; N, 21.8; found C, 55.9; H, 7.0, N, 21.7%.

### Example 102

**6-Methoxy-*N*-[2-(4-morpholinyl)ethyl]-1 ,2,4-benzotriazin-3-amine 1-oxide (86). 2**-(4-Morpholinyl)ethylamine (1.92 mL, 14 6 mmol) was added to a stirred solution of chloride **83** (1.03 g, 4.9 mmol) in DME (50 mL) and the solution stirred at reflux temperature for 16 h. The solvent was evaporated and the residue partitioned between DCM (100 mL) and dilute aqueous NH₃ (50 mL). The organic fraction was dried and the solvent evaporated. The residue was chromatographed, eluting with a gradient (0-5%) of MeOH/DCM, to give amine **86** (0.89 g, 60%) as a yellow powder, mp (MeOH/EtOAc) 186-188 °C, ¹H NMR [(CD₃)₂SO] δ 8.03 (d, *J* = 9.5 Hz, 1 H, H-8), 7.63 (br s, 1 H, NH), 6.90-6.95 (m, 2 H, H-5, H-7), 3.91 (s, 3 H, OCH₃), 3.55-3.58 (m, 4 H, 2 × CH₂O), 3 45-3.49 (m, 2 H, CH₂N), 2.50-2 55 (m, 2 H, CH₂N), 2.41-2.44 (m, 4 H, 2 × CH₂N), ¹³C NMR [(CD₃)₂SO) δ 164.8 (C-6), 159.4 (C-3), 150.9 (C-4a), 125.0 (C-8a), 121.5 (C-8), 116.8 (C-7), 104.2 (C-5), 66.1 (2 × CH₂O), 56.8 (CH₂N), 56.1 (OCH₃), 53 2 (2 × CH₂N), 37.7 (CH₂N); Anal. calc for C₁₄H₁₉N₅O₃.¼H₂O: C, 54 3; H, 6.3; N, 22 6; found C, 54 5; H, 6.2; N, 22.8%.

### Example 103

**3-Chloro-7-methyl-1,2,4-benzotriazine 1-oxide (87).** A solution of NaNO₂ (3.9 g, 56.3 mmol) in water (15 mL) was added dropwise to a stirred suspension of amine **3j** (4.95 g, 28.1 mmol) in 2 M HCl (200 mL) at 5 °C and the mixture stirred vigorously for 2 h at 20 °C. The suspension was filtered, the solid dissolved in dilute aqueous NH₃ (150 mL), filtered and the filtrate acidified with cHCl. The suspension was cooled, filtered and the solid washed with water (2 × 10 mL) and dried. The solid (3.76 g, 21.2 mmol) was suspended in dimethylaniline (6.7 mL, 53 mmol) and POCl₃ (14 mL, 149 mmol). The mixture was stirred at relux temperature for 1 h, the resulting solution poured on to ice (300 mL) The suspension was filtered, washed with water (2 x 20 mL), dissolved in EtOAc (200 mL), dried and the solvent evaporated. The residue was chromatographed, eluting with 5% EtOAc/DCM, to give chloride **87** (2.99 g, 72 %) as a yellow solid, mp 176.5-177 °C [lit (Foye et. al., *J Het Chem.* **1982,** *19*, 497) mp (toluene) 177-179 °C]; ¹H NMR δ 8.21 (d, *J* = 2.0 Hz, 1 H, H-8), 7.89 (d, *J* = 8.6 Hz, 1 H, H-5), 7.81 (dd, *J* = 8.6, 2.0 Hz, 1 H, H-6), 2.61 (s, 3 H, CH₃).

### Example 104

**7-Methyl-*N*-(2-(dimethylamino)ethyl]-1,2,4-benzotriazin-3-amine 1-oxide (88).** 2-(Dimethylamino)ethylamine (1.0 mL, 9.0 mmol) was added to a stirred solution of chloride **87** (700 mg, 3.6 mmol) in DME (50 mL) and the solution stirred at reflux temperature for 8 h The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-10%) MeOH/DCM, to give 1-oxide **88** (781 mg, 88%) as a yellow solid, mp (DCM) 143-144 °C; ¹H NMR [(CD₃)₂SO] δ 7.93 (br s, 1 H, H-8), 7.60-7.64 (m, 2 H, NH, H-6), 7 48 (d, *J* = 8.6 Hz, 1 H, H-5), 3.37-3.45 (m, 2 H, CH₂N), 2.46-2 52 (m, 2 H, CH₂N), 2 41 (s, 3 H, CH₃), 2.21 [m, 6 H, N(CH₃)_{2]}; ¹³C NMR [(CD₃)₂SO] δ 158.6 (C-3), 146.8 (C-4a), 137.6 (C-6), 134.6 (C-7), 129.6 (C-8a), 125.8 (C-5), 118.4 (C-8), 57.6 (CH₂N), 45.1 [N(CH₃)₂], 39.0 (CH₂N), 20.6 (CH₃); Anal. calc. for C₁₂Hₗ₇N₅O: C, 58.3; H, 6.9; N, 28.3; found C, 58.5: H, 7.2; N, 28.6%

### Example 105

**7-Methyl-*N*-[2-(1-piperidinyl)ethyl]-1,2,4-benzotriazin-3-amine 1-oxide (89).** 2-(1-Piperidinyl)ethylamine (0.83 mL, 5.8 mmol) was added to a stirred solution of chloride **87** (453 mg, 2.3 mmol) in DME (50 mL) and the solution stirred at reflux temperature for 2 h The solvent was evaporated and the residue partitioned between DCM (100 mL) and dilute aqueous NH₃ (50 mL). The organic fraction was dried and the solvent evaporated. The residue was chromatographed, eluting with a gradient (0-10%) of MeOH/DCM, to give 1-oxide **89** (635 mg, 95%) as a yellow powder, mp (MeOH) 166-168 °C; ¹H NMR δ 8.04-8.06 (m, 1 H, H-8), 7.52 (dd, *J* = 8.7, 1.8 Hz,1 H, H-6), 7.49 (d, *J* = 8.7 Hz, 1 H, H-5), 5.95 (br s, 1 H, NH), 3.52-3.56 (m, 2 H, CH₂N), 2.56-2.59 (m, 2 H, CH₂N), 2.45 (s, 3 H, CH₃), 2.40-2.44 (m, 4 H, 2 × CH₂N), 1.55-1.61 (m, 4 H, 2 × CH₂), 1.42-1.47 (m, 2 H, CH₂); ¹³C NMR δ 158.7 (C-3), 147.7 (C-4a), 137.6 (C-6), 135 3 (C-7), 130.4 (C-8a), 126.1 (C-5), 119.2 (C-8), 56.9 (CH₂N), 54 3 (2 × CH₂N), 37 9 (CH₂N), 26.0 (2 × CH₂), 24.4 (CH₂), 21.3 (CH₃); Anal. calc for C₁₅H₂₁N₅O: C, 62.7; H, 7.4; N, 24.4; found C, 62.8; H, 7.1; N, 24.7%.

### Example 106

**7-Methyl-*N*-[3-(4-morpholinyl)propyl]-1,2,4-benzotriazin-3-amine 1-oxide (90).** 3-(4-Morpholinyl)propylamine (1.4 mL, 9.4 mmol) was added to a stirred solution of chloride **87** (738 mg, 3.8 mmol) in DME (50 mL) and the solution stirred at reflux temperature for 8 h. The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-10%) of MeOH/DCM, to give 1-oxide **90** (1.12 g, 98%) as a yellow powder, mp 158-160 °C; ¹H NMR [(CD₃)₂SO] δ 7.94 (d, *J* = 1 7 Hz, 1 H, H-8), 7.80 (br s, 1 H, NH), 7.63 (dd, *J* = 8.6, 1.7 Hz, 1 H, H-6), 7.47 (d, *J* = 8.6 Hz, 1 H, H-5), 3.53-3.57 (m, 4 H, 2 × CH₂O), 3.36-3.39 (m, 2 H, CH₂N), 2.41 (s, 3 H, CH₃), 2.31-2.38 (m, 6 H, 3 × CH₂N), 1.71-1.77 (m, 2 H, CH₂); ¹³C NMR [(CD₃)₂SO] δ 158.6 (C-3), 146.8 (C-4a), 137 6 (C-6), 134.5 (C-7), 129.6 (C-8a), 125.7 (C-5), 118.4 (C-8), 66.1 (2 × CH₂O), 55.8 (CH₂N), 53 2 (2 × CH₂N), 38 9 (CH₂N), 25.3 (CH₂), 20 6 (CH₃); Anal. calc. for C₁₅H₂₁H₅O₂: C, 59.4; H, 7.0, N, 23.1; found C, 59.7: H, 7.2; N, 23.2%

### Example 107

**3-Chloro-7-methoxy-1 ,2,4-benzotriazine 1-oxide (91).** A solution of NaNO₂ (6.45 g, 93.6 mmol) in water (25 mL) was added dropwise to a stirred suspension of amine **3i** (9 0 g, 46.8 mmol) in 2 M HCl (500 mL) at 5 °C and the foaming suspension stirred vigourously for 2 h The solid was filtered, dissolved in dilute aqueous NH₃, filtered, the filtrate acidified with conc. HCl, and cooled. The resulting precipitate was filtered, washed with water and dried. The solid was dissolved in POCl₃ (100 mL) and DMF (0 5 mL) and heated at 100 °C for 1 h. The solution was cooled, poured into ice/water, stirred for 30 minutes, filtered, washed with water (3 x 30 mL) and dried. The solid was suspended in DCM (150 mL), dried and the solvent evaporated. The residue was chromatographed, eluting with 5% EtOAc/DCM, to give chloride **87** (7.10 g, 71%) as a pale yellow solid, mp (EtOAc/DCM) 177-179 °C [lit (Sasse et. al, Ger. *Offen.* 2,740,887, 1979) mp (benzene) 164-165 °C]; ¹H NMR δ 7.91 (d, *J* = 9.2 Hz, 1 H, H-5), 7.69 (d, *J* = 2.9 Hz, 1 H, H-8), 7.64 (dd, *J* = 9.2, 2.9 Hz, 1 H, H-6), 4.03 (s, 3 H, OCH₃); ¹³C NMR δ 161.7 (C-7), 154.7 (C-3), 143.6 (C-4a), 130.0 (C-8, C-8a), 129.6 (C-6), 97.8 (C-5), 56.6 (OCH₃).

### Example 108

***N***^{**1**}**-(7-Methoxy-1-oxido-1,2,4-benzotriazin-3-yl)-*N***^{**2**}**,*N***^{**2**}**-dimethyl-1,2**- **ethanediamine (92).** N,N-dimethylethylenediamine (1.0 mL, 9.3 mmol) was added to a stirred solution of chloride **91** (659 mg, 3.1 mmol) in DME (50 mL) and the solution stirred at reflux temperature for 16 h. The solvent was evaporated and the residue partitioned between DCM (100 mL) and dilute aqueous NH₃ (50 mL). The organic fraction was dried and the solvent evaporated. The residue was chromatographed, eluting with a gradient (0-10%) of MeOH/DCM, to give the amine **92** (820 mg, 90%) as a yellow powder, which was recrystallized as the hydrochloride salt, mp (MeOH/EtOAc) 231-235 °C; ¹H NMR [(CD₃)₂SO] δ 10 61 (br s, 1 H, NH⁺Cl⁻), 7.89 (br s, 1 H, NH), 7.59 (d, *J* = 8.9 Hz, 1 H, H-5), 7.49-7.53 (m, 2 H, H-6, H-8), 3.88 (s, 3 H, OCH₃), 3.69-3.73 (m, 2 H, CH₂N), 3.28-3.32 (m, 2 H, CH₂N), 2.82 [d, *J* = 4.9 Hz, 6 H, N(CH₃)₂], ¹³ C NMR [(CD₃)₂SO] δ 157.8 (C-7), 156.7 (C-3), 144.0 (C-4a), 130.3 (C-8a), 128.3 (C-5), 127.6 (C-6), 98.1 (C-8), 55.9 (OCH₃), 55.3 (CH₂N), 42.3 [N(CH₃)₂], 35.8 (CH₂N); Anal. calc. for C₁₂H₁₈ClN₅O₂: C, 48.1; H, 6.1; N, 23.4; Cl, 11.8; found C, 48.1; H, 6.2; N, 23.5; Cl, 12.0%.

### Example 109

**7-Methoxy-*N*-[2-(1-piperidinyl)ethyl)-1,2,4-benzotriazin-3-amine 1-oxide (93).** 2-(1-Piperidinyl)ethylamine (0.8 mL, 5.4 mmol) was added to a stirred solution of chloride **91** (453 mg, 2.1 mmol) in DME (50 mL) and the solution stirred at reflux temperature for 16 h. The solvent was evaporated and the residue partitioned between DCM (100 mL) and dilute aqueous NH₃ (50 mL). The organic fraction was dried and the solvent evaporated The residue was chromatographed, eluting with a gradient (0-10%) of MeOH/DCM, to give 1-oxide **93** (625 mg, 96%) as a yellow powder, mp (MeOH) 162-165 °C; ¹H NMR δ 7.58 (d, *J* = 2.8 Hz, 1 H, H-8), 7.53 (d, *J* = 9.2 Hz, 1 H, H-5), 7.38 (dd, *J* = 9.2, 2.8 Hz, 1 H, H-6), 5.90 (br s, 1 H, NH), 3.91 (s, 3 H, OCH₃), 3.52-3.56 (m, 2 H, CH₂N), 2.56-2.60 (m, 2 H, CH₂N), 2.46-2.56 (m, 4 H, 2 × CH₂N), 1.56-1.62 (m, 4 H, 2 × CH₂), 1 43-1.48 (m, 2 H, CH₂); ¹³C NMR 8 158.4 (C-7), 157.2 (C-3), 145.3 (C-4a), 130.7 (C-8a), 128.8 (C-5), 127.7 (C-6), 98.2 (C-8), 56.9 (CH₂N), 56.0 (OCH₃), 54.3 (2 x CH₂N), 38.2 (CH₂N), 26.0 (2 × CH₂), 24.4 (CH₂); Anal. calc. for C₁₅H₂₁N₅O₂: C, 59.4; H, 7.0; N, 23.1; found C, 59.1; H, 6.7; N, 23.2%.

### Example 110

**7-Methoxy-*N*-[2-(4-morpholinyl)ethyl]-1,2,4-benzotriazin-3-amine 1 -oxide (94).** 2-(4-Morpholinyl)ethylamine (1 2 mL, 9.0 mmol) was added to a stirred solution of chloride **91** (633 mg, 3.0 mmol) in DME (50 mL) and the solution stirred at reflux temperature for 16 h. The solvent was evaporated and the residue partitioned between DCM (100 mL) and dilute aqueous NH₃ (50 mL). The organic fraction was dried and the solvent evaporated. The residue was chromatographed, eluting with a gradient (0-5%) of MeOH/DCM, to give 1-oxide **94** (820 mg, 90%) as a yellow powder, mp (MeOH) 208-212 °C; ¹H NMR δ 7.58 (d, *J* = 2.7 Hz, 1 H, H-8), 7.53 (d, *J* = 9.3 Hz, 1 H, H-5), 7.38 (dd, *J* = 9.3, 2.7 Hz, 1 H, H-6), 5.77 (br s, 1 H, NH), 3.91 (s, 3 H, OCH₃), 3.70-3 73 (m, 4 H, 2 × CH₂O), 3.55-3.59 (m, 2 H, CH₂N), 2.63-2.67 (m, 2 H, CH₂N), 2.48-2.52 (m, 4 H, 2 × CH₂N); ¹³C NMR δ 158.3 (C-7), 157.3 (C-3), 145.2 (C-4a), 130 8 (C-8a), 128 9 (C-5), 127.7 (C-6), 98.2 (C-8), 66.9 (2 × CH₂O), 56.8 (CH₂N), 56.0 (OCH₃), 53.3 (2 × CH₂N), 37.5 (CH₂N); Anal. calc. for C₁₄H₁₉N₅O₃ ¼H₂O: C, 54.3; H, 6 3; N, 22.6; found C, 54.1; H, 6.2; N, 22.8%.

### Example 111

**3-Chloro-7-(2-methoxyethoxy)-1,2,4-benzotriazine 1-oxide (96).**
**3-Hydroxy-7-(2-methoxyethoxy)-1,2,4-benzotriazine 1-oxide (95).** A suspension of amine **14** (1.00 g, 4.2 mmol) in 2 N HCl (32 mL) was cooled to 5 °C and a solution of NaNO₂ (0.58 g, 8 5 mmol) in water (1.5 mL) was added over 1 h. More NaNO₂ (0.58 g, 8.5 mmol) in water (1 5 mL) was added and the suspension stirred 72 h at room temperature. The precipitate was filtered and washed with water. The solid was dissolved in 5% aqueous NH₃ and filtered. The filtrate was acidified with conc. HCl to give a precipitate which was filtered, dried and chromatographed, eluting with a gradient (0-5%) of MeOH/DCM, to give compound **95** (0.68 g, 68%) as a yellow solid, mp (DCM/pet.ether) 190-192 °C; ¹H NMR [(CD₃)₂SO] δ 12.52 (br, 1 H, OH), 7.69 (br s, 1 H, H-8), 7.53 (dd, *J* = 8 8, 2 8 Hz, 1 H, H-6), 7.33 (d, *J* = 8.8 Hz, 1 H, H-5), 4.19 (t, *J* = 4.4 Hz, 2 H, CH₂), 3.68 (t, *J* = 4.4 Hz, 2 H, CH₂), 3.33 (s, 3 H, OCH₃); ¹³C NMR [(CD₃)₂SO] δ 154 6, 152.9, 131 8, 129.5, 127.4, 117.8, 101.8, 70.0, 67.9, 58.1; Anal. calc for C₁₀H₁₁N₃O₄. C, 50.6; H, 4 2; N, 17.7; found: C, 50.5; H, 4.7; N, 17.7%.

**3-Chloro-7-(2-methoxyethoxy)-1,2,4-benzotriazine 1-oxide (96).** A mixture of **95** (1.00 g, 4 3 mmol) in POCl₃ (8 mL) was refluxed for 2 h. Excess reagent was evaporated under vacuum, and ice cold water (50 mL) was added to the residue, then solid Na₂CO₃ (1.0 g) was added portionwise. The resulting precipitate was filtered and chromatographed, eluting with a gradient (50-100%) of DCM/pet. ether, to give chloride **96** (0.90 g, 83%) as a pale yellow solid, mp (DCM/pet. ether) 121-125 °C; ¹H NMR [(CD₃)₂SO] δ 8.00 (d, *J* = 9.2 Hz, 1 H, H-5), 7.81 (dd, *J* = 9.2, 2.9 Hz, 1 H, H-6), 7.68 (d, *J* = 2.8 Hz, 1 H, H-8), 4.35 (t, *J* = 4.4 Hz, 2 H, CH₂), 3.74 (t, *J* = 4.4 Hz, 2 H, CH₂), 3 33 (s, 3 H, OCH₃); Anal. calc. for C₁₀H₁₀ClN₃O₃: C, 47.0; H, 3.9; N,16.4, Cl, 13.9; found C, 46.9; H, 4.3; N, 16.4; Cl, 13.7%.

### Example 112

**3-Ethyl-7-(2-methoxyethoxy)-1,2,4-benzotriazine 1-oxide (97).**
Pd(PPh₃)₄ (92 mg, 0.08 mmol) was added to a N₂ purged solution of chloride 96 (260 mg, 1.0 mmol) and tetraethyltin (0.4 mL, 2.0 mmol) in DMF (15 mL). The purged reaction mixture was heated at reflux temperature for 20 h under N₂. The solvent was evaporated and the residue chromatographed, eluting with 50% DCM/pet. ether, to give 1-oxide **97** (142 mg, 56%) as a white powder, mp (DCM/pet. ether) 95-97 °C; ¹H NMR [(CD₃)₂SO] δ 7.97 (d, *J* = 9.2 Hz, 1 H, H-5), 7.74 (dd, *J* = 9.2, 2 9 Hz, 1 H, H-6), 7 68 (d, *J* = 2 8 Hz, 1 H, H-8), 4 33 (t, *J* = 4.4 Hz, 2 H, CH₂), 3.74 (t, *J* = 4.4 Hz, 2 H, CH₂), 3.32 (s, 3 H, OCH₃), 2.75 (q, *J* = 7.5 Hz, 2 H, CH₂), 1.33 (t, *J* = 7.6 Hz, 3 H, CH₃); ¹³C NMR [(CD₃)₂SO] δ 164.9, 159.6, 143.1, 133.3, 129.8, 128.7, 98.1, 69.8, 68 2, 58.1, 29.5, 11 9; Anal. calc. for C₁₂H₁₅N₃O₃ C, 57.8; H, 6.1; N, 16.9; found C, 57.7; H, 6.1; N, 16.6%.

### Example 113

**3-Chloro-7-[2-(4-morpholinyl)ethoxy]-1,2,4-benzotriazine 1-oxide (98).** A solution of NaNO₂ (286 mg, 4.1 mmol) in water (2 mL) was added slowly to a solution of amine **17** (610 mg, 2.09 mmol) in 1 N HCl (16 mL) at 5°C. The reaction mixture was stirred at 5 °C for a further 2 h, neutralized with NaHCO₃ and solvent was evaporated. The residue was filtered through a short column of silica, eluting with MeOH. The filtrate was evaporated and the residue heated at reflux temperature in POCl₃ (2 mL) and dimethylaniline (0.6 mL, 2.5 eq) for 2 h The reaction mixture was cooled and poured into ice/water (50 mL). The cold slurry was neutralized with solid NaHCO₃ (2 g), extracted with EtOAc (3 x 50 mL), dried and the solvent evaporated. The residue was chromatographed, eluting with a gradient (0-20%) of EtOAc/DCM, to give chloride **98** (346 mg, 53%) as a white crystals, mp (DCM/pet. ether) 144-146 °C; ¹H NMR [(CD₃)₂SO] δ 7.99 (d, J = 9.2 Hz, 1 H, H-5), 7.80 (dd, *J* = 9.1, 2.8 Hz, 1 H, H-6), 7.70 (d, *J* = 2 8 Hz, 1 H, H-8), 4.34 (t, *J* = 5.8 Hz, 2 H, CH₂), 3.58 (t, J = 4.6 Hz, 4 H, 2 × CH₂), 2.77 (t, *J* = 5.5 Hz, 2 H, CH₂), 2.52-2.58 (m, 4 H, 2 × CH₂); ¹³C NMR [(CD₃)₂SO] δ 160.3, 153.1, 143.1, 134.6, 129.7, 129.4, 98.7, 66.8, 66.1 (2), 56.5, 53.4 (2); Anal. calc. for C₁₃H₁₅ClN₄O₃: C, 50.3; H, 4.9; N,18.0; found C, 50.7; H, 5.0; N, 18.1%.

### Example 114

**3-Ethyl-7-[2-(4-morpholinyl)ethoxy]-1,2,4-benzotriazine 1-oxide (99).** Pd(PPh₃)₄ (92 mg, 0.08 mmol) was added to a N₂ purged solution of chloride 96 (315 mg, 1.0 mmol) and tetraethyltin (0.4 mL, 2.0 mmol) in DMF (15 mL). The purged reaction mixture was heated at reflux temperature for 20 h under N₂. The solvent was evaporated and the residue chromatographed, eluting with a gradient (0-1%) of MeOH/DCM, to give 1-oxide **99** (204 mg, 67%) as a white powder, mp (DCM/pet. ether) 99-101 °C; ¹H NMR [(CD₃)₂SO] δ 7.96 (d, *J* = 9.0 Hz, 1 H, H-5), 7.72 (dd, *J* = 9.0, 2.8 Hz, 1 H, H-6), 7.70 (d, *J* = 2.6 Hz, 1 H, H-8), 4.36 (t, *J* = 5.8 Hz, 2 H, CH₂), 3.59 (t, *J* = 4.6 Hz, 4 H, 2 × CH₂), 2 94 (q, *J* = 7.6 Hz, 2 H, CH₂), 2.77 (t, *J* = 5.5 Hz, 2 H, CH₂), 2.50 (t, *J* = 4.2 Hz, 4 H, 2 × CH₂), 1.34 (t, *J* = 7.6 Hz, 3 H, CH₃), ¹³C NMR [(CD₃)₂SO] δ 164.8, 159.5, 143.0, 133.3, 129.8, 128 8, 98.3, 66.6, 66.1 (2), 55.6, 53.5 (2), 29.5, 11.9; Anal. calc. for C₁₅H₂₀N₄O₃: C, 59.2; H, 6.6; N, 18.4; found C, 59.3; H, 6.5; N, 18.4%.

### Example 115

**3-Chloro-8-methyl-1,2,4-benzotriazine 1-oxide (100).** Sodium nitrite (6.15 g, 89.1 mmol) was added in small portions to a stirred solution of 8-methyl-1,2,4-benzotriazin-3-amine 1-oxide (**3b**) (7.85 g, 44.6 mmol) in trifluoroacetic acid (80 mL) at 5 °C and the solution stirred at 20 °C for 3 h. The solution was poured into ice/water, stirred 30 minutes, filtered, washed with water (3 x 30 mL) and dried. The solid was suspended in POCl₃ (100 mL) and DMF (0.5 mL) and stirred at 100 °C for 1 h. The solution was cooled, poured into ice/water, stirred for 30 minutes, filtered, washed with water (3 x 30 mL) and dried. The solid was dissolved in DCM (150 mL), dried and the solvent evaporated The residue was chromatographed, eluting with 5% EtOAc/DCM, to give chloride **100** (4.25 g, 49%) as a pale yellow solid, mp (EtOAc/DCM) 170-173 °C; ¹H NMR δ 7.78-7.82 (m, 2 H, H-5, H-7), 7.47-7.51 (m, 1 H, H-6), 2.98 (s, 3 H, CH₃); ¹³C NMR δ 156.4 (C-3), 149.1 (C-4a), 135.7 (C-6), 134.5 (C-8), 133.9 (C-8a), 133.1 (C-5), 126.7 (C-7), 23.6 (CH₃); Anal. calc. for C₈H₆ClN₃0: C, 49.1; H, 3.1; N, 21.5; found: C, 49.4; H, 2.9; N, 21.6%.

### Example 116

**N**^{**1**}**,N**^{**1**}**-Dimethyl-*N***^{**2**}**-(8-methyl-1-oxido-1,2,4-benzotriazin-3-yl)-1,2-ethanediamine** (101). N,N-Dimethylethanediamine (530 µL, 4.9 mmol) was added to a stirred solution of chloride **100** (316 mg, 1.6 mmol) in DME (50 mL) and the solution stirred at reflux temperature for 2 h. The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-10%) of MeOH/DCM, to give 1-oxide **101** (341 mg, 85%) as a yellow solid, mp (MeOH/EtOAc) 121-123 °C; ¹H NMR δ 7.48 (dd, *J* = 8.1, 7.1 Hz, 1 H, H-6), 7.41 (d, *J* = 8.1 Hz, 1 H, H-5), 7.00 (d, *J* = 7.1 Hz, 1 H, H-7), 5.86 (br s, 1 H, NH), 3.51-3.57 (m, 2 H, CH₂N), 2.88 (s, 3 H, CH₃), 2.56-2.60 (m, 2 H, CH₂N), 2.29 [s, 6 H, N(CH₃)₂]; ¹³C NMR δ 158.5 (C-3), 150.7 (C-4a), 134.4 (C-6), 134.2 (C-8), 131.1 (C-8a), 127.5 (C-5), 124.7 (C-7), 57.6 (CH₂N), 45.0 [N(CH₃)₂], 38.6 (CH₂N), 24.0 (CH₃); Anal. calc. for C₁₂H₁₇N₅O; C, 58.3; H, 6 9; N, 28.3; found C, 58.0: H, 7.2; N, 28.1%.

### Example 117

**8-Methyl-*N*-[2-(1-piperidinyl)ethyl]-1,2,4-benzotriazin-3-amine 1-oxide (102).** 2-(1-Piperidinyl)ethylamine (1.26 mL, 8.9 mmol) was added to a stirred solution of chloride 100 (578 mg, 3.0 mmol) in DME (50 mL) and the solution stirred at reflux temperature for 2 h. The solvent was evaporated and the residue partitioned between DCM (100 mL) and dilute aqueous NH₃ (50 mL). The organic fraction was dried and the solvent evaporated. The residue was chromatographed, eluting with a gradient (0-10%) of MeOH/DCM, to give 1-oxide **102** (764 mg, 90%) as a yellow powder, mp (MeOH/EtOAc) 137-140 °C; ¹H NMR δ 7.48 (dd, *J* = 7.8, 7 8 Hz, 1 H, H-6), 7.41 (br d, *J* = 7.8 Hz, 1 H, H-5), 7.00 (d, *J* = 7.1 Hz, 1 H, H-7), 5.90 (br s, 1 H, NH), 3.52-3.56 (m, 2 H, CH₂N), 2.90 (s, 3 H, CH₃), 2.55-2.59 (m, 2 H, CH₂N), 2.40-2.45 (m, 4 H, 2 × CH₂N), 1.55-1.61 (m, 4 H, 2 × CH₂), 1.41-1.48 (m, 2 H, CH₂); ¹³C NMR δ 158.4 (C-3), 150.7 (C-4a), 134.5 (C-6), 134.2 (C-8), 131.1 (C-8a), 127.4 (C-5), 124.7 (C-7), 57.0 (CH₂N), 54.3 (2 x CH₂N), 37.9 (CH₂N), 26.0 (2 x CH₂), 24.4 (CH₂), 24.0 (CH₃).

### Example 118

**3-Chloro-6,7-dimethyl-1 -oxido-1,2,4-benzotriazine (104).** A mixture of 4,5-dimethyl-2-nitroaniline **103** (5.0 g, 30.1 mmol) and cyanamide (5.06 g, 120 mmol) were mixed together at 100 °C. The mixture was cooled to ca. 50 °C and cHCl (15 mL) added (CAUTION: exotherm) and the resulting solution heated at 100 °C for 1 h. The solution was cooled to ca. 50 °C and 7.5 M NaOH solution (50 mL) added carefully. The suspension was stirred at 100 °C for 2 h, cooled to 20 °C and diluted with water (100 mL). The suspension was filtered, washed with water (2 × 10 mL), washed with ether (2 × 10 mL) and dried. The yellow solid (4.50 g, 23.7 mmol) was suspended in 2 M HCl (250 mL), cooled to 5 °C, and a solution of NaNO₂ (3.27 g, 47.3 mmol) in water (20 mL) added dropwise. The mixture was stirred vigorously for 2 h at 20 °C. The suspension was filtered, the solid suspended in dilute aqueous NH₃ (200 mL) and filtered. The filtrate was acidified with cHCl, cooled at 5 °C for 16 h and the precipitate collected. The solid was washed with water (2 × 15 mL) and dried to give the 3-hydroxy-6,7-dimethyl-1,2,4-benzotriazine 1-oxide (1.21 g, 21%) which was used without further characterization. A mixture of the 3-hydroxide (1.21 g, 6.3 mmol), dimethylaniline (2.0 mL, 15.8 mmol) and POCl₃ (4.1 mL, 44.3 mmol) was heated at reflux temperature for 1 h. The solution was poured onto ice, stirred and filtered. The solid was dissolved in EtOAc (200 mL), dried, and the solvent evaporated. The residue was chromatographed, eluting with 5% EtOAc/DCM, to give the chloride **104** (1.07 g, 81 %) as colourless plates, mp 148-149 °C; ¹H NMR δ 8.16 (s, 1 H, H-8), 7.72 (s, 1 H, H-5), 2 51 (s, 3 H, CH3), 2.50 (s, 3 H, CH₃); ¹³C NMR δ 156.1 (C-3), 148.9 (C-6), 146.3 (C-4a), 142.5 (C-7), 132.0 (C-8a), 127.4 (C-5), 119.8 (C-8), 20.8 (CH₃), 20 5 (CH₃); Anal. calc. for C₉H₈ClN₃O: C, 51.6; H, 3.85; N, 20.0; found C, 51 8; H, 3.7; N, 20.2%

### Example 119

**tert-Butyl 2-[(6,7-dimethyl-1-oxido-1,2,4-benzotriazin-3-yl)amino]ethylcarbamate (105)** A solution of chloride **104** (842 mg, 4.0 mmol) and *tert*-butyl 2-aminoethylcarbamate (1.4 g, 8.8 mmol) in DME (50 mL) was heated at reflux temperature for 3 h. The solvent was evaporated and the residue partitioned between EtOAc (100 mL) and water (100 mL). The organic fraction was dried and the solvent evaporated The residue was chromatographed, eluting with a gradient (20-50%) of EtOAc/DCM, to give 1-oxide **105** (1.04 g, 78%) as a yellow solid, mp (EtOAc/DCM) 226-228 °C; ¹H NMR [(CD₃)₂SO] δ 7.90 (s, 1 H, H-8'), 7.66 (br s, 1 H, NH), 7.39 (s, 1 H, H-5'), 6.88 (t, *J* = 5 3 Hz, 1 H, NH), 3.33-3.38 (m, 2 H, CH₂N), 3.14-3.18 (m, 2 H, CH₂N), 2 36 (s, 3 H, CH₃), 2.33 (s, 3 H, CH₃), 1.37 [C(CH₃)₃]; ¹³C NMR [(CD₃)₂SO] δ 155 8 (NHCO₂), 155.6 (C-3'), 147.1 (C-4a'), 147.8 (C-7'), 134.8 (C-6'), 128.2 (C-8a'), 125.2 (C-5'), 118.5 (C-8'), 77.6 [C(CH₃)₃], 40.9 (CH₂N), 39.0 (CH₂N), 28.1 [C(CH₃)₃], 19.9 (CH₃), 19.2 (CH₃); Anal. calc. for C₁₆H₂₃N₅O₃: C, 57.6; H, 7.0; N, 21.0; found C, 57.9; H, 7.0; N, 20.8%.

### Example 120

***N***^{**1**}**-(6,7-Dimethyl-1-oxido-1,2,4-benzotriazin-3-yl)-*N***^{**2**}**,*N***^{**2**}**-dimethyl-1,2-ethanediamine (106)**. N,N-Dimethyl-1,2-ethanediamine (0.3 mL, 2.7 mmol) was added to a stirred solution of chloride **104** (190 mg, 0.9 mmol) in DME (30 mL) and the solution stirred at reflux temperature for 16 h. The solvent was evaporated and the residue partitioned between DCM (100 mL) and dilute aqueous NH₃ (50 mL). The organic fraction was dried and the solvent evaporated. The residue was chromatographed, eluting with a gradient (0-10%) of MeOH/DCM, to give amine **106** (181 mg, 76%), mp (MeOH) 175-178 °C; ¹H NMR δ 8.00 (s, 1 H, H-8), 7.36 (s, 1 H, H-5), 5 82 (br s, 1 H, NH), 3.52-3.56 (m, 2 H, CH₂N), 2.55-2.59 (m, 2 H, CH₂N), 2.38 (s, 3 H, CH₃), 2.36 (s, 3 H, CH₃), 2 27 [s, 6 H, N(CH₃)₂]; ¹³C NMR [(CD₃)₂SO] δ 158.9 (C-3), 147.8 (C-4a), 146.9 (C-6), 135.4 (C-7), 129.1 (C-8a), 125.7 (C-5), 119.4 (C-8), 57.6 (CH₂N), 45.1 [N(CH₃)₂], 38.7 (CH₂N), 20.5 (CH₃), 19.8 (CH₃); MS (EI) *m*/*z* 261 (M⁺, 5%), 224 (3), 217 (1), 58 (100); HRMS calc. for C₁₃H₁₉N₅O (M⁺) *m*/*z* 261.1590, found 261.1587.

### Example 121

**3-Chloro-6,8-dimethyl-1,2,4-benzotriazine 1-oxide (108).** A mixture 3,5-dimethyl-2-nitroaniline **(107)** (Andrews et al., *Aust. J. Chem.* **1972,** 25, 639) (6.61 g, 39.8 mmol) and cyanamide (6.7 g, 159 mmol) were mixed together at 100 °C, cooled to 50 °C, cHCl (30 mL) added carefully and the mixture heated at 100 °C for 4 h The mixture was cooled to 50 °C, 7.5 M NaOH solution added until the mixture was strongly basic and the mixture stirred at 100 °C for 3 h. The mixture was cooled, diluted with water (100 mL), filtered, washed with water (3 × 30 mL), washed with ether (3 × 20 mL) and dried to give crude 1-oxide (2.62 g, 35%) as a yellow powder. Sodium nitrite (1.55 g, 22.5 mmol) was added in small portions to a stirred solution of 1-oxide (2.14 g, 11.3 mmol) in trifluoroacetic acid (20 mL) at 5 °C and the solution stirred at 20 °C for 3 h. The solution was poured into ice/water, stirred 30 minutes, filtered, washed with water (3 × 30 mL) and dried. The solid was suspended in POCl₃ (50 mL) and DMF (0.2 mL) stirred at 100 °C for 1 h. The solution was cooled, poured into ice/water, stirred for 30 minutes, filtered, washed with water (3 × 30 mL) and dried. The solid was suspended in DCM (150 mL), dried and the solvent evaporated. The residue was chromatographed, eluting with 5% EtOAc/DCM, to give chloride 108 (1.58 g, 67%) as a pale yellow solid, mp (EtOAc/DCM) 120-122 °C; ¹H NMR δ 7.55 (s, 1 H, H-5), 7.30 (s, 1 H, 1 H, H-7), 2.93 (s, 3 H, CH₃), 2.52 (s, 3 H, CH₃); ¹³C NMR δ 156.4 (C-3), 149.4 (C-4a), 147.7 (C-6), 135.1 (C-5), 133.9 (C-8), 132.2 (C-8a), 125.5 (C-7), 23.4 (CH₃), 21 9 (CH₃); Anal. calc. for C₉H₈CIN₃O: C, 51.6; H, 3.9; N, 20.0; Cl, 16.9; found C, 51.8; H, 3.6; N, 20 2; Cl, 16.6%.

### Example 122

***N***^{**1**}**-(6,8-Dimethyl-1-oxido-1,2,4-benzotriazin-3-yl)-*N***^{**2**}***,N***^{**2**} **-dimethyl-1,2-ethanediamine (109).** N,N-Dimethylethanediamine (0.64 mL, 5.9 mmol) was added to a stirred solution of chloride **108** (494 mg, 2.4 mmol) in DME (80 mL) and the solution stirred at reflux temperature for 2 h. The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-10%) of MeOH/DCM, to give 1-oxide **109** (561 mg, 91%) as a yellow solid, mp (MeOH) 176-179 °C; ¹H NMR δ 7.20 (br s, 1 H, H-5), 6.84 (br s, 1 H, H-7), 5.76 (br s, 1 H, NH), 3.50-3 54 (m, 2 H, CH₂N), 2.85 (s, 3 H, CH₃), 2.52-2.56 (m, 2 H, CH₂N), 2.38 (s, 3 H, CH₃), 2.26 [s, 6 H, N(CH₃)₂], ¹³C NMR δ 158.7 (C-3), 150.9 (C-4a), 145.6 (C-6), 133.7 (C-8), 129.6 (C-5), 129.4 (C-8a), 123.7 (C-7), 57.6 (CH₂N), 45.1 [N(CH₃)₂], 38.7 (CH₂N), 23.8 (CH₃), 21.6 (CH₃); Anal. calc. for C₁₃H₁₉N₅O; C, 59.8; H, 7.3; N, 26.8; found C, 60.0: H, 7.6; N, 27.0%.

### Example 123

**6,8-Dimethyl-*N*-[2-(1-piperidinyl)ethyl]-1,2,4-benzotriazin-3-amine 1-oxide (110).**
2-(1-Piperidinyl)ethylamine (0.67 mL, 4 7 mmol) was added to a stirred solution of chloride **108** (395 mg, 1 9 mmol) in DME (50 mL) and the solution stirred at reflux temperature for 2 h. The solvent was evaporated and the residue partitioned between DCM (100 mL) and dilute aqueous NH₃ (50 mL). The organic fraction was dried and the solvent evaporated. The residue was chromatographed, eluting with a gradient (0-5%) of MeOH/DCM, to give 1-oxide **110** (517 mg, 91%) as a yellow powder, mp (MeOH) 177-178 °C; ¹H NMR δ 7.20 (s, 1 H, H-5), 6.84 (s, 1 H, H-7), 5.84 (br s, 1 H, NH), 3.49-3.55 (m, 2 H, CH₂N), 2.85 (s, 3 H, CH₃), 2.54-2.57 (m, 2 H, CH₂N), 2.39-2.44 (m, 4 H, 2 × CH₂N), 2.37 (s, 3 H, CH₃), 1.55-1.61 (m, 4 H, 2 × CH₂), 1.41-1.47 (m, 2 H, CH₂); ¹³C NMR δ 158.6 (C-3), 150.9 (C-4a), 145.6 (C-6), 133.7 (C-8), 129.5 (C-5), 129.4 (C-8a), 123.7 (C-7), 57 0 (CH₂N), 54 3 (2 × CH₂N), 37.9 (CH₂N), 26.0 (2 × CH₂), 24.4 (CH₂), 23.8 (CH₃), 21.7 (CH₃); Anal. calc. for C₁₆H₂₃N₅O: C, 63.8; H, 7.7; N, 23.2; found C, 63.9; H, 8.0; N, 23.5%.

### Example 124

**6-Methoxy-7-methyl-1,2,4-benzotriazin-3-amine 1-oxide (112).** A mixture of 5-methoxy-4-methyl-2-nitroaniline **(111)** (James & Felix, *U.S. Patent* 5,360,986) (8.9 g, 49 mmol) and cyanamide (8.2 g, 196 mmol) were mixed together at 100 °C, cooled to 50 °C, cHCl (50 mL) added carefully and the mixture heated at 100 °C for 4 h. The mixture was cooled to 50°C, 7.5 M NaOH solution added until the mixture was strongly basic and the mixture stirred at 100 °C for 3 h. The mixture was cooled, diluted with water (200 mL), filtered, washed with water (3 × 50 mL), washed with ether (3 x 30 mL) and dried. The solid was recrystallized from MeOH to give 1-oxide **112** (6.0 g, 59%) as a yellow powder, mp (MeOH) 289-292 °C; ¹H NMR [(CD₃)₂SO] δ 7.91 (d, *J* = 1.1 Hz, 1 H, H-8), 7.10 (br s, 2 H, NH₂), 6.84 (s, 1 H, H-5), 3.94 (s, 3 H, OCH₃), 2.23 (s, 3 H, CH₃); ¹³ C NMR [(CD₃)₂SO] δ 163.7 (C-6), 160.4 (C-3), 150.3 (C-4a), 127.3 (C-7), 124.4 (C-8a), 120.0 (C-8), 102.7 (C-5), 56.3 (OCH₃), 16.1 (CH₃); Anal. calc. for C₉H₁₀N₄O₂.¼MeOH: C, 51.9; H, 5 2; N, 26.2; found C, 52.1; H, 4.8; N, 264%.

### Example 125

**3-Chloro-6-methoxy-7-methyl-1,2,4-benzotriazine 1-oxide (113).** Sodium nitrite (3.38 g, 49.0 mmol) was added in small portions to a stirred solution of 1-oxide **112** (5 05 g, 24.5 mmol) in trifluoroacetic acid (30 mL) at 5 °C and the solution stirred at 20 °C for 3 h. The solution was poured into ice/water, stirred 30 minutes, filtered, washed with water (3 × 30 mL) and dried. The solid was suspended in POCl₃ (50 mL) and DMF (0.2 mL) stirred at 100 °C for 1 h. The solution was cooled, poured into ice/water, stirred for 30 minutes, filtered, washed with water (3 × 30 mL) and dried. The solid was suspended in DCM (150 mL), dried and the solvent evaporated. The residue was chromatographed, eluting with 5% EtOAc/DCM, to give chloride 113 (2 72 g, 49%) as a pale yellow solid, mp (EtOAc) 180-182 °C; ¹H NMR δ 8.44 (d, *J* = 0 9 Hz, 1 H, H-8), 7.14 (s, 1 H, 1 H, H-5), 4.03 (s, 3 H, OCH₃), 2.40 (d, *J* = 0.9 Hz, 3 H, CH₃); ¹³C NMR δ 165.4 (C-6), 156.8 (C-3), 149.2 (C-4a), 135.5 (C-7), 128.4 (C-8a), 120.4 (C-8), 104.3 (C-5), 56.6 (OCH₃), 17.2 (CH₃); Anal. calc. for C₉H₈ClN₃O₂: C, 47.9; H, 3 6; N, 18.6; Cl, 15.7; found C, 48.0; H, 3.5; N, 18.6; Cl, 15.7%.

### Example 126

***N***^{**1**}**-(6-Methoxy-7-methyl-1-oxido-1,2,4-benzotriazin-3-yl)-*N***^{**2**}**,*N***^{**2**}**-dimethyl-1,2**- **ethanediamine (114)**. *N,N*-Dimethylethanediamine (0.70 mL, 6.3 mmol) was added to a stirred solution of chloride **113** (474 mg, 2.1 mmol) in DME (50 mL) and the solution stirred at reflux temperature for 2 h. The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-15%) of MeOH/DCM, to give 1-oxide **114** (529 mg, 90%) as a yellow solid, mp (MeOH) 167-169 °C; ¹H NMR δ 7.99 *(d, J* = 1.0 Hz, 1 H, H-8'), 6.79 (s, 1 H, H-5'), 5.84 (br s, 1 H, NH), 3.94 (s, 3 H, OCH₃), 3.49-3.54 (m, 2 H, CH₂N), 2.52-2.56 (m, 2 H, CH₂N), 2.27 (d, *J* = 1.0 Hz, 3 H, CH₃), 2.26 [s, 6 H, N(CH₃)₂]; ¹³C NMR δ 164.5 (C-6'), 159.3 (C-3'), 150.5 (C-4a'), 128.5 (C-7'), 125.4 (C-8a'), 120 7 (C-8'), 120.7 (C-5'), 57.5 (CH₂N), 56.1 (OCH₃), 45.1 [N(CH₃)₂], 38.7 (CH₂N), 16.5 (CH₃); Anal. calc. for C₁₃H₁₉N₅O₂: C, 56.3; H, 6.9; N, 25 3; found C, 56.5: H, 7.2; N, 25.7%.

### Example 127

**7-Methoxy-6-methyl-1,2,4-benzotriazin-3-amine 1-oxide (116).** A mixture of 4-methoxy-5-methyl-2-nitroaniline (**115**) (Arnold & McCool, *J. Am. Chem.* Soc. **1942,** 64, 1315) (2.3 g, 12.6 mmol) and cyanamide (2.0 g, 50 mmol) were mixed together at 100 °C, cooled to 50 °C, cHCl (20 mL) added carefully and the mixture heated at 100 °C for 4 h. The mixture was cooled to 50 °C, 7.5 M NaOH solution added until the mixture was strongly basic and the mixture stirred at 100 °C for 3 h. The mixture was cooled, diluted with water (100 mL), filtered, washed with water (3 × 30 mL), washed with ether (3 × 20 mL) and dried to give crude 1-oxide **116** (2.52 g, 97%) as a yellow powder, mp (MeOH) >320 °C; ¹H NMR [(CD₃)₂SO] δ 7.42 (s, 1 H, H-8), 7.39 (d, J = 1.0 Hz, 1 H, H-5), 6.99 (br s, 2 H, NH₂), 3.90 (s, 3 H, OCH₃), 2.30 (s, 3 H, CH₃); ¹³C NMR [(CD₃)₂SO) δ 159.7 (C-7), 155.4 (C-3), 144.8 (C-4a), 139.3 (C-6), 128.4 (C-8a), 126 2 (C-5), 96.6 (C-8), 56.0 (OCH₃), 16.7 (CH₃).

### Example 128

**3-Chloro-7-methoxy-6-methyl-1,2,4-benzotriazine 1-oxide (117).** Sodium nitrite (1.7 g, 24.4 mmol) was added in small portions to a stirred solution of 1-oxide **116** (2.50 g, 12 2 mmol) in trifluoroacetic acid (20 mL) at 5 °C and the solution stirred at 20 °C for 3 h. The solution was poured into ice/water, stirred 30 minutes, filtered, washed with water (3 × 30 mL) and dried. The solid was suspended in POCl₃ (50 mL) and DMF (0.2 mL) stirred at 100 °C for 1 h. The solution was cooled, poured into ice/water, stirred for 30 minutes, filtered, washed with water (3 × 30 mL) and dried. The solid was suspended in DCM (150 mL), dried and the solvent evaporated. The residue was chromatographed, eluting with 5% EtOAc/DCM, to give chloride 117 (1.38 g, 50%) as a pale yellow solid, mp (EtOAc/DCM) 200-202 °C; ¹H NMR δ 7.70 (d, *J* = 0.9 Hz, 1 H, H-5), 7.59 (s, 1 H, 1 H, H-8), 4.03 (s, 3 H, OCH₃), 2.44 (d, *J* = 0.9 Hz, 3 H, CH₃); ¹³C NMR δ 160.7 (C-7), 154.7 (C-3), 143.5 (C-4a), 142.0 (C-6), 133.1 (C-8a), 128.4 (C-5), 96.4 (C-8), 56.6 (OCH₃), 17.4 (CH₃); Anal. calc. for C₉H₈CIN₃O₂: C, 47.9; H, 3.6, N, 18.6; found C, 48.1; H, 3.4; N, 18.7%.

### Example 129

***N***^{**1**}**,*N***^{**1**}**-Dimethyl-*N***^{**2**}**-(7-methoxy-6-methyl-1-oxido-1,2,4-benzotriazin-3-yl)-1,2-ethanediamine (118).** *N,N*-Dimethylethanediamine (0.48 mL, 4.3 mmol) was added to a stirred solution of chloride **117** (391 mg, 1.7 mmol) in DME (50 mL) and the solution stirred at reflux temperature for 2 h The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-10%) of MeOH/DCM, to give 1-oxide **118** (432 mg, 90%) as a yellow solid, mp (MeOH/EtOAc) 182-184 °C; ¹H NMR δ 7.49 (s, 1 H, H-8'), 7.36 (s, 1 H, H-5'), 5.72 (br s, 1 H, NH), 3.92 (s, 3 H, OCH₃), 3.49-3.54 (m, 2 H, CH₂N), 2 53-2.56 (m, 2 H, CH₂N), 2.34 (s, 3 H, CH₃), 2.26 [s, 6 H, N(CH₃)₂], ¹³C NMR δ 158.6 (C-7'), 156.4 (C-3'), 145.2 (C-4a'), 140.2 (C-6'), 129.4 (C-8a'), 124.9 (C-5'), 96.9 (C-8'), 57.6 (CH₂N), 56.0 (OCH₃), 45.1 [N(CH₃)₂], 38.8 (CH₂N), 17.2 (CH₃); Anal. calc. for C₁₃H₁₉N₅O₂: C, 56.3; H, 6.9; N, 25.3; found C, 56.5: H, 6.7; N, 25.5%.

### Example 130

**7-Methoxy-6-methyl-*N*-[2-(1-piperidinyl)ethyl]-1,2,4-benzotriazin-3-amine 1-oxide (119).** 2-(1-Piperidinyl)ethylamine (0.74 mL, 5.2 mmol) was added to a stirred solution of chloride **117** (467 mg, 2.1 mmol) in DME (50 mL) and the solution stirred at reflux temperature for 4 h. The solvent was evaporated and the residue partitioned between DCM (100 mL) and dilute aqueous NH₃ (50 mL). The organic fraction was dried and the solvent evaporated. The residue was chromatographed, eluting with a gradient (0-5%) of MeOH/DCM, to give 1-oxide **119** (574 mg, 87%) as a yellow powder, mp (MeOH/EtOAc) 195-197 °C; ¹H NMR δ 7.50 (s, 1 H, H-8), 7.36 (d, *J* = 0.8 Hz, 1 H, H-5), 5.83 (br s, 1 H, NH), 3 92 (s, 3 H, OCH₃), 3.49-3.54 (m, 2 H, CH₂N), 2.54-2 59 (m, 2 H, CH₂N), 2.39-2.43 (m, 4 H, 2 × CH₂N), 2.33 (s, 3 H, CH₃), 1.54-1.60 (m, 4 H, 2 × CH₂), 1.41-1.47 (m, 2 H, CH₂); ¹³C NMR δ 158.6 (C-7), 156.3 (C-3), 145.2 (C-4a), 140.2 (C-6), 129.4 (C-8a), 126.8 (C-5), 96.9 (C-8), 57.0 (CH₂N), 56.0 (OCH₃), 54.3 (2 x CH₂N), 38.0 (CH₂N), 26.0 (2 × CH₂), 24.4 (CH₂), 17.2 (CH₃); Anal. calc. for C₁₆H₂₃N₅O₂: C, 60.6; H, 7.3; N, 22.1; found C, 60.6; H, 7.1; N, 22.3%.

### Example 131

**N-(2-{2-[(1-Oxido-1,2,4-benzotriazin-3-yl)amino]ethoxy}ethyl)-4-acridinecarboxamide (121).** A solution of amine **36** (0.53 g, 2.1 mmol) in DCM (10 mL) was added dropwise to a stirred solution of imidazolide of acridine-4-carboxylic acid **120** (0.58 g, 2.1 mmol) in THF (25 mL) and the solution stirred at 20 °C for 72 h. The solvent was evaporated and the residue chromatographed, eluting with a gradiant (0-4%) of MeOH/DCM, to give **121** (642 mg, 66%) as a yellow powder, mp 178-182 °C; ¹H NMR [(CD₃)₂SO] δ 11.75 (t, *J =* 5.0 Hz, 1 H, NH), 9.27 (s, 1 H, H-9), 8.74 (dd, *J=* 8.4, 1.5 Hz, 1 H, H-3), 8.36 (dd, *J=* 8.4,1.4 Hz, 1 H, H-1), 8.18 (m, 2 H, H-5, H-8), 8.02 (dd, *J* = 8.6, 1.2 Hz, 1 H, H-8'), 7.90 (br s, 1 H, NH), 7.83 (t, *J* = 7.5 Hz, 1 H, H-6), 7.75 (dd, *J* = 8.6, 7.2 Hz, 1 H, H-2), 7.70 (ddd, *J* = 8.6, 7.1, 1.2 Hz, 1 H, H-6'), 7.61 (ddd, *J* = 8.9, 7.1, 0.7 Hz, 1 H, H-7), 7.42 (br d, *J* = 8.6 Hz, 1 H, H-5'), 7.28 (ddd, *J* = 8.4, 7.1, 1.4 Hz, 1 H, H-7'), 3.80-3.84 (m, 4 H, 2 × CH₂O), 3.73-3.77 (m, 2 H, CH₂N), 3.60-3.65 (m, 2 H, CH₂N); ¹³C NMR [(CD₃)₂SO] δ 164.6 (CONH), 158.8 (C-3'), 148.0 (C-4a'), 146.8 (C-4b), 145.3 (C-4a), 138.5 (C-9), 135.5 (C-6'), 134.5 (C-3), 132 8 (C-1), 131.7 (C-6), 129.9 (C-9a, C-8a'), 128.3 (C-8), 128.2 (C-5), 127 9 (C-7), 126.3 (C-5'), 125.8 (C-4), 125.5 (C-8a), 125.1 (C-7'), 124.4 (C-2), 119.7 (C-8'), 69 0 (CH₂O), 68.4 (CH₂O), 40 5 (CH₂N), 39.1 (CH₂N); Anal. calc. for C₂₅H₂₂N₆O₃.½H₂O: C, 64 8; H, 5.0; N, 18.1; found C, 65.2; H, 4.8; N, 18.4%.

### Example 132

**3-[(2-Methoxyethyl)amino]-2-quinoxalinecarbonitrile 1-oxide (123).** 2-Methoxyethylamine (0.32 mL, 3.0 mmol) was added to a stirred solution of 3-chloro-2-quinoxalinecarbonitrile 1-oxide **122** (Yoshida & Otomasu, *Chem. Pharm. Bull.* **1984,** 32, 3361) (203 mg, 1.0 mmol) in DME (50 mL) and the solution stirred at reflux temperature for 2 h. The solvent was evaporated and the residue partitioned between DCM (100 mL) and dilute aqueous NH₃ (50 mL). The organic fraction was dried and the solvent evaporated. The residue was chromatographed, eluting with 5% EtOAc/DCM, to give 1-oxide **123** (209 mg, 86%) as a yellow powder, mp (MeOH) 124-126 °C; ¹H NMR δ 8 28 (br d, *J* = 8.5 Hz, 1 H, H-8), 7.66-7.71 (m, 2 H, H-5, H-6), 7.36-7.42 (m, 1 H, H-7), 5.60 (br s, 1 H, NH), 3.78-3.84 (m, 2 H, CH₂N), 3.63-3.66 (m, 2 H, CH₂O), 3.42 (s, 3 H, OCH₃); ¹³C NMR δ 154.2 (C-3), 144.6 (C-4a), 134.1 (C-6), 132 6 (C-8a), 127.6 (C-5), 125.7 (C-7), 118.9 (C-8), 111.9 (C-2), 108.2 (CN), 70.4 (CH₂O), 59.0 (OCH₃), 41.2 (CH₂N); Anal. calc. for C₁₂H₁₂N₄O₂: C, 59.0; H, 5.0; N, 22.9; found C, 59.2; H, 5.2; N, 22.6%.

### Example 133

**1,2,4-Benzotriazine 1-oxide (124).** Isoamyl nitrite (1.05 mL, 7.8 mmol) was added to a stirred solution of 1-oxide **3** (254 mg, 1.6 mmol) in DMF (10 mL) and the solution stirred at 60 °C for 2 h. The solvent was evaporated and the residue partitioned between EtOAc (50 mL) and water (50 mL). The organic fraction was washed with water (2 × 25 mL), brine (20 mL), dried, and the solvent evaporated. The residue was chromatographed, eluting with a gradient (0-10%) of EtOAc/DCM, to give **124** (120 mg, 52%) as a pale yellow solid, mp (EtOAc/DCM) 138-139 °C [lit. (Robbins & Schofield, *J. Chem.* Soc. **1957, 3186)** mp (MeOH) 138-140 °C]; ¹H NMR δ 9.19 (s, 1 H, H-3), 8.41 (d, *J* = 8.6 Hz, 1 H, H-8), 8.10-8.13 (m, 2 H, H-5, H-6), 7.90-7.94 (m, 1 H, H-7).

### Examples 134 and 135

**1,2,4-Benzotriazin-3-amine 2-oxide (126) and 1,2,4-benzotriazin-3-amine 4-oxide (127).**
**1,2,4-Benzotriazin-3-amine (125).** A solution of 1-oxide **3** (1.98 g, 14.3 mmol) and Na₂S₂O₄ (4.99 g, 28.7 mmol) in 70% aqueous EtOH (100 mL) was heated at reflux temperature for 3 h. The hot suspension was filtered and the filtrate extracted with CHCl₃ (3 × 50 mL). The combined organic fraction was dried and the solvent evaporated The combined solid and extracts were chromatographed, eluting with a gradient (0-2%) of MeOH/CHCl₃, to give benzotriazine **125** (1.58 g, 67%) as a yellow solid, mp (CHCl₃/MeOH) 200-203 °C [lit. (Mason & Tennant, *J. Chem. Soc. (B)* **1970,** 911) mp 207 °C]; ¹H NMR δ 8.19 (dd, *J*= 8.3, 0.9 Hz, 1 H, H-8), 7.78-7.83 (m, 1 H, H-6), 7.62 (br s, 2 H, NH₂), 7.54 (d, *J =* 8.4 Hz, 1 H, H-5), 7.43-7.48 (m, 1 H, H-7).

**1,2,4-Benzotriazin-3-amine 2-oxide (126) and 1,2,4-benzotriazin-3-amine 4-oxide** (**127**). A solution of MCPBA (0 89 g, 3.6 mmol) in DCM (5 mL) was added dropwise to a stirred solution of **125** (0.50 g, 3.4 mmol) in 10% MeOH/DCM (50 mL) at 20 °C and the solution stirred at 20 °C for 3 h. The solution was washed with dilute aqueous NH₃ solution (50 mL), dried, and the solvent evaporated. The residue was chromatographed, eluting with a gradient (0-5%) of MeOH/CHCl₃, to give (i) 1,2,4-benzotriazin-3-amine 2-oxide (**126**) (405 mg, 70%) as a yellow powder, mp (MeOH/DCM) 175-180 °C [lit. (Mason & Tennant, J. *Chem. Soc. (B)* **1970**, 911) mp (HOAc) 200 °C]; ¹H NMR δ 8.20 (br s, 2 H, NH₂), 7.68 (d, J = 8.2 Hz, 1 H, H-8), 7.60-7.65 (m, 1 H, H-6), 7.53 (d, J = 7.6 Hz, 1 H, H-5), 7.45-7.59 (m, 1 H, H-7); followed by (ii) 1,2,4-benzotriazin-3-amine 1-oxide (3) (65 mg, 11%) as a yellow powder, mp 266-268 °C [lit (Arndt, Ber. 1913, 46, 3522) mp (EtOH) 269 °C]; spectroscopically identical with the sample prepared above; and (iii) 1,2,4-benzotriazin-3-amine 4-oxide (127) (51 mg, 9%) as pale yellow solid, [lit (Fuchs, et. al., *J*. *Org. Chem.* 2001, 66, 107)]; ¹H NMR δ 8.29 (d, *J* = 8.6 Hz, 1 H, H-8), 8.20 (br s, 2 H, NH₂), 8.16 (d, *J* = 8.6 Hz, 1 H, H-5), 7.91 (ddd, *J* = 8.6, 7.0, 1.2 Hz, 1 H, H-6), 7.65 (ddd, *J* = 8.6, 7.0, 1.1 Hz, 1 H, H-7); MS (EI) 162 (M⁺, 100%), 146 (10); HRMS (EI) calc. for C₇H₆N₄O (M⁺) m/z 162.0542, found 162.0540.

### Example 136

***tert*-Butyl bis{3-[(1-oxido-1,2,4-benzotriazin-3-yl)amino]propyL}carbamate (129).**
A solution of chloride **19** (0.86 g, 4.8 mmol), Et₃N (1.0 mL, 7.1 mmol) and *tert*-butyl bis(3-aminopropyl)carbamate (1.1 g, 4.8 mmol) in DCM was stirred at 20 °C for 3 days. The solvent was evaporated and the residue chromatographed, eluting with 20% EtOAc/DCM, to give bis-1-oxide **129** (457 mg, 18%) as a yellow oil, ¹H NMR δ 8.24 (d, *J* = 8 4 Hz, 2 H, 2 × H-8'), 7.65-7.69 (m, 2 H, 2 × H-6'), 7.56 (br d, *J* = 8.5 Hz, 2 H, 2 × H-5'), 7.23-7.28 (m, 2 H, 2 × H-7'), 6.26 (br s, 2 H, 2 × NH), 3.54-3.57 (m, 4 H, 2 × CH₂N), 2.32-2.39 (m, 4 H, 2 × CH₂N), 1.85-1.93 (m, 4 H, 2 × 2 × CH₂), 1.49 [s, 9 H, C(CH₃)₃]; ¹³C NMR δ 158 9 (2 × C-3'), 156.1 (NCO₂), 148.9 (2 × C-4a'), 135.4 (2 × C-6'), 130 9 (2 × C-8a'), 126.4 (2 × C-5'), 124.9 (2 × C-7'), 120.4 (2 × C-8'), 80.2 [OC(CH₃)₃], 60.4 (2 × CH₂N), 46.0 (2 x NCH₂), 28.5 (2 × CH₂), 28.4 [OC(CH₃)₃]; Anal. calc for C₂₅H₃₁N₉O₄: C, 57.6; H, 6.0; found C, 57.1; H, 6.1%.

### Example 137

***N***^{**1**}**-Methyl-*N***^{**2**}**-(1 -oxido-1,2,4-benzotriazin-3-yl)-1,2-ethanediamine (130).** *N*-Methylethanediamine (0.73 mL, 8.3 mmol) was added to a stirred solution of chloride 19 (1.0 g, 5.5 mmol) and Et₃N (1.2 mL, 8.3 mmol) in DCM (50 mL) and the solution stirred at 20 °C for 2 days. The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-10%) of MeOH/DCM, to give 1-oxide **130** (600 mg, 50%) as a yellow solid, mp (MeOH/EtOAc) 226-228 °C; ¹H NMR [(CD₃)₂SO] δ 8.40 (br s, 1 H, NH), 8.17 (dd, *J* = 8.6, 1.0 Hz, 1 H, H-8'), 7.84 (ddd, *J* = 8.4, 7.0, 1.0 Hz, 1 H, H-6'), 7.64 (br d, *J* = 8.4 Hz, 1 H, H-5'), 7.40 (ddd, *J* = 8.6, 7.0, 1.0 Hz, 1 H, H-7'), 3.93-3.97 (m, 2 H, CH₂N), 3.53 [br s, 1 H, NH), 3.21 (s, 3 H, NCH3), 3.13-3.17 (m, 2 H, CH₂N), ¹³C NMR [(CD₃)₂SO) δ 158.3 (C-3'), 148.2 (C-4a'), 135.9 (C-6'), 129.3 (C-8a'), 126 2 (C-5'), 125.2 (C-7'), 119.8 (C-8'), 45.5 (CH₂N), 36.7 (CH₂N), 35.6 (NCH₃).

### Example 138

**3-Chloro-6-fluoro-1,2,4-benzotriazine 1-Oxide (131).** NaNO₂ (4.26 g, 61.7 mmol) was added in small portions to a stirred solution of amine **3t** (5.56 g, 30.9 mmol) in trifluoroacetic acid (60 mL) at 5 °C and the solution stirred at 20 °C for 3 h. The solution was poured into ice/water, stirred 30 minutes, filtered, washed with water (3 × 30 mL) and dried. The solid was suspended in POCl₃ (80 mL) and DMF (0.5 mL) and stirred at 100 °C for 1 h. The solution was cooled, poured into ice/water, stirred for 30 minutes, filtered, washed with water (3 × 30 mL) and dried. The solid was suspended in DCM (150 mL), dried and the solvent evaporated. The residue was chromatographed, eluting with 5% EtOAc/DCM, to give chloride **131** (2.78 g, 45%) as a pale yellow solid, mp (EtOAc/DCM) 166-168 °C; ¹H NMR δ 8.45 (dd, *J* = 9.5, 5.3 Hz, 1 H, H-8), 7.61 (dd, *J* = 8.3, 2.6 Hz, 1 H, H-5), 7.45-7.52 (m, 1 H, H-7); ¹³C NMR δ 167.1 (q, *J* = 264 Hz), 158.4, 149.2, 131.0, 123.4 (d, *J* = 11 Hz), 120.1 (d, *J* = 26 Hz), 112.9 (d, *J* = 23 Hz). Anal. calcd for C₇H₃ClFN₃O: C, 42.1; H, 1.5; N, 21.1; Cl, 17 8; found C, 42.4; H, 1.6; N, 21.2; Cl, 17.8%.

### Example 139

***N***^{**1**}**-(6-Fluoro-1-oxido-1,2,4-benzotriazin-3-yl)-*N***^{**2**}**,*N***^{**2**}**-dipropyl-1,2-ethanediamine (132).** N,N-Dipropylethanediamine (0.76 g, 5.3 mmol) was added to a stirred solution of chloride **131** (455 mg, 2.1 mmol) in DME (50 mL) and the solution stirred at reflux temperature for 2 h. The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0―10%) of MeOH/DCM, to give 1-oxide **132** (645 mg, 96%) as a yellow solid, mp (MeOH) 77-79 °C; ¹H NMR δ 8.18 (d, *J* = 9.1 Hz, 1 H, H-8), 7.58 (d, *J* = 2 1 Hz, 1 H, H-5), 7 19 (dd, *J* = 9.1, 2.1 Hz, 1 H, H-7), 6.04 (br s, 1 H, NH), 3.47-3.52 (m, 2 H, CH₂N), 2.69 (dd, *J* = 6.0, 5.8 Hz, 2 H, CH₂N), 2.38-2.44 (m, 4 H, 2 × CH₂N), 1.42-1.51 (m, 4 H, 2 × CH₂), 0.89 (t, *J* = 7.3 Hz, 6 H, 2 × CH₃); ¹³C NMR δ 159.4, 149 6, 141.9, 129.4, 124.5 (2), 121.9, 55 8 (2), 52.4, 38.9, 20.3 (2), 11.9 (2). Anal. calcd for C₁₅H₂₂CIN₅O: C, 55.6; H, 6.9; N, 21.6; found C, 55.8; H, 7.0; N, 21.7%.

### Example 140

**3,6-Dich)oro-1,2,4-benzotriazine 1-Oxide (133). ).** NaNO₂ (2.78 g, 40.3 mmol) was added in small portions to a stirred solution of amine **3u** (3.96 g, 20.1 mmol) in trifluoroacetic acid (70 mL) at 5 °C and the solution stirred at 20 °C for 3 h. The solution was poured into ice/water, stirred 30 minutes, filtered, washed with water (3 × 30 mL) and dried. The solid was suspended in POCl₃ (60 mL) and DMF (0.5 mL) and stirred at 100 °C for 1 h. The solution was cooled, poured into ice/water, stirred for 30 minutes, filtered, washed with water (3 × 30 mL) and dried. The solid was suspended in DCM (150 mL), dried and the solvent evaporated. The residue was chromatographed, eluting with 5% EtOAc/DCM, to give dichloride 133 (3.59 g, 83%) as a pale yellow solid, mp (DCM) 149-151 °C; ¹H NMR δ 8.36 (d, *J* = 9.2 Hz, 1 H, H-8), 7.98 (d, *J* = 2.1 Hz, 1 H, H-5), 7.69 (dd, *J* = 9.2, 2.1 Hz, 1 H, H-7); ¹³C NMR δ 158.3, 147.8, 143.7, 132.4, 131.9, 127.5, 121.7. Anal calcd for C₇H₃Cl₂N₃O: C, 38.9; H, 1 4; N, 19.5; Cl, 32.8, found C, 39.1; H, 1.2; N, 19.6; Cl, 32.9%.

### Example 141

***N***^{**1**}**-(6-Chloro-1-oxido-1,2,4-benzotriazin-3-yl)-*N***^{**2**}**,*N***^{**2**}**-diethyl-1,2-ethanediamine (134).** *N*,*N*-Diethylethanediamine (0.55 mL, 3 9 mmol) was added to a stirred solution of chloride 133 (425 mg, 2.0 mmol) in DME (50 mL) and the solution stirred at reflux temperature for 2 h. The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-10%) of MeOH/DCM, to give 1-oxide **134** (566 mg, 97%) as a yellow solid, mp (MeOH) 128-130 °C; ¹H NMR δ 8.18 (d, *J* = 9.1 Hz, 1 H, H-8), 7.58 (br s, 1 H, H-5), 7.19 (dd, *J* = 9.1, 2.1 Hz, 1 H, H-7), 6.09 (br s, 1 H, NH), 3.48-3 53 (m, 2 H, CH₂N), 2.68 (dd, *J* = 6.1, 5.9 Hz, 2 H, CH₂N), 2.56 (q, *J* = 7.1 Hz, 4 H, 2 × CH₂N), 1 03 (t, *J* = 7 1 Hz, 6 H, 2 × CH₃); ¹³C NMR δ 158.9, 149 0, 141.4, 128.9, 124.8(2), 121 4, 50.5, 46.0 (2), 38.2, 11.3 (2). Anal. calcd for C₁₃H₁₈ClN₅O: C, 52.8; H, 6.1; N, 23.7; found C, 53.1; H, 6.3; N, 23.9%.

### Example 142

**6-Chloro-*N*-[2-(1-piperidinyl)ethyl]-1,2,4-benzotriazin-3-amine 1-Oxide (135).** *N*,*N*-2-(1-Piperidinyl)ethanamine (0.62 mL, 4.4 mmol) was added to a stirred solution of chloride **133** (382 mg, 1 8 mmol) in DME (50 mL) and the solution stirred at reflux temperature for 2 h. The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-10%) of MeOH/DCM, to give 1-oxide **135** (518 mg, 95%) as a yellow solid, mp (MeOH) 178-181 °C; ¹H NMR δ 8.17 (d, *J* = 9.1 Hz, 1 H, H-8), 7 57 (br s, 1 H, H-5), 7.18 (dd, *J* = 9.1, 2.1 Hz, 1 H, H-7), 6.15 (br s, 1 H, NH), 3.51-3.58 (m, 2 H, CH₂N), 2 57 (dd, *J* = 6.0, 5.9 Hz, 2 H, CH₂N), 2.39-2.45 (m, 4 H, 2 x CH₂N), 1.55-1.61 (m, 4 H, 2 × CH₂), 1.42-1.48 (m, 2 H, CH₂); ¹³C NMR δ 159.4, 149.6, 141.9, 129.4, 125.4 (2), 121.9, 56.7, 54.3 (2), 37.9, 26.0 (2), 24.4. Anal. calcd for C₁₄H₁₈ClN₅O: C, 54.6; H, 5.9; N, 22.7; found C, 54.6; H, 5.9; N, 22.7%.

### Example 143

***N***^{**1**}**-(6-Chloro-1-oxido-1,2,4-benzotriazin-3-yl)-*N***^{**2**}**,*N***^{**2**}**-dipropyl-1,2-ethanediamine (136).** *N,N*-Dipropylethanediamine (0.76 g, 5.3 mmol) was added to a stirred solution of chloride **133** (455 mg, 2.1 mmol) in DME (50 mL) and the solution stirred at reflux temperature for 2 h. The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-10%) of MeOH/DCM, to give 1-oxide **136** (645 mg, 96%) as a yellow solid, mp (MeOH) 77-79 °C; ¹H NMR δ 8.18 (d, *J* = 9.1 Hz, 1 H, H-8), 7 58 (d, *J* = 2.1 Hz, 1 H, H-5), 7.19 (dd, *J* = 9.1, 2.1 Hz, 1 H, H-7), 6.04 (br s, 1 H, NH), 3.47-3.52 (m, 2 H, CH₂N), 2 69 (dd, *J* = 6.0, 5 8 Hz, 2 H, CH₂N), 2.38-2.44 (m, 4 H, 2 × CH₂N), 1.42-1.51 (m, 4 H, 2 × CH₂), 0.89 (t, *J* = 7.3 Hz, 6 H, 2 × CH₃); ¹³C NMR δ 159.4, 149.6, 141.9, 129.4, 124 5 (2), 121.9, 55.8 (2), 52.4, 38.9, 20.3 (2), 11.9 (2). Anal. calcd for C₁₅H₂₂CIN₅O: C, 55.6; H, 6.9; N, 21.6; found C, 55.8; H, 7.0; N, 21.7%.

### Example 144

***N***^{**1**}**,*N***^{**1**}**-Diethyl-*N***^{**2**}**-(6-methyl-1-oxido-1,2,4-benzotriazin-3-yl)-1,2-ethanediamine (137).** N,N-Diethylethanediamine (0.68 mL, 4.9 mmol) was added to a stirred solution of chloride **73** (380 mg, 1 9 mmol) in DME (50 mL) and the solution stirred at reflux temperature for 2 h. The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-10%) MeOH/DCM, to give 1-oxide 137 (502 mg, 94%) as a yellow solid, mp (MeOH/EtOAc) 78-80 °C; ¹H NMR δ 8.13 (d, *J* = 8.7 Hz, 1 H, H-8), 7 36 (br s, 1 H, H-5), 7.08 (dd, *J* = 8.7, 1.6 Hz, 1 H, H-7), 5.98 (br s, 1 H, NH), 3.50-3.55 (m, 2 H, CH₂N), 2.60-2.73 (m, 2 H, CH₂N), 2.59 (q, *J* = 7.1 Hz, 4 H, 2 × CH₂N), 2 46 (s, 3 H, CH₃), 1.05 (t, *J* = 7.1 Hz, 6 H, 2 × CH₃); ¹³C NMR δ 159.1, 149.1, 146.9, 129.1, 126.9, 125.3, 120.1, 51.2, 46.7 (2), 38.7, 22.0, 11.7 (2). Anal. calcd for C₁₄H₂₁N₅O: C, 61 1; H, 7.7; N, 25.4; found C, 60.8 H, 8.0; N, 25.2%.

### Example 145

**6-Methyl-*N*-[2-(4-morpholinyl)ethyl]-1,2,4-benzotriazin-3-amine 1-Oxide (138).** 2-(1-Morpholinyl)ethylamine (1.57 mL, 12.0 mmol) was added to a stirred solution of chloride **73** (781 mg, 4.0 mmol) in DME (80 mL) and the solution stirred at reflux temperature for 2 h. The solvent was evaporated and the residue partitioned between DCM (100 mL) and dilute aqueous NH₃ (50 mL). The organic fraction was dried and the solvent evaporated. The residue was chromatographed, eluting with a gradient (0-10%) of MeOH/DCM, to give 1-oxide **138** (1.01 g, 87%) as a yellow powder, mp (EtOAc) 182-184 °C; ¹H NMR δ 8.13 (d, *J* = 88 Hz, 1 H, H-8), 7.36 (br s, 1 H, H-5), 7.10 (dd, *J* = 8.8, 1.6 Hz, 1 H, H-7), 5 85 (br s, 1 H, NH), 3.70-3.74 (m, 4 H, 2 × CH₂O), 3.54-3.59 (m, 2 H, CH₂N), 2.60-2.64 (m, 2 H, CH₂N), 2.48-2.52 (m, 4 H, 2 × CH₂N), 2 46 (s, 3 H, CH₃); ¹³C NMR δ 159.1, 149 1, 147.2, 129.2, 127.1, 125.3, 120.2, 67.0 (2), 56.7, 53.3 (2), 37.5, 22.0 Anal. calcd for C₁₄H₁₉N₅O₂: C, 58.1; H, 6.6; N, 24 2; found C, 58.1; H, 6.7; N, 24 2%.

### Example 146

**6-Methyl-N-[3-(4-morpholinyl)propyl]-1,2,4-benzotriazin-3-amine 1-Oxide (139).**
3-(1-Morpholinyl)propylamine (0.73 mL, 5.0 mmol) was added to a stirred solution of chloride **73** (327 mg, 1.7 mmol) in DME (50 mL) and the solution stirred at reflux temperature for 4 h. The solvent was evaporated and the residue partitioned between DCM (100 mL) and dilute aqueous NH₃ (50 mL). The organic fraction was dried and the solvent evaporated. The residue was chromatographed, eluting with a gradient (0-10%) of MeOH/DCM, to give 1-oxide **139** (347 mg, 68%) as a yellow powder, mp (EtOAc) 131-132 °C; ¹H NMR δ 8.13 (d, *J* = 8.8 Hz, 1 H, H-8), 7.36 (br s, 1 H, H-5), 7.09 (dd, *J* = 8.8, 1 6 Hz, 1 H, H-7), 6.25 (br s, 1 H, NH), 3.75-3.77 (m, 4 H, 2 × CH₂O), 3.57-3.62 (m, 2 H, CH₂N), 2.46-2.53 (m, 9 H, 3 × CH₂N, CH₃), 1.81-1.87 (m, 2 H, CH₂N); ¹³C NMR δ 159.2, 146.9, 147.0, 129.5, 126.9, 125.4, 120.2, 67.0 (2), 57.3, 53.8 (2), 40.9, 25.2, 22.0. Anal. calcd for C₁₅H₂₁N₅O₂: C, 59.4; H, 7.0; N, 23.1; found C, 59.5; H, 7.0; N, 22.8%.

### Example 147

***N***^{**1**}**-(6-Methyl-1-oxido-1,2,4-benzotriazin-3-yl)-*N***^{**2**}**,*N***^{**2**}**-dipropyl-1,2-ethanediamine** (140). *N,N*-dipropylethylenediamine (0.83 mL, 5.7 mmol) was added to a stirred solution of chloride 73 (448 mg, 2.3 mmol) in DME (50 mL) and the solution stirred at reflux temperature for 3 h. The solvent was evaporated and the residue partitioned between DCM (100 mL) and dilute aqueous NH₃ (50 mL). The organic fraction was dried and the solvent evaporated. The residue was chromatographed, eluting with a gradient (0-6%) of MeOH/DCM, to give 1-oxide **140** (697 mg, 100%) as a yellow powder, mp (EtOAc/pet ether) 88-90 °C; ¹H NMR δ 8.13 (d, *J =* 8.8 Hz, 1 H, H-8), 7.36 (br s, 1 H, H-5), 7.08 (dd, *J* = 8.8, 1.8 Hz, 1 H, H-7), 5.89 (br s, 1 H, NH), 3.48-3.52 (m, 2 H, CH₂N), 2.66-2.69 (m, 2 H, CH₂N), 2.46 (s, 3 H, CH₃), 2.39-2.43 (m, 4 H,2×CH₂N), 1.41-1.50 (m, 4 H, 2 × CH₂), 0.88 (t, *J* = 7.3 Hz, 6 H, 2 × CH₃); ¹³C NMR δ 159.2, 149.1, 146.8, 129.1, 126.9, 125.3, 120.2, 55.9 (2), 52.5, 38.9, 22.0, 20.3 (2), 11.9 (2). Anal. calcd for C₁₆H₂₅N₅O: C, 63.3; H, 8.3; N, 23.0; found C, 63.3; H, 8.6; N, 23.3%.

### Example 148

***N*-[2-(2,6-Dimethyl-1-piperidinyl)ethyl]- 6-methoxy-1,2,4-benzotriazin-3-amine 1-oxide (141).** 2-(2,6-Dimethyl-1-piperidinyl)ethylamine (580 mg, 3.7 mmol) was added to a stirred solution of chloride **83** (314 mg, 1.5 mmol) in DME (40 mL) and the solution stirred at reflux temperature for 4 h The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-10%) MeOH/DCM, to give 1-oxide **141** (416mg, 85%) as a yellow solid, mp (MeOH) 170-171 °C; ¹H NMR δ 8.15 (d, *J* = 9.4 Hz, 1 H, H-8), 6.87 (dd, *J* = 9.4, 2.6 Hz, 1 H, H-7), 6.83 (d, *J* = 2.6 Hz, 1 H, H-5), 5.60 (br s, 1 H, NH), 3.93 (s, 3 H, OCH₃), 3.51-3.56 (m, 2 H, CH₂N), 2.87-2 91 (m, 2 H, CH₂N), 2.49-2 57 (m, 2 H, 2 × CHN), 1.65-1.70 (m, 1 H, CH₂), 1.53-1.59 (m, 2 H, CH₂), 1.23-1.38 (m, 3 H, CH₂), 1.19 (d, *J =* 6.3 Hz, 6 H, 2 x CH₃); ¹³C NMR δ 165.4, 159.3, 151.5, 126.0, 122.0, 117.7, 104.0, 57.2, 56.0 (2), 47.4, 39.4, 34.2 (2), 24.4, 22.0 (2). Anal. calcd for C₁₇H₂₅N₅O₂: C, 61.6; H, 7.6; N, 21.1; found C, 61.6: H, 7.6; N, 21.1%.

### Example 149

**3-Chloro-6-trifluoromethyl-1,2,4-benzotriazine 1-Oxide (142)**. Sodium nitrite (690 mg, 10.0 mmol) was added in portions to a stirred solution of 1-oxide **3v** (1.15 g, 6.6 mmol) in trifluoroacetic acid (50 mL) at 5 °C and the solution stirred at 20 °C for 1 h. The solution was poured into ice/water, filtered, washed with water (2 x 50 mL) and dried. The solid was suspended in POCl₃ (20 mL), DMF (2 drops) added, and the mixture stirred at 100 °C for 3 h. The solution was poured into ice/water, stirred for 20 minutes and filtered. The solid was dissolved in DCM (150 mL), dried, and the solvent evaporated. The residue was purified by chromatography, eluting with 5% EtOAc/DCM, to give chloride **142** (375 mg, 33%) as a pale yellow solid, mp (DCM/pet. ether) 118-120 °C; ¹H NMR [(CD₃)₂SO] δ 8.52-8.57 (m, 2 H, H-5, H-8), 8.15 (dd, *J* = 9.0, 1.8 Hz, 1 H, H-7); ¹³ C NMR [(CD₃)₂SO] δ 156.6, 146.6, 135.8, 135.5 (q, *J* = 33 Hz), 126.6 (q, *J* = 3 Hz), 126.1 (q, *J* = 4 Hz), 126.6 (q, *J* = 274 Hz), 122.0. Anal. calcd for C₈H₃ClF₃N₃O: C, 38.5; H, 1.2; N, 16.8; F, 22.8; found C, 38.5; H, 1.1; N, 16.7; F, 14.4%.

### Example 150

***N***^{**1**}**-(6-Trifluoromethyl-1-oxido-1,2,4-benzotriazin-3-yl)-*N***^{**2**}**,*N***^{**2**}**-dimethyl-1,2-ethanediamine (143).** *N,N*-Dimethyl-1,2-ethanediamine (0.5 mL, 4.6 mmol) was added to a stirred solution of chloride **142** (305 mg, 1.2 mmol) in DME (20 mL) and the solution stirred at reflux temperature for 3 h. The solvent was evaporated and the residue partitioned between DCM (100 mL) and dilute aqueous NH₃ (50 mL). The organic fraction was dried and the solvent evaporated. The residue was purified by chromatography, eluting with a gradient (0-5%) of MeOH/DCM, to give the amine **143** (367 mg, 100%) as a yellow solid, mp (MeOH/DCM) 159-161 °C; ¹H NMR δ 8.33 (d, *J* = 8 9 Hz, 1 H, H-8), 7 87 (br s, 1 H, H-5), 7.40 (dd, *J* = 8.9, 1.5 Hz, 1 H, H-7), 6.24 (br s, 1 H, NH), 3.53-3.58 (m, 2 H, CH₂N), 2.57-2.60 (m, 2 H, CH₂N), 2.28 [s, 6 H, N(CH₃)₂]; ¹³C NMR δ 159.4, 148.5, 136.8 (q, *J* = 33 Hz), 132.0, 127.1, 124.3 (q, *J* = 4 Hz), 121.9, 120.0 (q, *J* = 3 Hz), 57.3, 45.0 (2), 38.7. Anal. calcd for C₁₂H₁₄F₃N₅O: C, 47.8; H, 4.7; N, 23.3; found C, 48.0; H, 4.4; N, 23.1%.

### Example 151

**6-Isopropyl-1,2,4-benzotriazin-3-amine 1-oxide (145).** A mixture of 5-isopropyl-2-nitroaniline **(144)** (Prasad, J. V. N. V. *Org. Lett.* **2000,** 2, 1069) (4.53 g, 25.1 mmol) and cyanamide (4.23 g, 100 mmol) were mixed together at 100 °C, cooled to 50 °C, cHCl (15 mL) added carefully and the mixture heated at 100 °C for 4 h. The mixture was cooled to 50 °C, 7.5 M NaOH solution added until the mixture was strongly basic and the mixture stirred at 100 °C for 3 h. The mixture was cooled, diluted with water (200 mL), filtered, washed with water (3 × 50 mL), washed with ether (3 x 30 mL) and dried. The residue was chromatographed, eluting with a gradient (0-10%) of MeOH/DCM, to give 1-oxide **145** (2.64 g, 52%) as a yellow powder, mp (CHCl₃) 220-222 °C; ¹H NMR [(CD₃)₂SO] δ 8.04 (d, *J* = 8.9 Hz, 1 H, H-8), 7.32 (d, *J* = 1.8 Hz, 1 H, H-5), 7.27 (dd, *J* = 8.9, 1.8 Hz, 1 H, H-7), 7.24 (br s, 2 H, NH₂), 3.01 (sept, *J* = 6.9 Hz, 1 H, CH), 1.24 (d, *J* = 6.9 Hz, 6 H, 2 × CH₃); ¹³C NMR [(CD₃)₂SO] δ 160.3, 156.7, 149.0, 128.3, 124.2, 121.9, 119.7, 33.5, 22.9 (2).

### Example 152

**3-Chloro-6-isopropyf-1,2,4-benzotriazine 1-oxide (146).** Sodium nitrite (1.71 g, 12.4 mmol) was added in small portions to a stirred solution of amine **145** (2.53 g, 12.4 mmol) in trifluoroacetic acid (80 mL) at 5 °C and the solution stirred at 20 °C for 3 h. The solution was poured into ice/water, stirred 30 minutes, filtered, washed with water (3 × 30 mL) and dried. The solid was suspended in POCl₃ (70 mL) and DMF (0.5 mL) and stirred at 100 °C for 1 h The solution was cooled, poured into ice/water, stirred for 30 minutes, filtered, washed with water (3 × 30 mL) and dried. The solid was suspended in DCM (150 mL), dried and the solvent evaporated. The residue was chromatographed, eluting with 5% EtOAc/DCM, to give chloride **146** (1.74 g, 63%) as a pale yellow solid, mp (EtOAc/DCM) 81-83 °C; ¹H NMR δ 8.32 (d, *J* = 8.9 Hz, 1 H, H-8), 7.78 (d, *J* = 1.8 Hz, 1 H, H-5), 7.63 (dd, *J* = 8.9, 1.8 Hz, 1 H, H-7), 3.15 (sept, *J* = 6 9 Hz, 1 H, CH), 1 35 (d, *J* = 6.9 Hz, 6 H, 2 × CH₃); ¹³C NMR δ 159.4, 157.0, 147.8, 132.2, 131.0, 124.6, 120.1, 34.6, 23.2(2). Anal. calcd for C₁₀H₁₀ClN₃O: C, 53.7; H, 4.5; N, 18.8; found C, 53.7; H, 4.4; N, 19.0%.

### Example 153

***N***^{**1**}**-(6-Isopropyl-1-oxido-1,2,4-benzotriazin-3-yl)-*N***^{**2**}**,*N***^{**2**}**-dimethyl-1,2-ethanediamine (147).** *N*,*N*-Dimethylethanediamine (0.37 mL, 3.4 mmol) was added to a stirred solution of chloride **146** (300 mg, 1.3 mmol) in DME (50 mL) and the solution stirred at reflux temperature for 2 h. The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-10%) of MeOH/DCM, to give 1-oxide **147** (348 mg, 94%) as a yellow solid, mp (MeOH/EtOAc) 87-89 °C; ¹H NMR δ 8.16 (d, *J* = 8.9Hz, 1 H, H-8), 7.40 (br s, 1 H, H-5), 7.16 *(dd, J =* 8.9, 1.7 Hz, 1 H, H-7), 5.91 (br s, 1 H, NH), 3.52-3.56 (m, 2 H, CH₂N), 3.00 (sept, *J* = 6.9 Hz, 1 H, CH), 2.54-2 58 (m, 2 H, CH₂N), 2.27 [s, 6 H, N(CH₃)₂], 1.29 (d, *J* = 6.9 Hz, 6 H, 2 × CH₃); ¹³C NMR δ 159.2, 157.5, 149.3, 129.3, 124.8, 122.6, 120.3, 57.5, 45.1 (2), 38.7, 34.5, 22.3 (2). Anal calcd for C₁₄H₂₁N₅O·¼CH₃OH: C, 60.4; H, 7.8; N, 24.7; found C, 60.5: H, 7.9; N, 25.0%.

### Example 154

***N***^{**1**}**-(6-Isopropyl-1-oxido-1,2,4-benzotriazin-3-yl)-*N***^{**2**}**,*N***^{**2**}**-diethyl-1,2-ethanediamine (148).** *N,N*-Diethylethanediamine (0.47 mL, 3.4 mmol) was added to a stirred solution of chloride **146** (308 mg, 1.4 mmol) in DME (50 mL) and the solution stirred at reflux temperature for 2 h. The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-10%) of MeOH/DCM, to give 1-oxide **148** (390 mg, 93%) as a yellow solid, mp (MeOH/EtOAc) 60-62 °C; ¹H NMR δ 8.16 (d, *J* = 8.9 Hz, 1 H, H-8), 7.40 (br s, 1 H, H-5), 7.16 (dd, *J* = 8.9, 1.8 Hz, 1 H, H-7), 5.98 (br s, 1 H, NH), 3.50-3.55 (m, 2 H, CH₂N), 3 00 (sept, *J* = 6.9 Hz, 1 H, CH), 2.71 (dd, *J* = 6.0, 5.9 Hz, 2 H, CH₂N), 2.58 (q, *J* = 7.1 Hz, 4 H, 2 × CH₂), 1.30 (d, *J* = 6.9 Hz, 6 H, 2 × CH₃), 1 04 (t, *J* = 7.1 Hz, 6 H, 2 x CH₃); ¹³C NMR δ 159.1, 157.5, 149.3, 129 3, 124.8, 122.6, 120.3, 51.2, 46.6 (2), 38.7, 34.5, 23.3 (2), 11.7 (2). Anal. calcd for C₁₆H₂₅N₅O^{.}½H₂O: C, 62.4; H, 8.4; N, 22.7; found C, 62.5: H, 8.5; N, 22.9%.

### Example 155

**6-Isopropyl-N-[2-(4-morpholinyl)ethyl]-1,2,4-benzotriazin-3-amine 1 -Oxide (149).** 2-(4-Morpholinyl)ethylamine (0.45 mL, 3.4 mmol) was added to a stirred solution of chloride **146** (306 mg, 1.4 mmol) in DME (50 mL) and the solution stirred at reflux temperature for 2 h. The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-10%) of MeOH/DCM, to give 1-oxide **149** (429 mg, 99%) as a yellow solid, mp (MeOH) 145-147 °C; ¹H NMR δ 8.17 (d, *J* = 8.9 Hz, 1 H, H-8), 7.40 (br s, 1 H, H-5), 7.17 (dd, *J* = 8.9, 1 8 Hz, 1 H, H-7), 5.86 (br s, 1 H, NH), 3.69-3 74 (m, 4 H, 2 × CH₂O), 3.56-3.60 (m, 2 H, CH₂N), 3.01 (sept, *J* = 6.9 Hz, 1 H, CH), 2.63 (dd, *J* = 6.0, 5.8 Hz, 2 H, CH₂N), 2.48-2.53 (m, 4 H, 2 × CH₂N), 1.30 (d, *J* = 6.9 Hz, 6 H, 2 × CH₃); ¹³C NMR δ 159.0, 157.6, 149.2, 129.3, 125.0, 122.6, 120.3, 67.0 (2), 56.7, 53.3 (2), 37.5, 34.5, 23 3 (2). Anal. calcd for C₁₆H₂₃N₅O₂·¼H₂O: C, 59.7; H, 7.4; N, 21.8; found C, 59.7: H, 7.5; N, 21.8%.

### Example 156

**6-isopropyl-*N*-[2-(1-piperidinyl)ethyl]-1,2,4-benzotriazin-3-amine 1-Oxide (150).** 2-(1-Piperidinyl)ethylamine (0.47 mL, 3.4 mmol) was added to a stirred solution of chloride **146** (297 mg, 1.3 mmol) in DME (50 mL) and the solution stirred at reflux temperature for 2 h. The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-10%) of MeOH/DCM, to give 1-oxide **150** (394 mg, 94%) as a yellow solid, mp (MeOH/EtOAc) 124-127 °C; ¹H NMR δ 8.16 (d, *J* = 8.9 Hz, 1 H, H-8), 7.41 (br s, 1 H, H-5), 7.15 (dd, *J* = 8.9, 1.8 Hz, 1 H, H-7), 5.79 (br s, 1 H, NH), 3.51-3.55 (m, 2 H, CH₂N), 3.00 (sept, *J* = 6.9 Hz, 1 H, CH), 2.56 (dd, *J* = 6.1, 5.9 Hz, 2 H, CH₂N), 2.39-2.44 (m, 4 H, 2 × CH₂), 1.54-1.60 (m, 4 H, 2 × CH₂), 1.41-1.47 (m, 2H, CH₂), 1.30 (d, *J* = 6.9 Hz, 6 H, 2 × CH₃); ¹³C NMR δ 159.1, 157.5, 149.3, 129.3, 124.8, 122.6, 120.3, 56.8, 54.3 (2), 37.9, 34.5, 26 0 (2), 24.4, 23.3 (2). Anal. calcd for C₁₇H₂₅N₅O: C, 64.7; H, 8.0; N, 22.2; found C, 64.5: H, 8.3; N, 22.4%.

### Example 157

***N***^{**1**}**-(6-Isopropyl-1-oxido-1,2,4-benzotriazin-3-yl)-*N***^{***2***}***N***^{**2**}**-dipropyl-1,2-ethanediamine (151).** *N*¹,*N*¹-Dipropyl-1,2-ethanediamine (0.49 mL, 3.4 mmol) was added to a stirred solution of chloride 146 (305 mg, 1.4 mmol) in DME (50 mL) and the solution stirred at reflux temperature for 2 h. The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-5%) of MeOH/DCM, to give 1-oxide **151** (434 mg, 96%) as a yellow solid, mp (MeOH) 79-81 °C; ¹H NMR δ 8.17 (d, *J* = 8.9 Hz, 1 H, H-8), 7.41 (br s, 1 H, H-5), 7.16 (dd, *J* = 8.9, 1.8 Hz, 1 H, H-7), 5.89 (br s, 1 H, NH), 3.48-3.53 (m, 2 H, CH₂N), 3.00 (sept, *J* = 6.9 Hz, 1 H, CH), 2.67 (dd, *J* = 6.0, 5.8 Hz, 2 H, CH₂N), 2.39-2.45 (m, 4 H, 2 × CH₂N), 1.41-1.50 (m, 4 H, 2 × CH₂), 1.30 (d, *J* = 6.9 Hz, 6 H, 2 × CH₃), 0.88 (t, *J* = 7.3 Hz, 6 H, 2 × CH₃); ¹³C NMR δ 159.2, 157.5, 149.3, 129.3, 124 8, 122.6, 120.3, 55.9 (2), 52.5, 38.9, 34.5, 23.3 (2), 20.4 (2), 11.9 (2). Anal. calcd for C₁₈H₂₉N₅O·¼H₂O: C, 64.4; H, 8.9; N, 20.9; found C, 64.6; H, 8.9; N, 21.3%.

### Example 158

**6-*tert*-Butyl-1,2,4-benzotriazin-3-amine 1-Oxide (153).** A mixture of 4-*tert*-butyl-2-nitroaniline **(152)** (Seko, S.; et. al. J. *Chem. Soc. Perkin Trans. 1* **1999**, 1437.) (4.11 g, 21.2 mmol) and cyanamide (3.56 g, 84.6 mmol) were mixed together at 100 °C, cooled to 50 °C, cHCl (15 mL) added carefully and the mixture heated at 100 °C for 4 h. The mixture was cooled to 50 °C, 7.5 M NaOH solution added until the mixture was strongly basic and the mixture stirred at 100 °C for 3 h. The mixture was cooled, diluted with water (200 mL), filtered, washed with water (3 x 50 mL), washed with ether (3 x 30 mL) and dried. The residue was chromatographed, eluting with a gradient (0-10%) of MeOH/DCM, to give 1-oxide **153** (2.56 g, 62%) as a yellow powder, mp (MeOH/EtOAc) 209-212 °C; ¹H NMR [(CD₃)₂SO] δ 8.04 (d, *J* = 9.0 Hz, 1 H, H-8), 7.45 (dd, *J* = 9.0, 2.0 Hz, 1 H, H-7), 7.22 (br s, 2 H, NH₂), 7.39 (d, *J* = 2.0 Hz, 1 H, H-5), 1.32 [s, 9 H, C(CH₃)₃]; ¹³C NMR [(CD₃)₂SO] δ 160.4, 158.9, 148.8, 127.9, 123.4, 120.8, 119.4, 35.0, 30.2 (3). Anal. calcd for C₁₁H₁₄N₄O: C, 60.5; H, 6.5; N, 25.7; found C, 60.8; H, 6.6; N, 25.8%.

### Example 159

**6-*tert*-Butyl-3-chloro-1,2,4-benzotriazine 1-oxide (154).** Sodium nitrite (285 mg, 4 1 mmol) was added in small portions to a stirred solution of amine **153** (0.45 g, 2.1 mmol) in trifluoroacetic acid (20 mL) at 5 °C and the solution stirred at 20 °C for 3 h. The solution was poured into ice/water, stirred 30 minutes, filtered, washed with water (3 × 30 mL) and dried. The solid was suspended in POCl₃ (50 mL) and DMF (0 5 mL) and stirred at 100 °C for 1 h. The solution was cooled, poured into ice/water, stirred for 30 minutes, filtered, washed with water (3 × 30 mL) and dried. The solid was suspended in DCM (150 mL), dried and the solvent evaporated. The residue was chromatographed, eluting with 5% EtOAc/DCM, to give chloride 154 (382 mg, 78%) as a pale yellow solid, mp (EtOAc/DCM) 91-94 °C; ¹H NMR δ 8.33 (d, *J* = 9.1 Hz, 1 H, H-8), 7.93 (d, *J* = 2.0 Hz, 1 H, H-5), 7.83 (dd, *J* = 9.1, 2.0 Hz, 1 H, H-7), 1.46 [s, 9H, C(CH₃)₃]; ¹³C NMR δ 161.2, 156.5, 147.1, 131.3, 129.4, 123.3, 119.2, 35.4, 30.1 (3). Anal. calcd for C₁₁H₁₂ClN₃O: C, 55.6; H, 5 1; N, 17.7, Cl, 14.9; found C, 55.3; H, 4.9; N, 17 5; Cl, 15.0%.

### Example 160

***N***^{**1**}**-(6-*tert*-Butyl-1-oxido-1,2,4-benzotriazin-3-yl)-*N***^{**2**}**,*N***^{**2**}**-dimethyl-1,2-ethanediamine (155).** *N*,*N*-Dimethylethanediamine (0.66 mL, 6.0 mmol) was added to a stirred solution of chloride **154** (476 mg, 2.0 mmol) in DME (50 mL) and the solution stirred at reflux temperature for 2 h. The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-10%) of MeOH/DCM, to give 1-oxide **155** (536 mg, 93%) as a yellow solid, mp (MeOH/EtOAc) 140-144 °C; ¹H NMR δ 8.16 (d, *J* = 9.0 Hz, 1 H, H-8), 7.53 (d, *J* = 2.0 Hz, 1 H, H-5), 7.35 (dd, *J* = 9.0, 2.0 Hz, 1 H, H-7), 6 01 (br s, 1 H, NH), 3.56-3.61 (m, 2 H, CH₂N), 2.62-2.65 (m, 2 H, CH₂N), 2.33 [s, 6 H, N(CH₃)₂], 1.38 [s, 9 H, C(CH₃)₃]; ¹³C NMR δ 159.8, 159.2,149.0, 129.0, 123.8, 121 8, 119.9, 57.5, 45.0 (2), 38.5, 35.5, 30.7 (3). Anal. calcd for C₁₅H₂₃N₅O·¼H₂O: C, 61.3; H, 8.1; N, 23.8; found C, 61.1: H, 8.1; N, 23.8%.

### Example 161

***N***^{**1**}**-(6-tert-Butyl-1-oxido-1,2,4-benzotriazin-3-yl)-*N***^{**2**}***N***^{**2**} **-diethyl-1,2-ethanediamine** (156). N¹,N¹-Diethyl-1,2-ethanediamine (0.52 mL, 3.7 mmol) was added to a stirred solution of chloride **154** (350 mg, 1.5 mmol) in DME (50 mL) and the solution stirred at reflux temperature for 2 h. The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-5%) of MeOH/DCM, to give 1-oxide **156** (412 mg, 88%) as a yellow gum, ¹H NMR δ 8.16 (d, *J* = 9.1 Hz, 1 H, H-8), 7.53 (d, *J* = 2.0 Hz, 1 H, H-5), 7.34 (dd, *J* = 9.1, 2.0 Hz, 1 H, H-7), 5.97 (br s, 1 H, NH), 3.50-3.55 (m, 2 H, CH₂N), 2.68-2.72 (m, 2 H, CH₂N), 2.55-2.60 (m, 4 H, 2 × CH₂N), 1.37 [s, 9 H, C(CH₃)₃], 1.04 (t, *J* = 7.1 Hz, 6 H, 2 × CH₃); ¹³C NMR δ 159.8, 159.2, 149.1, 128.9, 123.7, 121.8, 119.9, 51.2, 46.6 (2), 38.7, 35.5, 30.7 (3), 11.8 (2); MS (El⁺) *m*/*z* 317 (M⁺, 1%), 300 (10), 86 (100); HRMS (El⁺) calcd for C₁₇H₂₇N₅O (M⁺) m/z 317 2216, found 317.2215.

### Example 162

**6-*tert*-Butyl-*N*-[2-(4-morpholinyl)ethyl]-1,2,4-benzotriazin-3-amine 1-Oxide (157).** 2-(4-Morpholinyl)ethylamine (0.66 mL, 5.0 mmol) was added to a stirred solution of chloride **154** (476 mg, 2.0 mmol) in DME (50 mL) and the solution stirred at reflux temperature for 2 h. The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-5%) of MeOH/DCM, to give 1-oxide **157** (642 mg, 97%) as a yellow powder, mp (MeOH/EtOAc) 132-134 °C; ¹H NMR δ 8.18 (d, *J* = 9.1 Hz, 1 H, H-8), 7.55 (d, *J* = 2.0 Hz, 1 H, H-5), 7.37 (dd, *J* = 9.1, 2.0 Hz, 1 H, H-7), 5.86 (br s, 1 H, NH), 3.69-3.75 (m, 4 H, 2 × CH₂O) 3.56-3.61 (m, 2 H, CH₂N), 2.60-2.64 (m, 2 H, CH₂N), 2.48-2.53 (m, 4 H, 2 × CH₂N), 1.38 [s, 9 H, C(CH₃)₃]; ¹³C NMR δ 159.9, 159.1, 149.0, 129.0, 123.9, 121.8, 120.0, 67.0 (2), 56.7, 53.3 (2), 37.4, 35.6, 30.7 (3). Anal. calcd for C₁₇H₂₅N₅O₂: C, 61.1; H, 7.6; N, 21.1; found C, 61.2; H, 7.8; N, 21.2%.

### Example 163

**6-*tert*-Butyl-*N*-[2-(1-piperidinyl)ethyl]-1,2,4-benzotriazin-3-amine 1-Oxide (158).** 2-(1-Piperidinyl)ethylamine (0.57 mL, 4.0 mmol) was added to a stirred solution of chloride **154** (379 mg, 1.6 mmol) in DME (50 mL) and the solution stirred at reflux temperature for 2 h. The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-5%) of MeOH/DCM, to give 1-oxide 158 (500 mg, 95%) as a yellow powder, mp (MeOH/EtOAc) 145-148 °C; ¹H NMR δ 8.16 (d, *J* = 9.1 Hz, 1 H, H-8), 7.53 (d, *J* = 2.0 Hz, 1 H, H-5), 7.34 (dd, *J* = 9.1, 2.0 Hz, 1 H, H-7), 5.99 (br s, 1 H, NH), 3.52-3.57 (m, 2 H, CH₂N), 2.56-2.60 (m, 2 H, CH₂N), 2.38-2.45 (m, 4 H, 2 × CH₂N), 1.55-1.61 (m, 4 H, 2 × CH₂), 1.41-1.47 (m, 2 H, CH₂), 1.37 [s, 9 H, C(CH₃)₃]; ¹³C NMR δ 159.8, 159.1, 149.1, 128.9, 123.7, 121.8, 119.9, 56.8, 54.3 (2), 37.9, 35.5, 30.7 (3), 26.0 (2), 24.4. Anal. calcd for C₁₈H₂₇N₅O.¼H₂O: C, 64.7; H, 8.3; N, 21.0; found C, 64.5; H, 8.5; N, 21.0%.

### Example 164

***N***^{**1**}**-(6-*tert*-Butyl-1-oxido-1,2,4-benzotriazin-3-yl)-*N***^{**2**}**,*N***^{**2**}**-dipropyl-1,2-ethanediamine (159).** *N*¹*,N*¹-Dipropyl-1,2-ethanediamine (0.43 mL, 3.0 mmol) was added to a stirred solution of chloride **154** (283 mg, 1 2 mmol) in DME (50 mL) and the solution stirred at reflux temperature for 2 h. The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-5%) of MeOH/DCM, to give 1-oxide **159** (393 mg, 96%) as a pale yellow oil, ¹H NMR δ 8.17 (d, *J* = 9.1 Hz, 1 H, H-8), 7.54 *(d, J* = 1.8 Hz, 1 H, H-5), 7.35 (dd, *J* = 9.1, 1.8 Hz, 1 H, H-7), 5.90 (br s, 1 H, NH), 3.49-3.55 (m, 2 H, CH₂N), 2.67-2.71 (m, 2 H, CH₂N), 2.38-2.45 (m, 4 H, 2 × CH₂N), 1.43-1.52 (m, 4 H, 2 × CH₂), 1.37 [s, 9 H, C(CH₃)₃], 0.88 (t, *J* = 7.3 Hz, 6 H, 2 x CH₃); ¹³C NMR δ 159 2, 158.7, 148.6, 128.4, 123.1, 121.3, 119.4, 55.3 (2), 52.0, 38.4, 35.0, 30.7 (3), 19.8 (2), 11.4 (2); MS (El⁺) *m*/*z* 345 (M⁺, 1%), 328 (10), 114 (100), HRMS (El⁺) calcd for C₁₉H₃₁N₅O (M⁺) m/z 345.2529, found 345.2528.

### Example 165

**3-Ethyl-6-fluoro-1,2,4-benzotriazine 1-oxide** (**160).** Pd(PPh₃)₄ (196 mg, 0.17 mmol) was added to a stirred solution of chloride 131 (329 mg, 1.7 mmol) and tetraethyltin (0.7 mL, 3.3 mmol) in DME (20 mL), the solution degassed, and stirred under N₂ at reflux temperature for 16 h. The solvent was evaporated and the residue purified by chromatography, eluting with 20% EtOAc/pet. ether to give an oil which was further purified by chromatography, eluting with 5% EtOAc/DCM, to give 1-oxide **160** (295 mg, 93%) as a white solid, mp (EtOAc/pet. ether) 122―124 °C; ¹H NMR δ 8.48 (dd, *J* = 9.5, 5.5 Hz, 1 H, H-8), 7.60 (dd, *J* = 8.7, 2.6 Hz, 1 H, H-5), 7.38 (m, 1 H, H-7), 3.04 (q, *J* = 7.6 Hz, 2 H, CH₂), 1.43 (t, *J* = 7.6 Hz, 3 H, CH₃); ¹³C NMR δ 168.6 (q, *J* = 175 Hz), 165.1, 149.5 (d, *J* = 15 Hz), 130.5, 123.2 (d, *J* = 11 Hz), 120.0 (d, *J* = 26 Hz), 112.7 (d, *J* = 22 Hz), 30.7, 12.2. Anal. calcd for C₉H₈FN₃O: C, 56.0; H, 4.2; N, 21.8; found C, 56.0; H, 4.2; N, 21.8%.

### Example 166

**3-Ethyl-6-methoxy-1,2,4-benzotriazine 1-oxide (161).** Sodium (55 mg, 2 4 mmol) was added to a stirred solution of fluoride (310 mg, 1 6 mmol) in MeOH (10 mL) and the solution was stirred at 20 °C for 4 h under N₂. The solvent was evaporated and the residue partitioned between DCM (20 mL) and water (20 mL). The organic fraction was dried, and the solvent evaporated. The residue was purified by chromatography, eluting with 5% EtOAc/pet. ether, to give 1-oxide **161** (156 mg, 56%) as a white solid, mp (EtOAc/ pet. ether) 109-111 °C; ¹H NMR δ 8.32 (d, *J* = 9.5 Hz, 1 H, H-8), 7.24 (dd, *J* = 9.5, 2.6 Hz, 1 H, H-7), 7.19 (d, *J* = 2.6 Hz, 1 H, H-5), 3.98 (s, 3 H, OCH₃), 3 00 (q, *J* = 7.6 Hz, 2 H, CH₂), 1.43 (t, *J* = 7.6 Hz, 3 H, CH₃); ¹³C NMR δ 168.8, 165.3, 150.3, 128.5, 122.9, 121.7, 105.8, 56.2, 30.7, 12.2. Anal. calcd for C₁₀H₁₁N₃O₂: C, 58.5; H, 5.4; N, 20.5, found C, 58.6; H, 5.4; N, 20.5%.

### Example 167

***N***^{**1**}**,*N***^{**1**}**-Diethyl-*N***^{**2**}**-(8-methyl-1-oxido-1,2,4-benzotriazin-3-yl)-1,2-ethanediamine (162).** *N,N*-Diethylethanediamine (0.38 mL, 2.7 mmol) was added to a stirred solution of chloride **100** (214 mg, 1.1 mmol) in DME (50 mL) and the solution stirred at reflux temperature for 2 h The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-10%) MeOH/DCM, to give 1-oxide **162** (297 mg, 99%) as a yellow solid, mp (MeOH/EtOAc) 89-91 °C; ¹H NMR δ 7.49 (dd, J = 8.4, 7.1 Hz, 1 H, H-6), 7 42 (d, *J* = 8 4 Hz, 1 H, H-5), 7.01 (d, *J* = 7.1 Hz, 1 H, H-7), 5.86 (br s, 1 H, NH), 3.49-3.53 (m, 2 H, CH₂N), 2.90 (s, 3 H, CH₃), 2.69 (dd, *J* = 6.1, 5.9 Hz, 2 H, CH₂N), 2.57 (q, *J* = 7.1 Hz, 4 H, 2 × CH₂N), 1.04 (t, *J* = 7.1 Hz, 6 H, 2 × CH₃); ¹³C NMR δ 158.4, 150.7, 134 5, 134.2, 131.1, 127.4, 124.7, 51.3, 46.6 (2), 38.7, 24.0 11.8 (2). Anal. calcd for C₁₄H₂₁N₅O: C, 61.1; H, 7.7; N, 25.4; found C, 61.4; H, 7.8; N, 25.3%.

### Example 168

**8-Methyl-*N*-[2-(4-morpholinyl)ethyl]-1,2,4-benzotriazin-3-amine 1-Oxide (163).** N,N-2-(4-Morpholinyl)ethylamine (0.94 mL, 7.1 mmol) was added to a stirred solution of chloride **100** (465 mg, 2.4 mmol) in DME (50 mL) and the solution stirred at reflux temperature for 2 h. The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-10%) MeOH/DCM, to give 1-oxide **163** (545 mg, 79%) as a yellow solid, mp (MeOH/EtOAc) 168-170 °C; ¹H NMR δ 7.50 (dd, *J* = 8.3, 7.1 Hz, 1 H, H-6), 7.42 (d, *J* = 8.3 Hz, 1 H, H-5), 7.02 (d, *J* = 7.1 Hz, 1 H, H-7), 5.76 (br s, 1 H, NH), 3.69-3.73 (m, 4 H, 2 × CH₂O), 3.55-3.59 (m, 2 H, CH₂N), 2.89 (s, 3 H, CH₃), 2 60-2.63 (m, 2 H, CH₂N), 2.47-2.51 (m, 4 H, 2 × CH₂N); ¹³C NMR δ 158.4, 150.7, 134 6, 134.3, 131 2, 127.6, 124.7, 66.9 (2), 56.8, 55.3 (2), 37.4, 24.0. Anal. calcd for C₁₄H₁₉N₅O₂: C, 58.1; H, 6.6; N, 24.2; found C, 58.3; H, 6.7; N, 24.4%.

### Example 169

***N*-[2-(2,6-Dimethyl-1-piperidinyl)ethyl]-8-methyl-1,2,4-benzotriazin-3-amine 1-Oxide (164).** 2-(2,6-Dimethyl-1-piperidinyl)ethylamine (0.70 mL, 4.5 mmol) was added to a stirred solution of chloride **100** (352 mg, 1.8 mmol) in DME (50 mL) and the solution stirred at reflux temperature for 2 h. The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-10%) of MeOH/DCM, to give 1-oxide **164** (489 mg, 82%) as a yellow solid, mp (MeOH/EtOAc) 169-171 °C; ¹H NMR δ 7.48 (dd, *J* = 8.4, 7.1 Hz, 1 H, H-6), 7.37 (d, *J* = 8.4 Hz, 1 H, H-5), 7.00 (d, *J* = 7.1 Hz, 1 H, H-7), 5 56 (br s, 1 H, NH), 3.50-3.55 (m, 2 H, CH₂N), 2.86-2.90 (m, 5 H, CH₂N, CH₃), 2.48-2.56 (m, 2 H, CH₂N), 1.63-1.68 (m, 1 H, CH), 1.52-1.58 (m, 2 H, CH₂), 1.32-1 38 (m, 1 H, CH), 1.23-1.30 (m, 2 H, CH₂), 1.20 (d, *J* = 6.3 Hz, 6 H, 2 × CH₃); ¹³C NMR δ 158.5, 150.7, 134.5, 134.2, 131.1, 127.5, 124.8, 57.2 (2), 47.5, 39.3, 34.3 (2), 24.4, 24 0 (2), 21.6. Anal. calcd for C₁₇H₂₅N₅O: C, 64.7; H, 8.0; N, 22.2; found C, 64.7; H, 8.2; N, 22.3%.

### Example 170

***N***^{***1***}**-(8-Methyl-1-oxido-1,2,4-benzotriazin-3-yl)-*N***^{***2***}***,N***^{***2***}**-dipropyl-1,2-ethanediamine (165)** *N*^{*1*}*,N*^{*1*}-Dipropyl-1,2-ethanediamine (0.77 mL, 5.3 mmol) was added to a stirred solution of chloride **100** (415 mg, 2.1 mmol) in DME (50 mL) and the solution stirred at reflux temperature for 2 h. The solution was cooled, the solvent evaporated and the residue partitioned between dilute aqueous NH₃ (100 mL) and DCM (100 mL). The organic fraction was dried and the solvent evaporated. The residue was chromatographed, eluting with a gradient (0-10%) MeOH/DCM, to give 1-oxide **165** (597 mg, 93%) as a yellow solid, mp (MeOH/EtOAc) 91-93 °C; ¹H NMR δ 7.48 (dd, *J* = 8.4, 7.1 Hz, 1 H, H-6), 7.41 (d, *J* = 8.4 Hz, 1 H, H-5), 7.00 (d, *J* = 7.1 Hz, 1 H, H-7), 5.80 (br s, 1 H, NH), 3.47-3.52 (m, 2 H, CH₂N), 2.89 (s, 3 H, CH₃), 2.65-2.69 (m, 2 H, CH₂N), 2.39-2.43 (m, 4 H, 2 × CH₂N), 1.42-1.51 (m, 4 H, 2 × CH₂), 0.89 (t, *J* = 7.3 Hz, 6 H, 2 × CH₃); ¹³C NMR δ 158.5, 150.7, 134.4, 134.2, 131.1, 127.4, 124.7, 55.9 (2), 52.6, 38.8, 24.0, 20.3 (2), 11.9 (2). Anal. calcd for C₁₆H₂₅N₅O: C, 63.3; H, 8.3; N, 23.1; found C, 63.4; H, 8 3; N, 22.7%.

### Example 171

***N***^{**1**}***-(*6,7-Dimethyl-1-oxido-1,2,4-benzotriazin-3-yl)-*N***^{***2***}***,N***^{***2***} **-diethyl-1,2-ethanediamine (166).** N,N-Diethyl-1,2-ethanediamine (0.54 mL, 3.8 mmol) was added to a stirred solution of chloride **104** (322 mg, 1.5 mmol) in DME (30 mL) and the solution stirred at reflux temperature for 16 h. The solvent was evaporated and the residue partitioned between DCM (100 mL) and dilute aqueous NH₃ (50 mL). The organic fraction was dried and the solvent evaporated. The residue was chromatographed, eluting with a gradient (0-10%) of MeOH/DCM, to give 1-oxide **166** (438 mg, 98%), mp (MeOH/EtOAc) 130-132 °C; ¹H NMR δ 8.01 (s, 1 H, H-8), 7 36 (s, 1 H, H-5), 5.87 (br s, 1 H, NH), 3.47-3.52 (m, 2 H, CH₂N), 2.68 (dd, *J* = 6.1, 5.9 Hz, 2 H, CH₂N), 2.57 (g, *J* = 7 1 Hz, 4 H, 2 × CH₂N), 2.39 (s, 3 H, CH₃), 2.35 (s, 3 H, CH₃), 1.03 (t, *J* = 7.1 Hz, 6 H, 2 x CH₃); ¹³C NMR δ 158.9, 147.8, 146.9, 135.3, 129.1, 125.7, 119.4, 51.2, 46.6 (2), 38.7, 20.5, 19.8, 11.8(2). Anal. calcd for C₁₅H₂₃N₅O: C, 62 3, H, 8.0; N, 24.2; found C, 62.4; H, 8.1; N, 24.5%.

### Example 172

**6,7-Dimethyl-N-[2-(4-morpholinyl)ethyl)-1,2,4-benzotriazin-3-amine 1-Oxide (167).** 2-(4-Morpholinyl)ethylamine (0.76 mL, 5.8 mmol) was added to a stirred solution of chloride **104** (406 mg, 1.9 mmol) in DME (50 mL) and the solution stirred at reflux temperature for 6 h. The solvent was evaporated and the residue partitioned between DCM (100 mL) and dilute aqueous NH₃ (50 mL). The organic fraction was dried and the solvent evaporated. The residue was chromatographed, eluting with a gradient (0-10%) of MeOH/DCM, to give 1-oxide **167** (552 mg, 94%), mp (MeOH/EtOAc) 211-213 °C; ¹H NMR δ 8.01 (s, 1 H, H-8), 7.36 (s, 1 H, H-5), 5.79 (br s, 1 H, NH), 3.69-3.73 (m, 4 H, 2 × CH₂O), 3.54-3.58 (m, 2 H, CH₂), 2.62 (dd, *J* = 6.0, 5.9 Hz, 2 H, CH₂N), 2.48-2.52 (m, 4 H, 2 × CH₂N), 2.38 (s, 3 H, CH₃), 2.36 (s, 3 H, CH₃); ¹³C NMR δ 158.8, 147.8, 147.9, 135.3, 129.2, 125.7, 119.4, 66.9 (2), 56.8, 53.3 (2), 37.5, 20.5, 19.8. Anal. calcd for C₁₅H₂₁N₅O₂: C, 59.4; H, 7.0; N, 23.1; found C, 59.3; H, 6.9; N, 23.1%.

### Example 173

**6,7-Dimethyl-*N*-[3-(4-morpholinyl)propyl]-1,2,4-benzotriazin-3-amine 1-Oxide** (168). 3-(4-Morpholinyl)propylamine (1.16 mL, 7.9 mmol) was added to a stirred solution of chloride **104** (555 mg, 2.7 mmol) in DME (50 mL) and the solution stirred at reflux temperature for 6 h. The solvent was evaporated and the residue partitioned between DCM (100 mL) and dilute aqueous NH₃ (50 mL). The organic fraction was dried and the solvent evaporated. The residue was chromatographed, eluting with a gradient (0-10%) of MeOH/DCM, to give 1-oxide **168** (788 mg, 94%), mp (MeOH/EtOAc) 145-147 °C; ¹H NMR δ 8.00 (s, 1 H, H-8), 7.34 (s, 1 H, H-5), 6.16 (br s, 1 H, NH), 3.72-3.76 (m, 4 H, 2 × CH₂O), 3.55-3.60 (m, 2 H, CH₂), 2.44-2.52 (m, 6 H, 3 × CH₂N), 2.47 (s, 3H, CH₃), 2.35 (s, 3H, CH₃), 1.79-1.87 (m, 2 H, CH₂N); ¹³C NMR δ 159.0, 147 8, 146.9, 135.3, 129.2, 125.7, 119.4, 67.0 (2), 57.3, 53.8 (2), 40.8, 25.2, 20.5, 19.8. Anal. calcd for C₁₆H₂₃N₅O₂: C, 60.6; H, 7.3; N, 22.0; found C, 60.6; H, 7.2; N, 22.2%.

Wherein the foregoing description reference has been made to reagents or integers having known equivalents thereof, then those equivalents are herein incorporated as if individually set forth.

While this invention has been described with reference to certain embodiments and examples, it is to be appreciated that further modifications and variations may be made thereto without departing from the spirit or scope of the invention.

## Claims

1. A cytotoxic synergistic composition, comprising an effective amount of a benzoazine N-mono oxide compound of Formula A or a pharmacologically acceptable salt thereof and an effective amount of a benzoazine 1,4 dioxide compound of Formula B or a pharmacologically acceptable salt thereof 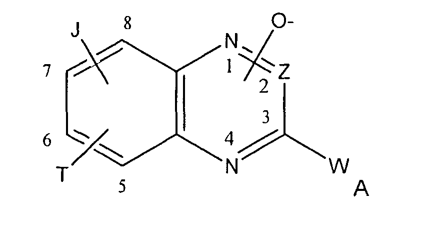 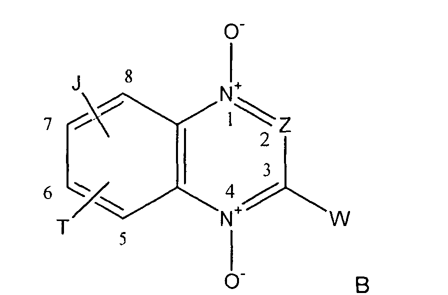 wherein in Formulae A or B
Z is N or C-CN, and
wherein in Formula A when Z represents N, the N-oxide moiety occupies one of the 1-, 2-, or 4-positions; and when Z represents C-CN, the N-oxide moiety occupies one of the 1-, or 4-positions;
wherein J in Formulae A or B represents at one or more of the available carbons 5-8 on the benzo ring the following groups:
halo, H, R, OH, OR, NO₂, NH₂, NHR, NR₂, SH, SR, SO₂R, CF₃, CN, CO₂H, CO₂R, CHO, COR, CONH₂, CONHR or CONRR, cyclic alkylamino, imidazolyl, alkylpiperazinyl, morpholino;
wherein each R is independently selected from an optionally substituted C₁₋₆ aliphatic or an optionally substituted C₃₋₆ cyclic alkyl group, and wherein the said optional substituents are each independently selected from; halo, OH, OR¹, NO₂, NH₂, NHR¹, NR¹R¹, SH, SR¹, imidazolyl, R¹-piperazinyl, morpholino, SO₂R¹, CF₃, CN, CO₂H, CO₂R¹, CHO, COR¹, CONH₂, CONHR¹, CONR¹R¹;
R can also represent an optionally substituted aryl or an optionally substituted heteroaryl group having up to 12 carbon atoms, and wherein the optional substituents are each independently selected from; halo, OH, OR¹, NH₂, NHR¹, NR¹R¹, SH, SR¹, imidazolyl, R'-piperazinyl, morpholino, SO₂R¹, CF₃, CN, CO₂H, CO₂R¹, CHO, COR¹, CONH₂, CONHR¹, CONR¹R¹, and each heteroaryl group contains one or more heteroatoms in its ring system which are each independently selected from O, N or S;
wherein each R¹ is independently selected from an optionally substituted C₁₋₄ alkyl or an optionally substituted C₂₋₄ alkenyl group and wherein the optional substituents are each independently selected from OH, OR, NH₂, NHR², NR²₂ or N(OH)R² wherein each R² is independently selected from C₁₋₄ alkyl, C₂₋₄ alkenyl, OH, NO₂, NH₂, CF₃, CN, CO₂H or SH, and
wherein W in Formulae A or B can represent -X-A, wherein -X-A together can represent H, or halogen; or
X represents O, S, NH, NMe, CH₂, SO, SO₂, CONH, NHCO, CO or CO₂, and
A represents H, an optionally substituted C₁₋₁₂ alkyl group wherein the optional substituents are each independently selected from OH, OR³, NH₂, NHR³, NR³₂, or N(OH)R³ wherein each R³ is independently selected from C₁₋₄ alkyl, C₂₋₄ alkenyl, OH, NO₂, NH₂, CF₃, CN, CO₂H or SH; and wherein the optionally substituted C₁₋₁₂alkyl chain can optionally interrupted or extended by one or more heteroatom containing linkage moieties selected from O, NH, NR⁴, CONH, CONR⁴, NHCO, NR⁴CO, where each R⁴ is independently selected from an optionally substituted C₁₋₄ alkyl or an optionally substituted C₂₋₄ alkenyl group and wherein the optional R⁴ substituents are each independently selected from OH, OR, NH₂, NHR⁵, NR⁵₂ or N(OH)R⁵ wherein each R⁵ is independently selected from C₁₋₄ alkyl, C₂₋₄ alkenyl, OH, NO₂, NH₂, CF₃, CN, CO₂H or SH, or a pharmacologically acceptable salt thereof, or
W can represent a group of Formula C 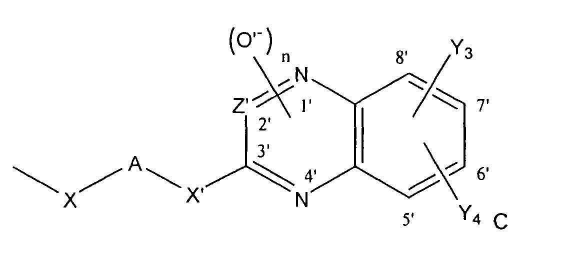 wherein in a group of Formula C
n represents either 1 or 2,
Z' is selected from N or C-CN, and when Z' represents N, and n represents 1 the N-oxide moiety occupies one of the 1'-, 2'-, or 4'-positions and when Z' represents C-CN, the N-oxide moiety occupies one of the 1'-, or 4'-positions; and when Z' represents N or C-CN, and n represents 2 the N-oxide moieties occupy the 1'and 4'-positions
Y₃ and Y₄ each represent at one or more of the available carbons 5'-8' on the benzo ring the following groups:
halo, H, R, OH, OR, NO₂, NH₂, NHR, NR₂, SH, SR, SO₂R, CF₃, CN, CO₂H, CO₂R, CHO, COR, CONH₂, CONHR or CONRR, cyclic alkylamino, imidazolyl, alkylpiperazinyl, morpholino;
wherein each R is independently selected from an optionally substituted C₁₋₆ aliphatic or an optionally substituted C₃₋₆ cyclic alkyl group, and wherein the said optional substituents are each independently selected from; halo, OH, OR¹, NO₂, NH₂, NHR¹, NR¹R¹, SH, SR¹, imidazolyl, R¹-piperazinyl, morpholino, SO₂R¹, CF₃, CN, CO₂H, CO₂R¹, CHO, COR¹, CONH₂, CONHR', CONR'R';
R can also represent an optionally substituted aryl or an optionally substituted heteroaryl group having up to 12 carbon atoms, and wherein the optional substituents are each independently selected from; halo, OH, OR¹, NH₂, NHR¹, NR¹R¹, SH, SR¹, imidazolyl, R¹-piperazinyl, morpholino, SO₂R¹, CF₃, CN, CO₂H, CO₂R¹, CHO, COR¹, CONH₂, CONHR¹, CONR¹R¹, and each heteroaryl group contains one or more heteroatoms in its ring system which are each independently selected from O, N or S;
wherein each R' is independently selected from an optionally substituted C₁₋₄ alkyl or an optionally substituted C₂₋₄ alkenyl group and wherein the optional substituents are each independently selected from OH, OR, NH₂, NHR², NR²₂ or N(OH)R² wherein each R² is independently selected from C₁₋₄ alkyl, C₂₋₄ alkenyl, OH, NO₂, NH₂, CF₃, CN, CO₂H or SH, and
X' represents O, NH, NMe, or CH₂,
A represents an optionally substituted C₁₋₁₂ alkyl group wherein the optional substituents are each independently selected from OH, OR³, NH₂, NHR³, NR³₂, or N(OH)R³ wherein each R³ is independently selected from C₁₋₄ alkyl, C₂₋₄ alkenyl, OH, NO₂, NH₂, CF₃, CN, CO₂H or SH; and wherein the optionally substituted C₁₋₁₂ alkyl chain is optionally interrupted or extended by one or more heteroatom containing linkage moieties selected from O, NH, NR⁴, CONH, CONR⁴, NHCO, NR⁴CO, where each R⁴ is independently selected from an optionally substituted C₁₋₄ alkyl or an optionally substituted C₂₋₄ alkenyl group and wherein the optional R⁴ substituents are each independently selected from OH, OR, NH₂, NHR⁵, NR⁵ ₂ or N(OH)R⁵ wherein each R⁵ is independently selected from C₁₋₄ alkyl, C₂₋₄ alkenyl, OH, NO₂, NH₂, CF₃, CN, CO₂H or SH, or
W can represent a group of Formula D 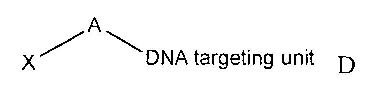 wherein X represents NH, NMe, CH₂, or O;
A represents an optionally substituted C₁₋₁₂ alkyl group wherein the optional substituents are each independently selected from OH, OR, NH_{2,} NHR³, NR³_{2,} or N(OH)R³ wherein each R³ is independently selected from C₁₋₄ alkyl, C₂₋₄ alkenyl, OH, NO_{2,} NH_{2,} CF_{3,} CN, CO₂H or SH; and wherein the optionally substituted C₁₋₁₂ alkyl chain is optionally interrupted or extended by one or more heteroatom containing linkage moieties selected from O, NH, NR⁴, CONH, CONR⁴, NHCO, NR⁴CO, where each R⁴ is independently selected from an optionally substituted C₁₋₄ alkyl or an optionally substituted C₂₋₄ alkenyl group and wherein the optional R⁴ substituents are each independently selected from OH, OR, NH_{2,} NHR⁵, NR⁵₂ or N(OH)R⁵ wherein each R⁵ is independently selected from C₁₋₄ alkyl, C₂₋₄ alkenyl, OH, NO_{2,} NH_{2,} CF_{3,} CN, CO₂H or SH, and
wherein the DNA-targeting unit is any moiety of a molecular weight below 700 Daltons that has an association constant (K) for binding to double-stranded random sequence DNA of >10³ M⁻¹ at an ionic strength of 0 01 M at 20 °C,
wherein T in Formulae A or B, represent at one of carbons 5-8 on the benzo ring the following groups:
halo, H, R, OH, OR, NO_{2,} NH₂, NHR, NR₂, SH, SR, SO₂R, CF₃, CN, CO₂H, CO₂R, CHO, COR, CONH₂, CONHR or CONRR, cyclic alkylamino, imidazolyl, alkylpiperazinyl, morpholino;
wherein each R is independently selected from an optionally substituted C₁₋₆ aliphatic or an optionally substituted C₃₋₆ cyclic alkyl group, and wherein the said optional substituents are each independently selected from; halo, OH, OR¹, NO₂, NH₂, NHR¹, NR¹R¹, SH, SR¹, imidazolyl, R¹-piperazinyl, morpholino, SO₂R¹, CF₃, CN, CO₂H, CO₂R¹, CHO, COR¹, CONH₂, CONHR¹, CONR¹R¹;
R can also represent an optionally substituted aryl or an optionally substituted heteroaryl group having up to 12 carbon atoms, and wherein the optional substituents are each independently selected from; halo, OH, OR¹, NH₂, NHR¹, NR¹R¹, SH, SR¹, imidazolyl, R¹-piperazinyl, morpholino, SO₂R¹, CF₃, CN, CO₂H, CO₂R¹, CHO, COR¹, CONH₂, CONHR¹, CONR¹R¹, and each heteroaryl group contains one or more heteroatoms in its ring system which are each independently selected from O, N or S;
wherein each R¹ is independently selected from an optionally substituted C₁₋₄ alkyl or an optionally substituted C₂₋₄ alkenyl group and wherein the optional substituents are each independently selected from OH, OR, NH₂, NHR², NR²₂ or N(OH)R² wherein each R² is independently selected from C₁₋₄ alkyl, C₂₋₄ alkenyl, OH, NO₂, NH₂, CF₃, CN, CO₂H or SH, or
T represents a group of Formula E 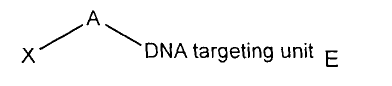 wherein X represents O, S, NH, NMe, CH₂, SO, SO₂, CONH, NHCO, CO, CO₂, or O and
A represents an optionally substituted C₁₋₁₂ alkyl group wherein the optional substituents are each independently selected from OH, OR, NH₂, NHR³, NR³₂, or N(OH)R³ wherein each R³ is independently selected from C₁₋₄ alkyl, C₂₋₄ alkenyl, OH, NO₂, NH₂, CF₃, CN, CO₂H or SH; and wherein the optionally substituted C₁₋₁₂ alkyl chain is optionally interrupted or extended by one or more heteroatom containing linkage moieties selected from O, NH, NR⁴, CONH, CONR⁴, NHCO, NR⁴CO, where each R⁴ is independently selected from an optionally substituted C₁₋₄ alkyl or an optionally substituted C₂₋₄ alkenyl group and wherein the optional R⁴ substituents are each independently selected from OH, OR, NH₂, NHR⁵, NR⁵₂ or N(OH)R⁵ wherein each R⁵ is independently selected from C₁₋₄ alkyl, C₂₋₄ alkenyl, OH, NO₂, NH₂, CF₃, CN, CO₂H or SH, and
wherein the DNA-targeting unit is any moiety of a molecular weight below 700 Daltons that has an association constant (K) for binding to double-stranded random-sequence DNA of >10³ M⁻¹ at an ionic strength of 0.01 M at 20 °C.

2. A composition according to claim 1 wherein the DNA targeting agent defined in claim 1 for a group of Formula D or Formula E is independently selected from any one of the formulae **III- XVII**, 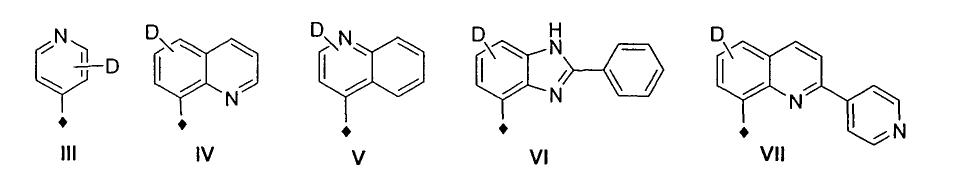 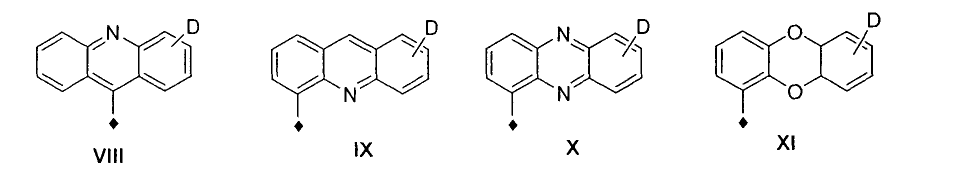 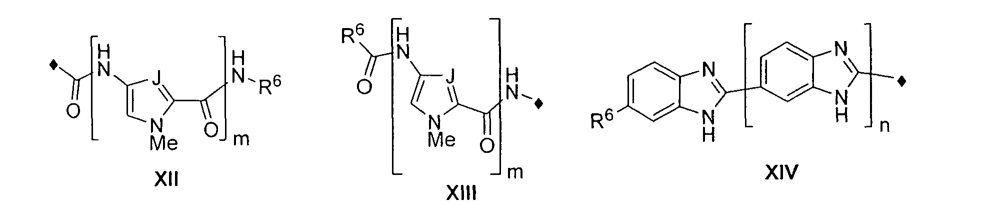 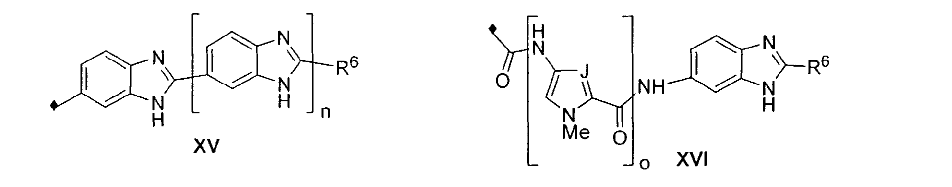 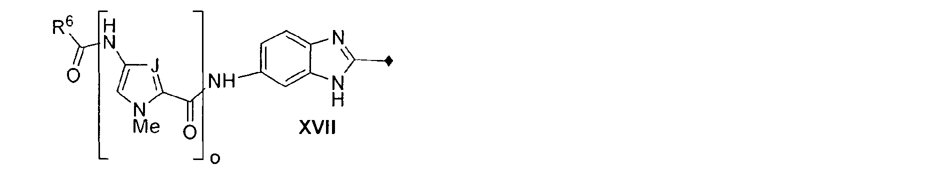 wherein in structures **XII-XVII** R⁶ is independently selected from an optionally substituted C₁₋₆ aliphatic or an optionally substituted C₃₋₆ cyclic alkyl group, and wherein the optional substituents are each independently selected from; halo, OH, OR⁷ NO₂, NH₂, NHR⁷, NR⁷R⁷, SR⁷, imidazolyl, R⁷-piperazinyl, morpholino, SO₂R⁷, CF₃, CN, CO₂H, CO₂R⁷, CHO, COR⁷, CONH₂, CONHR⁷, CONR⁷R⁷;
R⁶ can also represent an optionally substituted aryl or an optionally substituted heteroaryl group having up to 12 carbon atoms, and wherein the optional substituents are each independently selected from; halo, OH, OR⁷, NH₂, NHR⁷, NR⁷R⁷, SH, SR⁷, imidazolyl, R⁷-piperazinyl, morpholino, SO₂R⁷, CF₃, CN, CO₂H, CO₂R⁷, CHO, COR⁷, CONH₂, CONHR⁷, CONR⁷R⁷, and each heteroaryl group contains one or more heteroatoms in its ring system which are each independently selected from O, N or S;
wherein each R⁷ is independently selected from an optionally substituted C₁₋₄ alkyl or an optionally substituted C₂₋₄ alkenyl group and wherein the optional substituents are each independently selected from OH, OR⁸, NH₂, NHR⁸, NR⁸₂ or N(OH)R⁸ wherein each R⁸ is independently selected from C₁₋₄ alkyl, C₂₋₄ alkenyl, OH, NO₂, NH₂, CF₃, CN, CO₂H or SH;
D represents up to four of the following groups as substituents at any available ring carbon position; H, R⁹, hydroxy, alkoxy, halogen, NO₂, NH₂, NHR⁹, NR⁹₂, SH, SR⁹, SO₂R⁹, CF₃, CN, CO₂H, CO₂R⁹, CHO, COR⁹, CONH₂, CONHR⁹ or CONR⁹R⁹, cyclic alkylamino, imidazolyl, alkylpiperazinyl, morpholino, wherein each R⁹ is independently selected from an optionally substituted C₁₋₄ alkyl or an optionally substituted C₂₋₄ alkenyl group and wherein the optional substituents are each independently selected from OH, OR¹⁰, NH₂, NHR¹⁰, NR¹⁰₂ or N(OH)R¹⁰ wherein each R¹⁰ is independently selected from C₁₋₄ alkyl, C₂₋₄ alkenyl, OH, NO₂, NH₂, CF₃, CN, CO₂H or SH;
and wherein any available ring carbon position of formulae **III - XVII** can also be optionally replaced by -N- when the valency and configuration of the formula allows, the point of attachment of formulae **III- XVII** to the A group defined above is represented by **◆;** and
wherein in formulae **XII, XIII,** m is selected from 2, 3 or 4, and
wherein in formulae **XII, XIII, XVI** and **XVII,** J is selected from CH or N;
and wherein in formulae **XIV** and **XV** n is selected from 0, 1 or 2;
and wherein in formulae **XVI** and **XVII** o is selected from 1 and 2.

3. A composition according to claim 2 wherein the DNA targeting unit of Formula D or Formula E is selected from one of formulae **V, VI, VII, VIII, IX** or **X**.

4. A composition according to claim 2 or claim 3 wherein substituent D of the DNA targeting unit of Formulae **III** - **XI** is H or Me.

5. A composition according to any one of claims 1 to 4 wherein W in the compound of Formula A as defined in claim 1 represents a NH(C₀-C₁₂) optionally substituted alkyl or a O(C₀-C₁₂) optionally substituted alkyl.

6. A composition according to claim 5 wherein W represents NH₂, NHCH₂CH₂NHCH₃, NHCH₂CH₂N(CH₃)₂ or OCH₃.

7. The use in the manufacture of a medicament of a cytotoxic effective amount of a composition including an effective amount of one or more compounds of Formula A and one or more compounds of formula B as defined in any one of claims 1 to 6 for treating tumour cells in a subject.

8. A compound of Formula I, 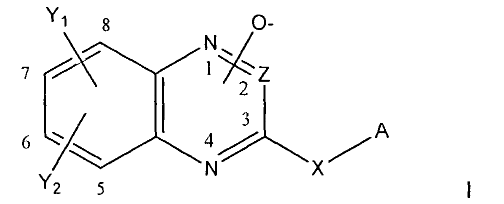 wherein
Z is selected from N or C-CN, and when Z represents N, the N-oxide moiety occupies one of the 1-, 2-, or 4-positions, and when Z represents C-CN, the N-oxide moiety occupies one of the 1-, or 4-positions;
Y₁ and Y₂ each represent at one or more of the available carbons 5-8 on the benzo ring the following groups:
halo, H, R, OH, OR, NO₂, NH₂, NHR, NR₂, SH, SR, SO₂R, CF₃, CN, CO₂H, CO₂R, CHO, COR, CONH₂, CONHR or CONRR, cyclic alkylamino, imidazolyl, alkylpiperazinyl, morpholino;
wherein each R is independently selected from an optionally substituted C₁₋₆ aliphatic or an optionally substituted C₃₋₆ cyclic alkyl group, and wherein the said optional substituents are each independently selected from; halo, OH, OR¹, NO₂, NH₂, NHR¹, NR¹R¹, SH, SR¹, imidazolyl, R¹-piperazinyl, morpholino, SO₂R¹, CF₃, CN, CO₂H, CO₂R¹. CHO, COR¹, CONH₂, CONHR¹, CONR¹R¹;
R can also represent an optionally substituted aryl or an optionally substituted heteroaryl group having up to 12 carbon atoms, and wherein the optional substituents are each independently selected from; halo, OH, OR¹, NH₂, NHR¹, NR¹R¹, SH, SR¹, imidazolyl, R¹-piperazinyl, morpholino, SO₂R¹, CF₃, CN, CO₂H, CO₂R¹, CHO, COR¹, CONH₂, CONHR¹, CONR¹R¹, and each heteroaryl group contains one or more heteroatoms in its ring system which are each independently selected from O, N or S;
wherein each R¹ is independently selected from an optionally substituted C₁₋₄ alkyl or an optionally substituted C₂₋₄ alkenyl group and wherein the optional substituents are each independently selected from OH, OR, NH₂, NHR², NR²₂ or N(OH)R² wherein each R² is independently selected from C₁₋₄ alkyl, C₂₋₄ alkenyl, OH, NO_{2,} NH₂, CF₃, CN, CO₂H or SH, and
wherein A and X together represent H, or halogen; or
X represents O, S, NH, NMe or CH₂ and
A represents H, an optionally substituted C₁₋₁₂ alkyl group wherein the optional substituents are each independently selected from OH, OR³, NH₂, NHR³, NR³ ₂ , or N(OH)R³ wherein each R³ is independently selected from C₁₋₄ alkyl, C₂₋₄ alkenyl, OH, NO₂ NH₂, CF₃, CN, CO₂H or SH; and wherein the optionally substituted C₁₋₁₂ alkyl chain can be optionally interrupted or extended by one or more heteroatom containing linkage moieties selected from O, NH, NR⁴, CONH, CONR⁴, NHCO, NR⁴CO, where each R⁴ is independently selected from an optionally substituted C₁₋₄ alkyl or an optionally substituted C₂₋₄ alkenyl group and wherein the optional R⁴ substituents are each independently selected from OH, OR, NH₂, NHR⁵, NR⁵ ₂ or N(OH)R⁵ wherein each R⁵ is independently selected from C₁₋₄ alkyl, C₂₋₄ alkenyl, OH, NO_{2,} NH_{2,} CF_{3,} CN, CO₂H or SH, or a pharmacologically acceptable salt thereof,
with the proviso that the following compounds are excluded
3-Amino-1,2,4-benzotriazine-1-oxide,
3-Amino-7-trifluoromethyl-1,2,4-benzotriazine-1-oxide,
3-Amino-7-carbamyl-1,2,4-benzotriazine-1-oxide,
3-Amino-7-chloro-1,2,4-benzotriazine-1-oxide,
3-Amino-7-nitro-1,2,4-benzotriazine-1-oxide
3-Chloro-1,2,4-benzotriazine-1-oxide,
3-(3-N,N-Diethylaminopropylamino)- 3-amino-1,2,4-benzotriazine-1-oxide,
3-Chloro-7-nitro-1,2,4-benzotriazine-1-oxide,
7-Nitro-(3-(2-N,N-diethylamino-ethylamino)-1,2,4-benzotriazine-1-oxide,
8-Methoxy-1,2,4-benzotriazin-3-amine 1-oxide,
8-Methyl-1,2,4-benzotriazin-3-amine 1-oxide,
8-Fluoro-1,2,4-benzotriazin-3-amine 1-oxide,
8-Chloro-1,2,4-benzotriazin-3-amine 1-oxide,
8-Trifluoromethyl-1,2,4-benzotriazin-3-amine 1-oxide,
8-(Methylsulfanyl)-1,2,4-benzotriazin-3-amine 1-oxide,
8-(Butylsulfanyl)-1,2,4-benzotriazin-3-amine 1-oxide,
3-Amino-1,2,4-benzotriazin-7-ol 1-oxide,
3-Amino-1,2,4-benzotriazin-7-ol 1-oxide,
7-Methyl-1,2,4-benzotriazin-3-amine 1-oxide,
7-Fluoro-1,2,4-benzotriazin-3-amine 1-oxide,
7-Chloro-1,2,4-benzotrazin-3-amine 1-oxide,
7-Trifluoromethyl-1,2,4-benzotriazin-3-amine 1-oxide,
7-(Methylsulfanyl)-1,2,4-benzotriazin-3-amine 1-oxide,
7-(Butylsulfanyl)-1,2,4-benzotriazin-3-amine 1-oxide,
7-Nitro-1,2,4-benzotriazin-3-amine 1-oxide,
6-Methoxy-1,2,4-benzotriazin-3-amine 1-oxide,
6-Methyl-1,2,4-benzotriazin-3-amine 1-oxide,
6-Phenyl-1,2,4-benzotriazin-3-amine 1-oxide,
6-Fluoro-1,2,4-benzotriazin-3-amine 1-oxide,
6-Chloro-1,2,4-benzotrazin-3-amine 1-oxide,
6-Trifluoromethyl-1,2,4-benzotriazin-3-amine 1-oxide,
6-(Methylsulfanyl)-1,2,4-benzotriazin-3-amine 1-oxide,
6-(Butylsulfanyl)-1,2,4-benzotriazin-3-amine 1-oxide,
5-Methoxy-1,2,4-benzotriazin-3-amine 1-oxide,
5-Methyl-1,2,4-benzotriazin-3-amine 1-oxide,
5-Chloro-1,2,4-benzotriazin-3-amine 1-oxide,
5-Fluoro-1,2,4-benzotriazin-3-amine 1-oxide,
*N*⁷,*N*⁷-Dimethyl-1,2,4-benzotriazine-3,7-diamine 1-oxide,
3-Chloro-1,2,4-benzotriazine 1-oxide,
3-Methyl-1,2,4-benzotriazine 1-oxide,
3-Ethyl-1,2,4-benzotriazine 1-oxide,
3-Phenyl-1,2,4-benzotriazine 1-oxide,
3-(4-Methoxyphenyl)-1,2,4-benzotriazine 1-oxide,
3-Vinyl-1,2,4-benzotriazine 1-oxide,
3-Allyl-1,2,4-benzotriazine 1-oxide,
3-(2-Hydroxyethyl)-1,2,4-benzotriazine 1-oxide,
3-(2-Methoxyethyl)-1,2,4-benzotriazine 1-oxide,
*N*-Phenyl-1,2,4-benzotriazin-3-amine 1-oxide,
3-Methoxy-1,2,4-benzotriazine 1-oxide,
3-Chloro-7-methyl-1,2,4-benzotriazine 1-oxide,
3-Chloro-7-methoxy-1,2,4-benzotriazine 1-oxide,
1,2,4-benzotriazine 1-oxide,
1,2,4-benzotriazin-3-amine 2-oxide, and
1,2,4-benzotriazin-3-amine 4-oxide.

9. The compound of Formula I according to claim 8 wherein Z is N.

10. The compound of Formula I according to claim 8 or claim 9 wherein X is NH or CH₂.

11. The compound of Formula I according to any one of claims 8 to 10 wherein ― X-A represents a NH(C₀-C₁₂) optionally substituted alkyl or an O(C₀-C₁₂) optionally substituted alkyl, such as NHCH₂CH₂NHCH_{3,} NHCH₂CH₂N(CH₃)₂ or OCH₃.

12. The compound of Formula I according to any one of claims 8 to 11 wherein Y₁ and Y₂ each represent H.

13. The compound of Formula I according to any one of claims 8 to 12 wherein the N-oxide moiety occupies the 1-position.

14. The use in the manufacture of a medicament of a cytotoxic effective amount of a compound of Formula I 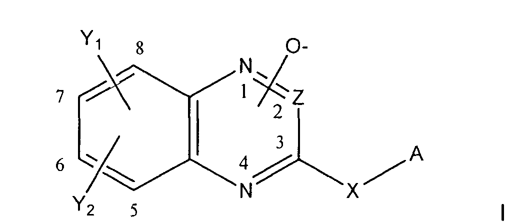 wherein
Z is selected from N or C-CN, and when Z represents N, the N-oxide moiety occupies one of the 1-, 2-, or 4-positions; and when Z represents C-CN, the N-oxide moiety occupies one of the 1-, or 4-positions;
Y₁ and Y₂ each represent at one or more of the available carbons 5-8 on the benzo ring the following groups:
halo, H, R, OH, OR, NO₂, NH₂, NHR, NR₂, SH, SR, SO₂R, CF₃, CN, CO₂H, CO₂R, CHO, COR, CONH₂, CONHR or CONRR, cyclic alkylamino, imidazolyl, alkylpiperazinyl, morpholino,
wherein each R can be independently selected from an optionally substituted C₁₋₆ aliphatic or an optionally substituted C₃₋₆ cyclic alkyl group, and wherein the said optional substituents are each independently selected from; halo, OH, OR¹, NO_{2,} NH_{2,} NHR¹, NR¹R¹, SH, SR¹, imidazolyl, R¹-piperazinyl, morpholino, SO₂R¹, CF₃, CN, CO₂H, CO₂R¹, CHO, COR¹, CONH_{2,} CONHR¹, CONR¹R¹;
R can also represent an optionally substituted aryl or an optionally substituted heteroaryl group having up to 12 carbon atoms, and wherein the optional substituents are each independently selected from; halo, OH, OR¹, NH₂, NHR¹, NR¹R¹, SH, SR¹, imidazolyl, R¹-piperazinyl, morpholino, SO₂R¹, CF₃, CN, CO₂H, CO₂R¹, CHO, COR¹, CONH₂, CONHR¹, CONR¹R¹, and each heteroaryl group contains one or more heteroatoms in its ring system which are each independently selected from O, N or S;
wherein each R¹ is independently selected from an optionally substituted C₁₋₄ alkyl or an optionally substituted C₂₋₄ alkenyl group and wherein the optional substituents are each independently selected from OH, OR, NH₂, NHR², NR²₂ or N(OH)R² wherein each R² is independently selected from C₁₋₄ alkyl, C₂₋₄ alkenyl, OH, NO₂, NH₂, CF₃, CN, CO₂H or SH, and
wherein A and X together represent H, or halogen; or
X represents O, S, NH, NMe or CH₂ and
A represents H, an optionally substituted C₁₋₁₂ alkyl group wherein the optional substituents are each independently selected from OH, OR, NH₂, NHR³, NR³₂, or N(OH)R³ wherein each R³ is independently selected from C₁₋₄ alkyl, C₂₋₄ alkenyl, OH, NO₂, NH₂, CF₃, CN, CO₂H or SH; and wherein the optionally substituted C₁₋₁₂ alkyl chain is optionally interrupted or extended by one or more heteroatom containing linkage moieties selected from O, NH, NR⁴, CONH, CONR⁴, NHCO, NR⁴CO, where each R⁴ is independently selected from an optionally substituted C₁₋₄ alkyl or an optionally substituted C₂₋₄ alkenyl group and wherein the optional R⁴ substituents are each independently selected from OH, OR, NH₂, NHR⁵, NR⁵₂ or N(OH)R⁵ wherein each R⁵ is independently selected from C₁₋₄ alkyl, C₂₋₄ alkenyl, OH, NO₂, NH₂, CF₃, CN, CO₂H or SH, or a pharmacologically acceptable salt thereof, for treating tumour cells in a subject.

15. A compound of Formula I', 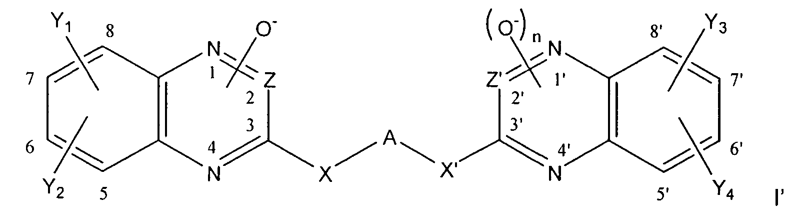 wherein
n represents either 1 or 2,
Z or Z' is selected from N or C-CN, and when Z or Z' represents N, and n represents 1 each N-oxide moiety occupies one of the 1-, 2-, or 4-positions or 1'-, 2'-, or 4'-positions respectively and when Z or Z' represents C-CN, each N-oxide moiety occupies one of the 1-, or 4-positions or 1'-, or 4'-positions respectively; and when Z' represents N, and n represents 2, the N'-oxide moieties occupy the 1'- and 4'-positions and when Z' represents C-CN, and n represents 2 the N'-oxide moieties occupy the 1'-, and 4'-positions;
Y₁, Y₂, Y₃ and Y₄ each represent at one or more of the available carbons 5-8 or one or more of the available carbons 5'-8' on the respective benzo ring the following groups:
halo, H, R, OH, OR, NO₂, NH₂, NHR, NR₂, SH, SR, SO₂R, CF₃, CN, CO₂H, CO₂R, CHO, COR, CONH₂, CONHR or CONRR, cyclic alkylamino, imidazolyl, alkylpiperazinyl, morpholino;
wherein each R is independently selected from an optionally substituted C₁₋₆ aliphatic or an optionally substituted C₃₋₆ cyclic alkyl group, and wherein the said optional substituents are each independently selected from; halo, OH, OR¹, NO₂, NH₂, NHR¹, NR¹R¹, SH, SR¹, imidazolyl, R¹-piperazinyl, morpholino, SO₂R¹, CF₃, CN, CO₂H, CO₂R¹, CHO, COR¹, CONH₂, CONHR¹, CONR¹R¹;
R can also represent an optionally substituted aryl or an optionally substituted heteroaryl group having up to 12 carbon atoms, and wherein the optional substituents are each independently selected from; halo, OH, OR¹, NH₂, NHR¹, NR¹R¹, SH, SR¹, imidazolyl, R¹-piperazinyl, morpholino, SO₂R¹, CF₃, CN, CO₂H, CO₂R¹, CHO, COR¹, CONH₂, CONHR¹, CONR¹R¹, and each heteroaryl group contains one or more heteroatoms in its ring system which are each independently selected from O, N or S;
wherein each R¹ is independently selected from an optionally substituted C₁₋₄ alkyl or an optionally substituted C₂₋₄ alkenyl group and wherein the optional substituents are each independently selected from OH, OR, NH_{2,} NHR², NR² ₂ or N(OH)R² wherein each R² is independently selected from C₁₋₄ alkyl, C₂₋₄ alkenyl, OH, NO_{2,} NH_{2,} CF_{3,} CN, CO₂H or SH, and
wherein X represents NH, NMe, CH_{2,} or O;
A represents an optionally substituted C₁₋₁₂ alkyl group wherein the optional substituents are each independently selected from OH, OR³, NH_{2,} NHR³, NR³ ₂ , or N(OH)R³ wherein each R³ is independently selected from C₁₋₄ alkyl, C₂₋₄ alkenyl, OH, NO₂ NH_{2,} CF_{3,} CN, CO₂H or SH; and wherein the optionally substituted C₁₋₂ alkyl chain is optionally interrupted or extended by one or more heteroatom containing linkage moieties selected from O, NH, NR⁴, CONH, CONR⁴, NHCO, NR⁴CO, where each R⁴ is independently selected from an optionally substituted C₁₋₄ alkyl or an optionally substituted C₂₋₄ alkenyl group and wherein the optional R⁴ substituents are each independently selected from OH, OR, NH_{2,} NHR⁵, NR⁵_{z}or N(OH)R⁵ wherein each R⁵ is independently selected from C₁₋₄ alkyl, C₂₋₄ alkenyl, OH, NO_{2,} NH_{2,} CF_{3,} CN, CO₂H or SH, or a pharmacologically acceptable salt thereof.

16. The compound of Formula I' as claimed in claim 15 wherein X is NH or CH_{2.}

17. The compound of Formula I' as claimed in claim 15 or claim 16 wherein Y₁ and Y₂each represent H.

18. The compound of Formula I' as claimed in any one of claims 15 to 17 wherein A is -(CH₂)₂NMe(CH₂)₂-.

19. The compound of Formula I' as claimed in any one of claims 15 to 18 wherein the N-oxides are positioned at the 1-position and the 1'-position.

20. The use in the manufature of a medicament of a cytotoxic effective amount of a compound of Formula I' as defined in any one of claims 15 to 19 for treating tumour cells in a subject.

21. A compound of Formula II, 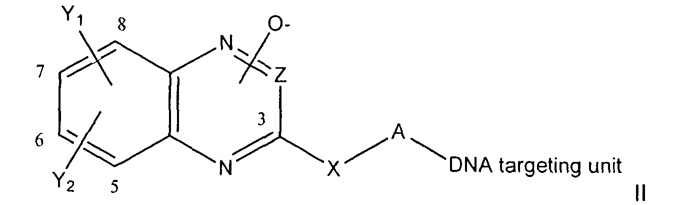 wherein
Z is selected from N or C-CN, and when Z represents N, the N-oxide moiety occupies one of the 1-, 2-, or 4-positions; and when Z represents C-CN, the N-oxide moiety occupies one of the 1-, or 4-positions;
Y₁ and Y₂ each represent at one or more of the available carbons 5-8 on the benzo ring the following groups:
halo, H, R, OH, OR, NO₂ NH_{2,} NHR, NR_{2,} SH, SR, SO₂R, CF_{3,} CN, CO₂H, CO₂R, CHO, COR, CONH₂, CONHR or CONRR, cyclic alkylamino, imidazolyl, alkylpiperazinyl, morpholino;
wherein each R is independently selected from an optionally substituted C₁₋₆ aliphatic or an optionally substituted C₃₋₆ cyclic alkyl group, and wherein the said optional substituents are each independently selected from; halo, OH, OR¹, NO_{2,} NH_{2,} NHR¹, NR¹R¹, SH, SR¹, imidazolyl, R¹-piperazinyl, morpholino, SO₂R¹, CF_{3,} CN, CO₂H, CO₂R¹, CHO, COR¹, CONH_{2,} CONHR¹, CONR¹R¹;
R can also represent an optionally substituted aryl or an optionally substituted heteroaryl group having up to 12 carbon atoms, and wherein the optional substituents are each independently selected from; halo, OH, OR¹, NH_{2,} NHR¹, NR¹R¹, SH, SR¹, imidazolyl, R¹-piperazinyl, morpholino, SO₂R¹, CF_{3,} CN, CO₂H, CO₂R¹, CHO, COR¹, CONH_{2,} CONHR¹, CONR¹R¹, and each heteroaryl group contains one or more heteroatoms in its ring system which are each independently selected from O, N or S;
wherein each R¹ is independently selected from an optionally substituted C₁₋₄ alkyl or an optionally substituted C₂₋₄ alkenyl group and wherein the optional substituents are each independently selected from OH, OR, NH_{2,} NHR², NR² ₂ or N(OH)R² wherein each R² is independently selected from C₁₋₄ alkyl, C₂₋₄ alkenyl, OH, NO_{2,} NH_{2,} CF_{3,} CN, CO₂H or SH, and
wherein X represents NH, NMe, CH₂, or O;
A represents an optionally substituted C₁₋₁₂ alkyl group wherein the optional substituents are each independently selected from OH, OR³, NH_{2,} NHR³, NR³ ₂ , or N(OH)R³ wherein each R³ is independently selected from C₁₋₄ alkyl, C₂₋₄ alkenyl, OH, NO_{2,} NH_{2,} CF_{3,} CN, CO₂H or SH; and wherein the optionally substituted C₁₋₁₂ alkyl chain can be optionally interrupted or extended by one or more heteroatom containing linkage moieties selected from O, NH, NR⁴, CONH, CONR⁴, NHCO, NR⁴CO, where each R⁴ is independently selected from an optionally substituted C₁₋₄ alkyl or an optionally substituted C₂₋₄ alkenyl group and wherein the optional R⁴ substituents are each independently selected from OH, OR, NH_{2,} NHR⁵, NR⁵ ₂or N(OH)R⁵ wherein each R⁵ is independently selected from C₁₋₄ alkyl, C₂₋₄ alkenyl, OH, NO_{2,} NH_{2,} CF_{3,} CN, CO₂H or SH; and
wherein the DNA-targeting unit is any moiety of a molecular weight below 700 Daltons that has an association constant (K) for binding to double-stranded random-sequence DNA of >10³ M⁻¹ at an ionic strength of 0.01 M at 20 °C,
or a pharmacologically acceptable salt thereof.

22. The compound of Formula II as claimed in claim 21, wherein Z is N.

23. The compound of Formula II as claimed in claim 21 or claim 22 wherein X is NH or CH₂.

24. The compound of Formula II as claimed in any one of claims 21 to 23 wherein the N-oxide is at the 1-position.

25. The compound of Formula II as claimed in any one of claims 21 to 24 wherein Y₁ and Y₂ each represent H.

26. The compound of Formula II as claimed in any one of claims 21 to 24 wherein Y₁ represents Me.

27. The compound of Formula II as claimed in any one of claims 21 to 26 wherein A is selected from -(CH₂)₆NH-, -(CH₂)₃NH(CH₂)₃NHCO-, -(CH₂)₃NMe(CH₂)₃NHCO-, -(CH₂)₃NH-, -(CH₂)₂NH(CH₂)₂NHCO- or -(CH₂)₂NMe(CH₂)₂NHCO-.

28. The compound of Formula II as claimed any one of claims 21 to 27 wherein the DNA-targeting unit is selected from one of formulae **III- XVII**, 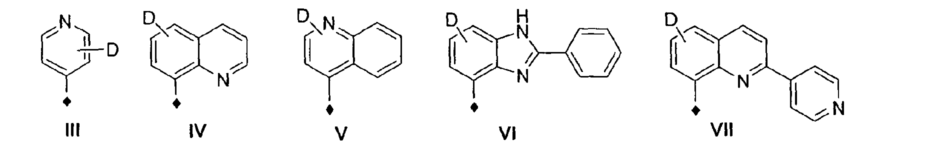 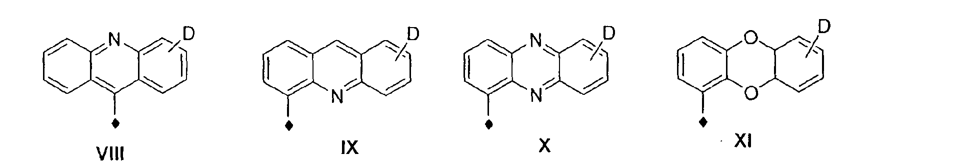 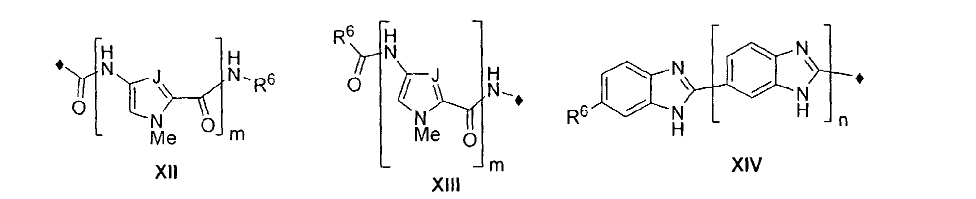 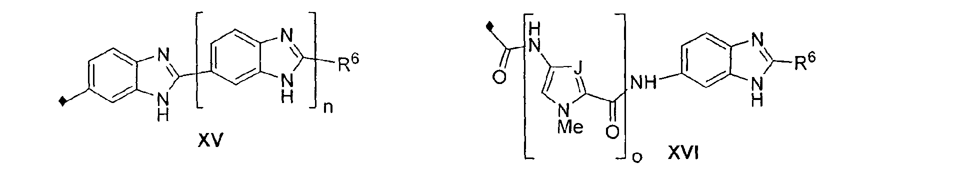 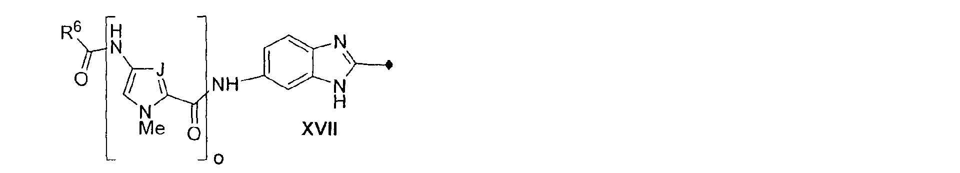 wherein in structures **XII-XVII** R⁶ is independently selected from an optionally substituted C₁₋₆ aliphatic or an optionally substituted C₃₋₆ cyclic alkyl group, and wherein the optional substituents are each independently selected from; halo, OH, OR⁷ NO₂, NH₂, NHR⁷, NR⁷R⁷, SR⁷, imidazolyl, R⁷-piperazinyl, morpholino, SO₂R⁷, CF₃, CN, CO₂H, CO₂R⁷, CHO, COR⁷, CONH₂, CONHR⁷, CONR⁷R⁷;
R⁶ can also be represent an optionally substituted aryl or an optionally substituted heteroaryl group having up to 12 carbon atoms, and wherein the optional substituents are each independently selected from; halo, OH, OR⁷, NH₂, NHR⁷, NR⁷R⁷, SH, SR⁷, imidazolyl, R⁷-piperazinyl, morpholino, SO₂R⁷, CF₃, CN, CO₂H, CO₂R⁷, CHO, COR⁷, CONH₂, CONHR⁷, CONR⁷R⁷, and each heteroaryl group contains one or more heteroatoms in its ring system which are each independently selected from O, N or S;
wherein each R⁷ is independently selected from an optionally substituted C₁₋₄ alkyl or an optionally substituted C₂₋₄ alkenyl group and wherein the optional substituents are each independently selected from OH, OR⁸, NH₂, NHR⁸, NR⁸₂ or N(OH)R⁸ wherein each R⁸ is independently selected from C₁₋₄ alkyl, C₂₋₄ alkenyl, OH, NO₂ NH₂, CF₃, CN, CO₂H or SH;
D represents up to four of the following groups as substituents at any available ring carbon position; H, R⁹, hydroxy, alkoxy, halogen, NO₂, NH₂, NHR⁹, NR⁹₂, SH, SR⁹, SO₂R⁹, CF₃, CN, CO₂H, CO₂R⁹, CHO, COR⁹, CONH₂, CONHR⁹ or CONR⁹R⁹, cyclic alkylamino, imidazolyl, alkylpiperazinyl, morpholino, wherein each R⁹ is independently selected from an optionally substituted C₁₋₄ alkyl or an optionally substituted C₂₋₄ alkenyl group and wherein the optional substituents are each independently selected from OH, OR¹⁰, NH₂, NHR¹⁰, NR¹⁰₂ or N(OH)R¹⁰ wherein each R¹⁰ is independently selected from C₁₋₄ alkyl, C₂₋₄ alkenyl, OH, NO₂, NH₂, CF₃, CN, CO₂H or SH;
and wherein any available ring carbon position of formulae **III** - **XVII** can also be optionally replaced by -N- when the valency and configuration of the formula allows, the point of attachment of formulae **III- XVII** to the A group defined above is represented by **◆;** and
wherein in formulae **XII, XIII,** m is selected from 2, 3 or 4, and
wherein in formulae **XII, XIII, XVI** and **XVII,** J is selected from CH or N;
and wherein in formulae **XIV** and **XV** n is selected from 0, 1 or 2;
and wherein in formulae **XVI** and **XVII** o is selected from 1 and 2.

29. The compound of formula **II** as claimed in claim 28 wherein the DNA targeting unit is selected from one of formulae V, **VI, VII, VIII, IX** or **X**.

30. The compound of formula II as claimed in claim 28 or claim 29 wherein D of the DNA targeting unit of Formulae **III** -**XI** is H or Me.

31. A compound of formula II as claimed in any one of claims 28 to 30 selected from a compound;
wherein X is NH-, Y is H, Z is N, position 1-oxide, A is -(CH₂)₂NH(CH₂)₂NHCO-, the DNA targeting unit represents formula IX and D is H;
wherein X is NH-, Y is H, Z is N, position 1-oxide, A is -(CH₂)₃NH(CH₂)₃NHCO-, the DNA targeting unit represents formula IX and D is H;
wherein X is NH-, Y is H, Z is N, position 1-oxide, A is -(CH₂)₂NMe(CH₂)₂NHCO-, the DNA targeting unit represents formula IX and D is H;
wherein X is NH-, Y is 6-Me, Z is N, position 1-oxide, A is -(CH₂)₂NMe(CH₂)₂NHCO-, the DNA targeting unit represents formula IX and D is H;
wherein X is NH-, Y is H, Z is N, position 1-oxide, A is -(CH₂)₃NMe(CH₂)₃NHCO-, the DNA targeting unit represents formula IX and D is H;
wherein X is NH-, Y is 6-Me, Z is N, position 1-oxide, A is -(CH₂)₃NMe(CH₂)₃NHCO-, the DNA targeting unit represents formula IX and D is H;
wherein X is NH-, Y is H, Z is N, position 1-oxide, A is -(CH₂)₂NMe(CH₂)₂NHCO-, the DNA targeting unit represents formula IX and D is Me; and
wherein X is NH-, Y is H, Z is N, position 1-oxide, A is -(CH₂)₃NMe(CH₂)₃NHCO-, the DNA targeting unit represents formula IX and D is Me.

32. The use in the manufacture of a medicament of a cytotoxic effective amount of a compound of Formula II as defined in any one of claims 21 to 31 for treating tumour cells in a subject.

33. A compound of Formula II', 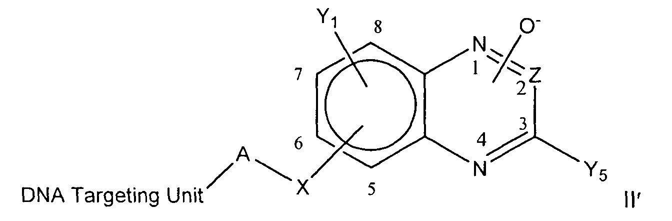 wherein
Z is selected from N or C-CN, and when Z represents N, the N-oxide moiety occupies one of the 1-, 2-, or 4-positions; and when Z represents C-CN, the N-oxide moiety occupies one of the 1-, or 4-positions;
Y₁ represents at one or more of the available carbons 5-8 on the benzo ring the following groups:
halo, H, R, OH, OR, NO₂, NH₂, NHR, NR₂, SH, SR, SO₂R, CF₃, CN, CO₂H, CO₂R, CHO, COR, CONH₂, CONHR or CONRR, cyclic alkylamino, imidazolyl, alkylpiperazinyl, morpholino;
Y₅ is selected from the following groups halo, H, R, OR, NH₂, NHR, NR₂, SO₂R, CF₃, CN, CO₂H, CO₂R, CHO, COR, CONH₂, CONHR or CONRR, cyclic alkylamino, imidazolyl, alkylpiperazinyl, morpholino;
wherein each R of groups Y₁ and Y₅ is independently selected from an optionally substituted C₁₋₆ aliphatic or an optionally substituted C₃₋₆ cyclic alkyl group, and wherein the optional substituents are each independently selected from; halo, OH, OR¹, NO₂, NH₂, NHR¹, NR¹R¹, SH, SR¹, imidazolyl, R¹-piperazinyl, morpholino, SO₂R¹, CF₃, CN, CO₂H, CO₂R¹, CHO, COR¹, CONH₂, CONHR¹, CONR¹R¹;
R can represent an optionally substituted aryl or an optionally substituted heteroaryl group having up to 12 carbon atoms, and wherein the optional substituents are each independently selected from; halo, OH, OR¹, NH₂, NHR¹, NR¹R¹, SH, SR¹, imidazolyl, R¹-piperazinyl, morpholino, SO₂R¹, CF₃, CN, CO₂H, CO₂R¹, CHO, COR¹, CONH₂, CONHR¹, CONR¹R¹, and each heteroaryl group contains one or more heteroatoms in its ring system which are each independently selected from O, N or S;
wherein each R¹ is independently selected from an optionally substituted C₁₋₄ alkyl or an optionally substituted C₂₋₄ alkenyl group and wherein the optional substituents are each independently selected from OH, OR, NH₂, NHR², NR² ₂ or N(OH)R² wherein each R² is independently selected from C₁₋₄ alkyl, C₂₋₄ alkenyl, OH, NO₂, NH₂, CF₃, CN, CO₂H or SH, and
wherein X represents NH, NMe, CH₂, S, SO, SO₂, CONH, NHCO, CO, CO₂, or O;
A represents an optionally substituted C₁₋₁₂ alkyl group wherein the optional substituents are each independently selected from OH, OR³, NH₂, NHR³, NR³ ₂ or N(OH)R³ wherein each R³ is independently selected from C₁₋₄ alkyl, C₂₋₄ alkenyl, OH, NO₂, NH₂, CF₃, CN, CO₂H or SH; and wherein the optionally substituted C₂₋₁₂ alkyl chain can be optionally interrupted by one or more heteroatom containing linkage moieties selected from O, NH, NR⁴, CONH, CONR⁴, NHCO, NR⁴CO, where each R⁴ is independently selected from an optionally substituted C₁₋₄ alkyl or an optionally substituted C₂₋₄ alkenyl group and wherein the optional R⁴ substituents are each independently selected from OH, OR, NH₂, NHR⁵, NR⁵₂ or N(OH)R⁵ wherein each R⁵ is independently selected from C₁₋₄ alkyl, C₂₋₄ alkenyl, OH, NO₂, NH₂, CF₃, CN, CO₂H or SH; and
wherein the DNA-targeting unit is any moiety of a molecular weight below 700 Daltons that has an association constant (K) for binding to double-stranded random-sequence DNA of > 10³ M⁻¹ at an ionic strength of 0.01 M at 20 °C,
or a pharmacologically acceptable salt thereof.

34. A compound of Formula II' as claimed in claim 33 wherein Z is N.

35. A compound of Formula II' as claimed in claim 33 or claim 34 wherein X is O or CH₂.

36. A compound of Formula II' as claimed in any one of claims 33 to 35 wherein the N-oxide is at the 1-position.

37. A compound of Formula II' as claimed in any one of claims 33 to 36 wherein Y₁ represents H.

38. A compound of Formula II' as claimed in any one of claims 33 to 37 wherein Y₅ represents NHR.

39. A compound of Formula II as claimed in any one of claims 33 to 38 wherein A is selected from
-(CH₂)₆NH-, -(CH₂)₃NH(CH₂)₃NHCO-, -(CH₂)₃NMe(CH₂)₃NHCO-, -(CH₂)₃NH-, -(CH₂)₂NH(CH₂)₂NHCO- or -(CH₂)₂NMe(CH₂)₂NHCO-.

40. A compound of Formula II as claimed in any one of claims 33 to 39 wherein the DNA-targeting unit is selected from one of formulae **III- XVII**, 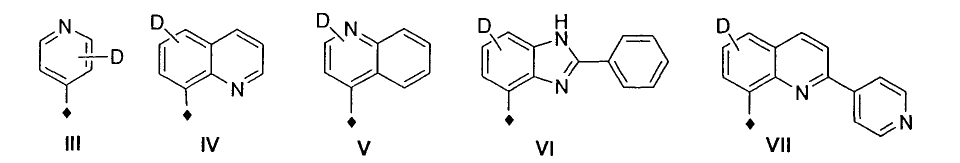 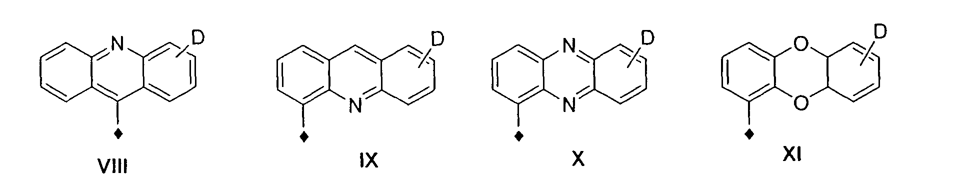 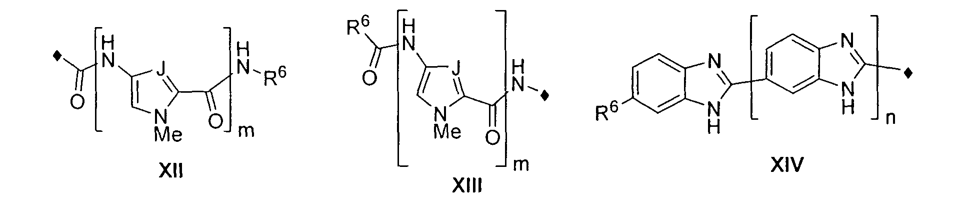 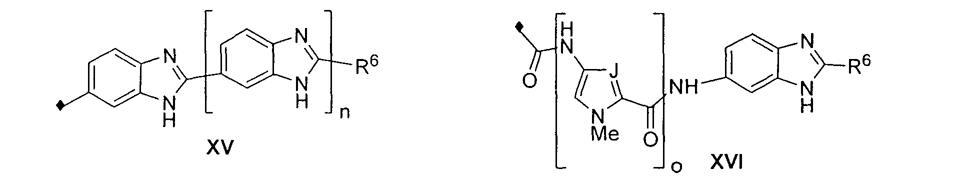 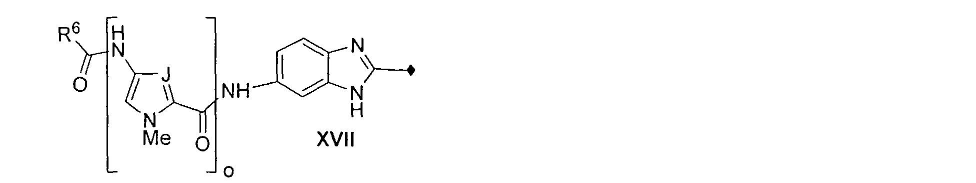 wherein in structures **XII - XVII** R⁶ is independently selected from an optionally substituted C₁₋₆ aliphatic or an optionally substituted C₃₋₆ cyclic alkyl group, and wherein the optional substituents are each independently selected from; halo, OH, OR⁷ NO_{2,} NH_{2,} NHR⁷, NR⁷R⁷, SR⁷, imidazolyl, R⁷-piperazinyl, morpholino, SO₂R⁷, CF₃, CN, CO₂H, CO₂R⁷, CHO, COR⁷, CONH_{2,} CONHR⁷, CONR⁷R⁷;
R⁶ represents an optionally substituted aryl or an optionally substituted heteroaryl group having up to 12 carbon atoms, and wherein the optional substituents are each independently selected from; halo, OH, OR⁷, NH_{2,} NHR⁷, NR⁷R⁷, SH, SR⁷, imidazolyl, R⁷-piperazinyl, morpholino, SO₂R⁷, CF₃, CN, CO₂H, CO₂R⁷, CHO, COR⁷, CONH_{2,} CONHR⁷, CONR⁷R⁷, and each heteroaryl group contains one or more heteroatoms in its ring system which are each independently selected from O, N or S;
wherein each R⁷ is independently selected from an optionally substituted C₁₋₄ alkyl or an optionally substituted C₂₋₄ alkenyl group and wherein the optional substituents are each independently selected from OH, OR⁸, NH_{2,} NHR⁸, NR⁸₂ or N(OH)R⁹⁸ wherein each R⁸ is independently selected from C₁₋₄ alkyl, C₂₋₄ alkenyl, OH, NO_{2,} NH_{2,} CF_{3,} CN, CO₂H or SH;
D represents up to four of the following groups as substituents at any available ring carbon position; H, R⁹, hydroxy, alkoxy, halogen, NO_{2,} NH_{2,} NHR⁹, NR⁹₂, SH, SR⁹, SO₂R⁹, CF_{3,} CN, CO₂H, CO₂R⁹, CHO, COR⁹, CONH_{2,} CONHR⁹ or CONR⁹R⁹, cyclic alkylamino, imidazolyl, alkylpiperazinyl, morpholino, wherein each R⁹ independently selected from an optionally substituted C₁₋₄ alkyl or an optionally substituted C₂₋₄ alkenyl group and wherein the optional substituents are each independently selected from OH, OR¹⁰, NH_{2,} NHR¹⁰, NR¹⁰₂ or N(OH)R¹⁰ wherein each R¹⁰ is independently selected from C₁₋₄ alkyl, C₂₋₄ alkenyl, OH, NO_{2,} NH_{2,} CF_{3,} CN, CO₂H or SH;
and wherein any available ring carbon position of formulae **III- XVII** can be optionally replaced by -N- when the valency and configuration of the formula allows, the point of attachment of formulae **III- XVII** to the A group defined above is represented by **◆**; and
wherein in formulae **XII** and **XIII,** m is selected from 2, 3 or 4,
and wherein in formulae **XII**, **XIII, XVI** or **XVII** J is selected from CH or N; and
wherein in formulae **XIV** and **XV** n is selected from 0, 1 or 2, and
wherein in formulae **XVI** and **XVII** o is selected from 1 or 2.

41. A compound of Formula II' as claimed in claim 40 wherein the DNA targeting unit is selected from one of formulae IV - X.

42. A compound of formula II' as claimed in claim 41 wherein D of the DNA targeting unit of Formulae III - XI is H or Me.

43. A compound of formula II' as claimed in any one of claims 40 to 42 selected from a compound;
wherein X is CH₂-, Y₁ is H, Y₅ is NHCH₂CH₂OMe, Z is -N-, A is -(CH₂)NMe(CH₂)₂NHCO-, the DNA targeting unit represents formula IX and D is H;
wherein X is CH₂-, Y₁ is H, Y₅ is NHCH₂CH₂OMe, Z is -N-, A is-(CH₂)₂NH(CH₂)₃NHCO-, the DNA targeting unit represents formula IX and D is H;
wherein X is CH₂-, Y₁ is H, Y₅ is NHCH₂CH₂OMe, Z is -N-, A is -(CH₂)NMe(CH₂)₂NHCO-, the DNA targeting unit represents formula IX and D is Me;
wherein X is CH₂-, Y₁ is H, Y₅ is NHCH₂CH₂OMe, Z is -N-, A is -(CH₂)₂NMe(CH₂)₃NHCO-, the DNA targeting unit represents formula IX and D is Me;
wherein X is CH₂-, Y₁ is H, Y₅ is NHCH₂CH₂OMe, Z is -N-, A is-(CH₂)NMe(CH₂)₂NHCO-, the DNA targeting unit represents formula X and D is Me; and
wherein X is CH₂-, Y₁ is H, Y₅ is NHCH₂CH₂OMe, Z is -N-, A is-(CH₂)₂NH(CH₂)₃NHCO-, the DNA targeting unit represents formula X and D is Me.

44. The use in the manufacture of a cytotoxic effective amount of a compound of Formula II' as defined in any one of claims 33 to 43 for treating tumour cells in a subject.
